# EUROPEAN PATENT SPECIFICATION

(11) **EP 4 267 969 B1**
(45) Date of publication and mention of the grant of the patent: **21.10.2026**
(21) Application number: 21912018.5
(22) Date of filing: 21.12.2021
(51) Int. Cl.: A61K 41/00, A61K 31/136, A61K 31/166, A61K 31/454, A61K 31/519, A61K 31/58, A61K 31/704, A61K 45/06, G01N 33/57555

(54) **METHODS AND MATERIALS FOR TREATING PROSTATE CANCER**
VERFAHREN UND MATERIALIEN ZUR BEHANDLUNG VON PROSTATAKREBS
PROCÉDÉS ET MATÉRIAUX POUR LE TRAITEMENT DU CANCER DE LA PROSTATE

(30) Priority: 22.12.2020 US 202063129393 P
(43) Date of publication of application: 01.11.2023
(73) Proprietor: Mayo Foundation for Medical Education and Research, Rochester, Minnesota 55905 (US)
(72) Inventor: WANG, Liewei, Rochester, Minnesota 55906 (US); GAO, Huanyao, Rochester, Minnesota 55901 (US)
(74) Representative: Fish & Richardson P.C.
(86) International application number: PCT/US2021/064539
(87) International publication number: WO 2022/140339

(56) References cited:
- EP-B1- 2 699 696
- WO-A1-2013/130177
- WO-A1-2015/089311
- WO-A1-2017/062505
- WO-A1-2020/169832
- WO-A1-2020/214718
- WO-A1-2022/055721
- US-B2- 8 877 445
- HORNING ET AL.: "Single- Cell RNA-seq Reveals a Subpopulation of Prostate Cancer Cells with Enhanced Cell -Cycle?", RELATED TRANSCRIPTION AND ATTENUATED ANDROGEN RESPONSE, GENOME AND EPIGENOME, vol. 78, no. 4, 15 February 2018 (2018-02-15), XP055557748, DOI: 10.1158/0008-5472.CAN-17-1924

## Description

### CROSS-REFERENCE To RELATED APPLICATIONS

This application claims the benefit of U.S. Patent Application Serial No. 63/129,393, filed on December 22, 2020.

### SEQUENCE LISTING

This document includes a Sequence Listing that has been submitted electronically as an ASCII text file named 07039-02014WO1_ST25.txt. The ASCII text file, created on November 5, 2021, is 7 kilobytes in size.

### 1. Technical Field

This document relates to methods and materials for assessing and/or treating mammals (*e.g.,* humans) having prostate cancer. For example, the methods and materials provided herein can be used to determine whether or not a prostate cancer is likely to respond to a particular cancer treatment (*e.g.,* treatment with one or more anti-androgen agents such as inhibitors of androgen biosynthesis and androgen receptor antagonists). Also provided are methods and materials for using one or more cancer treatments to treat a mammal (*e.g.,* a human) identified as likely to respond to a particular cancer treatment.

### 2. Background Information

Prostate cancer (PC) is the second most frequently diagnosed and the second most deadly cancer type for men in the United States (Siegel et al., CA Cancer J. Clin., 68, 7-30 (2018)). Approximately 10-20% of patients will develop metastatic prostate cancer (mPC) associated with a reduced survival rate. Androgen deprivation therapies (ADTs), including anti-androgen agents such as androgen biosynthesis inhibitors and antagonists for the androgen receptor (AR), the primary transcriptional regulator in normal and cancerous prostate cells, are the first-line systemic treatment of mPC (Knudsen et al., Clin. Cancer Res., 15, 4792-4798 (2009)). Despite initial response to ADT, a majority of these patients eventually develop hormone-refractory PC, castration-resistant prostate cancer (CRPC; Knudsen et al., Clin. Cancer Res., 15, 4792-4798 (2009); and Spratt et al., Prostate, 75, 175-182 (2015)). Second-generation ADT drugs such as abiraterone acetate (Abi), a cytochrome P450 17A1 (CYP17A1) inhibitor, have been shown to extend overall survival significantly (Azad et al., Clin. Cancer Res., 21, 2315-2324 (2015); and Romanel et al., Sci. Transl. Med., 7, 312re310 (2015)). However, at least 30% of patients do not respond to initial Abi treatment and nearly all patients will eventually develop acquired resistance.

WO 2020/214718 A1 describes signature genes useful for predicting patient prognosis and guiding treatment decisions in prostate cancer.

WO 2015/089311 A1 describes the use of SGK1 inhibitors for treating or reducing the risk of castration resistant prostate cancer and/or doubly resistant prostate cancer.

US 8,877,445 describes methods for identifying a tumor as an E2F-responsive gene (ERGO) over-expressing tumor and determining treatment for patients having an ERGO tumor.

WO 2020/169832 A1 describes assessing tumor aggressiveness and molecular subtype of tumor metastases in patients having prostate cancer based on subtype-associated gene transcript levels.

WO 2022/055721 A1 describes the identification of patients having prostate cancer that may benefit from treatment with a combination therapy based on expression of genes involved in meiosis and mitosis

### SUMMARY

The invention is defined by the independent claims. The dependent claims depict additional embodiments of the invention. This document provides methods and materials related to assessing and/or treating prostate cancer. In some cases, this document provides methods and materials for determining whether or not a mammal (*e.g.,* a human) having prostate cancer is likely to respond to a particular cancer treatment (*e.g.,* an anti-androgen agent), and, optionally, administering to the mammal one or more cancer treatments selected based, at least in part, on whether or not the mammal is likely to respond to a particular cancer treatment. For example, a sample (*e.g.,* a sample containing one or more cancer cells) obtained from a mammal having prostate cancer can be assessed to determine if the mammal is likely to respond to a particular cancer treatment based, at least in part, on the presence or absence of an increased level of expression of one or more (*e.g.,* one, two, three, four, five, six, seven, eight, nine, ten, eleven, or more) polypeptides in the sample.

As described herein, prostate cancers that respond to abiraterone (Abi responders) exhibit differential gene expression as compared to prostate cancers that do not respond to abiraterone (Abi non-responders). For example, increased expression of a cyclin A2 (CCNA2) nucleic acid (*e.g.,* resulting in increased level of expression of CCNA2 polypeptides), increased expression of a cyclin B1 (CCNB1) nucleic acid (*e.g.,* resulting in increased level of expression of CCNB1 polypeptides), increased expression of a cyclin B2 (CCNB2) nucleic acid (*e.g.,* resulting in increased level of expression of CCNB2 polypeptides), increased expression of a protein regulator of cytokinesis 1 (PRC1) nucleic acid (*e.g.,* resulting in increased level of expression of PRC1 polypeptides), increased expression of a structural maintenance of chromosomes protein 2 (SMC2) nucleic acid (*e.g.,* resulting in increased level of expression of SMC2 polypeptides), increased expression of a discs large-associated protein 5 (DLGAP5) nucleic acid (*e.g.,* resulting in increased level of expression of DLGAP5 polypeptides), increased expression of a epithelial cell transforming 2 (ECT2) nucleic acid (*e.g.,* resulting in increased level of expression of ECT2 polypeptides), increased expression of a F-box only protein 5 (FBXO5) nucleic acid (*e.g.,* resulting in increased level of expression of FBXO5 polypeptides), increased expression of a cyclin dependent kinase 1 (CDK1) nucleic acid (*e.g.,* resulting in increased level of expression of CDK1 polypeptides), increased expression of a non-SMC condensin I complex subunit G (NCAPG) nucleic acid (*e.g.,* resulting in increased level of expression of NCAPG polypeptides), and increased expression of a kinesin family member 4A (KIF4A) nucleic acid (*e.g.,* resulting in increased level of expression of KIF4A polypeptides) can be used to identify prostate cancer patients as having abiraterone resistance. Also as described herein, one or more DNA topoisomerase 2-alpha (TOP2A) inhibitors, one or more cyclin-dependent kinase (CDK) 4/6 inhibitors, one or more Mitogen-Activated Protein Kinase Kinase (MEK) inhibitors, and/or one or more pan-CDK inhibitors can sensitize prostate cancers to one or more anti-androgen agents. These results demonstrate that the presence or absence of an increased level of expression of one or more (*e.g.,* one, two, three, four, five, six, seven, eight, nine, ten, eleven, or more) polypeptides (*e.g.,* an increased level of expression of CCNA2 polypeptides, CCNB1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, and/or KIF4A polypeptides) in a sample from a mammal having prostate cancer can be used to determine anti-androgen agent (*e.g.,* abiraterone) responsiveness of that mammal.

Having the ability to identify a mammal having prostate cancer as being likely to respond to a particular cancer treatment based, at least in part, on the presence or absence of an increased level of expression of one or more polypeptides provides a unique and unrealized opportunity to provide an individualized approach in selecting effective prostate cancer therapies.

In general, one aspect of this document features *ex vivo* methods for assessing a mammal having prostate cancer. The methods include (a) detecting, in a sample from a mammal having prostate cancer, a presence or absence of an increased level of expression of a CCNA2 polypeptide, a CCNB1 polypeptide, a CCNB2 polypeptide, a PRC1 polypeptide, a SMC2 polypeptide, a DLGAP5 polypeptide, an ECT2 polypeptide, a FBXO5 polypeptide, a CDK1 polypeptide, a NCAPG polypeptide, and a KIF4A polypeptide; (b) classifying the mammal as being unlikely to respond to an anti-androgen agent if the presence of the increased level is detected; and (c) classifying the mammal as being likely to respond to the anti-androgen agent if the absence of the increased level is detected. The mammal can be a human. The sample can include cancer cells of the prostate cancer. The method can include detecting the presence of the increased level of the polypeptides. The method can include classifying the mammal as being unlikely to respond to the anti-androgen agent. The method can include detecting the absence of the increased level of the polypeptides. The method can include classifying the mammal as being likely to respond to the anti-androgen agent. The anti-androgen agent can be leuprolide, goserelin, triptorelin, histrelin, degarelix, abiraterone, ketoconazole, flutamide, bicalutamide, nilutamide, enzalutamide, apalutamide, or darolutamide. The prostate cancer can be a metastatic prostate cancer. The detecting can include a clustering analysis. The clustering analysis can be a machine learning based clustering analysis.

In another aspect, this document features a cancer drug for use in methods for treating a mammal having prostate cancer. The methods include (a) detecting, in a sample obtained from a mammal having prostate cancer, an increased level of expression of a CCNA2 polypeptide, a CCNB1 polypeptide, a CCNB2 polypeptide, a PRC1 polypeptide, a SMC2 polypeptide, a DLGAP5 polypeptide, an ECT2 polypeptide, a FBXO5 polypeptide, a CDK1 polypeptide, a NCAPG polypeptide, and a KIF4A polypeptide; and (b) administering the cancer drug to the mammal, where the cancer drug is not an anti-androgen agent. The mammal can be a human. The sample can include cancer cells of the prostate cancer. The cancer drug that is not an anti-androgen agent can be docetaxel, cabazitaxel, mitoxantrone, estramustine, doxorubicin, palbociclib, ribociclib, abemaciclib, PD-0325901, PHA-793887, or any combinations thereof.

In another aspect, this document features an anti-androgen agent for use in methods for treating a mammal having prostate cancer. The methods include (a) detecting, in a sample obtained from a mammal having prostate cancer, an absence of an increased level of expression of a CCNA2 polypeptide, a CCNB1 polypeptide, a CCNB2 polypeptide, a PRC1 polypeptide, a SMC2 polypeptide, a DLGAP5 polypeptide, an ECT2 polypeptide, a FBXO5 polypeptide, a CDK1 polypeptide, a NCAPG polypeptide, and a KIF4A polypeptide; and (b) administering the anti-androgen agent to the mammal. The mammal can be a human. The sample can include cancer cells of the prostate cancer. The anti-androgen agent can be leuprolide, goserelin, triptorelin, histrelin, degarelix, abiraterone, ketoconazole, flutamide, bicalutamide, nilutamide, enzalutamide, apalutamide, or darolutamide.

In another aspect, this document features a DNA TOP2A inhibitor for use in methods for treating a mammal having prostate cancer. The methods include (a) detecting, in a sample obtained from a mammal having prostate cancer, a presence of an increased level of expression of a CCNA2 polypeptide, a CCNB1 polypeptide, a CCNB2 polypeptide, a PRC1 polypeptide, a SMC2 polypeptide, a DLGAP5 polypeptide, an ECT2 polypeptide, a FBXO5 polypeptide, a CDK1 polypeptide, a NCAPG polypeptide, and a KIF4A polypeptide; (b) administering the TOP2A inhibitor to the mammal to increase the sensitivity of prostate cancer cells within the mammal to an anti-androgen agent; and (c) administering the anti-androgen agent to the mammal. The mammal can be a human. The sample can include cancer cells of the prostate cancer. The TOP2A inhibitor can be mitoxantrone, doxorubicin, teniposide, daunorubicin, amsacrine, ellipticines, aurintricarboxylic acid, or HU-331. The anti-androgen agent can be leuprolide, goserelin, triptorelin, histrelin, degarelix, abiraterone, ketoconazole, flutamide, bicalutamide, nilutamide, enzalutamide, apalutamide, or darolutamide.

In another aspect, this document features a cyclin-dependent kinase (CDK) 4/6 inhibitor for use in methods for treating a mammal having prostate cancer. The methods include (a) detecting, in a sample obtained from the mammal, a presence of an increased level of expression of a CCNA2 polypeptide, a CCNB1 polypeptide, a CCNB2 polypeptide, a PRC1 polypeptide, a SMC2 polypeptide, a DLGAP5 polypeptide, an ECT2 polypeptide, a FBXO5 polypeptide, a CDK1 polypeptide, a NCAPG polypeptide, and a KIF4A polypeptide; (b) administering the CDK 4/6 inhibitor to the mammal to increase the sensitivity of prostate cancer cells within the mammal to an anti-androgen agent; and (c) administering the anti-androgen agent to the mammal. The mammal can be a human. The sample can include cancer cells of the prostate cancer. The CDK 4/6 inhibitor can be palbociclib, abemaciclib, or ribociclib. The anti-androgen agent can be leuprolide, goserelin, triptorelin, histrelin, degarelix, abiraterone, ketoconazole, flutamide, bicalutamide, nilutamide, enzalutamide, apalutamide, or darolutamide.

In another aspect, this document features a pan-CDK inhibitor for use in methods for treating a mammal having prostate cancer. The methods include (a) detecting, in a sample obtained from a mammal having prostate cancer, a presence of an increased level of expression of a CCNA2 polypeptide, a CCNB1 polypeptide, a CCNB2 polypeptide, a PRC1 polypeptide, a SMC2 polypeptide, a DLGAP5 polypeptide, an ECT2 polypeptide, a FBXO5 polypeptide, a CDK1 polypeptide, a NCAPG polypeptide, and a KIF4A polypeptide; (b) administering a pan-CDK inhibitor to the mammal to increase the sensitivity of prostate cancer cells within the mammal to an anti-androgen agent; and (c) administering the anti-androgen agent to the mammal. The mammal can be a human. The sample can include cancer cells of the prostate cancer. The pan-CDK 4/6 inhibitor can be PHA-793887. The anti-androgen agent can be leuprolide, goserelin, triptorelin, histrelin, degarelix, abiraterone, ketoconazole, flutamide, bicalutamide, nilutamide, enzalutamide, apalutamide, or darolutamide.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below In case of conflict between the present specification and a reference mentioned herein, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and drawings, and from the claims.

### DESCRIPTION OF THE DRAWINGS

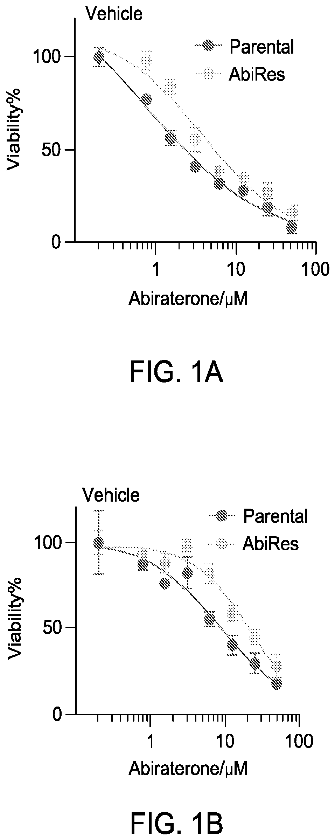

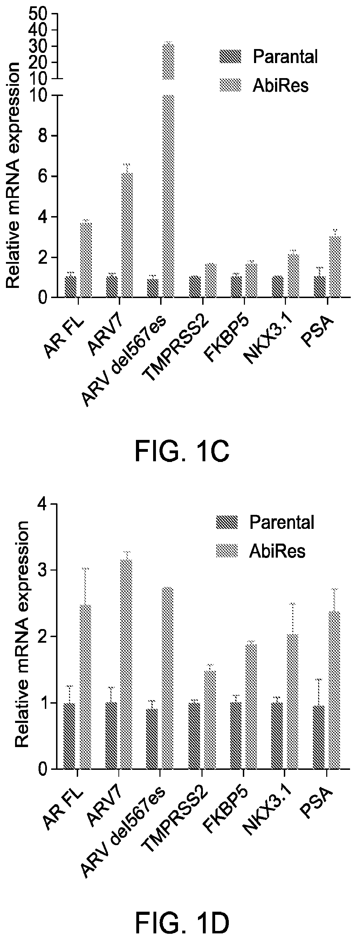

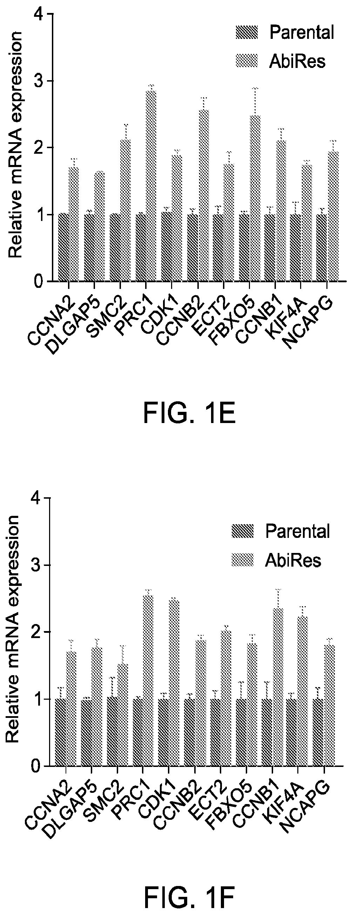

Figures 1A-1F show characteristics of abiraterone (Abi) resistant prostate cancer cell lines. Figure 1A-1B shows the cytotoxicity curve of Abi resistant and parental (Figure 1A) LNCaP and (Figure 1B) 22Rv1 cell line upon abiraterone treatment. Figure 1C-1D shows the expression of AR wild type (AR FL), AR variants (AR V7, AR del567es), and canonical AR targeted downstream genes (FKBP5, NKX3.1, PSA)in (Figure 1C) LNCaP and (Figure 1D) 22Rv1. (Figure 1E-1F) Baseline expression of the 11 drug targeted genes in Parental and Abi resistant cell lines in (Figure 1E) LNCaP and (Figure 1F) 22Rv1.

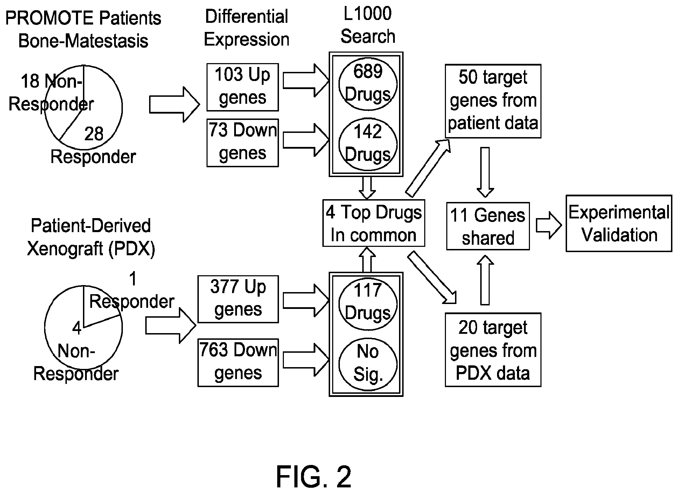

Figure 2 shows the workflow for the drug discovery-validation study. Differentially expressed genes between Abi-responders and non-responders, identified in either patient tumors or PDX tumors, were submitted to an Enrichr-LINCS L1000 Chemical Perturbation database to identify candidate drugs that can reverse the abiraterone resistant gene expression profiles. Four drugs were enriched and overlapped between the patient and PDX tumors. Eleven genes were shared target among all four drugs.

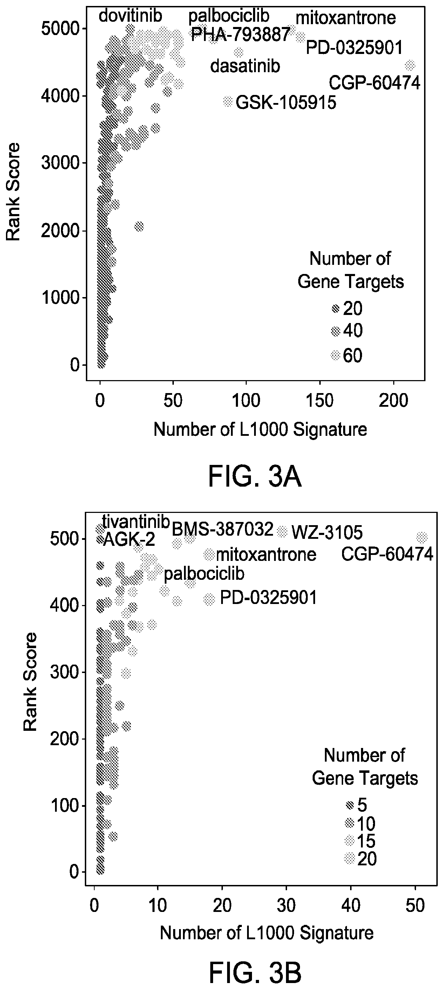

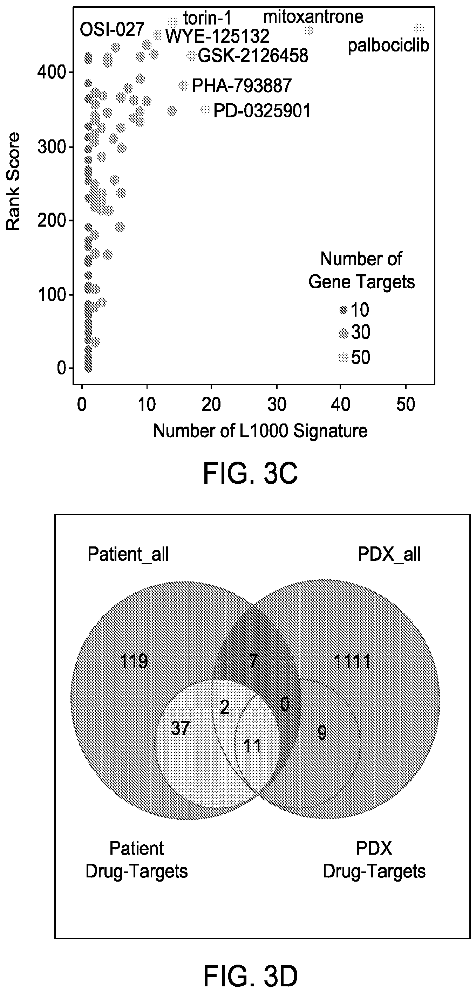

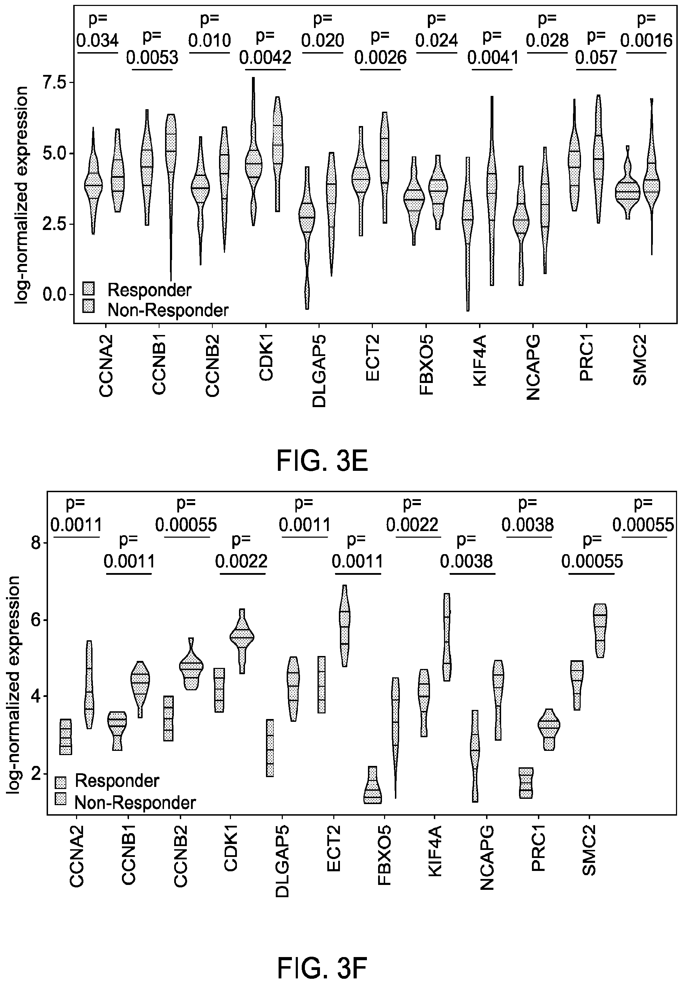

Figures 3A-3F show a drug discovery analysis based on patient tumor and xenograft genomic information. (Figures 3A-3C) Bubble plots for Enrichr-LINCS L1000 Chemical Perturbation analysis using significantly (Figure 3A) upregulated and (Figure 3B) downregulated genes in PROMOTE abiraterone non-responders, and (Figure 3C) upregulated genes in Abi resistant PROMOTE PDX model. Data was presented as Rank Score (see methods section) versus number of signatures for each drug returned by L1000 database search with FDR ≤ 0.05. Size of the bubbles represents number of gene targets overlapped between the submitted list genes and gene signature. (Figure 3D) Venn diagram for all differentially expressed genes (DEG) in PROMOTE patients (Patient_all), PDX models (PDX_all), and genes targeted by the top four candidate drugs that were also shared between patients (Patient Drug-Targets) and PDX models (PDX Drug-Targets). 11 genes were shared among all comparisons. (Figure 3E) Expression of the 11 shared genes targeted by all 4 drugs in PROMOTE patients' baseline biopsy samples including all tissue origins, grouped by AA/P response defined by composite score at 3 months post treatment. p-value was calculated based on one-sided Mann-whitney's test between responder/non-responder for each gene. (Figure 3F) Comparison of the 11 gene expression between PDX models derived from AA/P responder vs non-responder. p-value was calculated based on the one-sided Mann-whitney's test between responder/non-responder for each gene.

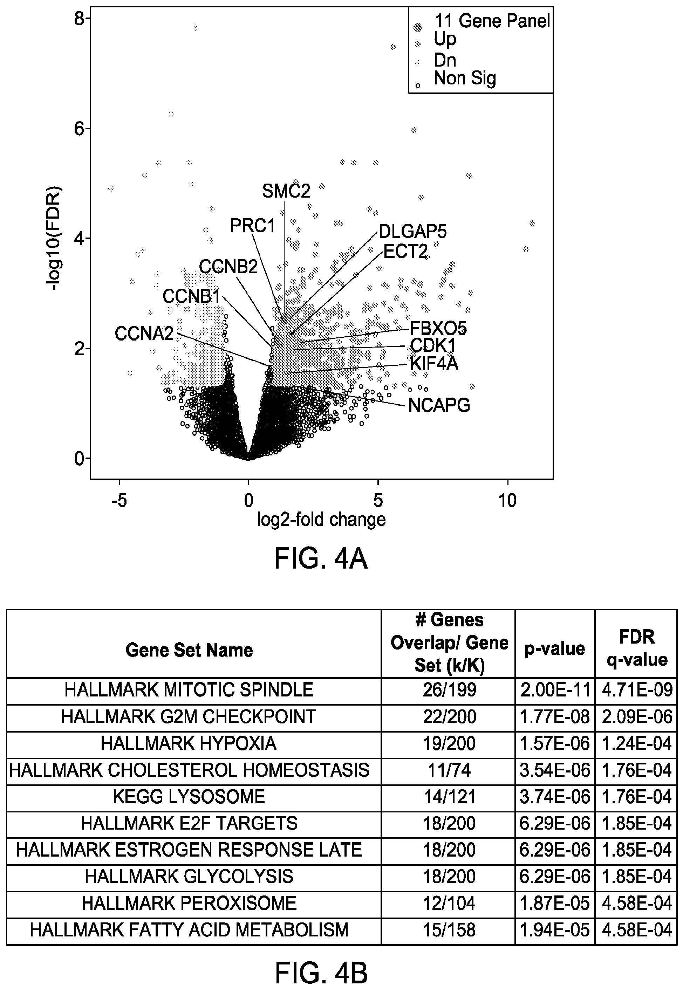

Figures 4A-4B show results from a RNAseq differential expression analysis using PDX models derived from the PROMOTE patients. (Figure 4A) Volcano plot for PDX differential expression, highlighting significantly up or down regulated genes (FDR≤0.05 and Fold change≥2) as indicated by different grayscales. The 11 genes are also labeled. (Figure 4B) GSEA analysis using the HALLMARK and KEGG pathway databases identify top pathways highlighting G2M and mitosis pathways.

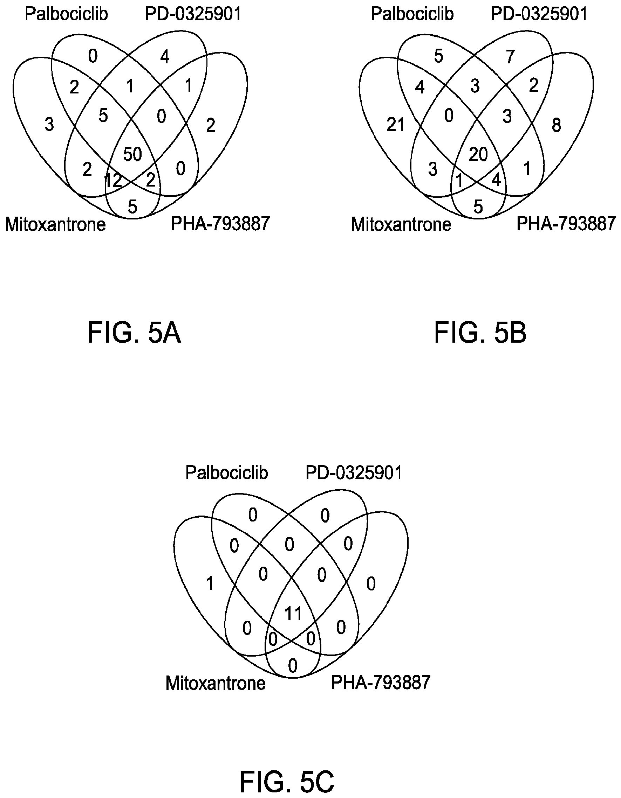

Figures 5A-5C show a Venn diagram of the number of shared genes identified from L1000 targeted by the 4 top candidate drugs. (Figure 5A) Number of gene targets by the 4 top drugs using patient differentially expressed genes (DEGs), (Figure 5B) Number of gene targets by the 4 top drugs using PDX DEGs; (Figure 5C) Number of overlapped genes among 4 drugs that are also common between DEGs of patient and PDX.

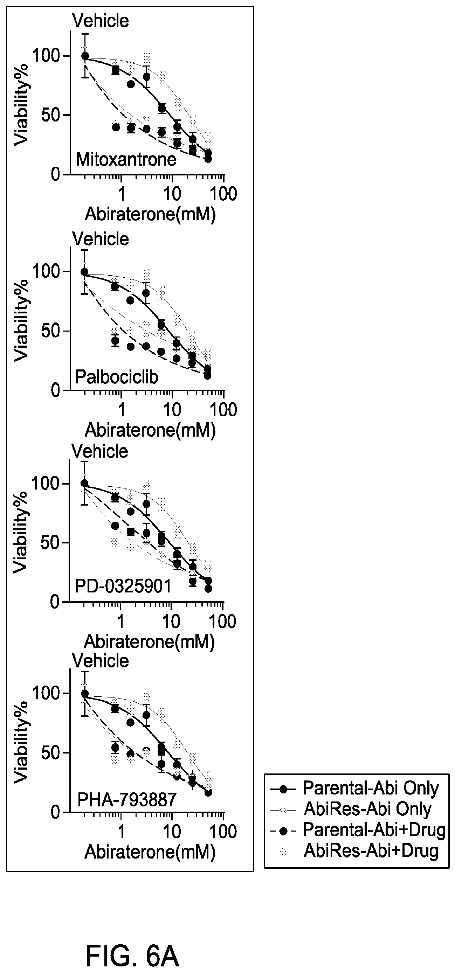

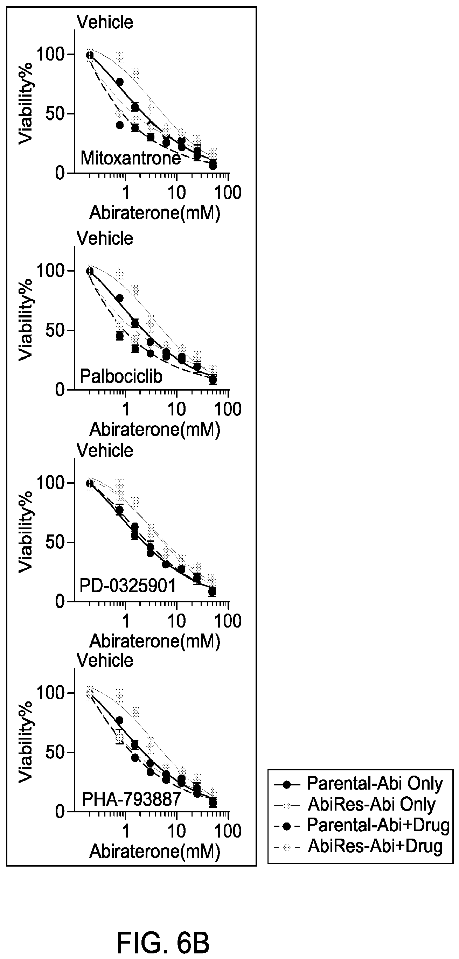

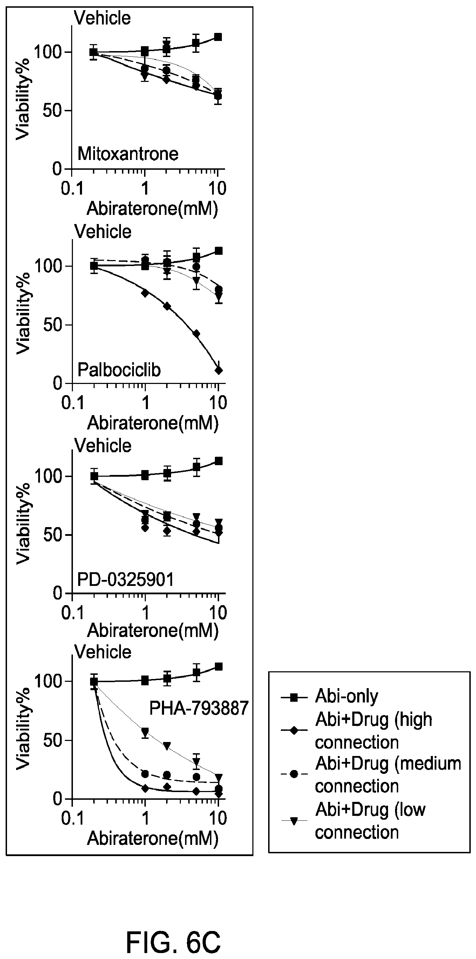

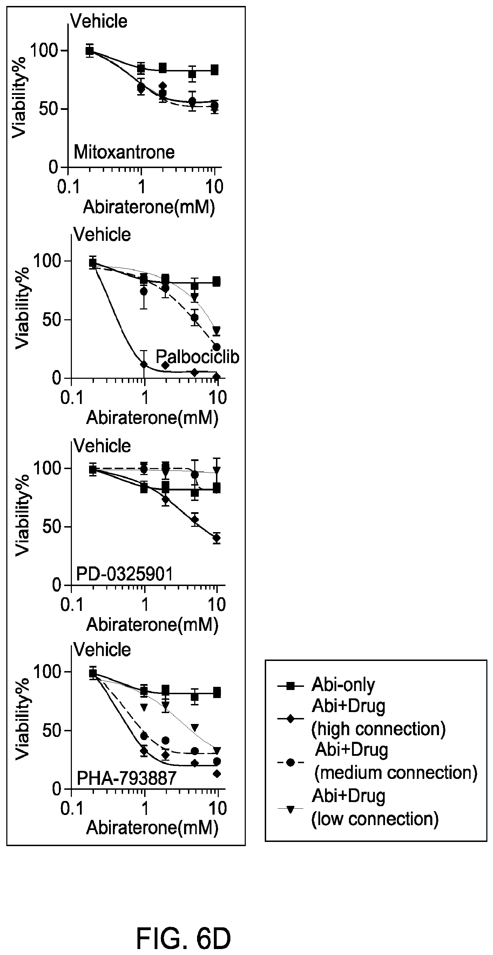

Figures 6A-6D show results from combination drug treatment experiments in Abi parental and resistant cell lines as well as in PDX derived organoid models. (Figure 6A-6B) Abiraterone alone or combined treatment with each of the four identified drugs in (Figure 6A) 22RV1 and (Figure 6B) LNCaP parental and Abi resistant (AbiRes) cell lines. Solid line indicates single drug and dash line indicates Abi + mitoxantrone (10 nM), palbociclib (250 nM), PD-0325901 (100 nM) or PHA-793882 (100 nM). (Figure 6C-6D) Abi treatment response in PDX organoids (Figure 6C) MC-PRX-01 and (Figure 6D) MC-PRX-05, as single drug or in combination with mitoxantrone (10, 20, or 30 nM), palbociclib (5, 10, or 20 µM), PD-0325901 (25, 50, or 100 µM) or PHA-793882 (5, 10, or 20 µM).

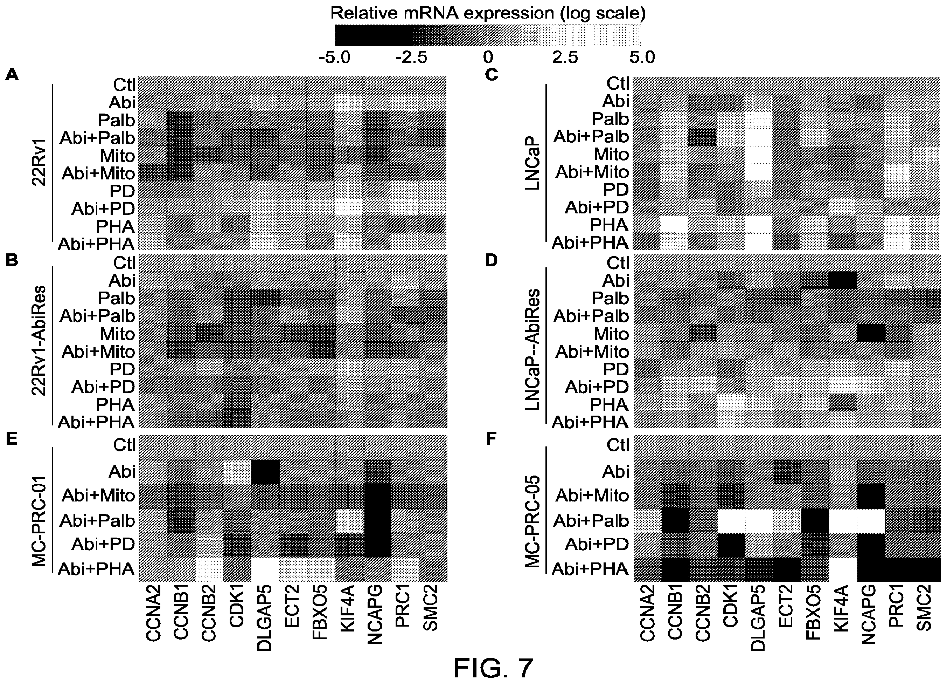

Figures 7A-7F shows modulation of gene expression by the 4 drugs in (Figure 7A) 22RV1 parental, (Figure 7B) 22RV1 AbiRes, (Figure 7C) LNCaP parental, (Figure 7D) LNCaP AbiRes cells, (Figure 7E) PDX organoids MC-PRX-01, and (Figure 7F) MC-PRX-05. Expression of the 11 genes was examined by qRTPCR after treatment with abiraterone (Abi), mitoxantrone (Mito), palbociclib (Palb), PD-0325901 (PD), PHA-793882 (PHA), or Abi combined with individual drug. Expression was normalized to vehicle treatment in each cell line or organoid model after normalization to housekeeping gene, β-Actin. Log2-fold change is represented as indicated on the scale.

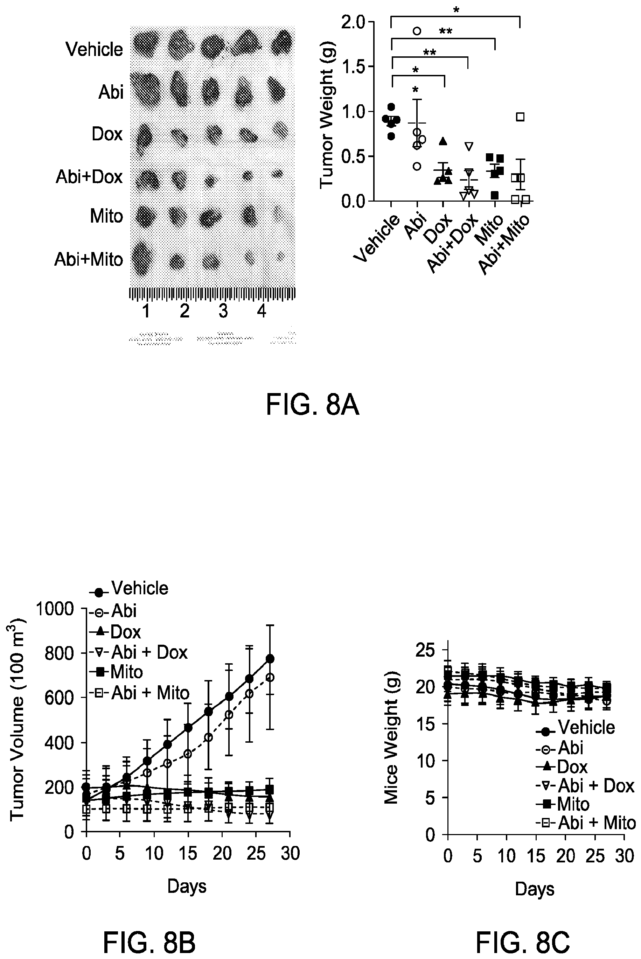

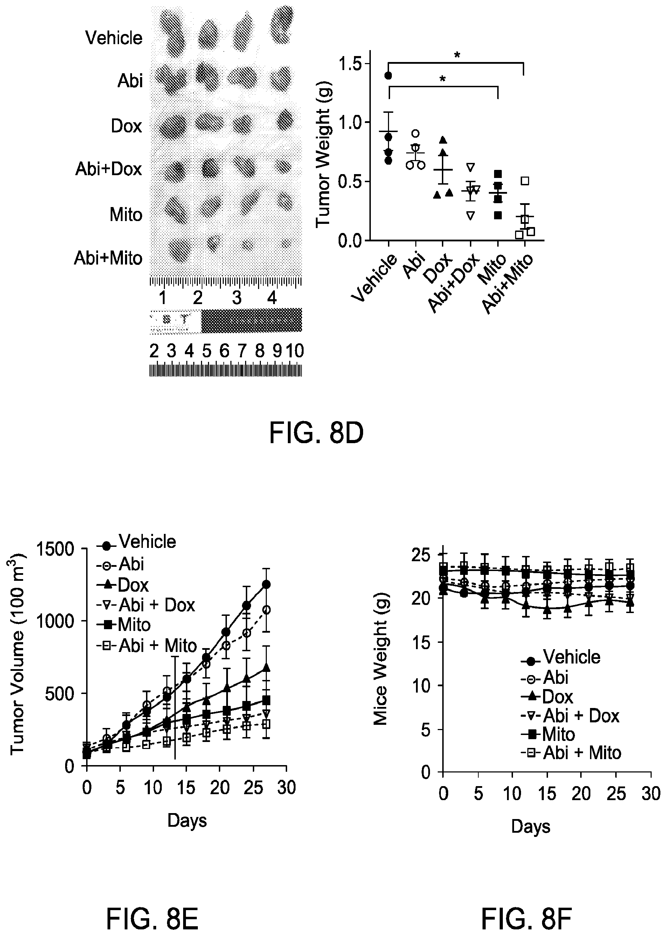

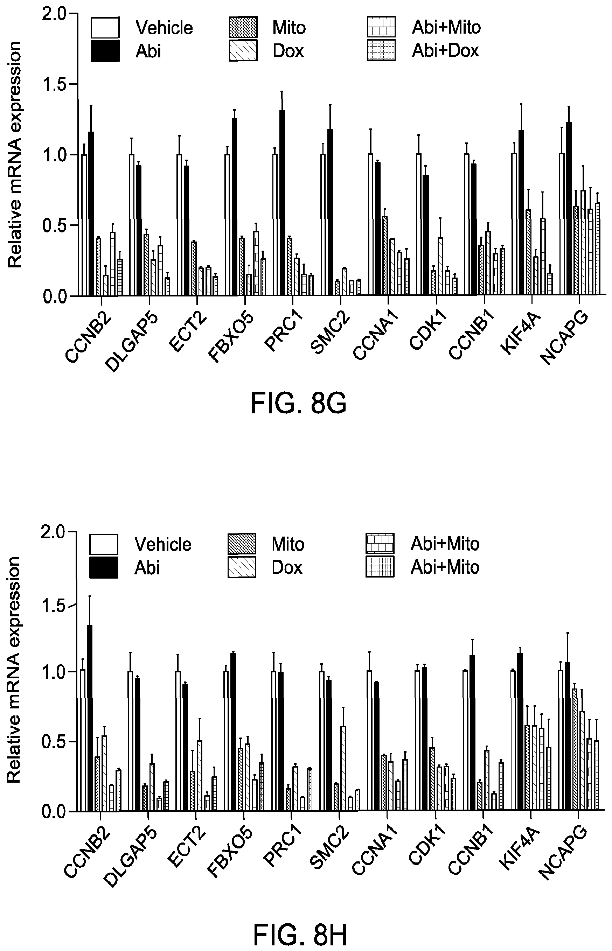

Figures 8A-8H show results from experiments about mitoxantrone (Mito) and doxorubicin (Dox) inhibit Abi resistant PDX tumor growth and modulate gene expression in PDX tumors. (Figure 8A, 8D) (Figure 8A) MC-PRX-01 and (Figure 8D) MC-PRX-06 tumors harvested after 28 days of treatments of Abi alone, TOP2 inhibitors (Mito, Dox) alone, or combination of the two. Tumor weights at the time of harvest were quantified. **p≤0.01, *p≤0.05. (Figure 8B, Figure 8E) Tumor growth plotted for (Figure 8B) MC-PRX-01 and (Figure 8E) MC-PRX-06 during the 28-day treatment period. (Figure 8C, Figure 8F) Mice weight plotted for (Figure 8C) MC-PRX-01 and (Figure 8F) MC-PRX-06 during the 28 days' treatment period. (Figure 8G, Figure 8H) qRT-PCR to validate the 11 genes in post treatment PDX tumors of (Figure 8G) MC-PRX-01 and (Figure 8H) MC-PRX-06.

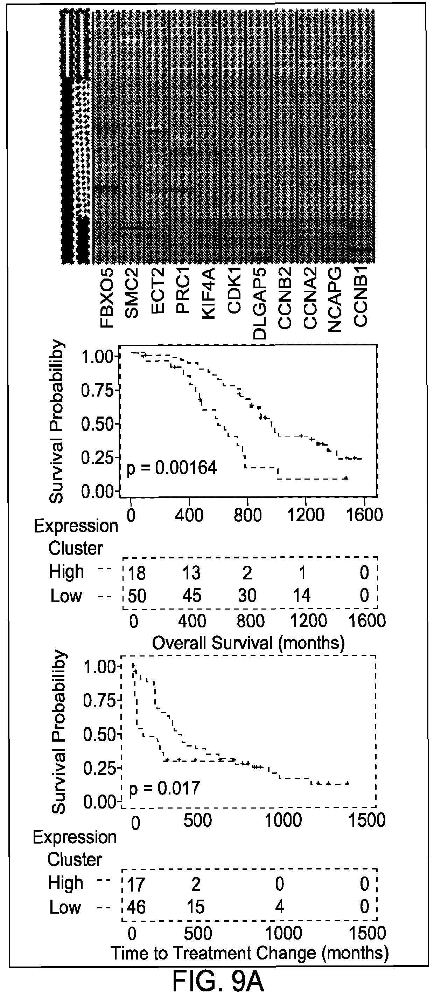

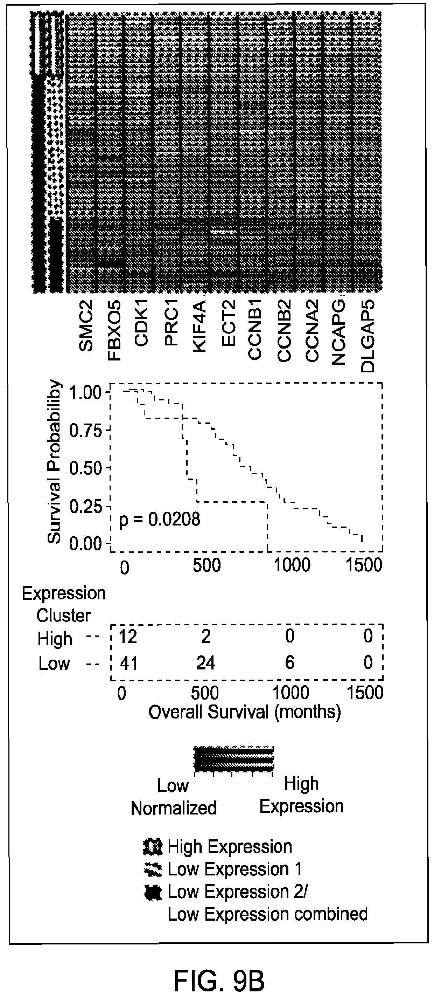

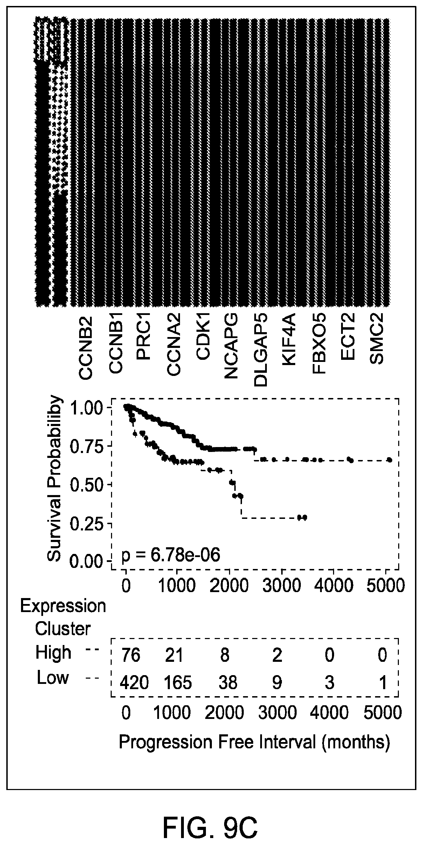

Figures 9A-9C show clustering analyses of the patients' profiles using the 11 gene panel. (Figure 9A, top Panel) Heatmap of expression of the 11 gene targets shared among the four candidate drugs. Patients were arranged after k-means clustering with the clusters arranged on the left indicated by patterned bar ("high expression" cluster as horizontal lines, and the two "low expression" clusters as tilted lines and solid black, respectively, or as solid black combined). Kaplan-Meier analysis of Overall survival (Figure 9A, middle Panel) and Time to treatment change (Figure 9A, bottom panel) based on high- and low expression clusters using the 11 gene panel. p-value of Gehan-Breslow-Wilcoxon test is calculated and indicated in the figure and number of patients in each risk group is indicated below the figures. (Figure 9B) Same analysis using the 11 gene panel with overall survival in the SU2C cohort. (Figure 9C) Same analysis using the 11 gene panels with progression free survival as an outcome in the TCGA prostate cancer cohort.

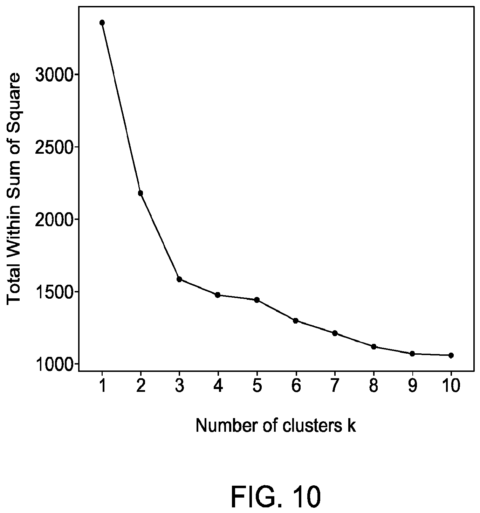

Figure 10 shows elbow plots determining optimal number of clusters in PROMOTE cohort using the 11 genes.

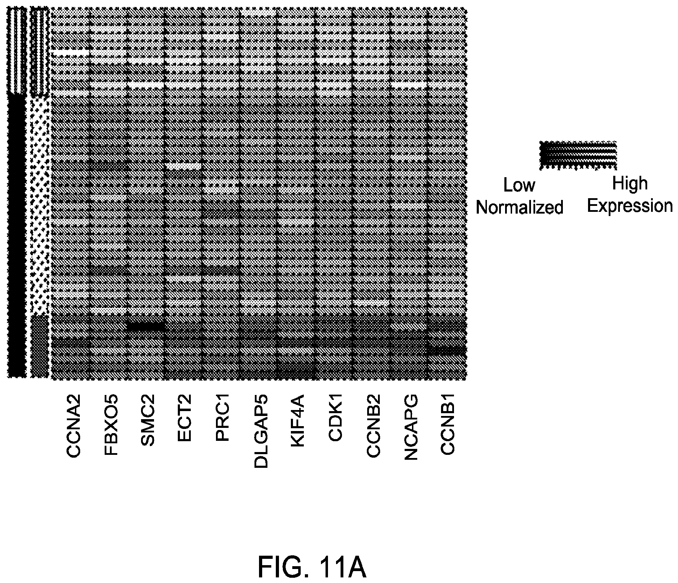

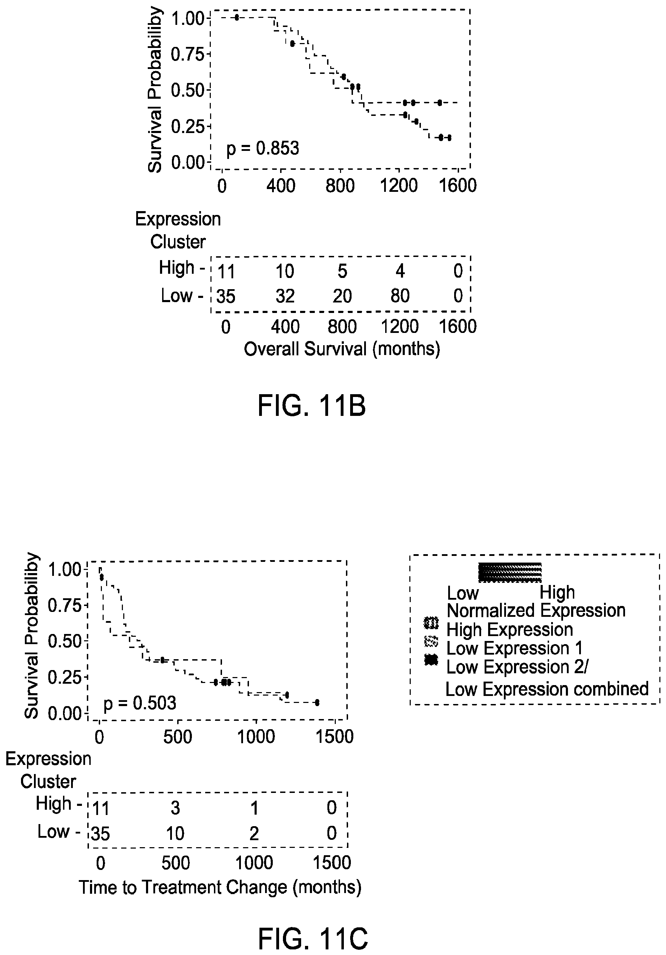

Figures 11A-11C show expression heatmaps and survival analyses using only the PROMOTE bone-metastasis samples. (Figure 11A) Heatmap of PROMOTE bone metastasis sample only, arranged by k-means clustering of samples based on the 11 genes. The clusters were pattern-labeled on the left side of the heatmap. (Figure 11B, Figure 11C) Kaplan-Meier curves for (Figure 11B) overall survival and (Figure 11C) TTC using the 11 gene panel. *p-*values of Gehan-Breslow-Wilcoxon test and number of patients in different risk groups are indicated.

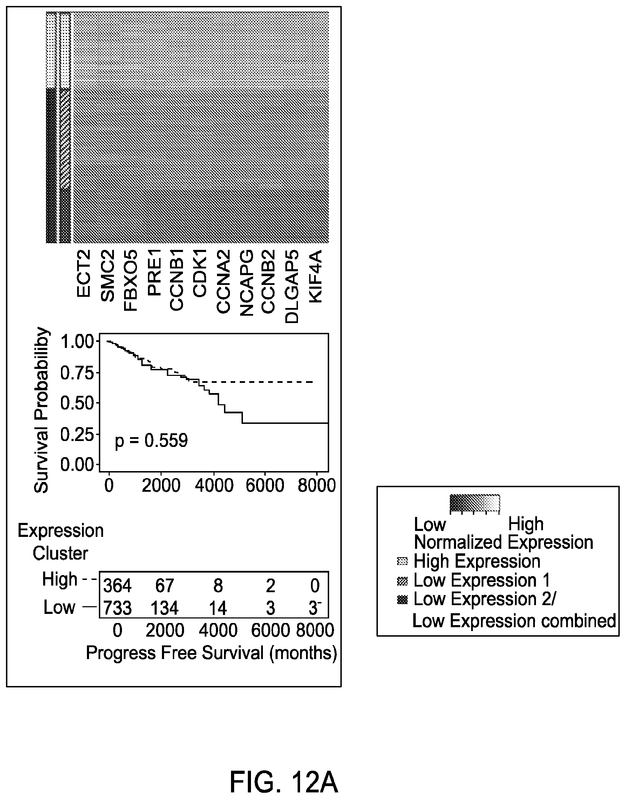

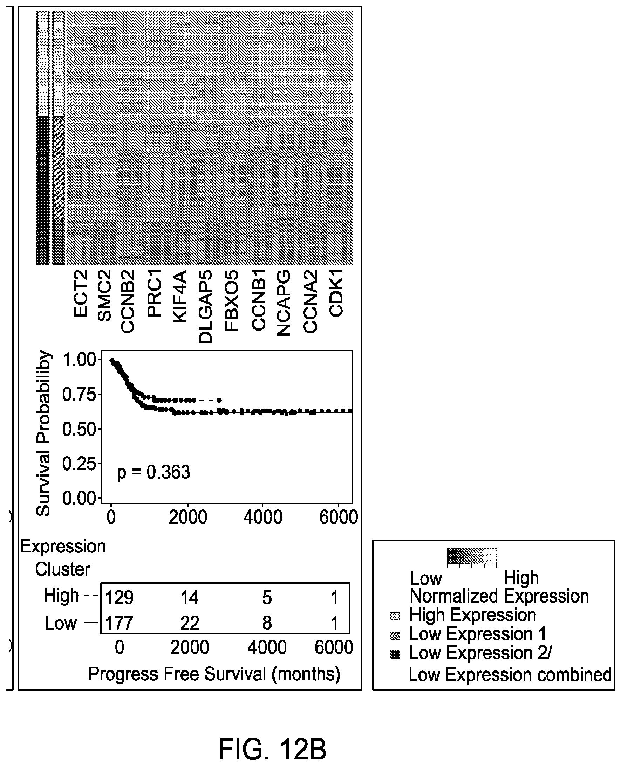

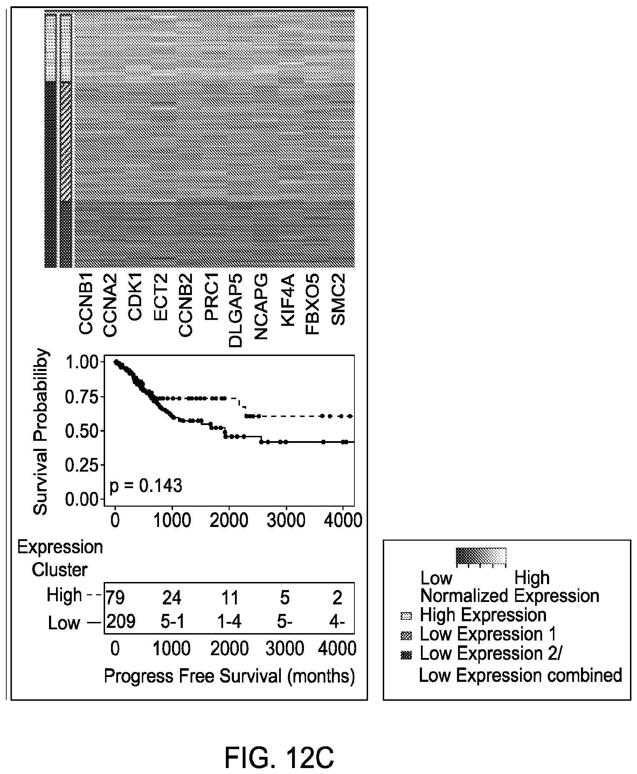

Figures 12A-12C show expression heatmaps and survival analyses using the 11 gene panel in TCGA cohorts. (Figure 12A) breast cancer, (Figure 12B) cervix cancer and (Figure 12C) colon cancer. (Top panels) Heatmaps of 11 gene expression using the RNA seq data from the TCGA breast, cervix and colorectal cancer cohorts. Samples are arranged based on the k-means clustering analysis, with different clusters pattern labeled on the left. (Bottom panels) Kaplan-Meier curves for progress free survival using three sample sets. P-values of Gehan-Breslow-Wilcoxon test and number of patients in different risk groups were indicated.

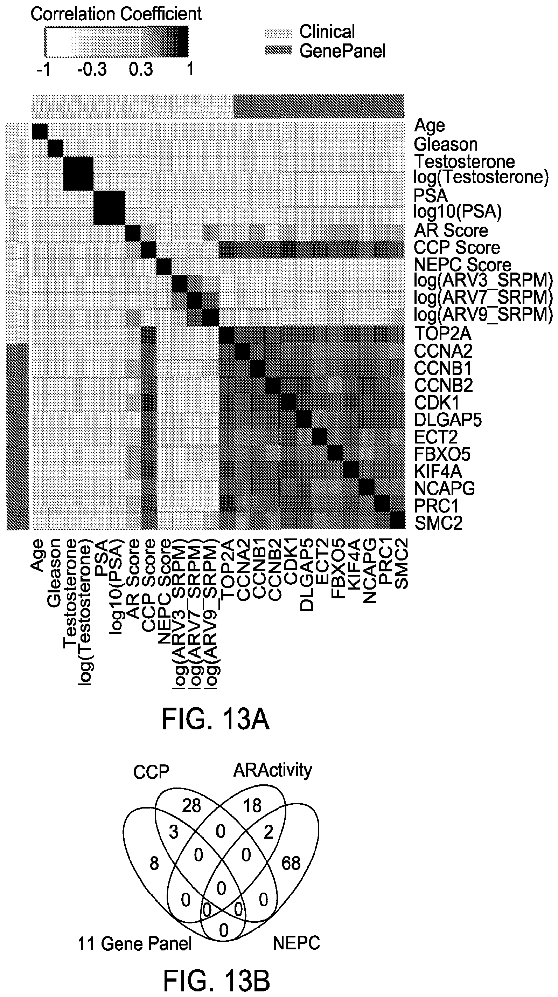

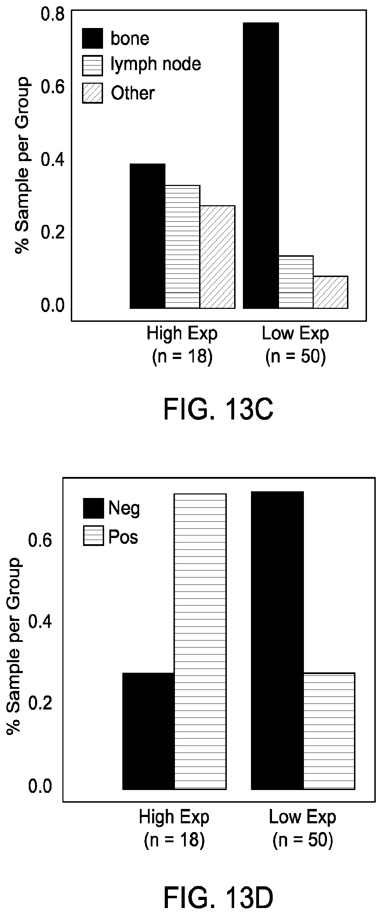

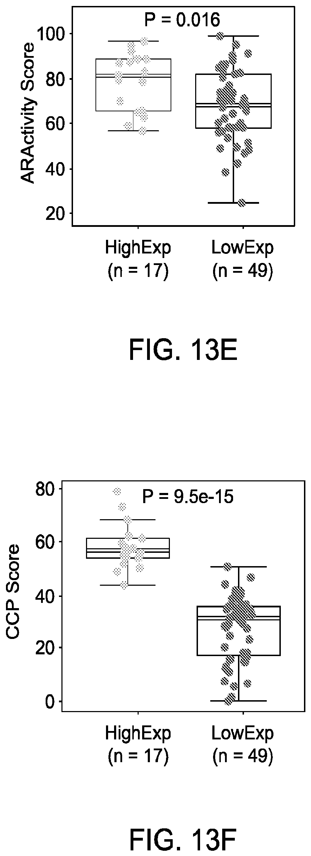

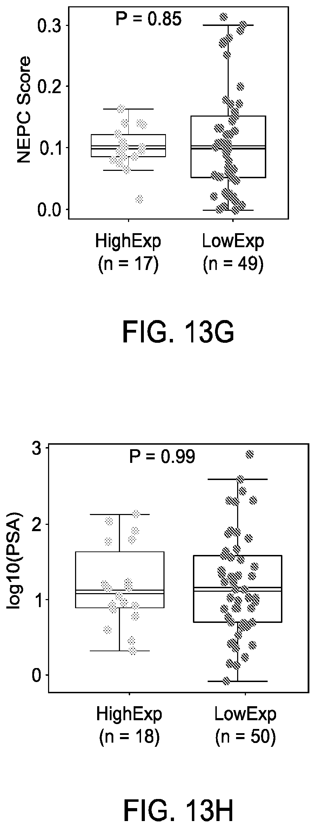

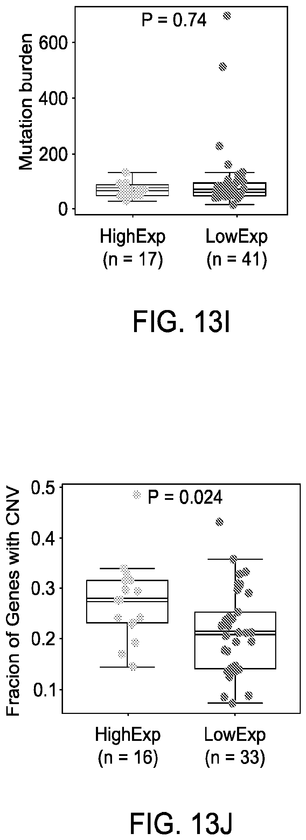

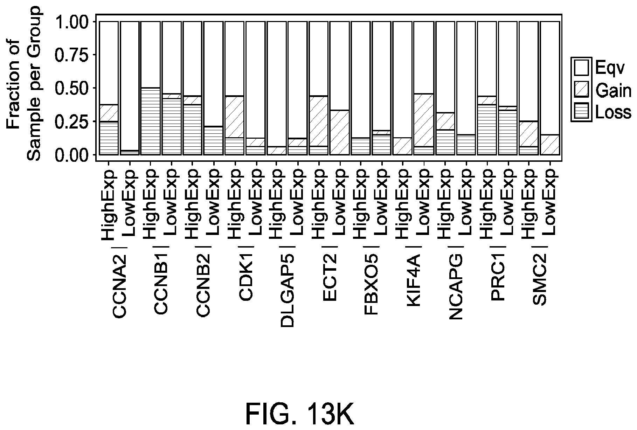

Figures 13A-13K show characteristics of the 11 gene panel and the 11-gene high-expression cluster (HighExp) vs low-expression cluster (LowExp) in PROMOTE cohort. (Figure 13A) Correlation matrix of the 11 gene panel and TOP2A with clinical variables as well as the CCP, NEPC and AR scores using the PROMOTE data. (Figure 13B) Overlapping genes between the 11 gene panel and CCP gene panel, AR activity gene panel or NEPC gene panel, respectively. (Figure 13C) Distribution of Biopsy sites by gene clusters based on the 11 gene panel. (Figure 13D) ETS fusion positivity by gene clusters based on the 11 gene expression. (Figure 13E, Figure 13F, Figure 13G, Figure 13H, Figure 13I, Figure 13J) AR activity score, CCP score, NEPC score, log₁₀ (PSA), mutation burden, and fraction of genes with gain or loss of copy numbers by gene clusters. P-values calculated based on Mann-Whitney test. (Figure 13K) Copy number variation of the 11 genes, comparing between high- and low-expression subgroup of patients.

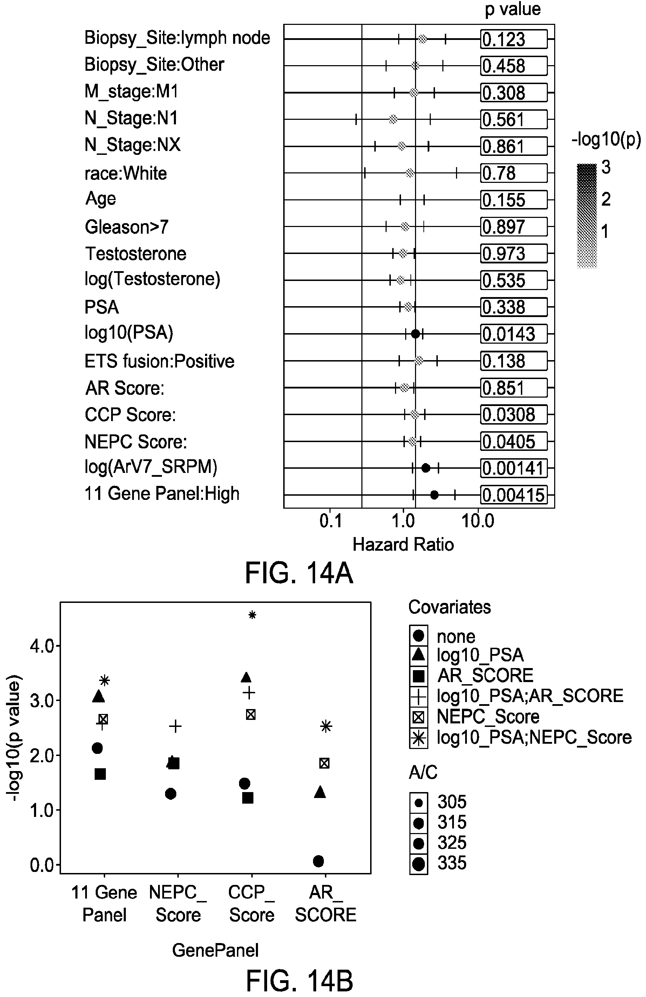

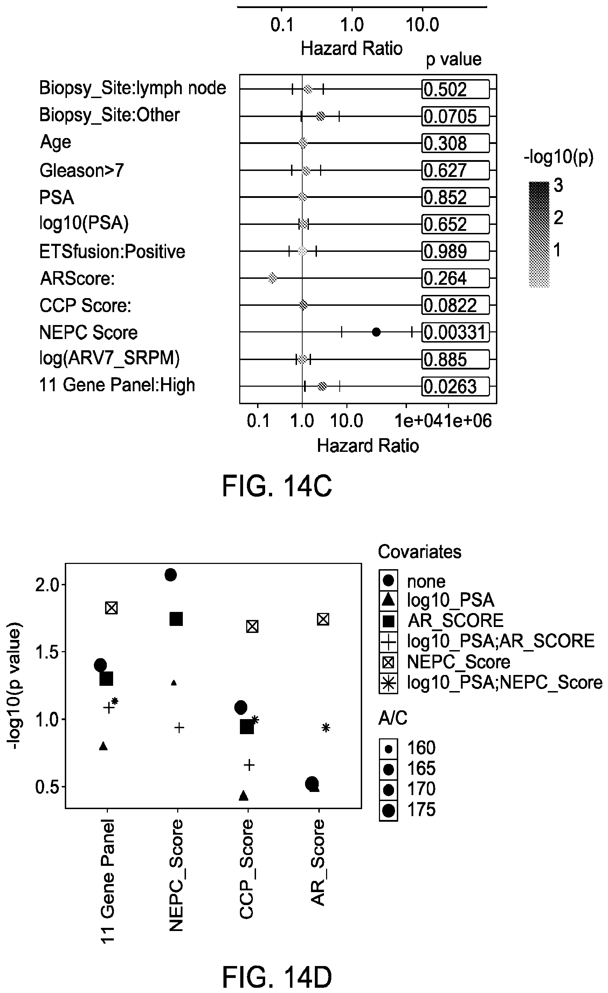

Figures 14A-14D show results from experiments about gene panels serving as independent prognosis predictors using the COX proportional hazard model. (Figure 14A, Figure 14C) Univariate analysis of overall survival against clinical variables and gene panels in (Figure 14A) PROMOTE cohort and (Figure 14C) SU2C cohort, respectively. X-axis represents the hazard ratio, plotted in a log scale, with error bars indicating 95% confidence interval. NEPC Score for SU2C cohort was out of range and thus plotted separately. Colors indicated -log10 (p-values) for univariate significance test, and actual p-values are indicated on the right side of each variable with p ≤0.05 highlighted in yellow. (Figure 14B, Figure 14D) COX multivariate model fitting overall survival with the 11 gene panel, AR score, CCP score or NEPC score alone or in combination with log10(PSA) or other scores, as indicated by different symbols, using (Figure 14B) PROMOTE and (Figure 14D) SU2C cohorts, respectively. Size represents Akaike information criterion (AIC) for each model.

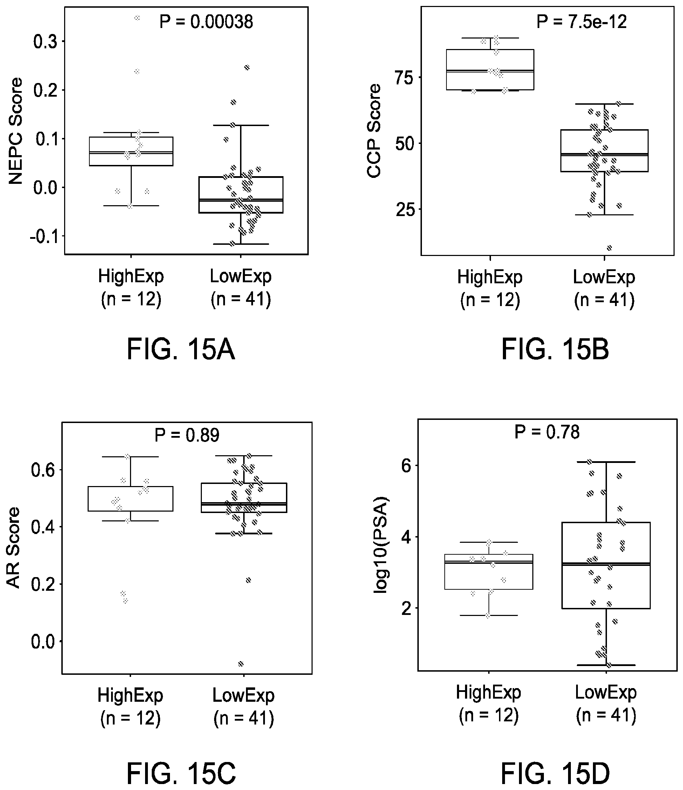

Figures 15A-15D show characteristics of the 11 gene panel and the 11-gene high-expression cluster (HighExp) vs low-expression cluster (LowExp) in SU2C cohort. (Figure 15A) NEPC score, (Figure 15B) CCP score, (Figure 15C) AR score, and (Figure 15D) log₁₀(PSA) by gene clusters based on the 11 gene panel in the SU2C cohort. P-values calculated based on the Mann-Whitney test.

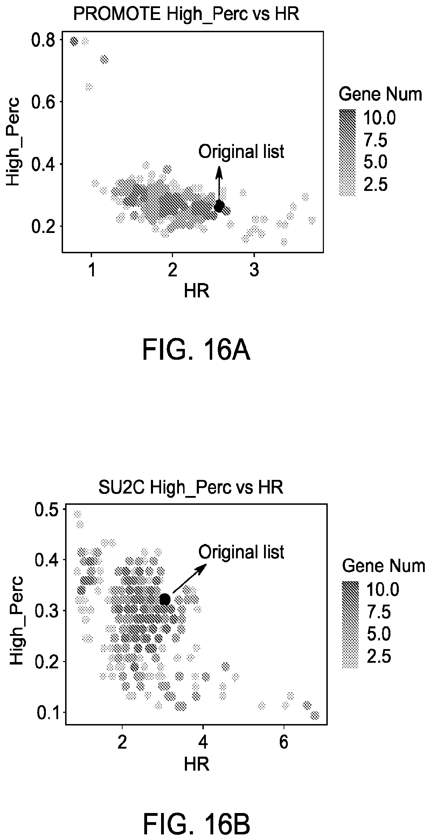

Figure 16A-B shows subsets of the 11 gene panel as Abi-prognostic marker. The figure was presented as percentage of patients classified to be high-expression cluster versus hazard ratio of overall survival between high- and low-expression cluster in (Figure 16A) PROMOTE and (Figure 16B) SU2C cohorts. Grayscale represents number of genes included in the analysis. Genes 1 to 10 out of the 11 (2047 combination) were selected, clustering was redone, and prognostic significance (hazard ratio) was tested.

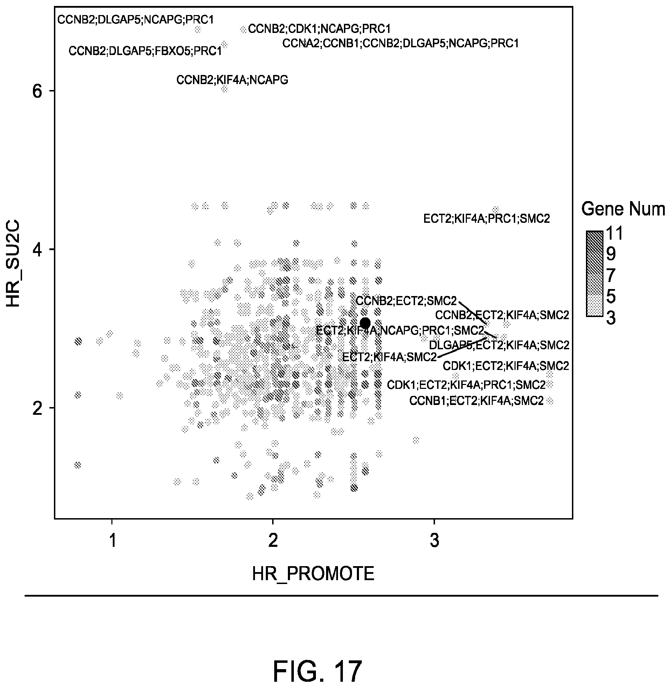

Figure 17 shows subsets of the 11 gene panel as Abi-prognostic marker. The figure was presented as hazard ratio of overall survival between high- and low-expression cluster in SU2C cohort versus in PROMOTE cohort.

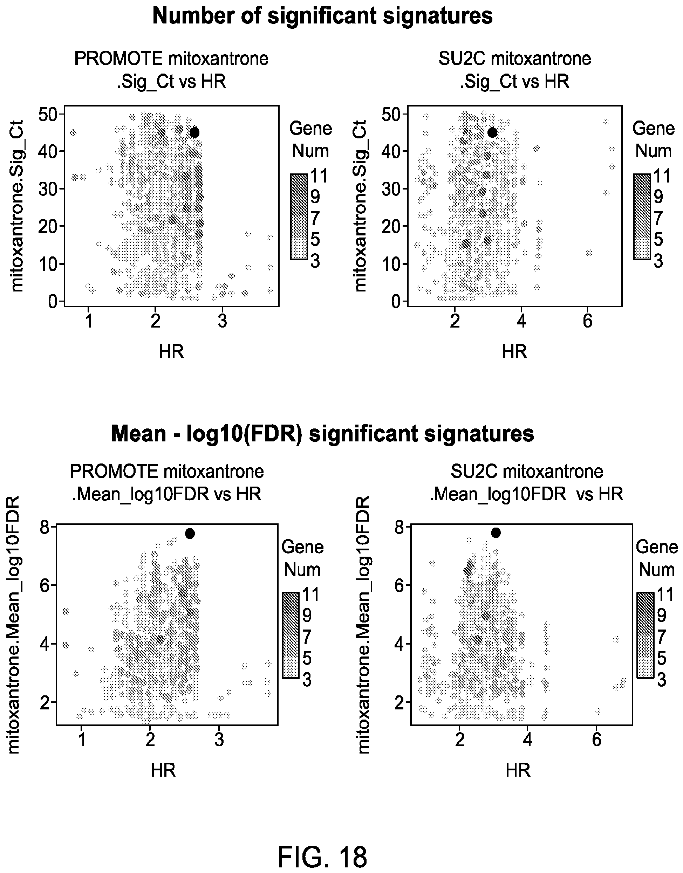

Figure 18 shows subsets of the 11 gene panel as markers for alternative therapy with mitoxantrone. Genes 1 to 10 out of the 11 (2047 combination) were selected and submitted to L1000 chem perturbation data base for significature search. Signatures of mitoxantrone significant at FDR≤0.05 were included. Patient was clustered using gene subset, and prognostic significance (hazard ratio) was tested. The figures were presented as either number of significant signatures returned from L1000 search (Figure 18A, Figure 18B) or mean -log₁₀(FDR) of significant signatures (Figure 18C, Figure 18D) versus hazard ratios of overall survival between high- and low-expression cluster in PROMOTE cohort (Figure 18A, Figure 18C) and in SU2C cohort (Figure 18B, Figure 18D).

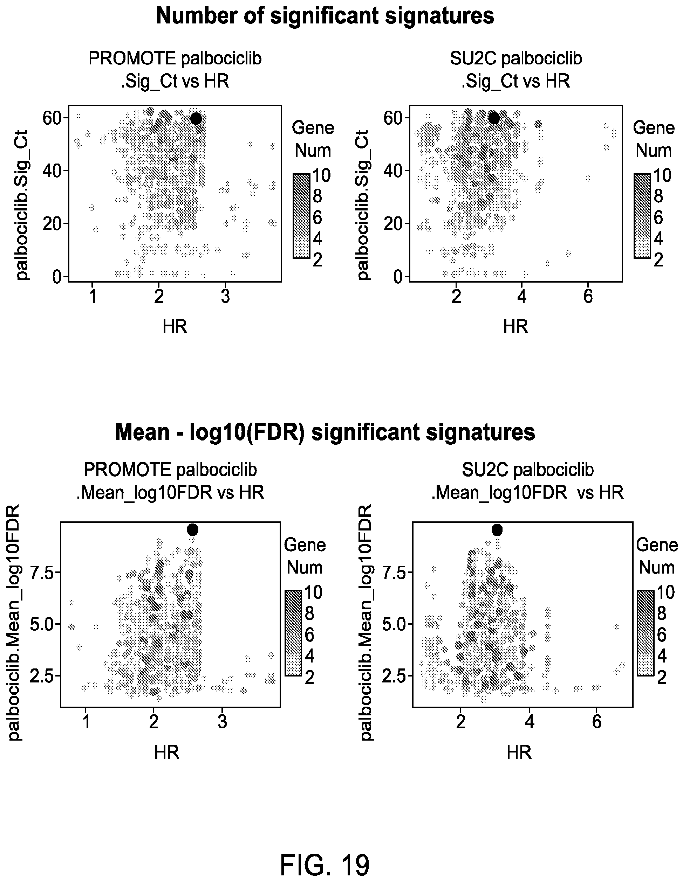

Figure 19 shows subsets of the 11 gene panel as markers for alternative therapy with palbociclib. Genes 1 to 10 out of the 11 (2047 combination) were selected and submitted to L1000 chem perturbation data base for significature search. Signatures of palbociclib significant at FDR≤0.05 were included. Patient was clustered using gene subset, and prognostic significance (hazard ratio) was tested. The figures were presented as either number of significant signatures returned from L1000 search (Figure 18A, Figure 18B) or mean -log₁₀(FDR) of significant signatures (Figure 18C, Figure 18D) versus hazard ratios of overall survival between high- and low-expression cluster in PROMOTE cohort (Figure 18A, Figure 18C) and in SU2C cohort (Figure 18B, Figure 18D).

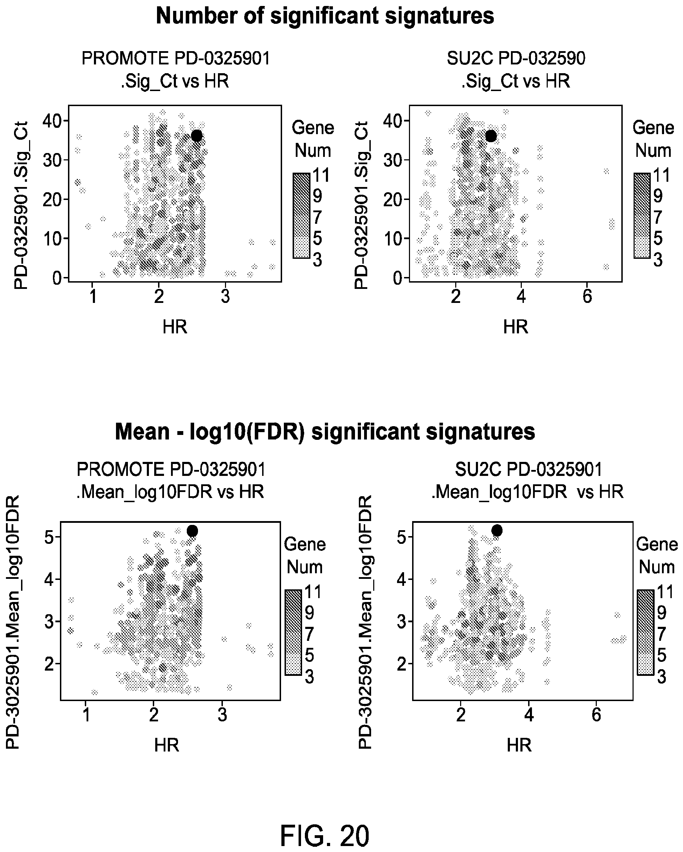

Figure 20 shows subsets of the 11 gene panel as markers for alternative therapy with PD-0325901. Genes 1 to 10 out of the 11 (2047 combination) were selected and submitted to L1000 chem perturbation data base for significature search. Signatures of PD-0325901 significant at FDR≤0.05 were included. Patient was clustered using gene subset, and prognostic significance (hazard ratio) was tested. The figures were presented as either number of significant signatures returned from L1000 search (Figure 18A, Figure 18B) or mean -log₁₀(FDR) of significant signatures (Figure 18C, Figure 18D) versus hazard ratios of overall survival between high- and low-expression cluster in PROMOTE cohort (Figure 18A, Figure 18C) and in SU2C cohort (Figure 18B, Figure 18D).

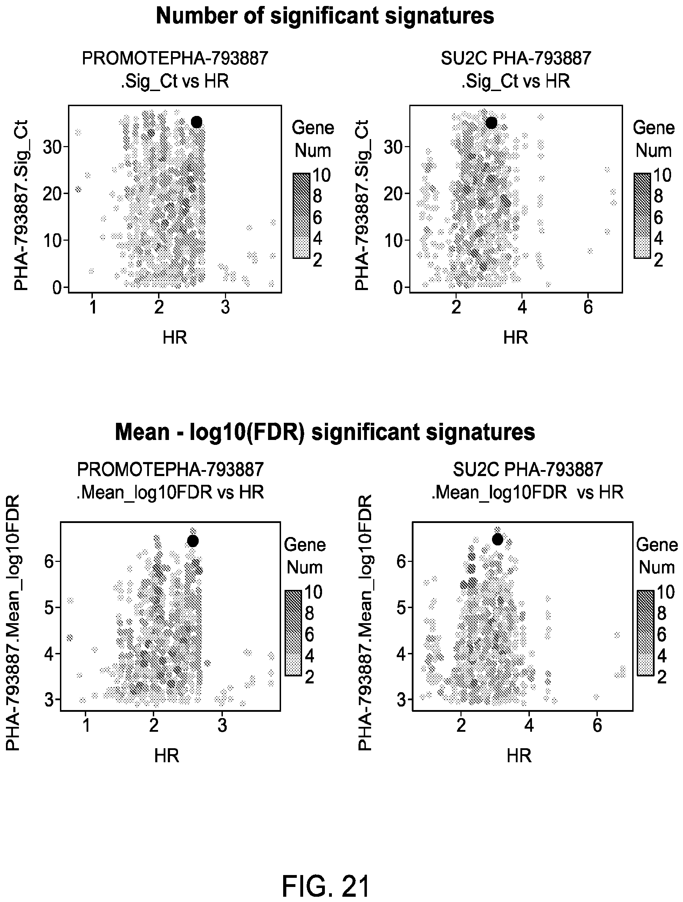

Figure 21 shows subsets of the 11 gene panel as markers for alternative therapy with PHA-793887. Genes 1 to 10 out of the 11 (2047 combination) were selected and submitted to L1000 chem perturbation data base for significature search. Signatures of PHA-793887 significant at FDR≤0.05 were included. Patient was clustered using gene subset, and prognostic significance (hazard ratio) was tested. The figures were presented as either number of significant signatures returned from L1000 search (Figure 18A, Figure 18B) or mean -log₁₀(FDR) of significant signatures (Figure 18C, Figure 18D) versus hazard ratios of overall survival between high- and low-expression cluster in PROMOTE cohort (Figure 18A, Figure 18C) and in SU2C cohort (Figure 18B, Figure 18D).

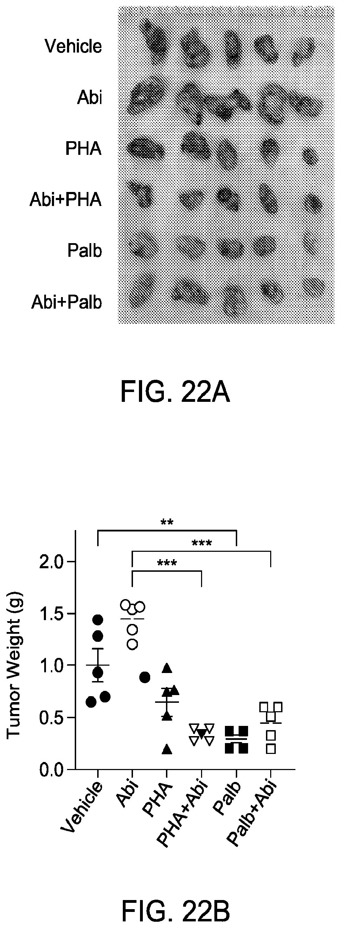

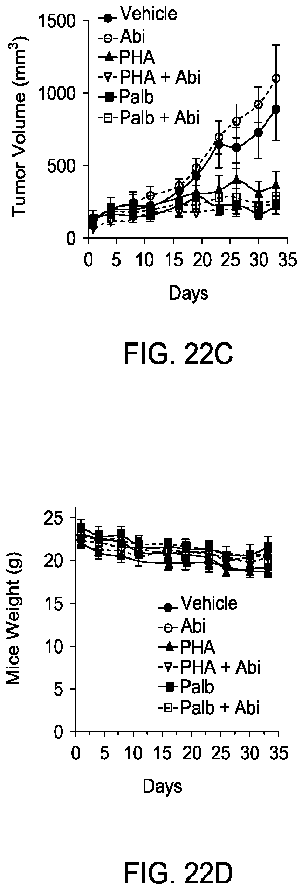

Figures 22A - 22D show results from a MC-PRX-01 PDX model treated with CDK inhibitors Palbociclib (Palb) and PHA-793887 (PHA). (Figure 22A) Tumors harvested after 35 days of treatments of Abi alone, CDK inhibitors (Palb, PHA) alone, or combination of the two. (Figure 22B) Tumor weights at the time of harvest were quantified. Statistical significance indicated unpaired t-test: **p≤0.01, ***p≤0.001. (n=5) (Figure 22C) Tumor growth during the CDK inhibitors treatment period. (Figure 22D) Mice body weight during the CDK inhibitors treatment period.

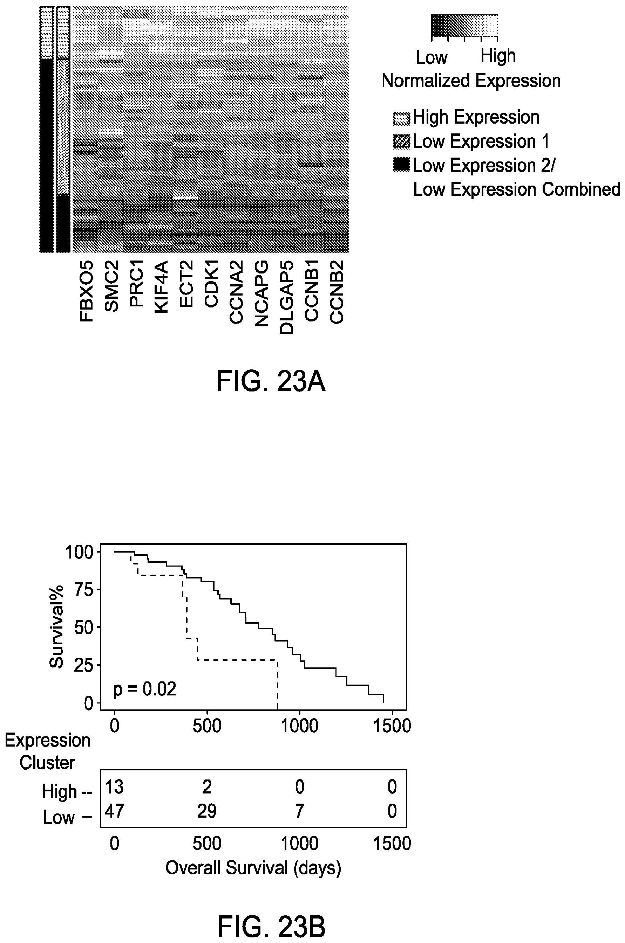

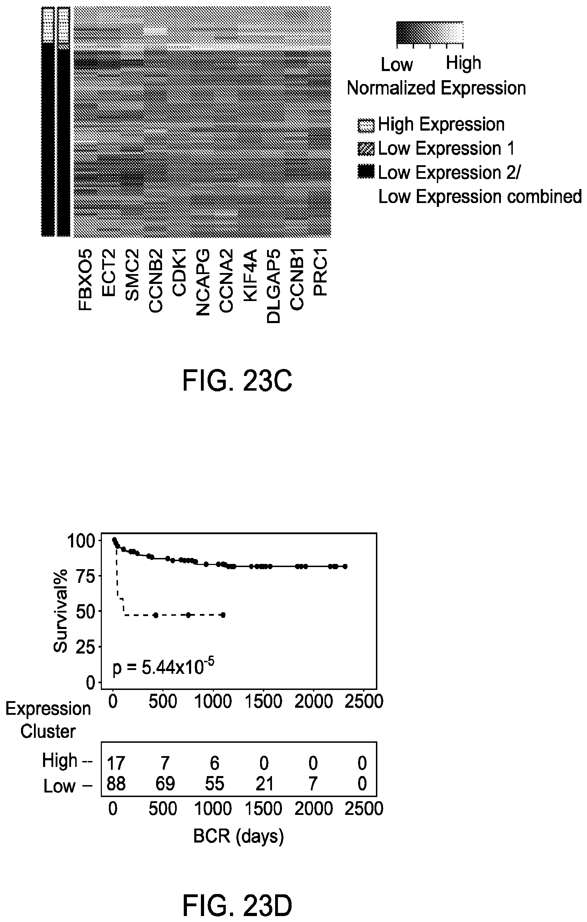

Figures 23A - 23D show clustering analyses of the patients' profiles using the 11 gene panel. (Figure 23A) Heatmap of expression and (Figure 23B) Kaplan-Meier analysis using the 11 gene panel with overall survival in the SU2C cohort including Enzalutamide treatment arm. (Figure 23C) Heatmap of expression and (Figure 23D) Kaplan-Meier analysis using the 11 gene panels with biochemical relapse survival as an outcome in the DKFZ early-onset prostate cancer cohort.

### DETAILED DESCRIPTION

This document provides methods and materials involved in assessing and/or treating mammals (e.g., humans) having prostate cancer. In some cases, the methods and materials provided herein can be used to determine whether or not a mammal having prostate cancer is likely to respond to a particular cancer treatment (*e.g.,* an anti-androgen agent). For example, a sample (*e.g.,* a sample containing one or more cancer cells) obtained from a mammal having prostate cancer can be assessed for the presence or absence of an increased level of expression of CCNA2 polypeptides, CCNB1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, and/or KIF4A polypeptides to determine whether or not the mammal is likely to respond to an anti-androgen agent (*e.g.,* abiraterone). In some cases, the methods and materials provided herein also can include administering one or more cancer treatments to a mammal having prostate cancer to treat the mammal (*e.g.,* one or more cancer treatments selected based, at least in part, on whether or not the mammal is likely to respond to a particular cancer treatment such as an anti-androgen agent).

A mammal (*e.g.,* a human) having prostate cancer can be assessed to determine whether or not the cancer is likely to respond to a particular cancer treatment (*e.g.,* an anti-androgen agent) by detecting the presence or absence of an increased level of expression of one or more polypeptides in a sample (*e.g.,* a sample containing one or more cancer cells) obtained from the mammal. As described herein, the presence of an increased level of expression of one or more polypeptides in a sample obtained from the mammal can be used to determine whether or not that mammal is likely to respond to a particular cancer treatment (*e.g.,* an anti-androgen agent). For example, the presence of an increased level of expression of CCNA2 polypeptides, CCNB1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, and/or KIF4A polypeptides in a sample obtained from a mammal having prostate cancer can be used to identify that mammal as being unlikely to respond to one or more anti-androgen agents. Also as demonstrated herein, one or more TOP2A inhibitors and/or one or more CDK 4/6 inhibitors can be used to sensitize prostate cancers to one or more anti-androgen agents. For example, one or more TOP2A inhibitors, one or more CDK 4/6 inhibitors, one or more MEK inhibitors, and/or one or more pan-CDK inhibitors can be administered to a mammal having prostate cancer and identified as having the presence of an increased level of expression of CCNA2 polypeptides, CCNB1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, and/or KIF4A polypeptides to sensitize the mammal to one or more anti-androgen agents, and optionally, the mammal can be administered one or more anti-androgen agents to treat the mammal.

Any appropriate mammal having prostate cancer can be assessed and/or treated as described herein. In some cases, a mammal having prostate cancer can have undergone no prior treatment for the prostate cancer. In some cases, a mammal having prostate cancer can have undergone treatment for the prostate cancer. For example, a mammal have prostate cancer can have undergone a surgical treatment for the prostate cancer. For example, a mammal having prostate cancer can have been administered one or more anti-cancer agents (e.g., one or more anti-androgen agents such as abiraterone and/or one or more cancer drugs that are not an anti-androgen agent such as docetaxel). Examples of mammals that can have prostate cancer and can be assessed and/or treated as described herein include, without limitation, humans, non-human primates (*e.g.,* monkeys), dogs, cats, horses, cows, pigs, sheep, rabbits, mice, rats, and Guinea pigs, hamsters. In some cases, a mammal can be a male mammal. For example, a male human having prostate cancer can be assessed and/or treated as described herein.

When assessing and/or treating a mammal (*e.g.,* a human) having prostate cancer as described herein, the prostate cancer can be any type of prostate cancer. A prostate cancer can be any stage of prostate cancer (*e.g.,* stage I, stage II, stage III, or stage IV). A prostate cancer can be any grade of prostate cancer (*e.g.,* grade 1, grade 2, or grade 3). A prostate cancer can have any Gleason score. In some cases, a prostate cancer can be a primary cancer (*e.g.,* a localized primary cancer). In some cases, a prostate cancer can have metastasized. In some cases, a prostate cancer can be castration-sensitive prostate cancer (CSPC). In some cases, a prostate cancer can be castration-resistant prostate cancer (CRPC). In some cases, a prostate cancer can be hormone-refractory prostate cancer (HRPC).

In some cases, the methods described herein can include identifying a mammal (*e.g.,* a human) as having prostate cancer. Any appropriate method can be used to identify a mammal as having prostate cancer. For example, physical examination (*e.g.,* a digital rectal examination (DRE)), laboratory testing (*e.g.,* blood tests for prostate-specific antigen (PSA) test), imaging techniques (*e.g.,* ultrasound, magnetic resonance imaging (MRI), bone scan, computerized tomography (CT) scan, and positron emission tomography (PET) scan), and biopsy techniques can be used to identify a mammal (*e.g.,* a human) as having prostate cancer.

In some cases, a mammal (*e.g.,* a human) having prostate cancer can be assessed to determine whether or not the cancer is likely to respond to a particular cancer treatment (*e.g.,* an anti-androgen agent such as abiraterone) based, at least in part, on the presence or absence of an increased level of expression of one or more (*e.g.,* one, two, three, four, five, six, seven, eight, nine, ten, eleven, or more) polypeptides in a sample (*e.g.,* a sample containing one or more cancer cells) obtained from the mammal. The term "increased level" as used herein with respect to a level of a polypeptide refers to any level that is greater than a reference level of that polypeptide. The term "reference level" as used herein with respect to a polypeptide refers to the level of that polypeptide typically observed in a sample *(e.g.,* a control sample) from one or more comparable mammals (*e.g.,* humans of comparable age) that do not have prostate cancer. In some cases, a reference level can be obtained using a machine learning based clustering method. Control samples can include, without limitation, comparable samples from mammals that do not have prostate cancer. Examples of polypeptides that can have increased levels of expression in a sample from a mammal having prostate cancer include, without limitation, CCNA2 polypeptides, CCNB1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, KIF4A polypeptides, androgen receptor (AR) polypeptides (e.g., AR splice variant polypeptides), PSA polypeptides, TOP2A polypeptides, ERV fusion polypeptides, tumor protein P53 (TP53) polypeptides, speckle type BTB/POZ Protein (SPOP) polypeptides, and forkhead box A1 (FOXA1) polypeptides. In some cases, an increased level of expression of a polypeptide can be a level that is at least 2 (*e.g.,* at least 5, at least 10, at least 15, at least 20, at least 25, at least 35, or at least 50) fold greater relative to a reference level of that polypeptide. In some cases, when control samples have an undetectable level of a polypeptide, an increased level can be any detectable level of that polypeptide. It will be appreciated that levels from comparable samples are used when determining whether or not a particular level is an increased level. In some cases, a polypeptide having an increased level of expression in a sample from a mammal having prostate cancer can be as described in Example 1.

In some cases, the methods described herein can include detecting the presence or absence of an increased level of expression of a panel of polypeptides. For example, a panel of polypeptides can include any two or more (*e.g.,* two, three, four, five, six, seven, eight, nine, ten, eleven, or more) of the polypeptides described herein. In some cases, the presence or absence of two or more (*e.g.,* two, three, four, five, six, seven, eight, nine, ten, eleven, or more) polypeptides in a sample (*e.g.,* a sample containing one or more cancer cells) obtained from a mammal (*e.g.,* a human) having prostate cancer can be used to determine whether or not that mammal is likely to respond to a particular cancer treatment (*e.g.,* an anti-androgen agent).

Any appropriate method can be used to detect the presence or absence of an increased level of expression of one or more polypeptides within a sample *(e.g.,* a sample containing one or more cancer cells) obtained from a mammal (*e.g.,* a human). In some cases, a level of polypeptide expression within a sample can be determined by detecting the presence, absence, or level of the polypeptide in the sample. For example, immunoassays (*e.g.,* immunohistochemistry (IHC) techniques, western blotting techniques, enzyme-linked immunosorbent assays (ELISAs), immunoprecipitation, and immunofluorescence such as immunofluorescence coupled flow cytometry), mass spectrometry techniques (*e.g.,* proteomics-based mass spectrometry assays or targeted quantification-based mass spectrometry assays), enzyme-linked immunosorbent assays (ELISAs), and radioimmunoassays can be used to determine the presence, absence, or level of a polypeptide in a sample. In some cases, a level of polypeptide expression within a sample can be determined by detecting the presence, absence, or level of mRNA encoding the polypeptide in the sample. For example, polymerase chain reaction (PCR)-based techniques such as quantitative RT-PCR techniques, nanoString ncounter techniques, gene expression panels or arrays (*e.g.,* next generation sequencing (NGS) such as RNA-seq, miRNAseq, amplicon sequencing, and nanopore sequencing), *in situ* hybridization (ISH) such as fluorescence *in situ* hybridization (FISH), and gel electrophoresis can be used to determine the presence, absence, or level of mRNA encoding the polypeptide in the sample. In some cases, a level of polypeptide expression within a sample can be determined using a machine learning based clustering algorithm. Examples of machine learning based clustering algorithms include, without limitation, kmeans, hierarchical clustering, support vector machines, decision trees, random forests, mean-shift clustering, density-based spatial clustering of applications with noise, and expectation-maximization (EM) clustering using Gaussian mixture models (GMM). In some cases, the presence or absence of an increased level of expression of one or more polypeptides within a sample from a mammal having prostate cancer can be determined as described in Example 1.

In some cases, a mammal (*e.g.,* a human) having prostate cancer can be identified as being unlikely to respond to a particular cancer treatment (*e.g.,* an anti-androgen agent such as abiraterone) based, at least in part, on the presence of an increased level of expression of CCNA2 polypeptides, CCNB1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, and/or KIF4A polypeptides in a sample (*e.g.,* a sample containing one or more cancer cells) obtained from the mammal. For example, a mammal having prostate cancer can be identified as being unlikely to respond to one or more anti-androgen agents (*e.g.,* abiraterone) based, at least in part, on the presence of an increased level of expression of CCNA2 polypeptides, CCNB 1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, and/or KIF4A polypeptides in a sample obtained from the mammal.

In some cases, a mammal (*e.g.,* a human) having prostate cancer can be identified as being likely to respond to a particular cancer treatment (*e.g.,* an anti-androgen agent such as abiraterone) based, at least in part, on the absence of an increased level of expression of CCNA2 polypeptides, CCNB1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, and/or KIF4A polypeptides in a sample (*e.g.,* a sample containing one or more cancer cells) obtained from the mammal. For example, a mammal having prostate cancer can be identified as being likely to respond to one or more anti-androgen agents (*e.g.,* abiraterone) based, at least in part, on the absence of an increased level of expression of CCNA2 polypeptides, CCNB1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, and/or KIF4A polypeptides in a sample obtained from the mammal.

In some cases, a mammal (*e.g.,* a human) having prostate cancer can be identified as being likely to respond to a particular cancer treatment (*e.g.,* a TOP2 inhibitor, a CDK4/6 inhibitor, a MEK inhibitor, and a pan-CDK inhibitor) based, at least in part, on the absence of an increased level of expression of CCNA2 polypeptides, CCNB1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, and/or KIF4A polypeptides in a sample (*e.g.,* a sample containing one or more cancer cells) obtained from the mammal. For example, a mammal having prostate cancer can be identified as being likely to respond to one or more TOP2 inhibitors, one or more CDK4/6 inhibitors, one or more MEK inhibitors, and/or one or more pan-CDK inhibitors based, at least in part, on the absence of an increased level of expression of CCNA2 polypeptides, CCNB1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, and/or KIF4A polypeptides in a sample obtained from the mammal.

Any appropriate sample from a mammal (*e.g.,* a human) having prostate cancer can be assessed as described herein (*e.g.,* for the presence or absence of an increased level of expression of one or more polypeptides). In some cases, a sample can be a biological sample. In some cases, a sample can contain one or more cancer cells. In some cases, a sample can contain one or more biological molecules (*e.g.,* nucleic acids such as DNA and RNA, polypeptides, carbohydrates, lipids, hormones, and/or metabolites). Examples of samples that can be assessed as described herein include, without limitation, tissue samples (*e.g.,* prostate tissue samples or prostate cancer tissue biopsies), fluid samples (*e.g.,* whole blood, serum, plasma, urine, and saliva), and cellular samples (e.g., samples containing circulating cancer cells). A sample can be a fresh sample or a fixed sample (*e.g.,* a formaldehyde-fixed sample or a formalin-fixed sample). In some cases, a sample can be a processed sample (*e.g.,* an embedded sample such as a paraffin or OCT embedded sample). In some cases, one or more biological molecules can be isolated from a sample. For example, nucleic acid (*e.g.,* DNA and RNA such as messenger RNA (mRNA)) can be isolated from a sample and can be assessed as described herein. In some cases, one or more polypeptides can be isolated from a sample and can be assessed as described herein.

When treating a mammal (*e.g.,* a human) having prostate cancer and identified as being likely to respond to one or more anti-androgen agents as described herein (*e.g.,* based, at least in part, on the absence of an increased level of expression of one or more polypeptides), the mammal can be administered or instructed to self-administer one or more (*e.g.,* one, two, three, four, five, or more) anti-androgen agents. For example, a mammal having cancer and identified as lacking an increased level of expression of CCNA2 polypeptides, CCNB1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, and/or KIF4A polypeptides in a sample (*e.g.,* a sample containing one or more cancer cells) obtained from the mammal can be administered or instructed to self-administer one or more anti-androgen agents. For example, a mammal having prostate cancer and identified as lacking an increased level of expression of CCNA2 polypeptides, CCNB1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, and KIF4A polypeptides in a sample (*e.g.,* a sample containing one or more cancer cells) obtained from the mammal can be administered or instructed to self-administer one or more anti-androgen agents. Examples of anti-androgen agents include, without limitation, leuprolide (*e.g.,* LUPRON DEPOT^{®} and ELIGARD^{®}), goserelin (*e.g.,* ZOLADEX^{®}), triptorelin (*e.g.,* TRELSTAR^{®}), histrelin (*e.g.,* VANTAS^{®}), degarelix (*e.g.,* FIRMAGON^{®}), abiraterone (*e.g.,* ZYTIGA^{®}), ketoconazole (*e.g.,* NIZORAL^{®}), flutamide *(e.g.,* EULEXIN^{®}), bicalutamide *(e.g.,* CASODEX^{®}), nilutamide *(e.g.,* NILANDRON^{®}), enzalutamide *(e.g.,* XTANDI^{®}), apalutamide *(e.g.,* ERLEADA^{®}), and darolutamide (*e.g.,* NUBEQA^{®}).

In some cases, a mammal (*e.g.,* a human) having prostate cancer and identified as being likely to respond to one or more anti-androgen agents as described herein (e.g., based, at least in part, on the absence of an increased level of expression of one or more polypeptides) can undergo a surgical hormone therapy *(e.g.,* in addition to or as an alternative to being administered one or more anti-androgen agents). For example, a mammal having prostate cancer and identified as lacking an increased level of expression of CCNA2 polypeptides, CCNB1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, and/or KIF4A polypeptides in a sample (*e.g.,* a sample containing one or more cancer cells) obtained from the mammal can undergo an orchiectomy (removal of the testicles).

When treating a mammal (*e.g.,* a human) having prostate cancer and identified as being unlikely to respond to one or more anti-androgen agents as described herein (*e.g.,* based, at least in part, on the presence of an increased level of expression of one or more polypeptides), the mammal can be administered or instructed to self-administer one or more (*e.g.,* one, two, three, four, five, or more) alternative cancer treatments (*e.g.,* one or more cancer treatments that are not an anti-androgen agent). For example, a mammal having prostate cancer and identified as having the presence of an increased level of expression of CCNA2 polypeptides, CCNB1 polypeptides, CCNB2 polypeptides, PRC1 polypeptides, SMC2 polypeptides, DLGAP5 polypeptides, ECT2 polypeptides, FBXO5 polypeptides, CDK1 polypeptides, NCAPG polypeptides, and/or KIF4A polypeptides in a sample (*e.g.,* a sample containing one or more cancer cells) obtained from the mammal can be administered or instructed to self-administer one or more alternative cancer treatments that are not anti-androgen agents. Examples of alternative cancer treatments that are not an anti-androgen agent include, without limitation, administering one or more cancer drugs (*e.g.,* chemotherapeutic agents, targeted cancer drugs, and immunotherapy drugs) other than an anti-androgen agent to a mammal in need thereof. Examples of cancer drugs that are not an anti-androgen agent and that can be administered to a mammal having prostate cancer and identified as being unlikely to respond to an anti-androgen agent include, without limitation, docetaxel *(e.g.,* TAXOTERE^{®}), cabazitaxel *(e.g.,* JEVTANA^{®}), mitoxantrone *(e.g.,* NOVANTRONE^{®}), doxorubicin (*e.g.* ADRIAMYCIN^{®}, CAELYX^{®}, and MYOCET^{®}) palbociclib (*e.g.,* IBRANCE^{®}), ribociclib (KISQALI^{®}), abemaciclib (VERZENIO^{®}), PD-0325901 (mirdametinib), and PHA-793887, and combinations thereof. In some cases, an alternative cancer treatment can include surgery. Examples of surgeries that can be performed on a mammal having prostate cancer include, without limitation, radical prostatectomy (removal of the prostate gland). In some cases, an alternative cancer treatment can include radiation treatment. In some cases, an alternative cancer treatment can include prostate tissue ablation. Examples of ablative therapies that can be performed on a mammal having prostate cancer to treat the mammal include, without limitation, freezing prostate tissue (e.g., cryoablation or cryotherapy) and heating prostate tissue.

When treating a mammal (*e.g.,* a human) having prostate cancer and identified as being unlikely to respond to one or more anti-androgen agents as described herein *(e.g.,* based, at least in part, on the presence of an increased level of expression of one or more polypeptides), the mammal can be administered or instructed to self-administer (a) one or more *(e.g.,* one, two, three, four, five, or more) anti-androgen agents and (b) one or more *(e.g.,* one, two, three, four, five, or more) agents that can sensitize prostate cancer to one or more anti-androgen agents. For example, a mammal having prostate cancer and identified as being unlikely to respond to one or more anti-androgen agents as described herein can be administered an anti-androgen agent (*e.g.,* abiraterone) and also can be administered one or more agents that can sensitize a prostate cancer to treatment with one or more anti-androgen agents. In some cases, an agent that can sensitize a prostate cancer to one or more anti-androgen agents can be a TOP2 inhibitor (*e.g.,* a TOP2A inhibitor). In some cases, an agent that can sensitize a prostate cancer to one or more anti-androgen agents can be a CDK 4/6 inhibitor. Examples of agents that can sensitize a prostate cancer to one or more anti-androgen agents *(e.g.,* abiraterone) include, without limitation, mitoxantrone *(e.g.,* NOVANTRONE^{®}), doxorubicin (*e.g.* ADRIAMYCIN^{®}, CAELYX^{®}, and MYOCET^{®}) palbociclib (*e.g.,* IBRANCE^{®}), ribociclib (KISQALI^{®}), abemaciclib (VERZENIO^{®}), PD-0325901 (mirdametinib), PHA-793887, teniposide, daunorubicin, amsacrine, ellipticines, aurintricarboxylic acid, and HU-331. In some cases, the one or more anti-androgen agents can be administered together with the one or more agents that can sensitize a prostate cancer to one or more anti-androgen agents. In some cases, the one or more anti-androgen agents can be administered independent of the one or more agents that can sensitize a prostate cancer to one or more anti-androgen agents. When the one or more anti-androgen agents are administered independent of the one or more agents that can sensitize a prostate cancer to one or more anti-androgen agents, the one or more agents that can sensitize a prostate cancer to one or more anti-androgen agents can be administered first, and the one or more anti-androgen agents administered second, or vice versa.

When treating a mammal (*e.g.,* a human) having prostate cancer by administering one or more (*e.g.,* one, two, three, four, five, or more) anti-androgen agents, the mammal also can be administered or instructed to self-administer one or more (*e.g.,* one, two, three, four, five, or more) steroids (*e.g.,* a corticosteroid), where the one or more cancer treatments are effective to treat the cancer within the mammal. Examples of steroids that can be administered to a mammal having prostate cancer together with one or more anti-androgen agents can include, without limitation, predisone, prednisolone, methylprednisolone, dexamethasone, and combinations thereof. In some cases, the one or more steroids can be administered together with the one or more anti-androgen agents. In some cases, the one or more steroids can be administered independent of the one or more anti-androgen agents. When the one or more steroids are administered independent of the one or more anti-androgen agents, the one or more steroids can be administered first, and the one or more anti-androgen agents administered second, or vice versa.

In some cases, when treating a mammal (*e.g.,* a human) having prostate cancer as described herein, the treatment can be effective to treat the cancer. For example, the number of cancer cells present within a mammal can be reduced using the materials and methods described herein. In some cases, the size (*e.g.,* volume) of one or more tumors present within a mammal can be reduced using the methods and materials described herein. For example, the methods and materials described herein can be used to reduce the size of one or more tumors present within a mammal having prostate cancer by, for example, 10, 20, 30, 40, 50, 60, 70, 80, 90, 95, or more percent. In some cases, the size (*e.g.,* volume) of one or more tumors present within a mammal does not increase.

In some cases, when treating a mammal (*e.g.,* a human) having prostate cancer as described herein, the treatment can be effective to improve survival of the mammal. For example, the methods and materials described herein can be used to improve overall survival. For example, the methods and materials described herein can be used to improve disease-free survival (*e.g.,* relapse-free survival). For example, the methods and materials described herein can be used to improve progression-free survival. For example, the methods and materials described herein can be used to improve the survival of a mammal having prostate cancer by, for example, 10, 20, 30, 40, 50, 60, 70, 80, 90, 95, or more percent. For example, the materials and methods described herein can be used to improve the survival of a mammal having prostate cancer by, for example, at least 6 months (*e.g.,* about 6 months, about 8 months, about 10 months, about 1 year, about 1.5 years, about 2 years, about 2.5 years, or about 3 years).

In some cases, when treating a mammal (*e.g.,* a human) having prostate cancer as described herein, the treatment can be effective to reduce one or more symptoms of the cancer. Examples of symptoms of prostate cancer include, without limitation, trouble urinating, decreased force in the stream of urine, blood in the urine, blood in the semen, bone pain, losing weight without trying, and erectile dysfunction. For example, the materials and methods described herein can be used to reduce one or more symptoms within a mammal having prostate cancer by, for example, 10, 20, 30, 40, 50, 60, 70, 80, 90, 95, or more percent.

The invention will be further described in the following examples, which do not limit the scope of the invention described in the claims.

### EXAMPLES

### Example 1: Biomarkers predicting abiraterone treatment prognosis and TOP2 inhibitor synergistic effect in castration resistant prostate cancer

This Example describes the identification of genes that are differentially expressed between prostate cancers that are abiraterone (Abi) responders and prostate cancers that are Abi non-responders. This Example also describes drugs that can be used to sensitize Abi-resistant prostate cancers to Abi treatment.

### Materials and Methods

### Patient-derived xenograft (PDX) mouse model and treatment

All patient specimens were collected as part of the PROMOTE study as described elsewhere (see, for example, Champoux. Annu. Rev. Biochem., 70, 369-413 (2001); and Kellner et al., Lancet Oncol., 3, 235-243 (2002)). Two pathology confirmed mouse PDX (patient-derived xenograft) models were generated from AA/P non-responder bone metastatic tumor biopsy tissues of metastatic castration-resistant prostate cancer (mCRPC), with one (MC-PRX-01) from baseline pretreated samples and one (MC-PRX-06) after 12-weeks of AA/P treatment as described elsewhere (see, for example, Kohli et al., PLoS One, 10, e0145176 (2015); and Wang et al., Ann. Oncol., 29, 352-360 (2018)). These two models were used to test the *in vivo* tumor response to abiraterone ± doxorubicin/mitoxantrone. Pieces of tumor (about 20 mg pieces having about 3-4 mm per cubed side) mixed with matrigel were implanted subcutaneously into 6-8 weeks old male CB17 NOD-SCID mice (Charles River Laboratories, Raleigh, North Carolina). After the tumor reached 100 mm³, mice were randomized to 6 groups. Each group of mice (n = 4 or 5) was administered, for 28 consecutive days, a vehicle control daily, 200 mg/kg abiraterone (dissolved in 5% benzyl alcohol and 95% safflower oil) daily, 2 mg/kg doxorubicin (saline solution) once every 14 days, 0.45 mg/kg mitoxantrone (saline solution) once every week, combination of Abi plus doxorubicin, or combination of Abi plus mitoxantrone. Body weight and tumor volumes (width² × length/2) were measured two to three times per week with a digital caliper, and the average tumor volumes were determined. At the end of the treatment period, mice were euthanized, and the tumors were removed, dissected, and frozen at -80 °C for further analysis.

### PDX derived organoid culture and cytotoxicity assay

PDX tumor dissociation, tumor cell isolation and organoid formation were done as described elsewhere (see, for example, Yu et al., J. Clin. Invest., 128, 2376-2388 (2018)). The tumor cell dissociation kit, cell strainer, and mouse cell depletion kit were purchased from Miltenyi Biotec. Harvested tumors were dissected into 2 mm sections, and were incubated with 5 mL of the human Tumor Dissociation enzyme mix. The tumor tissue was digested gently on a MACS Dissociator, and was passed through a 70 µm and a 40 µm MACS SmartStrainer sequentially. After centrifugation and washing with precooled washing buffer, mouse cells were removed using a Mouse Cell Depletion Purification Kit according to the manufacturer's protocol.

To culture tumor organoids, 1.5-2 x 10⁴ live cells were cultured in 96 well NanoCulture Plate (NCP) (Scivax Corp) in 100 µL modified MEF medium (Phenyl-red free DMEM supplemented with 10% charcoal-stripped FBS, 1% glutamax, 1% sodium pyruvate, 1% nonessential amino acids, 1% penicillin-streptomycin (Life Technologies, Grand Island, NY)), supplemented with 5 µM Y27632 ROCK inhibitor (Tocris Bioscience, Bristol, United Kingdom) and 50 nM pregnenolone. The medium was replaced every 3-5 days. Tumor organoids were allowed to grow for 3 to 7 days before drug testing. Organoids were then treated with a variable concentration of abiraterone with or without the indicated concentration of mitoxantrone, palbociclib, PD-0325901 or PHA-793887 (MedChemExpress) for 5 days before viability was examined by 3D cell titer kit (Promega, Madison, WI). Solvent was used as the control.

### Drug discovery analysis based on differentially expressed genes

Drugs potentially reversing abiraterone resistant expression profiles were identified using the Enrichr portal. Significantly up- or down-regulated genes were identified using transcriptomic data of PROMOTE patients (see, for example, Wang et al., Ann. Oncol., 29, 352-360 (2018)) and the 5 PDX models from this study. Those genes were used as input against LINCS L1000 Chem Pert down/up database using the Enrichr portal (amp.pharm.mssm.edu/Enrichr/) as described elsewhere (see, for example, Kuleshov et al., Nucleic Acids Res., 44, W90-97 (2016); and Chen et al., BMC Bioinformatics, 14, 128 (2013)). Search returned signatures with false discovery rate (FDR) adjusted p-values ≤ 0.05 were downloaded. Signatures of each drug were then combined by either the total number of significant signatures or weighted mean ranks (calculated using the formula as below). Target genes modulated by each drug were pooled from all significant signatures.

The Rank Score was calculated as following: Significant signatures were reverse ranked by p-values (signature with smallest p-value received largest rank), and weighted mean ranks (referred as Rank Score) for each drug i with total number of n significant signatures were calculated by $Rank {Score}_{i} = \frac{{\sum }_{j - 1}^{n} \left({rank}_{i , j} x {weight}_{i , j}\right)}{n}$ where weights_{i,j} = 2 ^{-i + 1} for the jth signature ordered by smallest p-values for drug i.

The Rank Score was calculated so that only the first few most significant signatures for each drug were considered.

### Transcriptomic data from PROMOTE, SU2C and TCGA cohorts

Baseline transcriptomic data and clinical data of the PROMOTE cohort were published elsewhere and can be accessed through dbGAP (phs001141.v1.p1) (see, for example, Champoux, Annu. Rev. Biochem., 70, 369-413 (2001)). 68 samples that passed quality control were included in the study, including 46 bones, 13 lymph nodes and 9 other metastatic sites. Raw count of PROMOTE RNAseq data was normalized by conditional quantile normalization (CQN) method and log2 transformed, as described (see, for example, Hansen et al., Biostatistics, 13, 204-216 (2012)). The drug response was measured by 12 weeks progression determined by composite scores as described earlier, as well as overall survival (46 deceased, 22 living) and time to treatment change (TTTC, 53 treatment changed, 15 not changed).

RNAseq data of Stand Up 2 Cancer (SU2C) cohort data was downloaded from cBioPortal (cbioportal.org,) as log2 transformed RPKM values. Patients with treatment naive target capture RNAseq data, overall survival, and having received Abi treatment were included. One sample (TP_2079_Tumor) was excluded due to the low reads. The final analyzable cohort included 53 samples including 23 bones, 22 lymph nodes, and 8 other metastatic sites. For treatment outcome, overall survival (32 deceased, 21 living) was used.

TCGA pan-cancer cohort batch effects normalized mRNA data was downloaded from UCSC Xenabrowser (xenabrowser.net) as log2 transformed RPKM values. Progression-free survival was used as the outcome. Prostate Cancer (93 progressed, 403 non-progressed), breast cancer (147 progressed, 951 non-progressed), cervix cancer (72 progressed, 234 non-progressed), and colon cancer (84 progressed, 204 non-progressed) were included.

### Patient subgroup identification and Clinical Outcome evaluation

Subgroups of patients who might be sensitive to the 4 top candidate drugs were identified by the k-means clustering method calculated from the z-score transformed 11 gene expression. The optimal number of clusters were determined by the elbow method (Figure 1). In the PROMOTE cohort, 3 clusters were observed using the gene panel. The cluster with the highest sum of z-score transformed expression of the gene panel was defined as "high-expression" cluster, whereas the rest two clusters were combined as "low-expression" cluster. Kaplan-Meier curve was plotted using survminer and survival packages, and The Gehan-Breslow-Wilcoxon test was used to test statistical significance for survival.

Prognosis prediction value was examined by the Cox proportional hazard model by either univariate or multivariate including various gene panels and log10(PSA). The 11 gene panel was input into the model as a binary variable representing "high expression" vs "low expression" groups. CCP score, AR score NEPC scores, copy number variation, and mutation calls of PROMOTE cohort were determined as described elsewhere (see, for example, Wang et al., Ann. Oncol., 29, 352-360 (2018)). AR activity score and NEPC scores for SU2C cohorts were downloaded from cBioPortal and CCP scores were calculated by sum up the z-score transformed expression of CCP genes, as described elsewhere (see, for example, Wang et al., Ann. Oncol., 29, 352-360 (2018)). COX modeling, Log-likelihood ratio test and Akaike information criterion (AIC) calculation were performed using a survival package from R software.

### Abiraterone resistant prostate cancer cell line generation

22Rv1 and LNCaP cells purchased from ATCC were routinely cultured in RPMI1640 medium (Gibco, Grand Island, NY) supplemented with 10% FBS (Atlanta Biologicals, Flowery Branch, GA) and 1% Pen-Strep. To develop Abi resistant cell line, cells were maintained in the medium supplemented with 5 µM of abiraterone (Sellect Chemicals, Houston, TX) for 3 months until the viability reached over 95%. Abi resistant cells (LNCaP-AbiRes or 22Rv1-AbiRes) were compared with parental cells for viability and gene expression after Abi and other treatments.

### Cytotoxicity and proliferation assay

Forty eight hours before abiraterone treatment, cell was seeded in phenol red-free RPMI (Gibco, Grand Island, NY) supplemented with 10% charcoal stripped FBS (Thermo Fisher Scientific, Waltham, MA), and 50 nM pregnenolone was added after 24 hours. Cells were then treated with abiraterone at various doses with or without mitoxantrone, palbociclib, PD-0325901 or PHA-793887 at indicated concentrations for 3 days before viability being examined by Cyquant direct assay (Thermo).

### qRT-PCR

Total RNA for qRT-PCR was extracted from tumor organoids or cell lines using Quick-RNA MiniPrep Kit (Zymo Research, Irvine, CA) according to the manufacturer's instructions. qRT-PCR was performed using the Power SYBR^{®} Green RNA-to-CT 1-Step Kit (Life Technologies, Grand Island, NY) and QuantiTect^{®} (QIAGEN, Germantown, MD) or PrimeTime^{®} (IDT, Inc., Coralville, Iowa) pre-designed qPCR primers (IDT Coralville, IA). Gene expression analyses were performed using ΔΔCt method, and β-actin was used as the internal reference. Three independent experiments were performed. Primer sequences are in Table 1.

**Table 1. Primer sequences.**

| Primer | Sequence | SEQ ID NO |
|---|---|---|
| PRC1 | CCCAATTAGCTCCCATATTTCC | 1 |
| | CGTGTGGAGTAGGTCTGGA | 2 |
| CCNA2 | GCATGTCACCGTTCCTCCTT | 3 |
| | GGGCATCTTCACGCTCTATTT | 4 |
| SMC2 | TGGAGCAGGTTTGAAGAGAAG | 5 |
| | TGTTTTGGATGGTCTGGAGTTC | 6 |
| CDK1 | GTCTACCCTTATACACAACTCCAT | 7 |
| | TGGCTGCTTTGAAAGTCTACG | 8 |
| KIF4A | TGAACTCCCAGTCGTCC | 9 |
| | GCACTGATTACATTTCCC | 10 |
| DLGAP5 | GATCACTTGAACCCAGGACTTC | 11 |
| | CTACTGAATGCCACCTTCTTGA | 12 |
| ECT2 | GCACTCCAGTCTAGGTAACAAG | 13 |
| | ATGGCGGTATTTGTGAGAGG | 14 |
| CCNB2 | CATCTCCTCATACTTGGAAGCC | 15 |
| | CCACTCCAAGTTTAGGCTTCT | 16 |
| FBXO5 | CTGGTTGAAGCATGAGGTGA | 17 |
| | TAATCAATGTGTCTAAAGTGAGCAC | 18 |
| CCNB1 | TGAACCTGTACTAGCCAGTCA | 19 |
| | TGTAGTGAATATGTGAAAGATATTTATGCT | 20 |
| TMPRSS2 | GTCAAGGACGAAGACCATGT | 21 |
| | TGCCAAAGCTTACAGACCAG | 22 |
| FKBP5 | CACAGTGAATGCCACATCTCT | 23 |
| | TGAAGATGGAGGCATTATCCG | 24 |
| NKX3-1 | CCTCTGTGAAAATCCAATACCTCT | 25 |
| | GACAGCTTGAGAAGGTCACTAC | 26 |
| PSA | AGGCCTTCCCTGTACACCAA | 27 |
| | GTCTTGGCCTGGTCATTTCC | 28 |
| AR FL | CCATCTTGTCGTCTTCGGAAATGTTATGAAGC | 29 |
| | CTGTTGTGGATGAGCAGCTGAGAGTCT | 30 |
| AR V7 | CCATCTTGTCGTCTTCGGAAATGTTATGAAGC | 31 |
| | TTTGAATGAGGCAAGTCAGCCTTTCT | 32 |
| AR del567ES | GCCTTGCCTGATTGCGAG | 33 |
| | CATGTGTGACTTGATTAGCAGGTCAAA | 34 |

### RNAseq and differential expression analysis on PDX models

RNAseq of PDX tumor was performed by ACGT, Inc. Total RNA from 5 PDX models (MC-PRX-01, MC-PRX-03, MC-PRX-04, MC-PRX-07, MC-PRX-08) was extracted using the RNeasy Plus Mini kit (QIAGEN, Germantown, MD), per manufacturer's instructions. At least 3 tumors were included for each PDX model from various passages of mice based on tumor availability and quality. mRNA libraries were enriched using a NEXTflex^{™} Rapid Directional qRNASeq^{™} Kit. Quantity and quality of RNA libraries were evaluated by Qubit fluorometry and Agilent 2100 Bioanalyzer. Individual libraries were pooled in equimolar ratios, and were run on HiSeq 4000 system (2x150 paired end, Illumina, San Diego, CA).

Low quality were trimmed by Trim Galore, and reads of mouse origin were removed by BBsplit. Filtered reads were aligned to hg19 human reference genome using Hisat2 with average mapping rate is 93%. Raw counts were then called by HTSeq excluding non-unique mapped reads. One sample (MC-PRX-08.1) was excluded due to poor consistency within replicates. Differential expression analysis was performed between 1 model derived from AA/P responder (MC-PRX-04) and 4 models derived from AA/P non-responders (MC-PRX-01, MC-PRX-03, MC-PRX-07, MC-PRX-08) EdgeR. Genes differentiate by fold change ≥ 2 and FDR ≤ 0.05 were considered significant.

### Results

### Four drugs were identified as candidate drugs to reverse Abi resistance expression phenotype

Drug identification workflow was illustrated in Figure 2. To identify drugs that might be able to overcome Abi resistance, gene enrichment analysis were performed using LINCS L1000 Chem Pert down/up database with two gene sets: 1) Differentially expressed (DE) genes between 3 months Abi- responder and non-responders from Mayo Clinic PROMOTE study, and 2) DE genes between PDX models generated from Abi- responder and non-responders enrolled in PROMOTE study.

The database was searched using DEGs between AA/P non-responder and responders in bone metastasis samples, which comprises 70% of the samples of PROMOTE cohort. Using 103 upregulated genes, 689 drugs were identified with at least one signature passing FDR 0.05, with a mean of 7.3 and a median of 2 signatures per drug (Figure 3A, Table 2). Using 73 downregulated genes, 141 drugs were identified with a mean of 3.7 and a median of 2 signatures per drug (Figure 3B, Table 3). The number of signatures and Rank Score and the weighted average rank of all signatures for a particular drug (see methods) were used to select top candidate drugs. As shown in Table 4, mitoxantrone and PD-0325901 were enriched in both analyses, with high number of signatures and Rank Scores. Out of the total 13 drugs, 4 are CDK inhibitors (palbociclib, PHA-793887, CGP-60474 and BMS-387032).

**Table 2. Summary of drugs search return from EnrichR-L1000 Chem Perturbation Down using upregulated genes in AA/P non-responders**

| Drug Name | Number of L1000 Signature | Rank Score | Number Gene Target | Gene targets |
|---|---|---|---|---|
| palbociclib | 69 | 4994.804 | 49 | RRM2;PKIA;NEB;CA12;PLK1;FBXO5;AURKA;GINS1;SLC7A5;FHL1;CC NA2;PPP1R3C;NUSAP1;ANP32E;KIF20A;CENPE;MKI67;DLGAP5;TOP2 A;CCNB2;CA2;CDK1;TPX2;ASPM;ESPL1;KIF4A;NCAPG;MCM4;KIF11; BUB1;CENPF;KIF18B;CIT;KIF15;MELK;KIF23;HMMR;PRC1;SMC2;ECT 2;DEPDC1;CCNB 1;BUB 1B;KIFC1;TROAP;MYBPC1;PRKACB;ITM2A;KI F2C |
| mitoxantrone | 130 | 4981.293 | 62 | CCNA2;RRM2;FHL1;ANP32E;ITM2A;HMGCS 1;PLK1;BCAT1;CCNB 1;KI F20A;CA12;PLIN2;DLGAP5;MELK;MKI67;AURKA;CENPF;CA2;CCNB2 ;MYBPC1;TTN;TOP2A;KIF2C;NUSAP1;ASPM;COL4A5;HMMR;CENPE; MB;COL11A1;SLC2A1;TFRC;CAMK2N1;CDK1;KIF23;ALDOC;PKIA;TP X2;CDH2;PRC1;BUB1B;GINS1;KIF11;PRKACB;BUB1;DEPDC1;FBX05; ECT2;CIT;MCM4;NCAPG;KIF18B;KIF15;KIF4A;THBS2;SMC2;ESPL1;KI FC1;TROAP;PPPIR3C;SLC7A5;ARHGAP11A |
| dovitinib | 21 | 4980.27 | 52 | RRM2;PLIN2;CA12;CDH2;FHL1;DLGAP5;CA2;CAMK2N1;ITM2A;CCN A2;PLK1;KIF20A;MELK;TOP2A;GINS1;NUSAP1;MKI67;PKIA;CDK1;M CM4;ASPM;CENPE;CENPF;MB;COL4AS;PRKACB;HMGCS1;PRC1;NCA PG;KIF23;KIF11;CCNB2;KIF18B;ANP32E;ECT2;BUB 1;KIF15;AURKA;T PX2;KIF4A;FBXO5;HMMR;CIT;DEPDC1;ESPL1;BUB1B;CCNB1;TROAP ;SMC2;KIFC1;ARHGAP11A;KIF2C |
| PHA-793887 | 71 | 4974.437 | 54 | CCNA2;CCNB2;AURKA;CA12;SLC7A5;RRM2;THBS2;HMGCS1;FBXO5; ASPM;PLIN2;CCNB1;CAMK2N1;NUSAP1;MKI67;CDK1;KIF20A;CIT;A NP32E;DLGAP5;PLK1;KIF18B;TOP2A;CA2;GINS1;C5;CENPF;CENPE;M YBPC1;CDH2;MELK;PRC1;SLC2A1;DEPDC1;TPX2;HMMR;KIF11;BUB 1;MB;ECT2;TFRC;MCM4;ITM2A;KIF23;KIF4A;KIF15;NCAPG;TROAP;FH L1;SMC2;ESPL1;BUB 1B;BCAT1;KIFC1 |
| NVP-BEZ235 | 67 | 4956.913 | 55 | RRM2;TFRC;ITM2A;GINS1;PPP1R3C;CCNA2;TPX2;HMGCS1;CDK1;MC M4;CA12;CDH2;COL4A5;ALDOC;BCAT1;MELK;NUSAP1;PRKACB;CA 2;SLC7AS;KIF20A;FHL1;SLC2A1;PRC1;SMC2;FADS2;ANP32E;KIF18B; CIT;PLKl;AURKA;DLGAP5;MYBPC1;PKIA;FBXO5;ASPM;BUB1;MKI6 7;NCAPG;CCNB1;TOP2A;CCNB2;ESPL1;CENPF;KIF4A;HMMR;KIF15;K IF11;CENPE;ECT2;KIF23;DEPDC1;TROAP;BUB1B;KIFC1 |
| torin-2 | 66 | 4948.468 | 60 | PPP1R3C;CAMK2N1;COL4A5;CA12;TFRC;CDH2;HMGCS1;FADS2;GIN S1;RRM2;CCNA2;MELK;MCM4;SCUBE2;COL11A1;THBS2;COX7A1;SL C2A1;ALDOC;ITM2A;KCNH2;CDK1;KIF20A;FHL1;NCAPG;PLK1;ANP3 2E:AURKA:PRC1:TTN:NUSAP1:PRKACB:KIF15:TOP2A:TPX2:CA2:KIF 18B;CENPE;CENPF;KIF4A;KIF1 1;MKI67;KIF23;BUB 1;DLGAP5;SMC2;S LC7AS;NEB;ASPM;CCNB2;CCNB1;ECT2;CIT;DEPDC1;ESPL1;PLIN2;FB XO5;HMMR;NUDT11;BUB1B |
| torin-1 | 63 | 4924.905 | 57 | PPP1R3C;CAMK2N1;COL4A5;GINS1;MELK;RRM2;TFRC;CDH2;HMGC S1;ALDOC;CA2;SLC7AS;CCNA2;CDK1;FHL1;ANP32E;MCM4;CCNB2;B CAT1;ITM2A;AURKA;FADS2;KIF20A;MKI67;TOP2A;TTN;PRC1 ;NUSA P1;TPX2;CA12;MB;CCNBl;SLC2Al;PKIA;NCAPG;FBXO5;NEB;KIF18B; DLGAP5;SMC2;KIF15;KCNH2;DEPDC1;PLK1;ECT2;BUB1;KIF11;KIF4A ;CIT;CENPE;MYBPC1;HMMR;KIF23;ASPM;ESPL1;CENPF;BUB1B |
| PI-103 | 52 | 4909.289 | 53 | TFRC;HMGCS1;ALDOC;BCAT1;CA12;SLC7AS;KIF20A;CCNA2;MELK; RRM2;MCM4;GINS1;ANP32E;PPP1R3C;FHL1;PLIN2;MYBPC1;SLC2A1; CDH2;CA2;NUSAPI;KIF18B;PLKI;ITM2A;PKIA;SMC2;AURKA;CDKI; BUB1B;ASPM;KIF23;DLGAP5;TPX2;CIT;CCNB1;FADS2;TOP2A;NCAP G;BUB 1;PRC1;DEPDC1;NEB;HMMR;ESPL1;CENPE;MKI67;CCNB2;KIF 4A;CENPF;KIF11;KIF15;FBXO5;ECT2 |
| radicicol | 64 | 4907.96 | 53 | GINS1;CAMK2N1;CDKl;CCNA2;CA12;RRM2;MCM4;MELK;PRKACB;T OP2A;NUSAPI1;PLIN2;:FHL1;THBS2;CCNB1;COL4A5;COL11A1;SLC7A5 ;CA2;BCAT1;CDH2;KIF20A;PRC1;CCNB2;PLK1;ASPM;ITM2A;IL2RB;ESPL1;DLGAP5;NEB;TPX2;NCAPG;HMMR;MYBPC1;KIF18B;ANP32E;B UB 1;CENPE;KIF23;MKI67;CENPF;SMC2;KIF15;SCUBE2;KIF4A;KIF11;E CT2;BUB1B;FBXO5;CIT;DEPDC1;KIFC1 |
| WYE-125132 | 82 | 4907.164 | 55 | GINS1;MELK;RRM2;SLC7A5;TFRC;PPP1R3C;CA2;MCM4;HMGCS1;AU RKA;BCATI;FHL1;COL4A5;ALDOC;CCNA2;NUSAP1;CAMK2N1;CA12; ITM2A;PLK1;CDKI1;FADS2;PRC1;TPX2;:SLC2A1;TOP2A;MB;NEB;ASP M;NCAPG;DLGAP5;GBE1;ANP32E;PRKACB;BUB1;KIF20A;KIF18B;KIF 15;CIT;SMC2;ESPL1;SCUBE2;MKI67;BUB1B;CENPE;DEPDC1;CCNB2; CCNB 1;KIF23;KIF4A;HMMR;KIF11;ECT2;MYBPC1;CENPF |
| NVP-TAE684 | 44 | 4906.641 | 55 | MYBPC1;CIT;GINS1;RRM2;CDK1;MELK;TFRC;CCNA2;PKIA;KIF20A;C A2;BCAT1;HMGCS 1;MCM4;TOP2A;ANP32E;AURKA;CCNB 1;CAMK2N 1;PLIN2;PRKACB;SMC2;PRC1;KIF4A;PLK1;DLGAPS;CA12;NUSAP1;TP X2;NCAPG;KIF 18B;HMMR;ITM2A;KIF15;PPP1R3C;MKI67;C5;CDH2;CE NPE;MB;NEB;BUB1;TTN;SLC7A5;ESPL1;CCNB2;KIF23;KIF11;ASPM;C ENPF;FHL1;BUB1B;FBXO5;ECT2;DEPDC1 |
| canertinib | 28 | 4902.742 | 50 | RRM2;ANP32E;PRKACB;CCNB2;HMGCS1;SLC2A1;CCNA2;TPX2;FHL1 ;KIF20A;CA12;CDK1;TOP2A;CDH2;PLK1;DLGAP5;CA2;AURKA;GINS1 ;CAMK2N1;MCM4;ASPM;MELK;MKI67;KIF11;PRC1;NUSAP1;KIF18B; BUB1;COL4A5;NCAPG;ITM2A;NUDT11;SLC7AS;THBS2;CENPE;CENP F;SCUBE2;FBXO5;KIF4A;HMMR;KIF15;DEPDC1;KIF23;ESPL1;SMC2;T ROAP;CCNB1;ECT2;CIT |
| AZD-8330 | 34 | 4896.949 | 49 | CA2;COL4A5;RRM2;MELK;TOP2A;PLK1;NCAPG;CCNA2;NUSAP1;AN P32E;DLGAP5;CA12;PRC1;CDK1;SCUBE2;GINS1;ASPM;CENPE;C5;KIF 18B;MCM4;ECT2;CIT;TFRC;KIF4A;AURKA;ESPL1;TROAP;PKIA;FBXO 5;CAMK2N1;KIF11;KIF15;SMC2;MKI67;CCNB2;TPX2;HMGCS1;LDHA; CENPF;DEPDC1;KIF20A;KIF23;BUB1;BUB1B;CCNB1;HMMR;MYBPC1; NUDT11 |
| ZSTK-474 | 46 | 4896.115 | 53 | TFRC;SLC2A1;ALDOC;GINS1;RRM2;MCM4;CA12;SLC7A5;PPP1R3C;M ELK;CCNB 1;BCAT1;CCNA2;ANP32E;ITM2A;CA2;KIF18B;CDK1;CENP E;HMGCS1;PLK1;KIF20A;AURKA;SMC2;PRC1;PRKACB;PKIA;FADS2; CDH2;NUSAP1;CCNB2;TPX2;DLGAP5;BUB1;MKI67;FHL1;NCAPG;ESP L1;KIF4A;TOP2A;HMMR;KIF15;NEB;TTN;DEPDC1;ASPM;CENPF;BUB 1B;ECT2;KIF11;CIT;FBXO5;KIF23 |
| GDC-0941 | 46 | 4892.664 | 53 | CA2;ANP32E;SLC7A5;TFRC;ITM2A;PLK1;AURKA;MELK;RRM2;KIF20 A;GINS1;MCM4;PRKACB;CDH2;ALDOC;HMGCS1;BCAT1;PPP1R3C;P KIA;CA12;CCNA2;PRC1;CDK1;MKI67;SMC2;NCAPG;FHL1;THBS2;SLC 2A1;NUSAP1;BUB1;TOP2A;CCNB2;CENPE;DLGAP5;TPX2;CCNB1;ECT 2;KIF18B;CENPF;ESPL1;HMMR;KIF15;NUDT11;KIF23;DEPDC1;ASPM; CIT;KIF4A:BUB1B:KIF11;FBXO5;FADS2 |
| trametinib | 34 | 4879.45 | 47 | RRM2;CA2;ITM2A;MYBPC1;TFRC;MELK;COL11A1;AGL;TOP2A;ANP3 2E;CDK1;MCM4;CCNA2;PLK1;NCAPG;GINS1;CA12;KIF18B;SMC2;ASP M;KIF15;CENPE;PKIA;MKI67;ECT2;AURKA;C5;KIF4A;BUB1B;TPX2;P RC1;KIF11;NUSAP1;CIT;DLGAP5;FBXO5;KIF20A;BUB1;CENPF;ESPL1; KIF23;CCNB1;HMMR;CCNB2;DEPDC1;PRKACB;TROAP |
| PD-0325901 | 137 | 4861.271 | 56 | RRM2;PKIA;CCNA2;PLK1;CDK1;MCM4;GINS1;COL11A1;THBS2;ITM2 A;TOP2A;MELK;CA2;CIT;TFRC;FHL1;NEB;TTN;KIF18B;CCNB2;KIF4A ;ANP32E;ECT2;PRKACB;TPX2;MKI67;AURKA;MYBPC1;SLC2A1;SLC7 A5;CENPE;ESPL1;CCNB1;BCATI1;ASPM;KIF20A;DLGAP5;PRC1:KIF15; C5;NCAPG;BUB1;BUB1B;NUSAP1;CA12;KIF23;SMC2;PLIN2;CAMK2N 1;KIF11;FBXOS;HMMR;CENPF;DEPDC1;NUDT11;TROAP |
| AZD-5438 | 54 | 4861.255 | 53 | ASPM;CAMK2N1;KIF20A;DLGAP5;MYBPC1;PLIN2;AURKA;CCNA2;P LK1;CCNB2;ITM2A;CA12;RRM2;CDH2;TOP2A;KIF18B;HMGCS 1;NCAP G;ANP32E;CDK1;MKI67;CENPE;CENPF;PKIA;BUB1B;CA2;NUSAP1;H MMR;DEPDC1;MB;ECT2;CCNB1;MELK;SLC2A1;GINS1;KIF23;TFRC;P RC1;KIF4A;FADS2;BUB1;COL4A5;TPX2;KIF11;CIT;ESPL1;SMC2;KIF2 C;FHL1;KIF15;MCM4;TROAP;KIFC1 |
| GDC-0980 | 46 | 4860.721 | 53 | RRM2;TFRC;HMGCS1;GINS1;PPP1R3C;SLC2A1;CA2;SLC7A5;MCM4:K IF20A;ITM2A;CCNA2;TPX2;MELK;CDK1;NEB;TTN;CDH2;CA12;ANP32 E;FHL1;PLK1;COL4AS;CIT;AURKA;MYBPC1;KIF18B;NUSAP1;ALDOC :NCAPG;TOP2A;PKIA;ESPL1;KIF23;DLGAP5;MKI67;:BUB1;PRC1;DEPDC1;CCNB2;SMC2;KIF15;KIF4A;ECT2;HMMR;CENPE;CCNB1;ASPM;KIF 11;CENPF;FBXOS;BUB 1B;KIFC1 |
| GSK-2126458 | 79 | 4851.577 | 56 | CAMK2N1;PLK1;COL4A5;HMGCS1;MCM4;BCAT1;RRM2;CDK1;ITM2 A;GINS1;PPP1R3C;TFRC;SCUBE2;CDH2;CA2;SLC7A5;ALDOC;CA12;M ELK;CCNA2;SLC2A1;AURKA;ANP32E;KIF20A;PKIA;NUSAP1;ACAT1; TOP2A;BUB1B;DEPDC1;TPX2;CENPE;SMC2;PRC1;CIT;FHL1;FADS2;E SPL1;BUB1;KIF18B;FBXO5;MKI67;NCAPG;KIF23;DLGAP5;HMMR;KIF 11;MYBPC1;KIF15;CENPF;CCNB2;CCNB1;ASPM;KIF4A;ECT2;KIFC1 |
| JW-7-24-1 | 38 | 4849.608 | 54 | PPP1R3C;CA2;KIF20A;SLC7A5;GBE1;ALDOC;CCNA2;ANP32E;CA12;F ADS2:ECT2;TOP2A;RRM2;PLK1;CDK1;DLGAPS5;FHL1;KIF2C;TFRC;CD H2;SLC2A1;BCAT1;GINS 1;MCM4;AURKA;MELK;HMGCS 1;PRC1;NCA PG;SMC2;NUSAP1;MKI67;CENPE;PRKACB;ITM2A;TPX2;KIF18B;ESPL 1;BUB1;CCNB2;CIT;KIF4A;CENPF;HMMR;CCNB1;CAMK2N1;CS;KIFC 1;KIF11;KIF15;KIF23;ASPM;DEPDC1;BUB1B |
| foretinib | 21 | 4847.551 | 44 | CA12;RRM2;CDH2;MCM4;SMC2;GINS1;CCNA2;CDK1;MELK;TFRC;FH L1;SLC7AS5;SLC2A1;TPX2;ESPL1;CA2;PRC1;TOP2A;ECT2;ANP32E;KIF 20A;NUSAP1;PRKACB;NCAPG;KIF18B;MKI67;DLGAP5;CIT;PLK1;KIF 23;CCNB2;BUB1;AURKA;ASPM;KIF4A;HMMR;KIF11;KIF15;CENPE;FB XOS;BUB1B;CENPF;TROAP;DEPDC1 |
| HG-6-64-01 | 31 | 4837.612 | 48 | CCNA2;GINS1;PLK1;SCUBE2;TFRC;CA12;CAMK2N1;PLIN2;MELK;HM GCS1;RRM2;CA2;KIF20A;AURKA;CCNB2;CCNB1;DLGAPS;CDK1;KIF1 8B;MCM4;PRC1 ;TPX2;ANP32E;NCAPG;MKI67 ;BUB IB;ITM2A;TOP2A; ASPM;KIF23;ALDOC;BUB1;SMC2;MYBPC1;ESPL1;KIF4A;ECT2;KIF15; NUDT11;CENPF;PKIA;KIF11;NUSAP1;HMMR;CENPE;DEPDC1;FBX05; CIT |
| MK-2206 | 20 | 4830.59 | 45 | TFRC;CA2;ITM2A;CA12;NEB;CAMK2N1;COL4A5;CCNA2;CCNB2;CDK 1;PPP1R3C;BCAT1;BUB1;DLGAP5;KIF15;TOP2A;RRM2;FHL1;NUSAP1; ANP32E;KIF20A;SCUBE2;GINS1;TPX2;MELK;PLK1;NCAPG;KIF23;ASP M;CENPE;AURKA;PRC1;KIF4A;MCM4;HMMR;MKI67;KIF18B;DEPDC1 ;SMC2;KIF11;CCNB1;PKIA;ECT2;CENPF;ESPL1 |
| WZ-4-145 | 40 | 4825.634 | 43 | CA12;CCNA2;RRM2;PKIA;MCM4;KIF20A;PRC1;PLK1;CA2;MELK;TFR C;TOP2A;CDK1;FHL1;AURKA;GINS1;CENPF;CDH2;DLGAP5;TPX2;NU SAP1;ASPM;NCAPG;PRKACB;KIF18B;KIF4A;MKI67;CENPE;ANP32E:E CT2;BUB1;ESPL1;KIFI5;HMMR;SMC2;CCNB1;CCNB2;KIF23;CIT;KIF1 1;FBXO5;BUB1B;DEPDC1 |
| gefitinib | 10 | 4828.477 | 36 | MELK;AMACR;AURKA;CCNB2;RRM2;CA2;PLK1;DLGAP5;TOP2A;CC NA2;GINS1;ASPM;ANP32E;CDK1;MCM4;TPX2;NUSAP1;KIF20A;ITM2 A;CENPF;PRC1;DEPDC1;NCAPG;HMMR;KIF11;MKI67;CDH2;BUB1;KI F18B;ESPL1;KIF4A;KIF15;KIF23;SMC2;ECT2;CENPE |
| selumetinib | 28 | 4818.486 | 48 | CA12;RRM2;CA2;FHL1;NEB;TTN;GINS1;CENPE;MCM4;CCNA2;CDK1; AURKA;KIF18B;PLK1;ITM2A;MELK;ECT2;TPX2;TFRC;BCAT1;ASPM; NUSAP1;KIF20A;PRKACB;TOP2A;SMC2;PRC1;CAMK2N1;NCAPG;CC NB2;ANP32E;KIF23;MKI67;SLC7A5;DLGAP5;KIF15;CENPF;KIF4A;BU B1;KIF11;ESPL1;BUB1B;CCNB1;DEPDC1;HMMR;CIT;FBXO5;MYBPC1 |
| TG-101348 | 19 | 4810.992 | 45 | CCNA2;GINS1;RRM2;MCM4;ITM2A;PLK1;KIF20A;AURKA;MELK;PRC 1;TOP2A;TPX2;CDK1;BUB 1;ANP32E;KIF11;KIF15;SMC2;SCUBE2;NUS AP1;PRKACB;CCNB2;KIF18B;CDH2;PPP1R3C;ESPL1;BUB1B;MKI67;KI F4A;DLGAP5;CA12;NCAPG;KIF23;HMMR;ASPM;CA2;ECT2;CENPE;CE NPF;C5;FBXO5;DEPDC1;TROAP;CIT;FHL1 |
| afatinib | 23 | 4794.333 | 46 | GINS1;FHL1;CDK1;MCM4;CCNB2;RRM2;ANP32E;TOP2A;CA3;CDH2;C AMK2N1;COL4A5;KIF20A;CCNA2;DLGAP5;CA2;AGL;COX7A1;PRKA CB;CA12;PLK1;NUSAP1;MELK;HMGCS 1;BUB 1;AURKA;SCUBE2;MKI 67;DEPDC1;NCAPG;MYBPC1;NEB;TTN;HMMR;KIF15;KIF23;TPX2;ASP M;CENPF;KIF18B;ESPL1;PRC1;KIF4A;KIF11;SMC2;CCNB 1 |
| OSI-027 | 44 | 4786.891 | 51 | TFRC;MB;PPP 1R3 C;FHL 1;SLC2A 1;CCNA2;HMGCS 1;CA2;CA 12;MYBP C1;RRM2;GINS1;SLC7A5;ALDOC;PKIA;BCAT1;MELK;MCM4;FADS2;C CNB2;ITM2A;TPX2;PLK1;CDK1;AURKA;ANP32E;TOP2A;SCUBE2;KIF 20A;BUB1;CIT;PRC1;NUSAP1;KIF4A;DLGAP5;CDH2;ESPL1;KIF15;NC APG;MKI67;KIF18B;HMMR;CCNB1;DEPDC1;SMC2;CENPE;ASPM;ECT 2;KIF23;KIF11;CENPF |
| erlotinib | 10 | 4790.827 | 43 | CCNA2;CAMK2N1;MCM4;MELK;GBE1;BCAT1;PRC1;PLK1;ANP32E;A SPM;CENPE;CENPF;RRM2;KIF18B;KIF20A;BUB1;TOP2A;TPX2;CDK1; NCAPG;DLGAP5;AURKA;GINS1;TFRC;ECT2;ITM2A;HMGCS1;MKI67; ESPL1;CA2;KIF4A;NUSAP1;HMMR;KIF11;CCNB2;DEPDC1;KIFI5;MYB PC1;KIF23;CIT;TROAP;SMC2;CCNB1 |
| geldanamycin | 39 | 4779.302 | 51 | TOP2A;COL11A1;AURKA;GINS1;MELK;RRM2;MCM4;CCNA2;PLK1;KI F20A;FHL1;THBS2;CA2;ANP32E;CAMK2N1;NUSAP1;FADS2;MYBPC1; ITM2A;PPP1R3C;CDK1;AMACR;HMGCS1;BUB1B;CCNB2;BCAT1;TFR C;CDH2;AGL;BUB 1;SCUBE2;KIF18B;MKI67;TPX2;ASPM;NCAPG;HM MR;CIT;CENPE;CENPF;PRC1;KIF4A;DEPDC1;CA12;KIF11;SMC2;KIF15 :DLGAP5.ECT2;FBXO5;KIF23 |
| AZD-8055 | 54 | 4760.557 | 51 | RRM2;TFRC;HMGCS1;PPP1R3C;CA2;CDH2;PLIN2;KIF20A;MELK;ALD OC;AURKA;SLC7A5;PKIA;FHL1;CA12;GINS1;NUSAP1;MCM4;CCNA2; CDK1;BCAT1;CCNB1;FADS2;MB;KIF18B;NEB;PRKACB;MYBPC1;PLK 1;TTN;SMC2;PRC1;ANP32E;MKI67;CIT;TPX2;DEPDC1;TOP2A;SCUBE2 ;CCNB2;ESPL1;NCAPG;KIF4A;BUB1;DLGAP5;KIF11;HMMR;ASPM;K1 F23;CENPE;CENPF |
| PD-184352 | 24 | 4709.785 | 41 | CCNA2;:RRM2;CDK1;MCM4;GINS1;MELK;PRC1;TFRC;CA2;DLGAP5;C AMK2N1;PLK1;ANP32E;KIF18B;HMGCS1;AURKA;TOP2A;CCNB2;NC APG;MKI67;TPX2;ESPL1;KIF11;ECT2;KIF15;ASPM;BUB1B;KIF20A;IT M2A;BUB1;CENPE;CIT;SLC7A5;NUSAP1;MYBPC1;SMC2;KIF4A;FBXO 5;CENPF;KIF23;DEPDC1 |
| BI-2536 | 33 | 4698.271 | 52 | TOP2A;CA12;CA2;CAMK2N1;CCNA2;GINS1;TIHBS2;SCUBE2;PLK1;IT M2A;CDK1;FHL1;MYL6B;ECT2;AMACR;CCNB1;CDH2;AURKA;MELK ;KIF20A;C5;PKIA;CCNB2;PRKACB;MCM4;RRM2;PPP1R3C;HMGCS1;A NP32E;COL11A1;SLC2A1;ASPM;CENPF;TPX2;KIF18B;KIF11;IL2RB; TF RC;KIF15;ACATI1;DLGAP5;MYBPC1;NCAPG;NUSAP1;BUB1;PRC1;MK I67;SMC2;ESPL1;HMMR;KIF23;KIF4A |
| XMD-1150 | 17 | 4689.441 | 38 | CA 12;RRM2;NUSAP 1;AURKA;CCNA2;GINS 1;MELK;ECT2;MYBPC 1;M CM4;FHL1;COL4A5;THSD7A;CCNB2;KIF20A;ASPM;DLGAP5;TOP2A;CAMK2N1;CDK1;KIF11;TPX2;PRC1;PLK1;KIF18B;CDH2;SMC2;NCAPG; HMMR;KIF4A;ANP32E;:DEPDC1;BUB1;KIF23;MKI67;ESPL1;KIF15;CEN PF |
| celastrol | 24 | 4684.658 | 48 | TOP2A;CCNA2;CENPF;PLK1;COL4AS;ACAT1;RRM2;TFRC;CDK1;CA1 2;CDH2;GBE1;CAMK2N1;ASPM:KIF20A;CA2;FHL1;FADS2;HMGCS1;P RC1;MCM4;KIF2C;NUDT11;G1NS1;MELK;KIF18B;DLGAP5;HMMR;CE NPE;THBS2;SCUBE2;CCNB2;TPX2;PKIA;NUSAP1;KIF11;MKI67;ECT2; AURKA;ESPL1;KIF4A;BUB 1;BUB 1B;NCAPG;KIF15;TROAP;CCNB 1;KI F23 |
| neratinib | 31 | 4654.557 | 46 | CA2;PLK1;RRM2;NUSAP1;CDH2;FHL1;HMGCS1;KIF20A;TOP2A;COL4 A5;TFRC;CCNA2;CDK1;MCM4;FADS2;ALDOC;SCUBE2;MELK;MKI67; GINS1;KIF18B;CAMK2N1;COL11A1;CCNB1;CCNB2;PRC1;NCAPG;ANP 32E;BUB1;AURKA;NEB;SLC2A1;ASPM;MYBPC1;TPX2;DEPDC1;DLGA PS;KIF11;SMC2;ECT2;CENPF;HMMR;ESPL1;THBS2;BUB 1B;KIF4A |
| KIN001-043 | 21 | 4651.959 | 49 | ASPM;NUSAP1;KIF20A;DLGAP5;CCNB2;HMGCS1;PPP1R3C;ALDOC;T OP2A;CA2;CAMK2N1;CCNA2;PLK1;PLIN2;RRM2;CDH2;ITM2A;CENPF ;TTN;PRC1;PKIA;CDK1;KIF11;THBS2;ESPL1;HMMR;NUDT11;FADS2; AURKA;MKI67;CA12;KIF23;COL4A5;PRKACB;GINS1;KIF15;TPX2;CC NB1;MELK;SMC2;KIF18B;MCM4;ECT2;NCAPG;KIF4A;BUB1;BUB1B;C ENPE;ANP32E |
| dasatinib | 95 | 4650.868 | 59 | TOP2A;CDK1;MKI67;PLIN2;THBS2;MELK;PLK1;TFRC;NUSAP1;KIF20 A:CA12:CDH2:HMGCS1:SLC7A5:ITM2A:RRM2:CCNB2:SLC2A1:PKIA; FHL1:CCNB1:COL11A1:CCNA2:CA2:BCAT1:MCM4:MYBPC1:NEB;TT N;DLGAP5;GINS1;AURKA;COX7A1;COL4AS;TPX2;KIF4A;ANP32E;NC APG;MEX3C;SCUBE2;CENPE;ECT2;MB;PPP1R3C;BUB1;PRC1;DEPDC1 ;ASPM;CENPF;ESPL1;KIF15;BUB1B;KIF18B;KIF23;PRKACB;KIF11;HM MR;SMC2;FBXO5 |
| lapatinib | 12 | 4650.388 | 39 | RRM2;CDH2;PRKACB;ANP32E;ITM2A;CCNA2;MELK;CDK1;MCM4;PL K1;KIF20A;DLGAP5;DEPDC1;NCAPG;AURKA;CA12;COL11A1;HMMR; GINS1;PRC1;THBS2;TOP2A;CCNB2;TPX2;CENPF;CA2;MKI67;ASPM;K1F18B;KIF4A;BUB1;CENPE;ESPL1;ECT2;NUSAP1;COL4A5;KIF11;KIF15; KIF23 |
| vemurafenib | 13 | 4646.889 | 36 | HMGCS1;NUSAP1;CA12;CA2;CCNA2;RRM2;PRC1;FHL1;CDK1;KIF20A ;DLGAP5;AURKA;MCM4;PRKACB;GINS1;CCNB1;MELK;PLK1;ECT2;N CAPG;MKI67;CIT;TPX2;KIF18B;BUB1;TOP2A;TFRC;KIF11;ASPM;ANP 32E;CENPE;CENPF:KIF23;ESPL1:KIF15;DEPDC1 |
| KIN001-127 | 7 | 4678.63 | 36 | SLC7A5;CA2;CCNA2;GINS1;CDK1;BUB1;DLGAP5;TOP2A;RRM2;TFRC ;PLK1;MCM4;:NCAPG;:HMMR:KIF11;SMC2;KIF15;AURKA;TPX2;CENP E;MELK;KIF18B;ESPL1;KIF4A;KIF20A;MKI67;CCNB2;NUSAP1;CIT;AS PM;PRC1;DEPDC1;FHL1;KIF23;CENPF;ANP32E |
| alvocidib | 51 | 4627.834 | 54 | CCNB1;PLIN2;FBX05;AURKA;CCNA2;RRM2;PRKACB;TOP2A;ANP32 E;KIF20A;MELK;MKI67;CCNB2;CA2;CDK1;SLC2A1;MYBPC1;HMGCS 1:PLK1:TPX2:NEB:MB:CAMK2N1:PKIA:THBS2:PRC1:DLGAP5:TFRC:C A12;GINS1;ALDOC;CDH2;BUB1;DEPDC1;ECT2;NCAPG;MCM4;CENPF ;KIF2C;NUSAP 1;ASPM;CENPE;COL4A5;KIF23;HMMR;TROAP;FHL1;KI F11;SMC2;KIF15;CIT;ESPL1;KIFC1;KIF4A |
| vorinostat | 41 | 4622.2 | 55 | TOP2A;PLK1;GCNT1;NUSAP1;ALDOC;HMGCS1;COL11A1;AURKA;A MACR;CCNB2;CENPE;FHL1;BUB 1;GINS 1;KIF20A;BUB 1B;ANK1;CDH2 ;SCUBE2;ANP32E;CCNA2;CA12;RRM2;SLC2A1;BCAT1;GBE1;THBS2;C DK1;COL4A5;ECT2;KIF4A;PRC1;MCM4;SMC2;KIF18B;CAMK2N1;ASP M:CCNB1:CENPF:HMMR:MYBPC1:TTN:MELK:KIF23:KIF11:TPX2:ITM 2A;ACAT1;DEPDC1;MKI67;DLGAP5;CIT;NCAPG;ESPL1;KIF15 |
| WH-4-025 | 23 | 4620.141 | 51 | CDH2;FHL1;CA2;ITM2A;TOP2A;RRM2;CDK1;MELK;PLK1;CA12;CAM K2N1;PRKACB;FADS2;SLC7AS; TFRC:HMGCS1;SCUBE2;COL11A1;TH BS2;PLIN2;BCAT1;CCNA2;PRC1;NUSAP1;GINS1;MCM4;NCAPG;CIT;A URKA;CCNB2;TPX2;KIF20A;DLGAP5;MKI67;KIF18B;ASPM;CCNB1;A NP32E;BUB 1;CENPF;KIF11;SMC2;ESPL1;KIF15;KIF4A;FBXO5;ECT2;T ROAP;HMMR;KIF23;CENPE |
| AZD-7762 | 36 | 4609.786 | 45 | MELK;RRM2;KIF2C;CCNA2;CDK1;CA2;AGL;TFRC;KIF23;HMGCS1;GI NS1;TOP2A;TTN;MCM4;ITM2A;MYBPC1;PRKACB;COL11A1;PPP1R3C;BCAT1;ANP32E;NUSAP1;PKIA;PLK1;NCAPG;KIF18B;MKI67;DLGAP5; SLC2A1;FHL1;FBXO5;TPX2;AURKA:KIF11;KIF15;ASPM;CENPF;KIF20 A;BUB1;CA12;SMC2;CENPE;PRC1;KIF4A;ECT2 |
| NU-7441 | 11 | 4596.143 | 38 | C5;DLGAP5;AURKA;RRM2;KIF20A;GINS1;MCM4;CCNA2;MELK;PLK1 ;CDK1;CA12;KIF18B;NUSAP1;DEPDC1;TPX2;CCNB1;ANP32E;TOP2A;F HL1;BUB 1B;NCAPG;MKI67;KIF15;CCNB2;ASPM;ESPL1;BUB 1;HMMR; KIF11;SMC2;ECT2;KIF23;PRC1;KIF4A;ITM2A;CENPE;CENPF |
| pelitinib | 26 | 4584.937 | 49 | RRM2;TFRC;CA2;CCNA2;MYBPC1;MCM4;CDK1;GBE1;PLIN2;C5;PPP1 R3C;FHL1;CA12;HMGCS1;PLK1;KIF20A;MKI67; TOP2A; THBS2;CAMK2 N1;ALDOC;THSD7A;GINS 1;CCNB 1;MELK;ANP32E;SCUBE2;NUSAP1; DLGAP5;ASPM;COL4A5;NCAPG;ECT2;SMC2;PRC1;BUB1;KIF18B;KIF2 3;AURKA;CCNB2;TPX2;KIF11;ITM2A;NEB;CIT;HMMR;CENPF;DEPDC 1;KIF4A |
| PF-562271 | 20 | 4542.109 | 44 | CAMK2N1;SLC2A1;PLIN2;AURKA;PLK1;SLC7AS;RRM2;TFRC;HMGCS 1;CCNA2;MB;CCNB2;MYBPC1;TOP2A;PPP1R3C;ANP32E;MELK;CDK1; MKI67;CA2;CA12;ASPM;DLGAP5;FHL1;ECT2;GINS1;NUSAP1;CENPE; KIF18B;KIF4A;TPX2;PRC1;KIF2C;CENPF;KIF20A;BUB 1;HMMR;KIF11; NCAPG;SMC2;KIF15;KIF23;CIT;MCM4 |
| PIK-93 | 13 | 4542.322 | 40 | HMGCS1;CA2;FADS2;FHL1;ALDOC;TOP2A;CCNA2;MYBPC1;MELK;C CNB2;ANP32E;COL4A5;AURKA;GINS1;RRM2;TFRC;CDK1;MCM4;SM C2;CCNB1;KIF23;MKI67;NUSAP1;KIF20A;DLGAP5;NCAPG;PLK1;BUB 1B;CIT;CENPE;TPX2;ESPL1;BUB1;KIF15;KIF18B;PRC1;KIF4A;ASPM;C ENPF;KIF 11 |
| PF-477736 | 27 | 4530.263 | 44 | CCNA2;MELK;RRM2;CA12;CDH2;HMGCS1;TFRC;CAMK2N1;PPP1R3C ;ITM2A;FHL1;PLK1;MCM4;GINS1;KIF15;ANP32E;KIF20A;CIT;TOP2A; ASPM;CENPF;CDK1;NUSAP1;MYBPC1;FBXO5;KIF11;CA2;DLGAP5;PR C1;TPX2;MKI67;SMC2;KIF18B;BUB1;ALDOC;NCAPG;AURKA;KIF23;P LIN2;HMMR;CENPE;NEB;SCUBE2;ESPL1 |
| YM-201636 | 14 | 4522.646 | 42 | CA2;KIF20A;ITM2A;RRM2;TFRC;MCM4;PRKACB;CDH2;SLC2A1;TPX2 ;CDK1;GINS1;MELK;CA12;PPP1R3C;FHL1;CCNA2;CCNB2;PLK1;ANP32E;AURKA;TOP2A;KIF18B;DLGAP5;PRC1;BUB1;NCAPG;ASPM;DEPD C1;BUB1B;MKI67;CCNB1;NUSAP1;KIF23;CENPE;HMMR;KIF11;SMC2; KIF15;FBXO5;ECT2;KIF4A |
| BMS509744 | 8 | 4531.843 | 35 | CCNA2;CA12;RRM2;MELK;NCAPG;MCM4;KIF20A;MKI67;TOP2A;GIN S1;KIF18B;TFRC;PRC1;CDK1;SMC2:PLK1;THBS2;CCNB2;CCNB1;ANP 32E;BUB1;DLGAP5;KIF15;CIT;AURKA;TPX2;CDH2;PRKACB;HMMR;A SPM;CENPF;NUSAP1;ECT2;ESPL1;DEPDC1 |
| HG-14-8-02 | 8 | 4531.698 | 35 | TOP2A;CCNA2;GINS1;RRM2;CDK1;CCNB2;FHL1;PLK1;KIF20A;ACAT 1;KIF18B;CA2;PRC1;PRKACB;MELK;MCM4;NCAPG;CIT;ASPM;NUSA P1;DLGAP5;HMMR;MKI67;AURKA;TPX2;KIF4A;ANP32E;BUB1;KIF11; SMC2;KIF15;ECT2;MYBPC1;CENPF;ESPL1 |
| pracinostat | 29 | 4507.156 | 51 | SCUBE2;CDH2;GBE1;PLK1;SLC7A5;HMGCS1;FHL1;CCNA2:CA12;CA MK2N1;AURKA;MCM4;ACAT1;CCNB2;COL11A1;KIF20A;BUB 1;RRM2 ;BCATI1;CDK1;ASPM;TOP2A;PRC1;ECT2;MKI67;CCNB1;HMMR;KIF23; KIF11;COL4A5;THBS2;SMC2;TPX2;ANP32E;MELK;MB;PRKACB;GINS 1;NUSAP1;NCAPG;TTN;KIF18B;KIF4A;DEPDC1;DLGAP5;KIF15;CENP E;CA2;FBXO5;CENPF;ESPLI |
| withaferin-a | 55 | 4501.987 | 53 | CAMK2N1;THBS2;CA12;GINS1;RRM2;NEB;PLIN2;COX7A1;TOP2A;FH L1;CDK1;CCNA2;CA2;COL4A5;HMGCS1;TFRC;PLKl;CENPF;SLC2A1; COL 11A1;KIF20A;CENPE;MCM4;ECT2;CDH2;FADS2;DLGAPS;ASPM; MKI67;SCUBE2;NUSAP1;C5;ANP32E;ITM2A;CCNB1;PPP1R3C;CCNB2; PRC 1;KIF 18B;AURKA;MELK;HMMR;BUB 1;AGL;FBXOS;KIF 11;DEPDC I;TPX2;NCAPG;ESPL1;KIF4A;CIT;BUB1B |
| sorafenib | 12 | 4497.945 | 43 | FADS2;HMGCS1;PRKACB;COL11A1;TIHBS2;ITM2A;CCNB2;PLIN2;TOP 2A;CCNA2;RRM2;PPP1R3C;PLK1;KIF20A;GINS1;ALDOC;DLGAP5;ASP M;NUSAP1;MKI67;CA12;MELK;PRC1;CDK1;FHL1;CENPF;KIF18B;MC M4;KIF11;AURKA;CCNB 1;BUB 1;HMMR;TPX2;BUB 1B;NCAPG;SMC2; KIF15;KIF23;CENPE;ESPL1;KIF4A;ANP32E |
| SB590885 | 14 | 4476.087 | 40 | COL11A1;PLIN2;CA12;C5;ITM2A;CCNB2;FADS2;HMGCS1;TOP2A;DL GAP5;CCNB1;RRM2;ANP32E;MCM4;ECT2;PLK1;AURKA;CCNA2;TPX2;MELK;CDK1;KIF11;MKI67;CENPE;PRC1;NCAPG;KIF20A;BUB 1;CENP F;KIF18B;SMC2;GINS1;ASPM;FHL1;ESPL1;NUSAP1;KIF23;HMMR;KIF 15;KIF4A |
| PD-173074 | 7 | 4506.378 | 36 | CA2;CAMK2N1;CCNA2;RRM2;CDK1;GINS1;PLK1;NUSAPI;KIF20A;TO P2A;BUB1;DLGAPS;CA12;ASPM;CCNB1;TFRC;PRC1;BUB1B;NCAPG;K IF23;HMMR;KIF11;MKI67;CCNB2;TPX2;CENPF;KIF4A;MCM4;KIF15;A URKA;CDH2;ECT2;CENPE;MELK;KIF18B;ANP32E |
| NVP-AEW541 | 12 | 4465.103 | 32 | CCNB2;RRM2;CDK1;CCNA2;ANP32E;PRKACB;GINS1;MCM4;PRC1;N USAP1;MELK;PLK1;DLGAP5;AURKA;TOP2A;MKI67:MYBPC1;KIF20A ;BUB1;KIF18B;CDH2;NCAPG;KIF4A;SMC2;CENPE;TPX2;CIT;COL4A5; HMMR;KIF15;KIF23;ASPM |
| PF-3758309 | 35 | 4462.843 | 50 | CCNA2;KIF20A;PLIN2;ITM2A;HMGCS1;CA2;THBS2;TOP2A;PLK1;CDK 1;RRM2;CDH2;PKIA;COL4A5;CA12;CCNB2;TFRC;GINS1;MCM4;SLC2 A1;CIT;ANP32E;SLC7A5;KIF11;FADS2;NUSAP1;DLGAP5;ASPM;CENP F;MELK;PRC1;KIF15;FHL1;CCNB 1;AURKA;CENPE;BCAT1;BUB 1;COL 11A1;MYL6B;ECT2;FBXO5;HMMR;MKI67;NCAPG;ESPL1;TROAP;KIF1 8B;KIF4A;DEPDC1 |
| SU-11274 | 7 | 4496.079 | 30 | RRM2;PKIA;MCM4;AGL;CDK1;CCNA2;GINS1;MELK;CA2;ANP32E;KIF 11;DLGAP5;SMC2;TPX2;KIF18B;KIF4A;PLK1;NCAPG;KIF20A;MKI67;T OP2A;KIF15;CENPF;NUSAP1;CIT;AURKA;ASPM;PRC1;BUB1;ITM2A |
| AS-605240 | 21 | 4459.784 | 38 | PLK1;BCAT1;MYBPC1;PRKACB;CCNA2;PRC1;CDK1;RRM2;TFRC;SLC 2A1;THBS2;GINS1;MCM4;ECT2;BUB1;MELK;CA2;ANP32E;NCAPG;CC NB2;CENPF;KIF18B;NUSAP1;AURKA;TPX2;PPP1R3C;ASPM;CAMK2N 1;MKI67;DLGAP5;TOP2A;KIF15;KIF20A;CDH2;HMMR;KIF11;KIF4A;D EPDC1 |
| CGP-60474 | 211 | 4459.348 | 57 | CCNA2;CDH2;AURKA;PLK1;MELK;RRM2;CA12;MB;TOP2A;CA2;CAM K2N1;CS;PLIN2;HMGCS1;CENPF;MKI67;DLGAPS;CCNB2;CDK1;GINS1 ;MYBPC1;KIF20A;NUSAP1;CCNB1;FBX05;ASPM;ECT2;SLC2A1;TFRC; PPP1R3C;KIF23;TPX2;ANP32E;MCM4;ITM2A;BUB 1 ;TTN;SLC7 A5;CENPE;THBS2;PRKACB;DEPDC1;CIT;PRC1;PKIA;LDHA;KIF2C;COX7A1;K IF18B;HMMR;NEB;KIF11;NCAPG;SMC2;KIF15;KIF4A;FHL1 |
| CGK-733 | 9 | 4464.748 | 39 | SCUBE2;CA12;FADS2;HMGCS1;PKIA;KIF20A; TOP2A;CCNA2;CA2;PL K1;CDK1;CCNB2;MELK;PRC1;PRKACB;ASPM;RRM2;KIF4A;NUSAP1; NCAPG;AURKA;MCM4;COL4A5;ALDOC;DLGAP5;GINS1;FHL1;CENPF ;PPP1R3C;TFRC;THBS2;TPX2;KIF11;HMMR;KIF18B;BUB1;MKI67;SMC 2;KIF15 |
| crizotinib | 13 | 4444.847 | 41 | COL4A5;PRKACB;AURKA;CA12;CDH2;COL11A1;TOP2A;RRM2;ITM2 A;CA2;CDK1;NUSAP 1;MCM4;GINS 1;MELK;ASPM;NCAPG;KIF20A;DL GAP5;MYBPC I;MB;PLKl ;CCNA2;PRC 1 ;ANP32E;SMC2;TPX2;FHL 1 ;MK I67;HMMR;KIF15;SCUBE2;ESPL1;KIF23;KIF11;CCNB2;KIF18B;CIT;CE NPE;KIF4A;BUB 1 |
| NVP-BGT226 | 35 | 4422.468 | 49 | CCNA2;GINS1;TFRC;BCAT1;PPP1R3C;COL11A1;FHL1;KIF20A;CA12;H MGCS 1;CA2;ALDOC;CDK1;MCM4;MKI67;BUB 1;RRM2;ACAT1;SLC7A 5;CCNB1;ITM2A;TOP2A;CDH2;PKIA;COL4A5;SMC2;PLK1;DLGAP5;M ELK;HMMR;NUSAP1;CCNB2;FADS2;SLC2A1;KIF18B;ANP32E;KIF4A; NCAPG;ECT2;TPX2;MB;FBXO5;KIF11;ASPM;AURKA;CIT;CENPF;ESP L1;PRC1 |
| saracatinib | 24 | 4423.393 | 50 | PLK1;COX7A1;TOP2A;CCNA2;CCNB2;GINS1;CDK1;HMGCS1;COL11A 1;TTN;MYBPC1;C5;RRM2;CA2;MCM4;ITM2A;CAMK2N1;MELK;MKI67 ;TPX2;DLGAP5;HMMR;ASPM;NEB;KIF11;PRKACB;NUSAP1;SLC7A5;C A12;PPP1R3C;PLIN2;AURKA;KIF18B;ANP32E;BUB 1B;PRC1;FHL1;BUB 1;KIF20A;TROAP;NCAPG;KIF15;CCNB1;CDH2;FBXO5;KIF23;CENPF;E SPL1;KIF4A;DEPDC1 |
| PLX-4720 | 8 | 4425.286 | 37 | FADS2;CCNB2;HMGCS1;PPP1R3C;COL11A1;PKIA;ITM2A;SCUBE2;TO P2A;CCNA2;GINS1;RRM2;MELK;PLK1;ANP32E;MCM4;KIF20A;AURK A;CDK1;NCAPG;ECT2;KIF23;KIF11;MKI67;CIT;TPX2;KIF18B;KIF15;AS PM;CENPE;CENPF;PRC1;DLGAPS;HMMR;NUSAP1;BUB1;DEPDC1 |
| sirolimus | 40 | 4393.774 | 50 | GINS 1;MELK;MCM4;CA12;ALDOC;RRM2;TFRC;CA2;CCNA2;MYBPC1 ;TTN;TOP2A;HMGCS1;PLK1;CDK1;BCAT1;PPP1R3C;NEB;PLIN2;SCUBE2;ITM2A;SLC7A5;CCNB1;AURKA;KIFI8B;PKIA;FADS2;PRC1;KIF20A ;DLGAP5;KIF4A;COL4A5;SLC2A1;LDHA;FHL1;ANP32E;NCAPG;BUB1; TPX2;NUSAP1;BUBIB;MKI67;CIT;CCNB2;ASPM;CENPF;ESPL1;KIF23; HMMR;FBXO5 |
| MK-1775 | 10 | 4369.96 | 39 | TFRC;PLIN2;THBS2;TOP2A;CA12;CAMK2N1;CDK1;PPP1R3C;MYBPC1 ;CDH2;COL11A1;FHL1;PRKACB;ITM2A;CCNA2;GINS1;MELK;RRM2;H MGCS1;ECT2;DLGAP5;KIF11;KIF15;ASPM;CA2;PRC1;NUSAP1;MCM4; KIF20A;TROAP;NCAPG;HMMR;MKI67;TPX2;CENPF;KIF18B;ESPL1;KI F4A;FBXO5 |
| NVP-TAE226 | 21 | 4337.281 | 35 | CCNA2;MELK;CDK1;KIF20A;PLK1;BCAT1;MYBPC1;ECT2;ASPM;CCN B2;ITM2A;AURKA;GINS1;HMGCS1;TFRC;MCM4;CDH2;RRM2;MKI67; DLGAP5;PRC1;SLC7A5;CCNB1;BUB1;CA2;NCAPG;TOP2A;FHL1;KIFI8 B;KIF11;SMC2;KIF23;TPX2;CENPF;PKIA |
| HG-5-113-01 | 18 | 4324.759 | 37 | RRM2;PRKACB;CCNA2;ASPM;MKI67;CAMK2N1;AURKA;TOP2A;CDK 1;CA2;CCNB2;GINS1;MCM4;PLK1;KIF23;CA12;NEB;SMC2;CIT;CDH2; NCAPG;MELK;HMGCS1;ECT2;PPP1R3C;PRC1;DLGAP5;ANP32E;HMM R;KIF20A;BUB1;KIF15;KIF11;CENPE;CENPF;KIF18B;FBXO5 |
| DCC-2036 | 11 | 4325.868 | 34 | TOP2A;CCNA2;MELK;HMGCS1;MCM4;PLK1;KIF20A;CA12;NEB;TTN; NUSAP1;GINS1;ASPM;RRM2;TFRC;MYBPC1;KIF18B;CCNB2;CENPF;C DK1;MKI67;THBS2;KIF11;HMMR;KIF15;TPX2;PRC1;KIF4A;ANP32E;B UB1;DLGAPS;CENPE;ESPL1;DEPDC1 |
| PF-431396 | 17 | 4313.388 | 40 | ASPM;DLGAP5;CA2;CAMK2N1;CA12;PRKACB;CCNA2;CCNB2;MYBP C1;AURKA;ANP32E;ITM2A;TOP2A;MELK:HMGCS1;ECT2;KIF20A;MK I67;BUB 1;RRM2;CDH2;PPP 1R3C;PLK1;CDK1;CCNB 1;PRC1;KIF18B;TF RC;TPX2;SLC2A1;NUSAP1;CENPE;FBX05;SMC2;CENPF;NCAPG;NEB; HMMR;KIF15;MB |
| BYL719 | 11 | 4313.98 | 34 | SLC7A5;ITM2A;HMGCS 1;PLK1;BCAT1;GINS 1;TFRC;CCNA2;KIF20A;A URKA;TPX2;MKI67;DEPDC1;COL4AS;CA12;MELK;RRM2;PRC1;ANP32 E:MCM4;CDK1;DLGAPS5;TOP2A;CENPE;FHL1;HMMR;NCAPG;KIF18B; KIF4A;SMC2;CCNB2;ASPM;ESPL1;BUB1 |
| INK-128 | 37 | 4298.783 | 45 | HMGCS1;CA2;CDK1;MYBPC1;RRM2;ITM2A;CA12;MELK;ANP32E;PPP 1R3C;GINS1;PKIA;FADS2;BCAT1;PRKACB;CCNA2;ALDOC;CCNB2;SL C7A5;FHL1;PLK1;MCM4;KIF20A;AURKA;MB;TTN;KIF18B;KIF4A;TOP 2A;TFRC;COL4A5;CIT:NUSAP1;NEB;HMMR;NCAPG;PRC1;ECT2;.DLG AP5;BUB 1;TPX2;CDH2;MKI67;SMC2;CENPF |
| sutent | 11 | 4292.268 | 34 | PLK1;KIF20A;CCNA2;CA12;SLC2A1;PKIA;FHL1;CCNB2;CDK1;DLGAP 5;AURKA;MELK;ANP32E;NCAPG;TOP2A;ASPM;RRM2;PRC1;MKI67;G INS1;TFRC;CA2;MCM4;ITM2A;C5;HMMR;SMC2;BUB1;KIF11;TPX2;CE NPF;NUSAP1;KIF15;CCNB1 |
| QL-XII-47 | 47 | 4277.174 | 53 | GINS1;HMGCS1;ALDOC;CCNA2;FHL1;KIF20A;CCNB2;C5;KCNH2;RR M2;TFRC;COL4AS;BCAT1;FADS2;COL11A1;CA12;CDH2;GBE1;KIF2C; CENPF;CDK1;PRKACB;PPP1R3C;CAMK2N1;THBS2;MYBPC1;MELK;M CM4;COX7A1;ACAT1;ANP32E;PLK1;DLGAP5;AURKA;KIF18B;NCAPG ;BUB 1;SCUBE2;PLIN2;AGL;PKIA;TOP2A;SMC2;ASPM;HMMR;MKI67; DEPDC1;NUSAP1;CIT;KIF11;TPX2;CCNB1;KIF4A |
| PHA-665752 | 11 | 4273.733 | 32 | CA2;COL4A5;RRM2;KIF20A;ITM2A;GINS1;MYBPC1;CDH2;DLGAP5;A URKA;CCNA2;PRC1;CENPE;NUSAP1;TOP2A;MELK;CIT;ASPM;CCNB2 ;TPX2;PLK1;NCAPG;MKI67;SMC2;CCNB 1;KIF18B;ANP32E;CDK1;MC M4;BUB1;HMMR;DEPDC1 |
| KW-2449 | 8 | 4283.404 | 34 | TOP2A;CIT;CA2;KIF20A;THBS2;GINS1;RRM2;CDK1;NUDT11;PLK1;DL GAP5;CCNA2;KIF18B;NCAPG;MCM4;KIF15;TPX2;CENPF;MELK;NEB; HMMR;ESPL1;TFRC;MKI67;SMC2;AURKA;ASPM;SLC7A5;CENPE;CC NB1;PRC1;KIF4A;NUSAP1;BUB1 |
| JWE-035 | 13 | 4260.713 | 31 | CA12;HMGCS 1;ITM2A;CCNA2;CDH2;SLC2A1;GINS 1;MCM4;AURKA; MYBPC1;CCNB1;RRM2;CCNB2;CDK1;TOP2A;PRC1;PLK1;CIT;TPX2;KI F18B;KIF11;MELK;KIF20A;NUSAP1;MKI67;KIF4A;SMC2;BCAT1;BUB1 ;DLGAP5;HMMR |
| LDN-193189 | 15 | 4249.403 | 40 | PPP1R3C;FHL1;THBS2;ASPM;RRM2;KIF20A;TOP2A;CCNA2;CCNB2;PL K1;TPX2;FADS2;CDH2;HMGCS 1;CENPE;KIF18B;DLGAPS;NUSAP 1;MY BPC1;CENPF;TFRC;SLC2A1;CA12;CAMK2N1;HMMR;MKI67;BUB 1;ACAT1;AURKA;GINS 1;PRC1;MCM4;NEB;MELK;CDK1;SMC2;KIF15;KIF4 A;ANP32E;ECT2 |
| AT-7519 | 45 | 4241.64 | 50 | CA2;FHL1;AURKA;CCNA2;HMGCS1;KIF20A;TOP2A;CA12;PLIN2;PLK 1;CCNB2;MELK;HMMR;ITM2A;DLGAP5;RRM2;C5;COL11A1;ANP32E; MYL6B;MYBPC1;TFRC;MB;PPP1R3C;NEB;MKI67;ECT2;TTN;CDK1;SL C2A1;GINS1 ;NUSAP 1 ;PRKACB;ASPM;CENPE;TPX2;CENPF;SLC7 A5;T HBS2;CDH2;BUB1;KIF11;SMC2;KIF15;CIT;KIF18B;ESPL1;PRC1;KIF4A; MCM4 |
| LY2603618 | 8 | 4231.565 | 33 | CCNA2;CDK1;NCAPG;MCM4;CCNB2;MELK;FHL1;KIF11;KIF15;TOP2A ;PLK1;KIF20A;BUB1;DLGAP5;MYBPC1;RRM2;KIF23;FBXO5;ECT2;AS PM;CENPF;KIF18B;HMGCS1;PRC1;MKI67;CENPE;NUSAP1;CA2;KIFC1 ;GINS1;TFRC;TTN;TPX2 |
| QL-X-138 | 34 | 4196.464 | 46 | RRM2;FHL1;COL4A5;MCM4;CCNA2;HMGCS1;PPP1R3C;GINS1;TFRC; CA12;FADS2;CDH2;MB;PLIN2;MKI67;PKIA;CA2;BCAT1;CAMK2N1;AL DOC;SLC7A5;MELK;ANP32E;KIF20A;DLGAP5;CENPF;CDK1;THBS2;P RKACB;NCAPG;PLK1;TPX2;CIT;TOP2A;CCNB2;KIF11;AURKA;BUB1; KIF23;HMMR;SMC2;ASPM;PRC1;KIF4A;NUSAP1;DEPDC1 |
| XMD-1499 | 7 | 4221.843 | 31 | TOP2A;PLK1;BUB 1;CCNB 1;CA2;COL11A1;CCNA2;CCNB2;CDK1;KIF2 0A;DLGAP5;RRM2;NUSAP1;MCM4;CIT;ASPM;CENPE;HMMR;KIF15;S MC2;NCAPG;KIF11;MKI67;KIF18B;CAMK2N1;PRC1;ANP32E;TPX2;CE NPF;ESPL1;KIF4A |
| BMS-387032 | 54 | 4184.756 | 48 | CCNA2;ASPM;MELK;AURKA;TOP2A;CA 12;MB;MYBPC 1;MYL6B;RR M2;CDK1;NUSAP1;MKI67;SLC2A1;CAMK2N1;PLIN2;HMGCS1;KIF20A ;ECT2;CCNB2;PLK1;GINS1;DLGAP5;MCM4;C5;HMMR;TPX2;NEB;CCN B1;ACATI1;DEPDCI1;ESPL1;ANP32E;CA2;KIF11;TFRC;PPP1R3C;NCAPG ;PRC1;KIF2C;CENPF;CENPE;COL11A1;THBS2;NUDT11;SMC2;KIF15;K IF23 |
| TWS-119 | 6 | 4230.571 | 35 | FHL 1;NUSAP 1;ITM2A;CCNA2;CCNB2;RRM2;PRC1;BCAT1;TOP2A;GIN S1;MELK;CDH2;CDK1;MCM4;TPX2;MYBPC1;PLK1;KIF20A;BUB1;AURKA;NCAPG;KIF11;MKI67;CIT;ASPM;KIF18B;KIF4A;ANP32E;ECT2;D LGAP5;NEB;TTN;FADS2;CENPF;MB |
| GSK-1070916 | 16 | 4161.588 | 39 | RRM2;MELK;PLK1;MKI67;CDH2;FHL1;ITM2A;GINS1;CCNA2;LDHA;T FRC;CDK1;SLC7A5;KIF20A;CA12;PPP1R3C;PRC1;CA2;TOP2A;CAMK2 N1;ANP32E;THBS2;CCNB2;MCM4;MYBPC1;NUSAP1;KIF15;SMC2;KIF 23;CCNB1;KIF18B;ECT2;BUB1;DLGAP5;NCAPG;KIF11;AURKA;TPX2; ASPM |
| dabrafenib | 19 | 4156.108 | 47 | MELK;HMGCS1;FHL1;CCNA2;GINS1;CENPE;MCM4;RRM2;TFRC;COL 11A1;SLC2A1;THBS2;TOP2A;CA12;ASPM;NUSAP1;SLC7AS;PLK1;KIF2 0A;FADS2;C5;CDK1;MKI67;PPP1R3C;NEB;TTN;CA2;PRC1;ITM2A;MY BPC1;ANP32E;ECT2;BCAT1;TPX2;KIF18B;CCNB2;CENPF;CAMK2N1;P LIN2;DLGAPS;NCAPG;KIF23;HMMR;KIF11;KIF15;KIF4A;BUB1 |
| QL-XI-92 | 6 | 4191.524 | 31 | ASPM;KIF20A;MKI67;RRM2;AMACR;HMGCS1;TOP2A;FHL1;DLGAPS; CCNB2;PRC1;CDK1;THBS2;GINS1;KIF23;KIF11;AURKA;CCNA2;NUSA P1;ECT2;PLK1;NCAPG;HMMR;KIF15;TPX2;CENPF;MELK;KIF18B;ESP L1;KIF4A;BUB1 |
| C646 | 6 | 4189 | 26 | TOP2A;ASPM;PLK1;CDK1;KIF20A;CCNA2;KIF18B;DLGAP5;GINS1;MY BPC1;CCNB1;RRM2;HMMR;CCNB2;TPX2;CENPF;MELK;HMGCS1;NU SAP1;NCAPG;MKI67;KIF15;AURKA;NEB;ESPL1;PRC1 |
| belinostat | 22 | 4118.242 | 43 | SCUBE2;CCNA2;CA12;BCAT1;KIF4A;BUB1;TFRC;HMGCS1;PRC1;TOP 2A;CCNB2;COL11A1;CDH2;GBE1;PLK1;ECT2;SLC7A5;RRM2;ACAT1; GINS 1 ;COL4A5;AURKA;ANP32E;KIF20A;CAMK2Nl ;CDKl ;HMMR;KIF 15;SMC2;CIT;PRKACB;DLGAP5;MELK;NCAPG;THBS2;KIF11;TPX2;M KI67;KIF23;NUSAP1;MYBPC1;ASPM;CENPF |
| AT7867 | 10 | 4110.017 | 34 | SLC7A5;TFRC;HMGCS1;CA12;SLC2A1;MYBPC1;BCAT1;CCNA2;PPP1 R3C;ALDOC;KIF20A;TOP2A;RRM2;TPX2;MELK;KIF18B;CDH2;CENPF; CA2;MKI67;SMC2;GINS1;CDK1;NCAPG;BUB1;DLGAP5;PLK1;CCNB2; PRKACB;KIF15;AURKA;ASPM;PRC1;MCM4 |
| GSK-1059615 | 92 | 4094.046 | 51 | GINS1;AURKA;TFRC;HMGCS1;PPP1R3C;FADS2;CA2;PLK1;CCNA2;RR M2;MCM4;CDK1;ACAT1;ALDOC;ITM2A;MYBPC1;COX7A1;NUSAP1; MELK;CA12;SCUBE2;SLC2A1;PLIN2;PKIA;MYL6B;FHL1;COL4A5;BC AT1;CAMK2N1;TOP2A;ANP32E;KIF20A;SLC7A5;CDH2;CIT;MKI67;PR KACB;TTN;CCNB2;GBE1;CCNB1;HMMR;PRC1;NCAPG;SMC2;BUB1;K IF18B;ECT2;TPX2;DLGAP5;KIF4A |
| HG-14-10-04 | 14 | 4067.134 | 29 | CDH2;PLK1;NEB;CCNA2;CDK1;MELK;TFRC;ACAT1;CA2;ITM2A;AUR KA;CA12;ANP32E;KIF20A;SMC2;GINS1;RRM2;PRKACB;HMMR;BUB1; DLGAP5;KIF18B;TOP2A;TPX2;PRC1;MCM4;MYBPC1;NCAPG;ECT2 |
| A443654 | 46 | 4064.866 | 49 | CCNB1;RRM2;SLC7A5;TFRC;HMGCS1;SLC2A1;CCNA2;CA12;CDH2;A URKA;CDK1;ACAT1;CA2;CAMK2N1;FHL1;FADS2;PKIA;PLK1;ECT2;A LDOC;ITM2A;MYBPC1;MELK;PPP1R3C;MB;COL4A5;NEB;ANP32E;KI F20A;TOP2A;GINS1;COL11A1;CCNB2;PRC1;HMMR:PLIN2;DLGAP5;CI T;BUB1;SMC2;KIF2C;MKI67;ASPM;DEPDC1;MCM4;NCAPG;KIF23;TP X2;CENPF |
| I-BET151 | 16 | 4049.673 | 39 | CCNA2;BCAT1;THBS2;CA12;C5;HMGCS1;CAMK2N1;AMACR;MELK;P LIN2;ITM2A;IL2RB;CDH2;GINS1;FHL1;TOP2A;CCNB2;RRM2;PLK1;CD K1;MCM4;ASPM;CA2;KIF11;ANP32E;SLC7A5;PPP1R3C;MKI67;MYBPC 1;ESPL1;PRC1;DLGAP5;ECT2;KIF23;HMMR;SMC2;AURKA;TPX2;COL 4A5 |
| AZ20 | 13 | 4048.23 | 34 | CCNA2;TFRC;SLC2A1;CA12;LDHA:HMGCS1;THBS2;GINS1;RRM2:CD K1;MYBPC 1;MELK;ITM2A;ALDOC;KIF20A;NUSAP 1;MCM4;TOP2A;C A2;PLK1;FHL1;DLGAP5;KIF18B;NCAPG;BUB1;KIF11;SMC2;KIF15;TT N;ASPM;MB;PRC1;CCNB2;ECT2 |
| CYT387 | 18 | 3995.63 | 32 | MYBPC1;RRM2;FHL1;THBS2;AURKA;SLC7A5;HMGCS1;KIF20A;CAM K2N1;PLK1;COL11A1;CA12;NUSAP1;CDK1;CCNB1;CENPE;TOP2A;CC NB2;ASPM;CCNA2;KIF23;HMMR;GINS1;MCM4;PRKACB;TPX2;CENPF ;SCUBE2;PRC1;BUB1;DLGAP5;NCAPG |
| BX-795 | 6 | 4029.952 | 26 | TOP2A;CENPE;PLK1;CA12;CDK1;SLC7A5;HMGCS1;COL11A1;SLC2A1 ;CCNA2;ASPM;MELK;RRM2;GINS1;KIF15;TPX2;CENPF;PRC1;NUSAP1 :KIF20A;HMMR;TTN;KIF18B;ESPL1;BUB1;DLGAP5 |
| methylstat | 7 | 3996.787 | 33 | CCNA2;PKIA;PLK1;CDK1;AMACR;CDH2;COL4AS5;PLIN2;TOP2A;DLG AP5;PRC1;SLC2A1;NUSAP1;KIF20A;BCAT1;GINS1;RRM2;FHL1;NEB;C CNB2;PPP 1R3C;MCM4;NCAPG;HMMR;KIF 11; SMC2;KIF 15;AURKA;TP X2;ASPM;CENPF;ITM2A;BUB 1 |
| WZ-4002 | 5 | 4059.161 | 22 | CCNA2;GINS1;RRM2;PLK1;NUSAP1;ANP32E;CDK1;TOP2A;PRC1;MKI 67;MCM4;KIF20A;PRKACB;BUB1;CCNB2;MELK;KIF18B;NCAPG;DLG AP5;FHL1;HMMR;SCUBE2 |
| SGI-1776 | 6 | 3930.27 | 25 | TOP2A;CCNA2;GINS 1;RRM2;CDK1;MCM4;CA2;KIF20A;PRKACB;ITM 2A;MELK;ANP32E;NCAPG;KIF23;CA12;MB;FHL1;PLK1;TTN;TPX2;HM MR;SMC2;AURKA;BUB1;DLGAP5 |
| linsitinib | 8 | 3878.753 | 27 | FHL 1;ALDOC;PPP 1R3C;CA2;AURKA;HMGCS 1;PLK1;CCNA2;MCM4;B UB1;SMC2;TPX2;ANP32E;COL4A5;CIT;SCUBE2;RRM2;MELK;PRC1;P KIA;MKI67;GINS1;NUSAP1;KIF20A;DLGAP5;CDK1;ITM2A |
| HG-5-88-01 | 8 | 3858.553 | 26 | RRM2;CA2;MELK;MCM4; SCUBE2;PPP 1R3 C;KIF 18B;HMGCS 1;PLK1;KI F20A;MYBPC1;AURKA;CCNA2;SLC7A5;TFRC;BCAT1;TPX2;CENPF;N USAP1;HMMR;DLGAP5;GINS1;MKI67;KIF15;ASPM;BUB1 |
| KIN001-265 | 5 | 3963.774 | 33 | TFRC;THBS2;ITM2A;TOP2A;CCNA2;PLK1;CDK1;CCNB2;TPX2;FHL 1; MCM4;KIF20A;MYBPC 1;ESPL 1;NUSAP 1;ANP32E;AURKA;HMMR;KIF 11;MKI67;KIF15;ECT2;BUB1;DLGAP5;CA12;RRM2;ASPM;CENPF;MEL K;KIF18B;PRC1;PKIA;DEPDC1 |
| CX-5461 | 5 | 3962 | 29 | CENPE;HMGCS1;KIF20A;CCNA2;MB;TOP2A;PRC1;PLK1;DLGAP5;NC APG;KIF23;MKI67;CCNB2;TPX2;NUSAP 1;DEPDC1;CDK1;MCM4;GINS 1;TROAP;HMMR;SMC2;KIF15;ASPM;CENPF;KIF18B;ESPL1;KIF4A;BU B1 |
| MLN-8054 | 8 | 3830.161 | 26 | CA2;CDK1;TOP2A;MELK;KIF20A;PRKACB;HMGCS 1;COL11A1;COX7 A1;THBS2;GINSl;RRM2;AURKA;KIF11;KIF15;CIT;ASPM;CCNB2;TPX2 ;KIF18B;ESPL1;PRC1;NUSAP1;PLK1;ITM2A;DLGAP5 |
| JNK-9L | 38 | 3812.349 | 40 | CCNA2;MELK;KIF20A;CCNB2;TFRC;AURKA;RRM2;HMGCS1;PRC1;C A12;MYBPC1;THBS2;ITM2A;TOP2A;PPP1R3C;KIF2C;GINS1;MCM4;PL K1;MB;GCNT1;CENPF;CA2;PKIA;DEPDC1;DLGAP5;NUSAP1;CDK1;PL IN2;CCNB1;ANP32E;MKI67;ASPM;CENPE;NCAPG;KIF11;ECT2;TPX2;B UB1;KIF15 |
| XMD16-144 | 19 | 3769.741 | 23 | CCNA2;GINS1;MELK;RRM2;BCAT1;MCM4;ITM2A;CA2;CDH2;ANP32E ;CA12;C5;CDK1;CAMK2N1;TOP2A;KIF18B;PLK1;AURKA;KIF20A;TPX 2;KIF4A;NUSAP1;KIF15 |
| AZD4547 | 6 | 3816.032 | 25 | TOP2A;CA12;CA2;KIF20A;RRM2;PRC1;NUSAP1;DLGAP5;CCNA2;PLK 1;CDK1;MCM4;ACAT1;GINS1;PRKACB;CCNB2;TPX2;MELK;FHL1;TF RC;NCAPG;ASPM;KIF18B;PKIA;ANP32E |
| bosutinib | 5 | 3864.613 | 28 | MELK;CDH2;FHL1;TOP2A;CCNA2;CDK1;CCNB1;RRM2;ASPM;KIF20A ;DLGAP5;NCAPG;HMMR;KIF11;SMC2;KIF15;CCNB2;NUSAP1;ECT2;B UB1;MYBPC1;PLK1;CIT;TPX2;CENPF;KIF18B;PRC1;MCM4 |
| nintedanib | 12 | 3723.869 | 28 | CCNA2;GINS1:MCM4;MYBPC1;CDH2;CA2;MELK;RRM2;KIF18B;CDK1 ;HMGCS1;THBS2;PLK1;SLC2Al;KIF20A;BCAT1;DLGAP5;CA12;DEPD C 1;AURKA;TOP2A;TPX2;PRC 1;NUSAP 1;SLC7AS;ESPL 1;BUB 1;KIF 15 |
| BS-181 | 4 | 3946 | 24 | TPX2;PLK1;CDK1;KIF20A;DLGAP5;TOP2A;CA12;CCNA2;CCNB1;MK16 7;RRM2;KIF11;HMMR;AURKA;ASPM;CCNB2;CENPF;MELK;KIF18B;C A2;PRC 1;KIF4A;ANP32E;BUB 1 |
| SAR-245408 | 4 | 3941.867 | 24 | RRM2;KIF 11;MKI67;TOP2A;CCNA2;MELK;PLK 1;CDK 1;NEB;KIF20A;B UB1;ASPM;CENPF;KIF18B;PRC1;KIF4A;NUSAP1;DLGAP5;AURKA;GI NS1;HMGCS1;HMMR;CCNB2;MCM4 |
| azacitidine | 11 | 3668.169 | 30 | TOP2A;AMACR;ALDOC;KIF20A;CA12;CCNB1;HMGCS1;NEB;CCNB2; RRM2;MYBPC1;CAMK2N1;PPP1R3C;COL4A5;CCNA2;GINS1;ASPM;TFRC;FHL1;HMMR;DLGAP5;MELK;CDK1;KIF11;MKI67;SMC2;CENPF;M B;ANP32E;BUB1 |
| I-BET | 16 | 3660.64 | 39 | CA12;PPP1R3C;ITM2A;COL4A5;SLC7A5;CDH2;BCAT1;CCNA2;HMGC S1;FHL1;PLIN2;PRKACB;ACAT1;TOP2A;ASPM;MYBPC1;CA2;C5;AMA CR;CCNB2;MELK;CAMK2N1;CDK1;SLC2A1;ECT2;PLK1;KIF20A;KIF18 B:GINS1;TPX2;RRM2;SCUBE2;CCNB1;THBS2;DLGAP5;MKI67;PRC1;N USAP1;MCM4 |
| CP-724714 | 9 | 3654.389 | 25 | CCNB2; CA2;SCUBE2;ITM2A;CCNA2;PLK1;KIF20A;MYBPC 1;MB;FHL 1 ;NEB;TTN;MELK;MKI67;RRM2;HMMR;NUSAP1;ANP32E;AURKA;GIN S1;KIF18B;CDK1;SLC7A5;HMGCS1;NCAPG |
| AKT-inhibitor-1-2 | 8 | 3617.659 | 22 | CDH2;ANP32E;TFRC;CA2;MELK;RRM2;MCM4;PRC1;NUSAP1;KIF20A; PLK1;BUB1;CA12;CCNB2;DLGAP5;CCNA2;TPX2;CDK1;MKI67;TOP2A; HMMR;SMC2 |
| entinostat | 10 | 3583.139 | 22 | CCNA2;PLK1;CCNB2;KIF20A;SCUBE2;GBE1;CA12;GINS1;PRC1;CDK1; PPP1R3C;CAMK2N1;ANP32E;AURKA;TPX2;ACAT1;TOP2A;RRM2;ME LK;HMMR;ESPL1;MCM4 |
| PFI-1 | 10 | 3574.82 | 35 | AMACR;HMGCS1;PRKACB;SCUBE2;CA12;BCAT1;MELK;THBS2;FHL1 ;PLIN2;C5;CAMK2N1;ITM2A;RRM2;MYL6B;CCNA2;MYBPC1;PLK1;N USAP1;TOP2A;CDK1;MCM4;PPP1R3C;COL4A5;DLGAP5;KIF15;GINS1; KIF23;HMMR;TPX2;ASPM;CCNB1;PRC1;FBXOS;ECT2 |
| rocilinostat | 6 | 3622.984 | 30 | CCNB2;GBE1;PLK1;ECT2;CCNA2;GINS1;THBS2;SMC2;TOP2A;RRM2; KIF20A;SCUBE2;ANP32E;ITM2A;HMGCS1;KIF23;KIF11;MKI67;TPX2; ASPM;CDK1;BUB 1;DLGAPS;MELK;KIF18B;ESPL1;PRC 1;KIF4A;MCM4 ;COL4A5 |
| GSK-2334470 | 9 | 3550.186 | 33 | CCNA2;RRM2;KIF20A;GINS1;MCM4;ITM2A;CA12;SLC7A5;PRKACB;M B:NUSAP1;TTN;CDH2;AURKA;CDK1;BUB1;FHL1;SLC2A1;TPX2;PLK1; CIT;TOP2A;HMMR;MKI67;KIF15;CCNB2;ASPM;CENPF;MELK;ESPL1;P RC1;KIF4A;DLGAP5 |
| WZ-3105 | 38 | 3521.619 | 28 | MYBPC1;NEB;TTN;PPP1R3C;PLIN2;AURKA;TOP2A;CCNA2;MELK;CD H2;CIT;PLK1;RRM2;HMGCS1;KIF20A;GINS1;CCNB1;CDK1;CCNB2;M B;TPX2;DLGAP5;PRC1;MKI67;CA12;KIF23;NUSAP1;CENPF |
| dinaciclib | 29 | 3518.842 | 28 | CCNA2;HMGCS 1;PLK1;MELK;AURKA;TOP2A;CCNB2;MYBPC1;CA2; MK167;CDKI;MB;CA12;KIF20A;PLIN2;GINS1;CCNB1;COL11A1;CIT;PK IA;FBXO5;ECT2;NUSAP1;SLC2A1;TPX2;CENPF;DLGAP5;NEB |
| BX-912 | 6 | 3540.524 | 22 | CDK1;NCAPG;MKI67;MELK:KIF18B;TFRC;MCM4;PLIN2;CCNA2;GINS 1;RRM2;CA2;CCNB1;ALDOC;TOP2A;PRC1;NUSAP1;ASPM;C5;ANP32E ;HMMR;BUB1 |
| mocetinostat | 21 | 3436.27 | 30 | CCNA2;CA12;AURKA;CCNB2;PLK1;KIF11;KIF20A;ECT2;GBE1;TOP2A ;PRC1;GINS1;ACAT1;MELK;RRM2;COL4A5;CA2;PRKACB;HMGCS1;B CAT1;NUSAP1;MYBPC1;CDK1;SCUBE2;ANP32E;ESPL1;CAMK2N1;M CM4;TPX2;PLIN2 |
| sunitinib | 23 | 3419.319 | 20 | TOP2A;CDK1;TTN;COL11A1;CIT;RRM2;NUSAP1;FHL1;CAMK2N1;TH BS2;CA12;PLIN2;PPP1R3C;MELK;GINS1;PKIA;MYBPC1;ITM2A;C5;KIF 11 |
| ON-01910 | 22 | 3405.445 | 23 | TOP2A;HMGCS1;CDK1;TTN;RRM2;COX7A1;GINS1;MELK;CAMK2N1; PLIN2;SLC7A5;PPP1R3C;C5;COL11A1;NUSAP1;THBS2;SCUBE2;CA12; CIT;ITM2A;CDH2;CCNB1;ACAT1 |
| JNK-IN-5A | 31 | 3405.883 | 22 | TOP2A;HMGCS1;CDK1;SCUBE2;RRM2;CA12;PLIN2;GINS1;MELK;AM ACR;PPP1R3C;THBS2;CAMK2N1;CDH2;NUSAP1;ITM2A;ASPM;C5;SLC 2A1;CCNA2;KIF18B;KIF11 |
| GSK-461364 | 29 | 3373.57 | 35 | CDK1;THBS2;CA12;RRM2;COL11A1;ITM2A;TOP2A;CAMK2N1;SLC7A 5;CDH2;CA2;SLC2A1;KIF20A;HMGCS1;PPP1R3C;FHL1;PLK1;CCNA2;T FRC;NUSAP1;MKI67;ECT2;CENPE;MYBPC1;GINS1;MCM4;CCNB1;MB; TTN;AMACR;KIF11;MELK;ASPM;ALDOC;PLIN2 |
| SYK-inhibitor | 13 | 3362.855 | 27 | CA12;CA2;PLK1;CAMK2N1;PLIN2;TFRC;CDH2;PPP1R3C;MB;GBE1;KI F20A;TOP2A;KIF11;CCNB2;ITM2A;RRM2;NUSAP1;HMGCS1;MYBPC1; ANP32E;DLGAP5;CCNA2;PRC1;CDK1;THBS2;ACAT1;GINS1 |
| vandetanib | 4 | 3584.867 | 18 | CCNA2;GINS 1;MELK;RRM2;TFRC;CDK1;MCM4;TOP2A;PLK1;CA12;M YBPC1;FHL1;CCNB2;ASPM;CA2;KIF20A;BUB1;DLGAP5 |
| roscovitine | 6 | 3406.762 | 21 | MELK;KIF2C;AURKA;GBE1;NUSAP 1;BUB 1;TOP2A;CDK1;PRKACB;H MGCS1;PPP1R3C;KIF20A;CCNA2;RRM2;KIF4A;ITM2A;DLGAP5;ASPM ;CCNB2;TFRC;PLK1 |
| AZD-6482 | 4 | 3554.733 | 20 | MYBPC1;HMGCS1;PLK1;GINS1;MELK;RRM2;CCNA2;CCNB2;CENPF; ANP32E;DEPDC1;KIF20A;DLGAP5;AURKA;KIF18B;ESPL1;NUSAP1;M CM4;ALDOC;MKI67 |
| ponatinib | 5 | 3435.419 | 24 | CCNA2;RRM2;PLIN2;KIF2C;CDK1;MCM4;KIF20A;TOP2A;ASPM;TFRC; GINS 1;PKIA;PLK 1;NCAPG;KIF23;CENPF;MELK;KIF 18B;PRC 1;NUSAP 1 ;DEPDC1;ECT2;BUB1;DLGAP5 |
| buparlisib | 17 | 3301.221 | 25 | RRM2;TFRC;CA2;SLC7AS;ITM2A;PPP 1R3C;HMGCS 1;GINS 1;MELK;CO X7A1;THBS2;BCAT1;TOP2A;CCNB2;PLK1;NUSAP1;CDK1;MCM4;CA1 2;ALDOC;CCNA2;KIF20A;DLGAP5;MB;PLIN2 |
| XMD-892 | 5 | 3380.742 | 26 | MYBPC1;CDH2:HMGCS1;COL11A1;BCAT1;CCNA2;MCM4;KIF20A;TO P2A;ASPM;PLK1;CDK1;DLGAP5;KIF15;CA12;CENPE;FHL1;KIF23;HM MR;GINS1;MELK;KIF18B;RRM2;CA2;KIF11;ITM2A |
| ZM-447439 | 5 | 3376 | 21 | CENPE;MYBPC1;FHL1;COX7A1;PPP1R3C;PLK1;MCM4;ITM2A;TOP2A; CCNA2;GINS1;RRM2;CDK1;DLGAP5;SLC7A5;ASPM;MELK;NUSAP1; MKI67;AURKA;CA2 |
| NVP-AUY922 | 25 | 3265.82 | 28 | CCNA2;RRM2;MCM4;KIF20A;CA12;CA2;PLK1;CDH2;PKIA;FHL1;GINS 1;MELK;ITM2A;TOP2A;CDK1;PRKACB;TFRC;CAMK2N1;MKI67;BCAT 1;KIF18B;ANP32E;CIT;TPX2;ASPM;CENPF;NCAPG;FBXO5 |
| XMD 11-85H | 2 | 4352.333 | 21 | TOP2A;CCNA2;CA12;GINS1;TPX2;MELK;RRM2;PLK1;NUSAP1;CDK1; MCM4;MKI67;KIF11;AURKA;CCNB2;CENPE;KIF18B;PRC1;KIF4A;BUB 1;DLGAP5 |
| PF_573228 | 5 | 3348.161 | 25 | CCNA2;SLC7A5;BCAT1;ITM2A;CA12;MELK;PLK1;CENPF;MCM4;KIF2 0A;THBS2;TOP2A;TFRC;PRC1;ANP32E;HMMR;AURKA;CCNB2;ASPM; CCNB1;KIF18B;PPP1R3C;NUSAP1;CDK1;DLGAP5 |
| PHA-767491 | 9 | 3240.859 | 22 | RRM2;NUSAP1;ITM2A;TOP2A;CCNB2;KIF20A;CCNB1;TFRC;CCNA2;C A12;MYBPC1;HMGCS1;GBE1;CIT;PKIA;PLK1;PLIN2;CDH2;ANP32E;M ELK;CDK1;DLGAP5 |
| tivantinib | 18 | 3228.222 | 19 | RRM2;CDK1;TOP2A;MYBPC 1;CA 12;AMACR;NUSAP 1;KIF 11;HMGCS 1; CAMK2N1;THBS2;GINS1;PKIA;MELK;PPP1R3C;KIF18B;CDH2;NEB;TT N |
| AST1306 | 5 | 3330.516 | 14 | TOP2A;GINS 1 ;RRM2;KIF 18B; CCNA2;KIF20A;DLGAP5; CCNB2;HMGCS 1;CDK1;HMMR;MELK;PRC1;PLK1 |
| GW-843682X | 10 | 3229.502 | 20 | CA2;FHL1;TOP2A;CDK1;ALDOC;RRM2;NUSAP1;SCUBE2;CAMK2N1; MKI67;HMGCS1;PPP1R3C;KIF20A;CA12;MELK;PLIN2;THBS2;ASPM;C ENPF;KIF 11 |
| (+)-JQ1 | 18 | 3226.161 | 29 | CA12;C5;HMGCS1;CCNA2;BCAT1;ACAT1;CAMK2N1;CDH2;MYBPC1; GINS 1;CDK1;THBS2;TOP2A;MELK;ECT2;MCM4;SCUBE2;SLC7AS;CA2 ;RRM2;PLK1;ASPM;DLGAP5;KIF15;CCNB2;MKI67;ITM2A;ANP32E;HM MR |
| tozasertib | 7 | 3250.055 | 18 | RRM2;MELK;MCM4;GINS1;NUSAP1;BCAT1;CDK1;ITM2A;CCNA2;TF RC;ANP32E;ASPM;KIF18B;NCAPG;DLGAP5;KIF15;SMC2;CIT |
| serdemetan | 4 | 3407.6 | 17 | CCNA2;CA2;PLK1;KIF20A;TOP2A;ASPM;CA12;CCNB2;ITM2A;HMMR; MKI67;CIT;CENPF;KIFC1;BCAT1;BUB1;DLGAP5 |
| RAF_265 | 9 | 3195.562 | 22 | CA12;FHL1;ALDOC;PLIN2;KIF20A;TOP2A;CCNA2;GINS1;RRM2;HMG CS1;PKIA;PRKACB;CCNB2;TFRC;ECT2;ASPM;MKI67;CDK1;NCAPG;K IF18B;PLK1;NUSAP1 |
| CP466722 | 6 | 3206.19 | 30 | RRM2;TFRC;ALDOC;CA12;KIF20A;ITM2A;CCNA2;CDK1;BUB1;COL11 A1;NUSAP 1;HMGCS 1;CAMK2N1;PLIN2;TOP2A;GINS 1;PLK1;KIF11;M KI67;KIF15;AURKA;ASPM;CENPF;MELK;PPP1R3C;PRC1;KIF4A;ANP3 2E;MCM4;DLGAP5 |
| AZ-628 | 11 | 3100.734 | 16 | MELK;RRM2;MCM4;ECT2;CCNA2;CA12;COL11A1;SLC7A5;PLK1;TFR C;MYBPC1;CDK1;ANP32E;PRKACB;BUB1B;CA2 |
| OTSSP167 | 13 | 3075.209 | 19 | MYBPC1;NEB;TTN;CCNA2;HMGCS1;PLK1;GINS1;CDK1;AURKA;SLC2 A1;CCNB2;TOP2A;CA2;MB;CA 12;MELK;CENPF;NUSAP 1;MKI67 |
| UNC0638 | 4 | 3279.333 | 18 | GBE1;MCM4;CA12;CA2;NUSAP1;TOP2A;CCNA2;CCNB2;CDK1;KIF20 A;MYBPC1;RRM2;PLK1;MKI67;TTN;TPX2;MELK;DLGAP5 |
| XMD-885 | 5 | 3134.581 | 21 | CENPF;RRM2;ITM2A;CCNA2;SLC7A5;MYBPC1;MCM4;CA12;CCNB1;P PP1R3C;TOP2A;GINS1;CA2;CDK1;KIF11;THBS2;BUB1;ASPM;MELK;P LK1;ECT2 |
| KU-60019 | 3 | 3437.714 | 14 | TOP2A;MELK;PRC1;CCNA2;PLK1;CDK1;DLGAPS;GINS1;TPX2;RRM2; CDH2;MCM4;KIF20A;THBS2 |
| VE821 | 3 | 3435.857 | 17 | CCNA2;MELK;CDH2;COL4A5;KIF20A;DLGAP5;TOP2A;CA12;GINS1;A SPM;CCNB2;CCNB1;CENPF;KIF18B;PRC1;PLK1;CDK1 |
| fostamatinib | 8 | 2994.51 | 22 | TOP2A;CDK1;THBS2;CA12;SLC7A5;PPP1R3C;TFRC;PRC1;ECT2;ALDO C;GINS1;CDH2;PLIN2;CCNA2;ANP32E;CCNB2;CENPE;HMGCS1;MB;P LK1;KIF20A;DLGAP5 |
| regorafenib | 6 | 3022.698 | 19 | HMGCS1;FHL1;NUSAP1S2;GINS 1;MELK;CD K1;TOP2A;CCNA2;PKIA;CENPE;PLK1;MCM4;ASPM;CCNB2;CAMK2N 1 |
| linifanib | 6 | 3021.349 | 20 | CCNA2;ASPM;RRM2;CIT;TOP2A;CCNB2;MYBPC1;MELK;PLK1;MKI67 ;CA12;GINS1;FHL1;NEB;PLIN2;C5;TTN;CCNB1;KIF20A;DLGAP5 |
| BMS-345541 | 8 | 2949.157 | 19 | CCNA2;KIF2C;AURKA;TOP2A;PPP1R3C;NEB;CA12;DLGAP5;MELK;N USAP1;RRM2;CDH2;CIT;SCUBE2;HMGCS1;CA2;PLK1;TPX2;MYBPC1 |
| AZD7762 | 4 | 3123.867 | 16 | TOP2A;CA12;GINS1;CAMK2N1;PLK1;NUSAP1;KIF20A;MKI67;CCNA2; PPP 1R3C;KIF 11;PRKACB;CENPE;CCNB 1;PRC1;DLGAPS |
| HG-9-91-01 | 3 | 3318.143 | 14 | TOP2A;CCNA2;PLK1;CCNB2;TPX2;HMGCS1;SLC2A1;KIF20A;RRM2;N USAP1;ANP32E;CDK1;HMMR;DLGAP5 |
| NPK76-II-72-1 | 5 | 2884.548 | 13 | MELK;RRM2;CDK1;PPP1R3C;THBS2;TOP2A;GINS1;TFRC;NUSAP1;CA MK2N1;PLIN2;ASPM;KIF11 |
| BIX-01294 | 4 | 2956.2 | 16 | CA12;PLK1;ITM2A;TOP2A;CCNA2;CDH2;PPP1R3C;ACAT1;CCNB2;MC M4;ASPM;RRM2;CDK1;COL4A5;KIF20A;DLGAP5 |
| quizartinib | 5 | 2813.742 | 15 | MYBPC1;THBS2;CCNA2;PRC1;PLK1;CDK1;NUDT11;CA12;KIF20A;DL GAP5;TTN;TOP2A;ASPM;CCNB2;NUSAP1 |
| GDC-0879 | 6 | 2721.492 | 22 | MYBPC1;PLIN2;BCAT1;TFRC;SLC2A1;THBS2;ITM2A;CA12;MELK;CA 2;HMGCS1;PPP1R3C;FHL1;TTN;SLC7A5;RRM2;ANP32E;MCM4;CCNA2 ;PLK1;CDK1;KIF20A |
| imatinib | 2 | 3569.333 | 12 | TPX2;CCNB2;COL4A5;KIF20A;TOP2A;CCNA2;MELK;RRM2;KIF18B;PP P1R3C;NUSAP1;CDK1 |
| AS-601245 | 6 | 2697.19 | 20 | TFRC;AURKA;TOP2A;CCNA2;RRM2;CCNB1;COL11A1;PLK1;MELK;H MGCS1;GINS1;C5;MYBPC1;NEB;TTN;ASPM;CENPF;NCAPG;BUB1;DL GAP5 |
| nilotinib | 5 | 2726 | 17 | CA2;FHL1;PRKACB;CA12;PPP1R3C;CAMK2N1;SCUBE2;DNAJC1;C5;C DK1;AURKA;TOP2A;CCNA2;GINS 1;PLK1;KIF20A;DLGAPS |
| GSK-1904529A | 3 | 2941.143 | 11 | CCNA2;PPP1R3C;PLK1;ANP32E;BCAT1;BUB1;AURKA;KIF18B;CA2;M CM4;KIF20A |
| GSK1059615 | 7 | 2573.827 | 16 | SLC7A5;PLK1;PPP1R3C;COL11A1;PLIN2;TOP2A;CAMK2N1;CDK1;MY BPC1;CA12;THBS2;GINS1;MELK;RRM2;HMGCS1;NUSAP1 |
| TPCA-1 | 4 | 2705.6 | 15 | CCNA2;FHL1;BUB1;TOP2A;SLC2A1;KIF20A;RRM2;TFRC;CDK1;ASPM :MELK;CDH2;HMGCS1;PRC1;PLK1 |
| CC-401 | 3 | 2826.143 | 13 | TOP2A;CA12;CDK1;PLIN2;THBS2;RRM2;CAMK2N1;ALDOC;GINS1;M YBPC 1;MELK;NUSAP 1;ITM2A |
| GDC-0068 | 2 | 3292 | 13 | CA12;CA2;CDK1;NEB;ITM2A;CCNA2;GINS1;SLC7A5;TFRC;PLK1;KIF2 0A;BUB1;AURKA |
| KIN001-220 | 6 | 2492.111 | 12 | TOP2A;CDK1;ITM2A;KIF20A;MELK;PLK1;GINS1;CA2;THBS2;CCNA2; CAMK2N1;AGL |
| TGX-221 | 2 | 3221 | 19 | CCNA2;TFRC;CIT;TOP2A;RRM2;NCAPG;MKI67;AURKA;TPX2;ASPM; CENPE;CENPF;MELK;KIF18B;ANP32E;CDK1;MCM4;KIF20A;DLGAP5 |
| cabozantinib | 4 | 2553.8 | 15 | CCNA2;CDK1;MCM4;PRC1;AURKA;SLC7A5;CA2;PLK1;ITM2A;MYBP C1;MELK;KIF18B;RRM2;FHL1;ECT2 |
| everolimus | 11 | 2387.299 | 21 | CA2;ALDOC;PRKACB;CCNB2;PPP1R3C;HMGCS1;BCAT1;ITM2A;CA12 ;FADS2;SLC2A1;SLC7A5;MYBPC1;COL4A5;TFRC;MB;CDK1;MCM4;PL K1;NEB;TTN |
| THZ-2-98-01 | 2 | 3179.667 | 16 | CCNB2;RRM2;MB;TOP2A;PLK1;NCAPG;KIF23;HMMR;KIF11;AURKA; ASPM;CENPE;CCNB1;NUSAP1;KIF20A;DLGAP5 |
| chelerythrine_chloride | 4 | 2491.733 | 15 | RRM2;CDH2;PLK1;HMGCS1;PPP1R3C;NUSAP1;TOP2A;CA12;GINS1;M ELK;FADS2;CAMK2N1;COL4A5;KIF20A;HMMR |
| ABT-737 | 2 | 3077 | 10 | CA12;MELK;TFRC;PRKACB;TOP2A;CAMK2N1;PKIA;CDK1;KIF20A;T HBS2 |
| dacomitinib | 5 | 2322.032 | 14 | TOP2A;CA12;CA2;MELK;RRM2;COL4A5;CDK1;CCNA2;FHL1;SLC2A1; KIF20A;GINS1;KIF18B;TFRC |
| NVP-BGJ398 | 1 | 4455 | 17 | TOP2A;RRM2;NCAPG;KIF23;KIF11;HMMR;SMC2;CCNA2;ASPM;MEL K;KIF18B;NUSAP1;CDK1;MCM4;ECT2;BUB1;DLGAP5 |
| Y-39983 | 5 | 2280.387 | 15 | CA12;RRM2;AGL;CCNA2;PLK1;COX7A1;AURKA;DLGAP5;CCNB1;HM GCS1;DEPDC1;ANP32E;CCNB2;PKIA;KIF20A |
| MGCD-265 | 3 | 2469.714 | 11 | TOP2A;CA12;MELK;RRM2;CDK1;MKI67;PRKACB;CCNA2;ESPL1;PLK 1;KIF20A |
| amuvatinib | 3 | 2461.571 | 10 | CA12;KIF20A;MELK;HMGCS1;KIFC1;CCNA2;GINS1;FADS2;PKIA;PLK 1 |
| garcinol | 1 | 4284 | 14 | TOP2A;GINS1;RRM2;HMGCS1;PLK1;TTN;CCNA2;ASPM;CCNB2;MEL K;CA2;PRC1;NUSAP1;CDK1 |
| GSK-J4 | 2 | 2802.333 | 6 | TOP2A;FHL1;PLK1;KIF20A;CCNA2;AURKA |
| pazopanib | 1 | 4199 | 13 | TOP2A;RRM2;PLK1;MKI67;CCNA2;ASPM;CCNB2;TPX2;CENPF;PRC1; NUSAP1;CDK1;DLGAP5 |
| hy-11001 | 4 | 2211.133 | 16 | CA12;DNAJC1;MELK;GBE1;ALDOC;AMACR;ESPL1;ANK1;BUB1;ACA T1;AURKA;TOP2A;CDH2;CENPE;KIF2C;BCAT1 |
| trichostatin_a | 27 | 2060.779 | 19 | TOP2A;CENPE;PLK1;GCNT1;ALDOC;COL11A1;NUSAP1;BUB1B;AGL; ANK1;AMACR;KIF20A;AURKA;FHL1;BUB1;CCNB2;GINS1;CDK1;HM MR |
| tivozanib | 3 | 2244.714 | 13 | CA12;MB;IL2RB;RRM2;MELK;COL11A1;GINS1;SLC7A5;TFRC;PLK1;S LC2A1;MCM4;BCAT1 |
| BIX-02189 | 2 | 2609 | 12 | MELK;FHL1;TOP2A;ASPM;CCNB2;CENPF;RRM2;PLK1;NUSAP1;CDK1 ;MKI67;DLGAP5 |
| curcumin | 6 | 1972.508 | 13 | CA12;GBE1;ALDOC;ACAT1;CENPE;BUB1;ESPL1;BCAT1;AMACR;KIF 20A;DEPDC1;FBXO5;AURKA |
| GSK-690693 | 5 | 1961.161 | 12 | RRM2;TFRC;PPP1R3C;CA12;MYBPC1;ANP32E;CCNA2;C5;CDK1;GINS 1;MCM4;TOP2A |
| cycloheximide | 6 | 1894.078 | 14 | C5;MELK;AURKA;FHL1;CDK1;FBXO5;PLK1;TOP2A;ARHGAP11A;CA1 2;THBS2;BUB1;CCNA2;ANK1 |
| barasertib | 3 | 2124.571 | 10 | COL4A5;THBS2;MYBPC1;MYL6B;NEB;BCAT1;CCNB2;ASPM;CENPF; MKI67 |
| chaetocin | 5 | 1923.759 | 14 | CCNB2;CS;AMACR;TOP2A;CENPF;ARHGAP11A;DNAJC1;CDH2;BCAT 1;FBXO5;HMGCS1;FHL1;BUB1B;AURKA |
| quinacrine_dihydrochloride_dihydr ate | 6 | 1863.556 | 15 | MELK;AMACR;DEPDC1;AURKA;CA12;CDK1;BCAT1;GINS1;ESPL1;B UB1;BUB1B;C5;HMGCS1;GBE1;ANK1 |
| BRD-K38615104 | 6 | 1857.302 | 11 | CA12;MELK;AMACR;AGL;AURKA;C5;NUSAP1;DNAJC1;ESPL1;ANK1; ACAT1 |
| ag14361 | 7 | 1813.016 | 14 | MELK;GBE1;ACAT1;CA12;C5;ANK1;BUB1;AURKA;CCNB2;NUSAP1;D LGAP5;FHL1;KIF2C;CENPE |
| tanespimycin | 9 | 1748.523 | 17 | HMGCS1;FHL1;PLIN2;THBS2;GINS1;CAMK2N1;MCM4;TFRC;BCAT1; MELK;COL11A1;ITM2A;TOP2A;CCNA2;CA12;RRM2;PLK1 |
| digitoxin | 6 | 1728.369 | 15 | MELK;AMACR;AURKA;ANK1;CCNB 1;AGL;ARHGAP 11A;HMGCS 1;GB E1;BUB1;ACAT1;BCAT1;CCNB2;FHL1;CDK1 |
| BRD-U00779237 | 6 | 1708.587 | 10 | CA12;DNAJC1;AMACR;ACAT1;AURKA;MELK;GBE1;NUSAP1;ALDOC ;ANK1 |
| hy-11004 | 3 | 1913.429 | 8 | CCNB1;AMACR;AURKA;AGL;BCAT1;DNAJC1;MELK;PLK1 |
| 16-hydroxytriptolide | 5 | 1725.968 | 15 | CA12;C5;DNAJC1;MELK;CENPF;PRC1;AMACR;ANK1;AURKA;AGL;B CAT1;BUB1;ACAT1;FHL1;KIF2C |
| pp-110 | 6 | 1691.111 | 11 | MELK;AGL;BCAT1;CA12;HMGCS 1;BUB 1;NUSAP1;CCNB 1;AURKA;D NAJC1;ACAT1 |
| hy-11009 | 3 | 1875.429 | 9 | CA12;DNAJC1;AMACR;BUB1;AURKA;MELK;NUSAP1;ESPL1;ACAT1 |
| menadione | 4 | 1689.2 | 12 | ARHGAP 11A;CA12;HMGCS 1;BUB 1;CDH2;COL11A1;GBE1;AGL;CDK1; BCAT1;GINS1;MELK |
| forskolin | 2 | 2103.333 | 7 | ARHGAP11A;AGL;ANP32E;TOP2A;CA12;CDH2;BUB1 |
| BRD-K00313977 | 2 | 2095.667 | 5 | MELK;NUSAP1;C5;PLK1;AURKA |
| irinotecan _hydrochloride_trihydrat e | 2 | 2056.333 | 6 | CENPE;FHL1;PLK1;ANK1;CA12;MELK |
| GW-5074 | 1 | 3066 | 6 | CCNB2;COL11A1;CDK1;HMMR;THBS2;DLGAPS |
| doxorubicin_hydrochloride | 8 | 1532.004 | 18 | CENPE;GBE1;COL 11A 1;AURKA;MELK;NUSAP 1;ANK1;CA 12;ALDOC; AMACR;DEPDC1;ACAT1;CDK1;BUB1;FADS2;C5;AGL;FHL1 |
| BRD-K54256913 | 4 | 1617.933 | 13 | MELK;AURKA;AGL;FHL1;CA12;GINS1;HMGCS1;DEPDC1;BUB1;DLG AP5;CDH2;KIF20A;BCAT1 |
| CTB | 1 | 3024 | 6 | TOP2A;CCNB 1;MELK;HMGCS 1;PRC1;TTN |
| enzastaurin | 4 | 1580.933 | 10 | CA12;C5;NUSAP1;CA2;PLIN2;PRC1;ASPM;CCNB1;KIF20A;DLGAP5 |
| A66 | 1 | 2950 | 5 | CCNA2;TFRC;PLK1;KIF20A;BCAT1 |
| tpca-1 | 3 | 1683.143 | 8 | MELK;BCAT1;AURKA;CA12;AMACR;FHL1;CDK1;KIF20A |
| a-275 | 1 | 2940 | 4 | MELK;CDK1;BCAT1;AURKA |
| CHIR-99021 | 5 | 1517.387 | 13 | CCNB2;MB;CAMK2N1;COL11A1;RRM2;ALDOC;KIF20A;THBS2;MYBP C1;ITM2A;PPP1R3C;FHL1;NUSAP1 |
| CH5424802 | 1 | 2917 | 8 | TOP2A;CA12;ASPM;HMGCS1;COL11A1;FHL1;COL4A5;KIF20A |
| r406 | 3 | 1665.857 | 10 | CA 12;DNAJC 1;HMGCS 1;AGL;BCAT1;ARHGAP 11A;GBE 1;AURKA;AM ACR;BUB1B |
| temsirolimus_[ci779] | 2 | 1928 | 5 | CA12;DNAJC1;MELK;BCAT1;AURKA |
| 1-6307 | 3 | 1612.286 | 11 | MELK;AMACR;BCAT1;DLGAP5;CA12;DNAJC1;ACAT1;AURKA;C5;NU SAP1;KIF2C |
| BRD-K56343971 | 4 | 1497.867 | 11 | CA12;MELK;BUB1;CCNB1;AMACR;BCAT1;AGL;DNAJC1;HMGCS1;C OL11A1;NUSAP1 |
| bms-536924 | 3 | 1604.143 | 7 | CA12;DNAJC1;BCAT1;MELK;AMACR;CDH2;AURKA |
| BRD-K15563106 | 3 | 1610.26 | 14 | ASPM;MELK;FHL1;KIF2C;CCNA2;GBE1;DEPDC1;ACAT1;TOP2A;CA12 ;CENPE;DNAJC1;COL11A1;BCAT1 |
| BRD-U51951544 | 4 | 1464.533 | 12 | C5;AMACR;PLK1;AURKA;MELK;FHL1;NUSAP1;ANK1;ACAT1;CA12; DNAJC1;BUB1 |
| ku_0060648_trihydrochloride | 5 | 1385.548 | 8 | TOP2A;BCAT1;AURKA;GINS1;MELK;NUSAP1;AGL;FHL1 |
| digitoxigenin | 3 | 1524.571 | 12 | ARHGAP 11A;MELK;CDK1;CCNB2;GDF 11;AMACR;ANK1;DLGAPS;TO P2A;DNAJC1;BUB 1;ACAT1 |
| hy-10459 | 3 | 1515.143 | 9 | CA12;NUSAP1;ALDOC;ACAT1;MELK;AMACR;ESPL1;AURKA;ANK1 |
| BRD-K17953061 | 2 | 1753.667 | 8 | MELK;AMACR;NUSAP1;ACAT1;AURKA;CA12;GBE1;ALDOC |
| BRD-K57080016 | 5 | 1348.774 | 11 | DNAJC 1;MELK;AMACR;HMGCS 1;COL 11A 1;NUSAP 1;BCAT1;AURKA; AGL;FHL1;CCNB1 |
| t3205 | 1 | 2611 | 4 | CA12;MELK;GBE1;AGL |
| ncgc00242557-01 | 1 | 2604 | 7 | ARHGAP11A;ASPM;CENPE;MELK;CDH2;HMGCS1;ANK1 |
| azd8055 | 6 | 1320.81 | 8 | CA12;MELK;BCAT1;NUSAP1;GBE1;C5;CDH2;AGL |
| cyclosporine | 3 | 1485 | 7 | AGL;CDK1;BCAT1;MELK;AURKA;CDH2;DEPDC1 |
| ei-293 | 3 | 1470.143 | 10 | CA12;BUB1;ACAT1;AURKA;DNAJC1;MELK;AMACR;ESPL1;DEPDC1; NUSAP1 |
| erythrosine_sodium | 2 | 1713.667 | 9 | AMACR;NUSAP1;PRKACB;BUB1;CIT;CA12;C5;CDK1;ANK1 |
| hy-50940 | 2 | 1681.667 | 9 | CA12;MELK;AMACR;GBE1;AURKA;PLK1;NUSAP1;ALDOC;ACAT1 |
| ro_31-8220_mesylate | 6 | 1280.889 | 13 | DNAJC1;AMACR;BCAT1;ACAT1;AURKA;CA12;GBE1;NUSAP1;MELK; ANK1;C5;ESPL1;FBXO5 |
| trap_101 | 1 | 2520 | 5 | TOP2A;MELK;DEPDC1;BCAT1;BUB1 |
| BRD-K31342827 | 4 | 1340.2 | 10 | MELK;NUSAP1;AURKA;AGL;CENPE;GBE1;COL11A1;KIF2C;FBXO5;K IF15 |
| ku-0063794 | 4 | 1323.267 | 8 | CA12;DNAJC1;MELK;BUB1;BCAT1;FHL1;CDK1;ANK1 |
| h7270 | 2 | 1648.333 | 8 | TOP2A;CENPE;AMACR;AURKA;HMGCS1;FHL1;ANK1;BUB1 |
| ncgc00165199-01 | 2 | 1645.667 | 6 | TOP2A;HMGCS1;COL11A1;AGL;BUB1;AURKA |
| BRD-K04923131 | 3 | 1409.571 | 11 | MELK;GBE 1;AURKA;TOP2A;CA 12;AMACR;ANK1;CCNA2;FADS2;BC AT1;BUB1 |
| tranilast | 2 | 1638 | 5 | CDH2;BCAT1;AURKA;TOP2A;DEPDC1 |
| BRD-K04853698 | 1 | 2456 | 8 | CCNA2;AMACR;CDH2;COL11A1;BCAT1;PRKACB;BUB1;AURKA |
| BRD-K68202742_(trichostatin_a) | 5 | 1267.097 | 11 | TOP2A;CENPE;AMACR;BUB 1;AURKA;CA3;NUSAP 1;GCNT1;ALDOC;F HL1;CCNB2 |
| ym-155 | 2 | 1617.667 | 7 | MELK;ANK1;BUB 1;CDH2;GBE1;FHL1;ACAT1 |
| pik-90 | 1 | 2423 | 4 | CA12;C5;MELK;BCAT1 |
| sb_218078 | 3 | 1384.286 | 7 | CCNA2;BCAT1;AURKA;MELK;BUB1;C5;PLK1 |
| strophanthidin | 3 | 1380.571 | 11 | MELK;AMACR;ANK1;TOP2A;ARHGAP 11A;CA 12;AGL;CCNB2;FHL1;C DK1;ACAT1 |
| BRD-K12184916 | 3 | 1372.429 | 7 | CA 12;MELK;CDH2;DNAJC 1;CDK1;AURKA;NUSAP 1 |
| strophantine_octahydrate | 2 | 1596.333 | 4 | MELK;AMACR;ANK1;FHL1 |
| BRD-K99582188 | 1 | 2387 | 5 | MELK;CDH2;GBE1;PLK1;BUB 1 |
| leucine_enkephalin | 1 | 2378 | 4 | KIF18B;CDH2;AGL;CDK1 |
| cay10594 | 2 | 1582 | 6 | DNAJC1;MELK;CDH2;AGL;ANK1;AURKA |
| mk-2206 | 2 | 1576.667 | 7 | MELK;CDK1;BCAT1;AURKA;CA12;HMGCS 1;BUB 1 |
| t542500 | 2 | 1565.333 | 8 | CA 12;CENPE;AMACR;BUB 1;AURKA;CENPF;MELK;NUSAP 1 |
| pi_103_hydrochloride | 2 | 1542.333 | 6 | MELK;KIF18B;AURKA;CA12;DNAJC1;NUSAP1 |
| 15-delta_prostaglandin_j2 | 1 | 2311 | 3 | COL11A1;CDK1;AURKA |
| BRD-K01868942 | 1 | 2310 | 3 | HMGCS1;AURKA;CIT |
| KIN236 | 2 | 1537.333 | 6 | CA12;PPP1R3C;CA2;FHL1;BCAT1;ACAT1 |
| pf_750 | 3 | 1305.286 | 7 | DNAJC1;MELK;BCAT1;ACAT1;CA12;FBXO5;BUB1 |
| hy-10992 | 2 | 1514.667 | 7 | MELK;AMACR;BCAT1;ARHGAP 11A;DNAJC 1;PLK1;ANP32E |
| ouabain | 6 | 1145.857 | 14 | MELK;AGL;FHL 1;CDK1;BCAT1;ARHGAP 11A;DNAJC 1;HMGCS 1;ANK 1;BUB 1;ACAT1;AMACR;AURKA;CCNB2 |
| eugenol | 1 | 2249 | 4 | CA3;AGL;CDK1;AURKA |
| BRD-K49371609 | 9 | 1123.859 | 15 | CA12;CENPE;DNAJC1;NUSAP1;MELK;ESPL1;ACAT1;AGL;CENPF;KIF 15;AURKA;DLGAP5;BCAT1;C5;ANK1 |
| triclosan | 1 | 2234 | 4 | MELK;GBE 1;BCAT1;ANK1 |
| SB-525334 | 1 | 2227 | 5 | CA12;HMGCS1;PPP1R3C;MB;TTN |
| BRD-K85853281 | 2 | 1484 | 10 | KIF18B;ANK1;BUB1;AURKA;CA12;C5;MELK;ESPL1;PLK1;CDK1 |
| daunorubicin _hydrochloride | 4 | 1178.733 | 10 | CA12;NUSAP1;ALDOC;ACAT1;FADS2;BUB1;AURKA;MELK;AMACR; ANK1 |
| imd_0354 | 5 | 1140.194 | 8 | DNAJC1;HMGCS1;FHL1;AURKA;MELK;PLK1;BCAT1;KIF18B |
| KIN001-055 | 2 | 1462.667 | 6 | COL 11A1;THBS2;PRKACB;MELK;HMGCS 1;ALDOC |
| BRD-K19220233 | 4 | 1154.2 | 10 | CA12;AMACR;BUB 1;AURKA;MELK;ESPL1;DEPDC1;ACAT1;GBE1;NU SAP1 |
| zardaverine | 1 | 2163 | 3 | CDK1;AURKA;ACAT1 |
| k784-3131 | 2 | 1432.667 | 7 | GINS1;FBXO5;BCAT1;BUB1;GBE1;ACAT1;AURKA |
| thiazolopyrimidine | 1 | 2142 | 7 | CA12;CENPE;CDH2;HMGCS1;BCAT1;BUB1;ACAT1 |
| gsk-3b_inhibitor_viii | 1 | 2122 | 5 | AMACR;CDH2;ANK1;BUB1;ACAT1 |
| BRD-K28907958 | 3 | 1210.429 | 8 | ARHGAP11A;MELK;KIF18B;DNAJC1;NUSAP1;BCAT1;CDH2;COL11A1 |
| bufalin | 4 | 1126.467 | 13 | CCNB2;MELK;FHL 1;CDK1;ANK1;AURKA;ARHGAP 11A;DNAJC 1;HMG CS1;ESPL1;BUB1;ACAT1;AMACR |
| ncgc00182608-01 | 1 | 2112 | 6 | CA12;ASPM;MELK;HMGCS1;AGL;FBXO5 |
| BRD-K05649647 | 4 | 1124.8 | 6 | DNAJC1;MELK;FBXO5;CCNA2;CA12;BCAT1 |
| tgx-115 | 1 | 2090 | 4 | MELK;HMGCS1;PRC1;BUB1 |
| prednisolone_acetate | 1 | 2088 | 4 | CA12;CA2;DEPDC1;BCAT1 |
| dorsomorphin_dihydrochloride | 4 | 1104.933 | 13 | MELK;AMACR;AGL;CCNB2;DNAJC1;BCAT1;PRKACB;BUB1;ACAT1; GBE1;AURKA;CDK1;KIF20A |
| BRD-K93605482 | 1 | 2063 | 4 | CCNB1;AGL;BCAT1;AURKA |
| BRD-K88708949 | 1 | 2062 | 4 | TOP2A;GINS1;AGL;BCAT1 |
| t7765 | 1 | 2055 | 5 | CA12;DNAJC1;AMACR;HMGCS1;BCAT1 |
| naltrexone hydrochloride | 1 | 2054 | 5 | TOP2A;KIF18B;HMGCS1;CDK1;BCAT1 |
| cyclopiazonic_acid | 1 | 2052 | 2 | FHL 1;AURKA |
| BRD-K61341215 | 1 | 2042 | 4 | CCNB1;AMACR;BCAT1;AURKA |
| GSK-429286A | 2 | 1358.667 | 5 | HMGCS1;COL11A1;THBS2;TFRC;ECT2 |
| flavokawain_b | 1 | 2035 | 5 | TOP2A;CA12;RMGCS1;BUB1B;BCAT1 |
| BRD-K71726959 | 2 | 1356.333 | 9 | CA12;C5;HMGCS1;ANK1;DNAJC1;MELK;KIF2C;BCAT1;ACAT1 |
| BRD-K39232234 | 1 | 2031 | 3 | DNAJC1;LDHA;ANK1 |
| tipifarnib-p2 | 2 | 1352.667 | 6 | AGL;FHL1;AURKA;CA12;HMGCS1;BCAT1 |
| BRD-K72238567 | 5 | 1043.581 | 11 | AMACR;GBE1;AURKA;CA12;MELK;ESPL1;DEPDC1;PLK1;NUSAP1;A LDOC;ACAT1 |
| BRD-K73261812 | 4 | 1074.867 | 9 | CA12;DNAJC1;FHL1;AURKA;MELK;PLK1;BUB1;HMGCS1;PRKACB |
| emetine_dihydrochloridehydrate_( 74) | 5 | 1044.54 | 10 | MELK;KIF18B;FHL1;ANK1;CA12;C5;GBE1;AGL;BUB1;CA3 |
| mln4924 | 2 | 1337.667 | 8 | ARHGAP11A;FHL1;BCAT1;ASPM;AMACR;CDK1;BUB1B;AURKA |
| 1de225_(nvp-1de225) | 1 | 2000 | 4 | CDH2;AGL;KIF20A;BCAT1 |
| chr_2797 | 4 | 1062.4 | 16 | DNAJC 1;AURKA;CCNB 1;MELK;CDK1;ARHGAP 11A;COL 11A 1;HMMR; BCAT1;DLGAP5;CA12;GINS1;CENPE;HMGCS1;GBE1;BUB1 |
| JNJ-38877605 | 1 | 1992 | 3 | MYBPC1;NEB;TTN |
| BRD-K31841256 | 1 | 1978 | 4 | MELK;GBE1;BCAT1;AURKA |
| cinobufagin | 2 | 1317.667 | 8 | MELK;FHL1;ANK1;AURKA;AMACR;GBE1;DLGAP5;ACAT1 |
| bx-795 | 1 | 1972 | 6 | DNAJC 1;MELK;HMGCS 1;GBE 1;NUSAP 1;BCAT1 |
| methylene_blue | 2 | 1310 | 7 | CA12;CDH2;ANK1;LDHA;C5;GBE1;DEPDC1 |
| s1018 | 1 | 1957 | 4 | MELK;PLK1;NUSAP 1;AURKA |
| tw_37 | 2 | 1300 | 10 | KIF18B;AMACR;ACAT1;AURKA;DNAJC1;MELK;CDH2;FBXO5;BCAT1 ;DLGAP5 |
| gr_127935_hydrochloride | 1 | 1932 | 4 | MELK;HMGCS1;COL11A1;CDK1 |
| d-64131 | 1 | 1927 | 5 | MELK;CDK1;DEPDC1;BCAT1;ANK1 |
| BRD-K83670234 | 1 | 1908 | 5 | CA12;AMACR;ESPL1;BCAT1;AURKA |
| BRD-K28366633 | 1 | 1907 | 5 | TOP2A;HMGCS1;CDK1;KIF20A;BCAT1 |
| cot-10b | 4 | 1016.6 | 10 | C5;MELK;AMACR;AURKA;GBE1;CCNA2;BCAT1;ACAT1;ARHGAP11A ;CENPE |
| TAK-715 | 1 | 1897 | 4 | FADS2;HMGCS1;FHL1;PRKACB |
| BRD-K68007270 | 1 | 1892 | 2 | KIF18B;KIF15 |
| ei-346 | 2 | 1261.333 | 5 | CENPE;AMACR;GBE 1;AURKA;MELK |
| BRD-A36630025 | 3 | 1079.857 | 9 | CENPE;CDH2;PLK1;KIF18B;GBE1;BUB1B;CA12;RMGCS1;DLGAP5 |
| BRD-K92317137 | 2 | 1250.667 | 7 | ALDOC;HMMR;ACAT1;CDH2;GBE1;COL11A1;FHL1 |
| penitrem_a | 1 | 1874 | 4 | AMACR;CDH2;GBE1;BCAT1 |
| thm-i-94 | 1 | 1863 | 6 | AMACR;HMGCS1;COL11A1;CDK1;BUB1B;AURKA |
| dactinomycin | 4 | 991 | 10 | CA12;MELK;DEPDC1;BCAT1;NUSAP1;ALDOC;ACAT1;AMACR;ANK1; AURKA |
| BRD-K63068307 | 2 | 1236 | 6 | C5;BUB1;AURKA;CA12;DNAJC1;MELK |
| niclosamide | 3 | 1055.286 | 9 | DNAJC1;MELK;COL11A1;BCAT1;ANK1;DLGAP5;HMGCS1;FBXO5;AU RKA |
| BRD-K06666320 | 2 | 1226.333 | 8 | CA12;DNAJC1;NUSAP1;BUB1;CENPE;FHL1;BCAT1;AURKA |
| s6005 | 1 | 1836 | 5 | TOP2A;CA12;AMACR;BCAT1;AURKA |
| auranofin | 2 | 1215 | 6 | C5;GBE1;CDK1;BUB1;DEPDC1;KIF20A |
| wortmannin | 6 | 918.5397 | 10 | MELK;AMACR;BCAT1;ARHGAP11A;AGL;HMGCS1;BUB1;AURKA;PL K1;CA12 |
| **tubocurarine**_chloride_pentahydrat e_(+) | 1 | 1808 | 4 | AGL;CDK1;BUB1B;AURKA |
| OSI-930 | 2 | 1195 | 6 | HMGCSI1;THBS2;COX7A1;ITM2A;MELK;KIF20A |
| su11652 | 1 | 1787 | 5 | CDK1;DEPDC1;BCAT1;BUB1;AURKA |
| s1003 | 1 | 1774 | 4 | CENPE;FHL1;NUSAP1;AURKA |
| carprofen | 1 | 1766 | 2 | AGL;DEPDC1 |
| nicardipine hydrochloride | 1 | 1765 | 2 | AGL;AURKA |
| BRD-K07877311 | 1 | 1752 | 3 | CCNB1;ANK1;AURKA |
| BRD-K37021324 | 1 | 1751 | 3 | PLK1;CDK1;BUB1 |
| BRD-K96076993 | 1 | 1730 | 4 | CENPE;GBE1;AGL;KIF20A |
| ncgc00188714-01 | 1 | 1728 | 5 | ASPM;MELK;HMGCS 1;CDK1;ANK1 |
| zm_336372 | 3 | 982.5714 | 5 | CA12;DNAJC1;MELK;C5;CA3 |
| s1205 | 2 | 1143.333 | 7 | MELK;AMACR;DEPDC1;BUB1;AURKA;CA12;NUSAP1 |
| clobetasol_propionate | 2 | 1141.667 | 6 | TOP2A;MELK;BCAT1;AMACR;AGL;ANP32E |
| t5212475 | 1 | 1711 | 3 | CA12;PLK1;AURKA |
| BRD-K23478508_(digoxin) | 2 | 1130 | 6 | CCNB2;MELK;CDK1;AMACR;AGL;ANK1 |
| BRD-K53780220 | 1 | 1692 | 4 | CA12;MELK;ANK1;AURKA |
| BRD-K43620258 | 3 | 954 | 8 | DNAJC1;CCNB1;ANK1;GINS1;BCAT1;BUB1;MELK;KIF18B |
| pj_34_hydrochloride | 1 | 1667 | 4 | TOP2A;CA12;CDH2;BCAT1 |
| BRD-K05104363 | 3 | 940.5714 | 5 | MELK;AMACR;BCAT1;AGL;AURKA |
| s1042 | 1 | 1644 | 3 | MELK;GBE1;AGL |
| ncgc00167094-01 | 2 | 1094.333 | 6 | CCNB2;ANK1;MELK;DEPDC1;CDK1;BCAT1 |
| tyrphostin_ag_1478 | 3 | 936 | 10 | MELK;AURKA;TOP2A;DNAJC1;AMACR;COL11A1;FBXO5;BCAT1;AN K1;CA12 |
| BRD-K28916077 | 1 | 1629 | 4 | AMACR;BCAT1;AURKA;ACAT1 |
| BRD-K69852452 | 1 | 1626 | 5 | AMACR;BCAT1;ACAT1;CIT;AURKA |
| stk397047 | 1 | 1618 | 5 | CA12;FADS2;ESPL1;CDK1;BCAT1 |
| brompheniramine_maleate | 1 | 1612 | 3 | AMACR;AGL;BCAT1 |
| ski_ii | 2 | 1071.667 | 4 | BUB 1B;AURKA;DNAJC1;COL11A1 |
| BRD-A31107743 | 2 | 1048.667 | 7 | DNAJC1;MELK;CDH2;AMACR;BCAT1;BUB1;AURKA |
| er_27319_maleate | 2 | 1047.333 | 8 | C5;MELK;DEPDC1;ANK1;BUB1;AMACR;NUSAP1;AURKA |
| s1030 | 2 | 1045.333 | 8 | AMACR;NUSAP1;AURKA;TOP2A;CENPE;PLK1;GCNT1;ALDOC |
| xylazine | 1 | 1562 | 4 | C5;MELK;COL11A1;BCAT1 |
| mitomycin c | 3 | 880.1429 | 10 | CA 12;CDH2;BUB 1;AURKA;CENPE;AMACR;PLK 1;MELK;NUSAP 1;ALD OC |
| s1475 | 1 | 1531 | 4 | MELK;AMACR;BCAT1;AURKA |
| u-0126 | 1 | 1527 | 4 | ARHGAP11A;AMACR;CDK1;AURKA |
| BRD-K24754728 | 1 | 1517 | 3 | IL2RB;CDK1;KIF15 |
| BRD-K98731749 | 1 | 1516 | 3 | TOP2A;CDH2;AGL |
| BRD-K26664453 | 2 | 997.6667 | 6 | CA12;DNAJC1;HMGCS1;PLK1;BUB1;MELK |
| ncgc00184713-01 | 1 | 1480 | 3 | FHL1;DEPDC1;FBXO5 |
| hdac6_inhibitor_isox | 1 | 1477 | 6 | TOP2A;CCNB2;COL11A1;FHL1;BUB1B;AURKA |
| pd_407824 | 1 | 1469 | 4 | GINS1;AMACR;HMGCS1;AGL |
| pnu_74654 | 1 | 1465 | 4 | CA12;MELK;BCAT1;AURKA |
| BRD-K01896723 | 3 | 830.1429 | 11 | CA12;MELK;CDK1;AURKA;AMACR;AGL;PLK1;BCAT1;GBE1;NUSAP1 ;ALDOC |
| s1216 | 3 | 827.4286 | 10 | CA 12;NUSAP 1;ALDOC;ACAT1;AMACR;AGL;PLK1;AURKA;CCNB2;B UB1 |
| KIN001-242 | 1 | 1444 | 3 | SCUBE2;MYBPC1;FHL1 |
| su_5416 | 1 | 1443 | 3 | AGL;PLK1;BUB1 |
| c8273 | 1 | 1440 | 3 | KIF18B;FHL1;AURKA |
| BRD-K35004659 | 1 | 1439 | 3 | NUSAP1;ANP32E;CDK1 |
| BRD-K00541156 | 1 | 1438 | 3 | BCAT1;BUB1;AURKA |
| BRD-K43782924 | 1 | 1437 | 3 | MELK;GBE1;PLK1 |
| BRD-K34711351 | 1 | 1436 | 3 | TOP2A;BCAT1;BUB1 |
| sid_26681509 | 2 | 935.3333 | 4 | BCAT1;ANK1;AURKA;AMACR |
| HY-11007 | 1 | 1399 | 3 | CA12;PPP1R3C;ITM2A |
| mitoxantrone_dihydrochloride | 3 | 799 | 9 | MELK;AMACR;NUSAP1;AURKA;CA12;C5;ANK1;GBE1;ALDOC |
| ncgc00188536-01 | 1 | 1394 | 3 | CDH2;CDK1;BCAT1 |
| losartan_potassium | 1 | 1393 | 3 | GBE1;AGL;BCAT1 |
| BRD-K77681376 | 2 | 920.6667 | 8 | CA12;MELK;ANK1;AURKA;CS;AMACR;CAMK2N1;ACAT1 |
| BRD-K59962020 | 2 | 916.6667 | 6 | DNAJC1;MELK;ACAT1;PLK1;CDK1;BUB1 |
| cccp | 1 | 1369 | 3 | MELK;KIFI18B;AURKA |
| p5172 | 1 | 1368 | 3 | AMACR;CDK1;BUB1 |
| mbcq | 1 | 1365 | 3 | TOP2A;MELK;CDH2 |
| mls-0435526.0001 | 1 | 1351 | 4 | MELK;GBE1;BCAT1;AURKA |
| ei-156 | 6 | 676.2381 | 10 | MELK;AMACR;AURKA;ESPL1;DEPDC1;CA12;NUSAP1;ALDOC;ACAT 1;DNAJC1 |
| penfluridol | 1 | 1328 | 3 | AGL;BCAT1;AURKA |
| s1266 | 1 | 1327 | 3 | CA12;MELK;NUSAP1 |
| methyl_benzethonium_chloride | 1 | 1324 | 5 | DNAJC1;MELK;KIF18B;HMGCS 1;AURKA |
| cgp_53353 | 1 | 1318 | 2 | KIF18B;AURKA |
| mwp00339 | 1 | 1317 | 2 | MELK;PLK1 |
| s1095 | 1 | 1310 | 4 | TOP2A;PLK1;NUSAP 1;ALDOC |
| km_00927 | 1 | 1307 | 5 | TOP2A;CENPE;AMACR;FHL1;AURKA |
| prostaglandin_a1 | 1 | 1301 | 4 | GINS1;AGL;BCAT1;BUB1 |
| vu0365114-2 | 1 | 1280 | 5 | ASPM;MELK;HMGCS1;AGL;BCAT1 |
| gr-108 | 1 | 1275 | 4 | MELK;AGL;ANK1;AURKA |
| **BRD-**K62310379_(fluticasone_propionat e) | 1 | 1269 | 4 | MELK;KIF18B;AMACR;BUB1 |
| BRD-K30849099 | 1 | 1256 | 3 | AMACR;BCAT1;AURKA |
| BRD-U74615290 | 2 | 829.6667 | 5 | TOP2A;CCNA2;MELK;AURKA;FADS2 |
| BRD-K68191783 | 2 | 829.3333 | 3 | MELK;BCAT1;AURKA |
| BRD-K14609189 | 1 | 1241 | 2 | AMACR;AURKA |
| BRD-K15640888 | 1 | 1240 | 2 | CDK1;BCAT1 |
| vu0410183-2 | 2 | 820 | 7 | GINS 1;HMGCS 1;FHL 1;AURKA;DNAJC 1;MELK;BCAT1 |
| hy-10044 | 1 | 1225 | 3 | TOP2A;MELK;CDK1 |
| s1028 | 1 | 1224 | 3 | CCNB 1;AMACR;AURKA |
| BRD-K26818574 | 1 | 1223 | 3 | C5;MELK;AURKA |
| hy-11067 | 2 | 811.3333 | 3 | MELK;AMACR;AURKA |
| BRD-K56653679 | 1 | 1211 | 4 | CENPE;AMACR;BUB1;ACAT1 |
| BRD-K19724398 | 1 | 1210 | 4 | CCNB2;CENPF;NUSAP1;ALDOC |
| ALW-II-49-7 | 2 | 804.3333 | 5 | MELK;CDH2;HMGCS1;PPP1R3C;CAMK2N1 |
| SB-203580 | 1 | 1189 | 3 | MYBPC1;MB;NEB |
| BRD-K87909389 | 3 | 678.7143 | 8 | CA12;AMACR;BUB1;ACAT1;C5;DNAJC1;AURKA;PLK1 |
| niridazole | 1 | 1185 | 3 | TOP2A;CDH2;BUB1 |
| nefazodone hydrochloride | 1 | 1184 | 3 | TOP2A;CA12;CDH2 |
| digoxin | 3 | 674.4286 | 11 | MELK;FHL 1;CDK1;TOP2A;FADS2;GDF11;CDH2;BUB 1;ARHGAP 11A;H MGCS1;ACAT1 |
| BRD-K01436366 | 1 | 1179 | 4 | TOP2A;AGL;PLK1;AURKA |
| salermide | 1 | 1178 | 4 | CCNB1;BCAT1;ANK1;AURKA |
| BRD-K31851742 | 1 | 1175 | 5 | TOP2A;PLK1;GCNT1;NUSAP1;ALDOC |
| BRD-U29336476 | 2 | 776.6667 | 7 | GINS1;NUSAP1;AURKA;TOP2A;CCNB1;AMACR;PLK1 |
| BRD-U25771771 | 1 | 1149 | 3 | CDK1;BCAT1;AURKA |
| s-8599 | 1 | 1147 | 3 | MELK;AMACR;BCAT1 |
| gr_159897 | 1 | 1145 | 3 | MELK;CDH2;AURKA |
| (-)-gallocatechin_gallate | 1 | 1143 | 5 | CA12;CDH2;HMGCS 1;GBE1;ANK1 |
| nortriptyline _hydrochloride | 1 | 1135 | 2 | KIF18B;AURKA |
| pimozide | 1 | 1134 | 2 | KIF18B;AURKA |
| BRD-K33211335 | 1 | 1133 | 2 | GBE1;AURKA |
| u0126 | 2 | 750.6667 | 4 | CCNB 1;AMACR;MELK;AURKA |
| nsc_23766 | 2 | 749.3333 | 4 | MELK;HMGCS1;BUB1;C5 |
| KU-55933 | 1 | 1115 | 3 | TOP2A;ASPM;DLGAP5 |
| unc0321 | 2 | 742.6667 | 7 | GINS 1;AMACR;AURKA;MELK;HMGCS 1;COL 11A1;NUSAP 1 |
| flubendazole | 1 | 1110 | 3 | MELK;ALDOC;ANK1 |
| BRD-K53610452 | 1 | 1109 | 3 | GBE1;AGL;ALDOC |
| BRD-U82589721 | 1 | 1108 | 3 | DNAJC1;MELK;CDK1 |
| BRD-K78385490 | 1 | 1107 | 3 | MELK;CDH2;PLK1 |
| rs_67333_hydrochloride | 1 | 1106 | 3 | KIF18B;AGL;BUB1 |
| tws_119 | 1 | 1097 | 4 | CA12;AMACR;HMGCS1;COL11A1 |
| elesclomol | 1 | 1095 | 4 | ALDOC;HMMR;BCAT1;ACAT1 |
| ch_55 | 1 | 1094 | 4 | ARHGAP 11A;CA 12;HMGCS 1;BUB 1 |
| BRD-U68942961 | 2 | 724.6667 | 6 | CDH2;KIF 15;AURKA;DNAJC 1;MELK;NUSAP 1 |
| BRD-K39829853 | 2 | 723.3333 | 6 | MELK;GBE1;AGL;BCAT1;KIF18B;ANK1 |
| CG-930 | 1 | 1075 | 3 | MELK;FHL 1;COL4A5 |
| a8674 | 1 | 1071 | 3 | CA12;MELK;AURKA |
| BRD-A60245366 | 4 | 563.5333 | 11 | CENPF;ESPL1;NUSAP1;ALDOC;ACAT1;C5;MELK;AURKA;AMACR;CA 12;BUB 1 |
| emetine | 1 | 1056 | 4 | C5;DNAJC1;MELK;AURKA |
| BRD-K79222491 | 1 | 1053 | 4 | C5;NUSAP1;ALDOC;BCAT1 |
| BRD-K18724229 | 1 | 1052 | 4 | CCNB2;CA2;NUSAP1;ALDOC |
| bibr1532 | 1 | 1051 | 4 | DNAJC1;MELK;GBE1;DLGAP5 |
| vu0365118-1 | 1 | 1036 | 3 | CA12;HMMR;ANK1 |
| ncgc00184834-01 | 1 | 1035 | 3 | CA12;NUSAP1;AURKA |
| 7b-cis | 2 | 689 | 5 | CA12;AMACR;BUB1;TOP2A;BCAT1 |
| BRD-K18595892 | 1 | 1033 | 3 | KIF18B;CA3;AGL |
| cyt997 | 1 | 1031 | 3 | MELK;ANK1;AURKA |
| BRD-K78373679 | 1 | 1023 | 2 | GDF11;NUSAP1 |
| BRD-K73945663 | 1 | 1022 | 2 | KIF18B;AGL |
| BRD-K79404599 | 2 | 680.6667 | 5 | AMACR;AURKA;CCNA2;FADS2;BCAT1 |
| s1452 | 1 | 1021 | 2 | KIF18B;BCAT1 |
| BRD-A32214171 | 1 | 1020 | 2 | CA12;MELK |
| t5840683 | 1 | 1019 | 2 | CCNB1;ANK1 |
| retinoic_acid | 1 | 1017 | 2 | AMACR;AURKA |
| ursolic_acid | 1 | 1016 | 2 | AGL;BCAT1 |
| abt-751 | 1 | 1015 | 2 | ANK 1;AURKA |
| hy-11005 | 1 | 1013 | 4 | ARHGAP 11A;AMACR;KIF2C;ACAT1 |
| KIN001-244 | 1 | 1007 | 4 | FADS2;TFRC;HMGCS1;SLC2A1 |
| mebendazole | 2 | 661.6667 | 3 | MELK;AURKA;ANK1 |
| s1019 | 1 | 992 | 3 | AGL;FHL1;BCAT1 |
| BRD-K77987635 | 1 | 991 | 3 | GINS1;AGL;FHL1 |
| inca-6 | 1 | 990 | 3 | CENPE;CDH2;BCAT1 |
| BRD-K36055864_(cycloheximide) | 2 | 659 | 6 | MELK;AMACR;CDK1;AURKA;TOP2A;FHL1 |
| oxibendazole | 1 | 985 | 4 | MELK;KIF18B;ANK1;AURKA |
| BRD-K88832793 | 1 | 984 | 4 | TOP2A;HMGCS1;CDK1;KIF20A |
| gitoxigenin | 1 | 975 | 3 | TOP2A;MELK;AGL |
| BRD-K27170250 | 1 | 973 | 3 | MELK;AGL;BCAT1 |
| m-3m3fbs | 1 | 972 | 3 | TOP2A;CA12;ACAT1 |
| deoxycorticosterone | 1 | 971 | 3 | TOP2A;MELK;DEPDC 1 |
| curcubitacin_i | 2 | 647 | 9 | TOP2A;DNAJC1;AMACR;HMGCS1;BCAT1;PRKACB;MELK;PLK1;AUR KA |
| s1249 | 3 | 554.5714 | 8 | CA12;FADS2;BUB1;AURKA;MELK;NUSAP1;AMACR;PLK1 |
| olaparib | 1 | 970 | 3 | PLK1;PLIN2;KIF20A |
| cct_018159 | 1 | 960 | 4 | CA12;DEPDC1;BCAT1;BUB1 |
| bms-754807 | 2 | 637 | 4 | MELK;BCAT1;AURKA;FHL1 |
| BRD-K52522949 | 1 | 955 | 4 | GINS1;CCNB2;AMACR;NUSAP1 |
| chloroxine | 2 | 636.3333 | 5 | DNAJC1;MELK;CA12;GBE1;ANK1 |
| v4877 | 1 | 954 | 5 | TOP2A;AMACR;NUSAP1;ACAT1;AURKA |
| compound_58 | 1 | 949 | 2 | ANK 1;AURKA |
| BRD-K92419424 | 1 | 948 | 2 | CDK1;BCAT1 |
| BRD-K70161581 | 1 | 937 | 3 | MELK;PLK1;ALDOC |
| BRD-K19295594 | 1 | 932 | 3 | MELK;PLK1;ANK1 |
| idarubicin_hcl | 1 | 930 | 5 | CA12;MELK;AMACR;ESPL1;AURKA |
| vx-680 | 1 | 923 | 4 | MELK;CDH2;KIF20A;ACAT1 |
| hy-10161 | 2 | 613 | 5 | MELK;AMACR;HMGCS1;COL11A1;BCAT1 |
| ncgc00182942-03 | 1 | 914 | 4 | MELK;HMGCS1;CDK1;BCAT1 |
| piplartine | 1 | 911 | 4 | CA12;GBE1;KIF20A;BCAT1 |
| BRD-A58924247 | 3 | 516.2857 | 7 | BCAT1;BUB1;AURKA;GBE1;ACAT1;HMGCS1;FHL1 |
| doxorubicin | 1 | 896 | 3 | MELK;AMACR;AURKA |
| kirromycin | 1 | 895 | 3 | TOP2A;CDH2;DEPDC1 |
| mw-ras12 | 3 | 509.5714 | 10 | FBXO5;BCAT1;BUB1;CA12;CDH2;HMGCS1;PRKACB;AURKA;GBE1;A CAT1 |
| cymarin | 1 | 885 | 6 | ARHGAP 11A;DNAJC 1;HMGCS 1;ANK1;BUB 1;ACAT1 |
| BRD-K05197617 | 1 | 877 | 3 | AGL;DEPDC 1;AURKA |
| BRD-K63399564 | 1 | 862 | 2 | HMGCS1;BCAT1 |
| BRD-A15914070 | 1 | 861 | 2 | ANK 1;AURKA |
| protriptyline _hydrochloride | 1 | 860 | 2 | CCNB1;NUSAP1 |
| hts_12526 | 1 | 859 | 2 | ALDOC;BCAT1 |
| BRD-K58547240 | 1 | 858 | 2 | CCNB2;MELK |
| BRD-K97764662 | 1 | 857 | 2 | CCNB1;MELK |
| naltrindole | 1 | 856 | 2 | ASPM;AURKA |
| BRD-A62809825 | 1 | 851 | 6 | DNAJC1;MELK;COL11A1;FBXO5;BCAT1;AURKA |
| BRD-A11702965 | 3 | 474.4286 | 8 | CA12;MELK;AMACR;DEPDC1;ALDOC;BCAT1;GBE1;BUB 1 |
| BRD-A19248578 | 1 | 830 | 3 | NUSAP 1;KIF2C;AURKA |
| delta1-hydrocortisone_21-hemisuccinate | 1 | 829 | 3 | DNAJC1;MELK;BCAT1 |
| ro_28-1675_? | 1 | 827 | 3 | CA12;DNAJC1;BCAT1 |
| ha-1004 | 1 | 826 | 3 | CA12;CDK1;BUB1 |
| BRD-K75081836 | 1 | 824 | 4 | TOP2A;CCNB2;CENPE;AURKA |
| SB-216763 | 1 | 820 | 3 | NUDT11;CA2;FHL1 |
| ALW-II-38-3 | 1 | 810 | 3 | CA12;HMGCS1;ALDOC |
| vinblastine_sulfate | 1 | 805 | 3 | MELK;HMMR;ANK1 |
| f9428 | 1 | 804 | 3 | CCNB1;AGL;BCAT1 |
| reichstein/c,aeos_substance/c-*s | 1 | 803 | 3 | PLK1;DEPDC1;BUB1 |
| BRD-K35716340 | 1 | 800 | 3 | CCNB1;AGL;BCAT1 |
| arachidonyl_trifluoromethyl_ketone | 1 | 792 | 4 | GINS1;BCAT1;BUB1;ACAT1 |
| BRD-K02113016 | 1 | 791 | 4 | TOP2A;CENPE;DNAJC1;MELK |
| s1053 | 1 | 773 | 2 | FHL 1;NUSAP 1 |
| ncgc00183696-01 | 1 | 771 | 2 | BCAT1;BUB1 |
| BRD-K23673040 | 1 | 770 | 2 | BCAT1;AURKA |
| selamectin | 1 | 753 | 3 | DNAJC1;MELK;AGL |
| BRD-A75931230 | 1 | 752 | 3 | AMACR;AGL;BCAT1 |
| bi_2536 | 1 | 749 | 3 | NUSAP1;DEPDC1;CDK1 |
| bibu_1361_dihydrochloride | 1 | 747 | 3 | HMGCS1;BUB1B;AURKA |
| teniposide | 1 | 744 | 5 | TOP2A;AMACR;GBE1;BCAT1;ACAT1 |
| BRD-A94377914 | 2 | 493.6667 | 5 | NUSAP1;ANK1;AURKA;CA3;ECT2 |
| BRD-K56411643 | 1 | 736 | 4 | CA12;CDH2;COL11A1;AURKA |
| BRD-K39944607 | 1 | 733 | 4 | CA12;MELK;PLK1;AURKA |
| budesonide | 1 | 713 | 3 | C5;HMGCS1;AGL |
| BRD-K03122949 | 1 | 711 | 3 | CA12;MELK;GBE1 |
| BRD-K32501161 | 1 | 685 | 3 | MELK;AGL;AURKA |
| cc-100 | 1 | 683 | 3 | DNAJC1;BCAT1;AURKA |
| fk506 | 1 | 682 | 3 | CA12;CDH2;BUB1 |
| norcyclobenzaprine | 1 | 681 | 3 | TOP2A;MELK;DEPDC 1 |
| w-13_hydrochloride | 1 | 680 | 3 | CA12;CDK1;BUB1 |
| BRD-K11611839 | 1 | 677 | 4 | TOP2A;CDK1;DEPDC1;KIF20A |
| t_98475 | 1 | 671 | 2 | DNAJC1;HMGCS1 |
| s1485 | 1 | 670 | 2 | ANK 1;AURKA |
| cv_1808 | 1 | 669 | 2 | AGL;BCAT1 |
| epirubicin _hydrochloride | 2 | 443.3333 | 7 | CA12;COL11A1;AURKA;MELK;AMACR;ESPL1;ACAT1 |
| s1011 | 1 | 665 | 4 | PLK1;ALDOC;BCAT1;ANK1 |
| pkcbeta_inhibitor | 2 | 441.6667 | 4 | AGL;BCAT1;KIF18B;NUSAP1 |
| BRD-K83336168 | 2 | 434.3333 | 6 | ALDOC;ANK1;ACAT1;CENPE;AMACR;BCAT1 |
| hy-13001 | 1 | 647 | 3 | DNAJC1;CCNB1;MELK |
| BRD-K31484631 | 1 | 646 | 3 | COL11A1;BCAT1;AURKA |
| BRD-K94390040 | 1 | 644 | 3 | GBE1;AGL;BCAT1 |
| 6-diazo-5-oxo-1-norleucine | 1 | 640 | 3 | TOP2A;GINS 1;AGL |
| osi-906 | 1 | 639 | 3 | HMGCS1;BUB1B;AURKA |
| BRD-A84102390 | 1 | 637 | 5 | CA12;MELK;GBE1;NUSAP1;ACAT1 |
| BRD-K00317371 | 1 | 625 | 4 | CA12;NUSAP1;ALDOC;DLGAP5 |
| t1_hras26_brd-k01877528 | 1 | 624 | 4 | CDH2;BUB1;ACAT1;AURKA |
| mammea_a/ba_+_a/bb | 1 | 607 | 3 | MELK;AGL;AURKA |
| daunorubicin | 1 | 599 | 4 | FADS2;C5;PLK1;ANK1 |
| mls-0437633.0003 | 1 | 597 | 4 | MELK;CDH2;HMGCS1;FBXO5 |
| manumycin_a | 2 | 396.6667 | 5 | CA12;BCAT1;ARHGAP11A;BUB1;ACAT1 |
| BRD-K69569876 | 1 | 589 | 2 | MELK;GBE1 |
| t6129388 | 1 | 587 | 2 | MELK;NUSAP 1 |
| BRD-U44700465 | 1 | 586 | 2 | FHL 1;AURKA |
| ncgc00183412-01 | 1 | 584 | 2 | BCAT1;BUB1 |
| BRD-K83972459 | 1 | 570 | 3 | TOP2A;GCNT1;NUSAP1 |
| BRD-K68174511 | 1 | 569 | 3 | MELK;ANK1;AURKA |
| gsk-3_inhibitor_ii | 1 | 564 | 3 | CCNA2;DNAJC1;AGL |
| s1122 | 2 | 374 | 4 | NUSAP1;AURKA;ACAT1;MELK |
| kenpaullone | 1 | 554 | 4 | TOP2A;CCNA2;FADS2;AURKA |
| perhexiline_maleate | 2 | 366 | 6 | TOP2A;DNAJC1;HMGCS1;COL11A1;BCAT1;DEPDC1 |
| BRD-K16541732 | 1 | 540 | 3 | GINS1;HMGCS1;BCAT1 |
| cilostazol | 1 | 539 | 3 | TOP2A;C5;AURKA |
| BRD-K11540476 | 1 | 534 | 4 | TOP2A;HMGCS1;FBXO5;KIF20A |
| malonoben | 1 | 520 | 3 | CA12;DNAJC1;MELK |
| hli_373 | 2 | 346.3333 | 6 | CA12;CDH2;AURKA;MELK;AMACR;ANK1 |
| diflorasone_diacetate | 1 | 515 | 3 | CA12;BCAT1;BUB1 |
| gw_843682x | 1 | 511 | 4 | CA12;AMACR;GBE1;BUB1 |
| purvalanol_a | 1 | 510 | 4 | DNAJC1;MELK;FBXO5;BCAT1 |
| lasalocid_sodium | 1 | 509 | 4 | DNAJC1;HMGCS1;BCAT1;AURKA |
| mdl_72222 | 1 | 502 | 2 | AGL;FHL1 |
| rlm-2-12 | 1 | 501 | 2 | DEPDC1;BCAT1 |
| BRD-K14027855 | 1 | 497 | 4 | CCNB 1;MELK;HMGCS 1;CDK1 |
| rev-5901 | 1 | 488 | 3 | CCNB1;AGL;AURKA |
| mln2238 | 1 | 484 | 3 | TOP2A;BCAT1;ANK1 |
| sulconazole_nitrate | 1 | 481 | 3 | CA12;CDH2;BUB1 |
| ro_08-2750 | 2 | 315 | 6 | CA12;MELK;GBE1;ACAT1;BCAT1;ANK1 |
| ei-332 | 1 | 472 | 4 | AMACR;GBE1;AGL;AURKA |
| pi_828 | 1 | 453 | 3 | CA12;MELK;NUSAP1 |
| BRD-K91701654 | 1 | 451 | 3 | AMACR;BCAT1;AURKA |
| ibrutinib | 1 | 444 | 2 | CA12;TFRC |
| pkc-412 | 1 | 435 | 2 | ANK 1;AURKA |
| BRD-K74931283 | 1 | 434 | 2 | MELK;BUB 1 |
| s1130 | 1 | 433 | 2 | MELK;PLK1 |
| calcitriol | 1 | 432 | 2 | BUB1;AURKA |
| BRD-K56477805 | 1 | 414 | 3 | DEPDC1;BCAT1;AURKA |
| ver155008 | 1 | 412 | 3 | MELK;FBXO5;AURKA |
| doxepin _hydrochloride | 1 | 408 | 3 | TOP2A;CA12;HMGCS1 |
| hexylcaine hydrochloride | 1 | 407 | 3 | CA12;CDK1;BCAT1 |
| gamma-linolenic_acid_(18:3_n-6) | 1 | 406 | 3 | CDH2;BCAT1;BUB1 |
| sulpiride | 1 | 405 | 3 | CDH2;HMGCS1;BUB1 |
| pac_1 | 2 | 267.6667 | 4 | DNAJC1;MELK;BCAT1;ACAT1 |
| akti-1/2 | 2 | 263.6667 | 6 | HMGCS1;BCAT1;BUB1;AURKA;MELK;KIF18B |
| ivermectin | 1 | 374 | 3 | MELK;HMGCS1;AURKA |
| BRD-K76698671 | 1 | 372 | 3 | CA12;MELK;AMACR |
| s1367 | 1 | 371 | 3 | MELK;NUSAP1;BUB1 |
| s1362 | 1 | 370 | 3 | MELK;BCAT1;AURKA |
| BRD-K34968258 | 1 | 369 | 3 | MELK;PLK1;CDK1 |
| 1-sulforophane | 1 | 366 | 3 | AMACR;AGL;BUB 1 |
| pd_158780 | 1 | 365 | 3 | MELK;AMACR;AURKA |
| hki-272 | 1 | 363 | 5 | CENPE;DNAJC1;HMGCS1;PLK1;BCAT1 |
| BRD-K80348542 | 1 | 362 | 5 | TOP2A;C5;MELK;COL11A1;BUB1 |
| BRD-K20401833 | 1 | 357 | 4 | TOP2A;HMGCS1;KIF2C;ACAT1 |
| peitc | 1 | 356 | 4 | CA12;AMACR;HMGCS1;BUB1 |
| glyburide | 1 | 354 | 1 | AURKA |
| as605240 | 2 | 234.6667 | 6 | BCAT1;BUB1;AURKA;AMACR;AGL;ANK1 |
| BRD-K31706415 | 1 | 349 | 2 | KIF18B;ANK1 |
| hemado | 1 | 348 | 2 | AGL;BCAT1 |
| brefeldin_a | 1 | 346 | 2 | CCNB1;MELK |
| kuc103428n | 1 | 344 | 2 | GBE1;AURKA |
| pd_98059 | 1 | 342 | 2 | AGL;BCAT1 |
| sib_1893 | 1 | 333 | 3 | CENPE;C5;PLK1 |
| BRD-U43867373 | 1 | 330 | 3 | CCNB1;AMACR;AURKA |
| BRD-K06765193 | 1 | 327 | 3 | CDH2;PLK1;BUB1 |
| mw-a1-12 | 1 | 323 | 3 | CDK1;BUB1;AURKA |
| t_0901317 | 1 | 319 | 3 | CDH2;BUB1;AURKA |
| cucurbitacin_i | 1 | 318 | 3 | TOP2A;AMACR;AURKA |
| BRD-K53932786 | 1 | 301 | 3 | HMGCS1;PLK1;CIT |
| fenofibrate | 1 | 297 | 3 | MELK;CDH2;AURKA |
| ncgc00184830-01 | 1 | 290 | 4 | TOP2A;AMACR;NUSAP 1;AURKA |
| sb_202190 | 1 | 271 | 3 | AMACR;PLK1;AURKA |
| isotretinoin | 1 | 270 | 3 | AGL;BCAT1;AURKA |
| ncgc00182364-01 | 1 | 269 | 3 | TOP2A;HMGCS 1;AGL |
| BRD-K45399554 | 1 | 268 | 3 | MELK;HMGCS1;CDK1 |
| ag_555 | 1 | 263 | 3 | CA12;CDH2;BCAT1 |
| oxymetazoline_hydrochloride | 1 | 262 | 3 | CDK1;BUB1;AURKA |
| tg101348 | 1 | 258 | 4 | MELK;COL11A1;FHL1;BCAT1 |
| c3930 | 1 | 247 | 2 | GINS1;AURKA |
| BRD-K68588778 | 1 | 246 | 2 | MELK;AURKA |
| thioridazine hydrochloride | 1 | 244 | 2 | HMGCS1;BCAT1 |
| BRD-K90826279 | 1 | 243 | 2 | FBXO5;ANK1 |
| BRD-K84203638 | 1 | 236 | 4 | CA12;FHL1;NUSAP1;KIF2C |
| cercosporin | 1 | 235 | 4 | BUB1B;ANK1;ACAT1;AURKA |
| BRD-K49865102 | 1 | 219 | 3 | AMACR;AGL;AURKA |
| BRD-K50140147 | 1 | 217 | 3 | DNAJC1;MELK;PLK1 |
| p8624 | 3 | 122.5714 | 11 | CA12;NUSAP1;ALDOC;BCAT1;C5;AMACR;DEPDC1;AURKA;TOP2A;C DH2;ESPL1 |
| mesoridazine_besylate | 1 | 212 | 3 | CCNB1;AMACR;BCAT1 |
| hy-10518 | 1 | 205 | 4 | CA12;GBE1;NUSAP1;ALDOC |
| masitinib | 2 | 133.3333 | 4 | CA12;CA2;SCUBE2;TFRC |
| akt_inhibitor_iv | 1 | 197 | 4 | CA12;DNAJC1;HMGCS1;BCAT1 |
| idelalisib | 1 | 195 | 3 | SLC7A5;CA2;FHL1 |
| s(-)-timolol | 1 | 181 | 3 | TOP2A;BCAT1;BUB1 |
| cyclosporin_a | 1 | 165 | 2 | MELK;AURKA |
| a-1065 | 1 | 164 | 2 | MELK;NUSAP 1 |
| BRD-A20697603 | 1 | 163 | 2 | KIF18B;CDK1 |
| BRD-K92754162 | 1 | 162 | 2 | AMACR;AURKA |
| calpeptin | 1 | 161 | 2 | GINS1;MELK |
| dexamethasone | 1 | 160 | 2 | KIF18B;AURKA |
| ei-215 | 2 | 106.3333 | 4 | MELK;BCAT1;AURKA;ARHGAP11A |
| nutlin-3 | 1 | 141 | 3 | HMGCS 1;BUB 1B;AURKA |
| importazole | 1 | 138 | 4 | CA12;CENPE;ANK1;DLGAPS |
| aloisine_a | 1 | 125 | 3 | CA12;PRC1;ANK1 |
| indatraline | 1 | 109 | 2 | KIF18B;AURKA |
| BRD-A68065211 | 1 | 108 | 2 | BCAT1;BUB1 |
| 4-fluoro-n-pyridin-3-yl-benzamide | 1 | 106 | 2 | MELK;BCAT1 |
| KIN001-266 | 1 | 100 | 3 | CA2;IL2RB;PRKACB |
| BRD-K68548958 | 1 | 82 | 3 | CDH2;AGL;DEPDC1 |
| calyculin a | 1 | 81 | 3 | GINS1;BCAT1;AURKA |
| betamethasone_17,21-dipropionate | 1 | 80 | 3 | MELK;AMACR;AURKA |
| thioproperazine_dimesylate | 1 | 64 | 3 | AGL;DEPDC1;AURKA |
| carbidopa | 2 | 41.33333 | 4 | AGL;BCAT1;TOP2A;ASPM |
| gemcitabine | 1 | 62 | 3 | CA12;AGL;BCAT1 |
| BRD-K94325918 | 1 | 57 | 3 | CCNB1;CDH2;BCAT1 |
| BRD-K92158425 | 1 | 46 | 5 | C5;MELK;CAMK2N1;PRC1;BCAT1 |
| fenbendazole | 1 | 29 | 2 | KIF18B;AURKA |
| paroxetine hydrochloride | 1 | 27 | 2 | KIF18B;AURKA |
| s1035 | 1 | 26 | 2 | CCNB1;AURKA |
| BRD-K45044657 | 1 | 25 | 2 | NUSAP1;ACAT1 |
| rottlerin_ncgc00025228-11 | 1 | 23 | 2 | DNAJC1;MELK |
| cis-(z)-flupenthixol | 1 | 21 | 2 | MELK;GBE1 |
| fccp | 1 | 10 | 3 | MELK;AGL;CDK1 |

**Table 3. Summary of drugs search return from EnrichR-L1000 Chem Perturbation Up using downregulated genes in AA/P non-responders.**

| Drug Name | Number of L1000 Signature | Rank Score | Number Gene Target | Gene targets |
|---|---|---|---|---|
| WZ-3105 | 25 | 510.4512 | 21 | CSTA;S100A9;S100A8;CFD;GPX3;WIF1;SERPINI1;CCL5;RAMP1;DKK3;ISG15;F3;TSPA N8;BST2;MT1F;MT1E;SLC4A4;TF;PRSS8;NEFH;LST1 |
| BMS-387032 | 15 | 501.0502 | 19 | GPX3;WIF1;ISG15;SERPINI1;MT1F;MT1E;CFD;BST2;SLC4A4;TF;CSTA;LST1;S100A9;S 100A8;F3;DKK3;TSPAN8;RAMP1;NCAM1 |
| CGP-60474 | 51 | 500.8804 | 23 | CFD;GPX3;MT1F;ISG15;WIF1;CCL5;RAMP1;DKK3;TF;MT1E;CSTA;SLC4A4;SERPINI1; S100A8;TSPAN8;F3;NCAM1;ITGA8;S100A9;BST2;LST1;PRSS8;EMID1 |
| AT-7519 | 13 | 492.6546 | 17 | CSTA;GPX3;MT1F;S100A9;S100A8;CFD;NEFH;ISG15;RAMP1;MT1E;TF;CCL5;F3;WIF1; DKK3;NCAM1;TSPAN8 |
| PHA-793887 | 7 | 487.5512 | 18 | WIF1;ISG15;NEFH;CFD;GPX3;MT1F;MT1E;LST1;NCAM1;RAMP1;CSTA;CCL5;SERPIN I1;F3;DKK3;BST2;TSPAN8;SLC4A4 |
| mitoxantrone | 18 | 475.91 | 19 | CSTA;NCAM1;ISG15;DKK3;BST2;GPX3;CCL5;MT1E;WIF1;S100A9;S100A8;CFD;MT1F; SLC4A4;RAMP1;SERPINII1;TF;TSPAN8;NEFH |
| AZD-7762 | 9 | 468.6125 | 20 | CFD;CSTA;MT1F;S100A8;MTI1E;LST1;S100A9;RAMP1;WIF1;CCL5;ISG15;MID1;TSPAN 8;DKK3;TF;ITGA8;F3;GPX3;PRSS8;NEFH |
| AZD-5438 | 8 | 469.3373 | 18 | BST2;CCL5;SERPINI1;MLPH;TSPAN8;SLC4A4;CSTA;GPX3;MT1F;ISG15;CFD;MT1E;N CAM1;RAMP1;TF;WIF1;DKK3;F3 |
| BMS-345541 | 8 | 458.9412 | 18 | GPX3;WIF1;MT1F;MT1E;CFD;CSTA;TF;S100A8;DKK3;LST1;ISG15;NEFH;SLC4A4;RA MP1;F3;FCN1;S100A9;BST2 |
| alvocidib | 10 | 453.4076 | 15 | CSTA;GPX3;CCL5;ISG15;MT1F;MT1E;CFD;TF;S100A8;DKK3;RAMP1;WIF1;F3;LST1;S1 00A9 |
| PF-562271 | 9 | 444.0196 | 15 | MTI1F;S100A8;MT1E;CFD;GPX3;WIF1;CCL5;ISG15;CSTA;S100A9;DKK3;F3;RAMP1;TF; NEFH |
| WZ-4-145 | 7 | 444.8508 | 12 | MT1F;ISG15;MT1E;GPX3;S100A9;S100A8;BST2;CFD;CCL5;CSTA;PRSS8;NEFH |
| PF-431396 | 7 | 438.0709 | 19 | GPX3;CCL5;S100A9;WIF1;ISG15;RAMP1;DKK3;BST2;MID1;CFD;S100A8;MT1F;F3;MT 1E;CSTA;TF;TSPAN8;SERPINI1;SLC4A4 |
| dasatinib | 15 | 434.0944 | 20 | CFD;BST2;S100A9;S100A8;CSTA;ISG15;GPX3;LST1;MZB1;TF;SLC4A4;MLPH;PRSS8;M ID 1;TSPAN8;NEFH;MT1F;MT1E;RAMP 1;SERPINI1 |
| sunitinib | 6 | 437.1429 | 10 | MLPH;DKK1;MT1E;MT1F;F3;ISG15;SLC4A4;CFD;CCL5;S100A9 |
| palbociclib | 4 | 458.2667 | 11 | BST2;TF;ISG15;MT1E;CSTA;S 100A9;S 100A8;TSPAN8;NEFH;GPX3;MT1F |
| PF-477736 | 5 | 436.7742 | 11 | SERPINI1;S100A9;S100A8;ISG15;SLC4A4;RAMP1;MT1E;BST2;GPX3;CFD;CSTA |
| JNK-9L | 11 | 420.6913 | 17 | CFD;GPX3;MT1F;DKK3;CSTA;CCL5;RAMP1;BST2;TF;ISG15;MT1E;MLPH;TSPAN8;DK K1;F3;WIF1;SLC4A4 |
| TGX-221 | 4 | 448.2 | 11 | ISG15;RAMP1;MT1E;CFD;CSTA;S100A9;S100A8;MLPH;MT1F;PRSS8;DKK3 |
| AS-601245 | 6 | 419.9365 | 14 | CSTA;TSPAN8;RAMP1;GPX3;CCL5;NEFH;S100A9;S100A8;CFD;WIF1;ISG15;MT1F;DK K3;MT1E |
| PD-0325901 | 18 | 407.521 | 19 | BST2;ISG15;SLC4A4;NEFH;CSTA;TSPAN8;CCL5;TF;GPX3;SERPINI1;S100A9;S 100A8; DKK3;MT1E;MID1;DKK1;PRSS8;CFD;WIF1 |
| A443654 | 13 | 405.7796 | 17 | TF;LST1;MT1F;MT1E;CFD;CSTA;S100A8;DKK3;TSPAN8;GPX3;ISG15;F3;SLC4A4;NEF H;WIF1;CCL5;S100A9 |
| AS-605240 | 4 | 422.0667 | 8 | CFD;CSTA;GPX3;ISG15;PRSS8;S100A9;BST2;S100A8 |
| canertinib | 6 | 395.9524 | 10 | CFD;BST2;ISG15;S100A8;CSTA;PRSS8;S100A9;WIF1;NEFH;TF |
| JW-7-24-1 | 4 | 408 | 14 | MT1F;S100A8;DKK3;MT1E;CFD;MLPH;ISG15;PRSS8;BCAS1;TSPAN8;CCL5;SLC4A4;C STA;S100A9 |
| linifanib | 5 | 388.9677 | 12 | CFD;CSTA;WIF1;S100A9;S100A8;MT1F;PRSS8;GPX3;NEFH;MT1E;ISG15;SLC4A4 |
| BI-2536 | 9 | 371.6536 | 19 | MLPH;TSPAN8;PRSS8;S100A8;BCAS1;MT1F;SERPINI1;RAMP1;MT1E;ISG15;GPX3;WI F1;NCAM1;DKK3;CCL5;NEFH;CFD;CSTA;S100A9 |
| withaferin-a | 7 | 367.937 | 16 | MZB1;S100A9;S100A8;DKK3;CFD;CSTA;TF;MT1F;MLPH;SERPINI1;SLC4A4;MT1E;GP X3;CCL5;BST2;ISG15 |
| GSK-1059615 | 6 | 370.4044 | 12 | WIF1;MT1F;MT1E;DKK1;TF;TSPAN8;CCL5;CSTA;RAMP1;GPX3;MID1;DKK3 |
| selumetinib | 4 | 370.4 | 10 | BST2;GPX3;ISG15;PRSS8;SLC4A4;WIF1;S100A9;S100A8;NEFH;CSTA |
| HG-5-113-01 | 4 | 362.4 | 12 | CCL5;NCAM1:ISG15;F3;MT1E;LST1;SERPINI1;S100A9;S100A8;TSPAN8;GPX3;NEFH |
| pracinostat | 5 | 346.6774 | 10 | CFD;BST2;CSTA;GPX3;ISG15;NCAM1;PRSS8;NEFH;CCL5;MID1 |
| dinaciclib | 4 | 357.6667 | 11 | CSTA;TF;MT1F;MT1E;CFD;GPX3;ISG15;BST2;CCL5;SERPINI1;DKK3 |
| LDN-193189 | 6 | 331.2381 | 17 | CCL5;NEFH;F3;RAMP1;MT1E;MT1F;NCAM1;TF;SERPINI1;SLC4A4;CFD;CSTA;S100A9 ;S100A8;BST2;MLPH;ISG15 |
| NVP-TAE684 | 2 | 434.3333 | 9 | CSTA;MLPH;TSPAN8;S 100A8;BST2;GPX3;CCL5;ISG15;NEFH |
| HG-5-88-01 | 2 | 433.6667 | 9 | CSTA;F3;DKK1;S100A9;S100A8;WIF1;MT1F;ISG115;PRSS8 |
| PD-184352 | 3 | 368.8571 | 8 | BST2;ISG15;S100A9;S100A8;CFD;CSTA;MID1;PRSS8 |
| CX-5461 | 4 | 337.1333 | 12 | GPX3;MT1F;ISG15;BST2;NEFH;F3;MT1E;CFD;MLPH;TSPAN8;PRSS8;DKK1 |
| TWS-119 | 2 | 403.6667 | 6 | CCL5;SLC4A4;MT1E;GPX3;SERPINI1;S100A8 |
| GSK-690693 | 3 | 336.5714 | 8 | CFD;DKK1;S 100A8;GPX3;ISG15;NEFH;TSPAN8;RAMP 1 |
| trametinib | 5 | 298.0323 | 13 | NCAM1;NEFH;S100A9;S100A8;BST2;CSTA;WIF1;CFD;TF;MT1F;GPX3;CCL5;SLC4A4 |
| gefitinib | 5 | 294.2258 | 11 | BST2;ISG15;RAMP 1;DKK3;CSTA;WIF 1;S 100A8;CFD;S 100A9;GPX3;PRSS8 |
| GSK-429286A | 2 | 360 | 4 | S100A9;S100A8;CSTA;TSPAN8 |
| PHA-665752 | 2 | 354.3333 | 9 | CSTA;TSPAN8;RAMP1;TF;MT1F;SERPINI1;F3;S100A8;MT1E |
| buparlisib | 2 | 349.6667 | 9 | CFD;GPX3;CCL5;DKK3;MLPH;WIF1;MT1F;DKK1;MT1E |
| tivantinib | 1 | 515 | 8 | TSPAN8;CCL5;MT1F;DKK1;S100A9;S100A8;DKK3;MT1E |
| JNK-IN-5A | 4 | 273.3333 | 7 | CCL5;MT1F;S100A9;MT1E;CFD;F3;DKK1 |
| geldanamycin | 2 | 340.6667 | 8 | CFD;BST2;ISG15;GPX3;GFRA1;NCAM1;S100A9;S100A8 |
| OSI-027 | 2 | 340 | 6 | TF;CCL5;DKK3;CSTA;S100A9;S100A8 |
| AZD-8330 | 4 | 271.7278 | 10 | TSPAN8;NEFH;GPX3;S 1 00A8;CFD;BST2;CSTA;ISG 15;PRSS8;S 100A9 |
| AGK-2 | 1 | 500 | 5 | TSP AN8;ISG 15;F3;S 100A9;S 100A8 |
| decitabine | 1 | 498 | 5 | BST2;GPX3;ISG15;F3;DKK1 |
| dovitinib | 2 | 331.3333 | 9 | CFD;MTI1F;ISG15;PRSS8;SLC4A4;CSTA;TF;WIF1;F3 |
| CYT387 | 3 | 283.5714 | 10 | CFD;CSTA;DKK1;GPX3;MT1F;ISG15;MT1E;BST2;S100A9;S100A8 |
| BX-912 | 2 | 328.6667 | 8 | BST2;TSPAN8;ISG15;NEFH;MT1F;S100A9;S100A8;MT1E |
| crizotinib | 2 | 325 | 7 | CFD;GPX3;ISG15;S100A8;TSPAN8;DKK1;S100A9 |
| I-BET | 2 | 313 | 8 | BST2;MT1F;MT1E;CFD;ISG15;SERPINI1;NEFH;DKK3 |
| radicicol | 4 | 249.4667 | 10 | CFD;BST2;CSTA;ISG15;GPX3;NEFH;S100A9;S100A8;DKK1;WIF1 |
| WYE-125132 | 2 | 307 | 6 | CSTA;GPX3;DKK1;CFD;TSPAN8;NEFH |
| LY2603618 | 1 | 459 | 5 | CSTA;CCL5;NEFH;S100A9;S100A8 |
| nilotinib | 2 | 298 | 7 | FCN1;CCL5;ISG15;S100A8;CSTA;MT1F;MT1E |
| KI20227 | 1 | 435 | 5 | CSTA;MT1F;NEFH;S100A9;MT1E |
| GSK-2126458 | 5 | 219.1613 | 10 | MT1F;ISG15;MT1E;CFD;MLPH;TSPAN8;GPX3;WIF1;PRSS8;BST2 |
| KIN001-043 | 2 | 275.6667 | 7 | BST2;TSPAN8;NEFH;MT1F;ISG15;F3;RAMP1 |
| C646 | 1 | 404 | 4 | CFD;MZB1;DKK1;S100A8 |
| pyrazolanthrone | 1 | 400 | 4 | BST2;MT1F;SLC4A4;MT1E |
| GDC-0879 | 1 | 395 | 5 | CFD;BST2;TF;GPX3;MID 1 |
| entinostat | 2 | 263 | 8 | CFD;MT1F;DKK1;MLPH;GPX3;ISG15;MID1;F3 |
| rocilinostat | 1 | 394 | 6 | CFD;BST2;CCL5;ISG15;PRSS8;MID1 |
| pelitinib | 2 | 256.3333 | 9 | BST2;MT1F;SLC4A4;MT1E;CFD;TF;CCL5;ISG15;PRSS8 |
| afatinib | 3 | 216.1429 | 10 | CFD;BST2;CSTA;PRSS8;S 100A9;ISG15;GPX3;MT1F;NEFH;S 100A8 |
| WH-4-025 | 2 | 244.6667 | 6 | TF;MT1F;S100A8;MT1E;CFD;ISG15 |
| THZ-2-98-01 | 1 | 361 | 4 | FCN1;ISG15;S100A9;S100A8 |
| KU-55933 | 1 | 351 | 4 | MT1F;NEFH;DKK1;MT1E |
| MK-1775 | 1 | 350 | 4 | BST2;ISG15;DKK1;MT1E |
| OTSSP167 | 2 | 233 | 7 | MT1F;ITGA8;DKK3;MT1E;CCL5;ISG15;SLC4A4 |
| GW-843682X | 2 | 227 | 6 | CFD;TSPAN8;DKK1;MT1F;F3;MT1E |
| (-)-JQ1 | 1 | 337 | 4 | TSPAN8;GPX3;SLC4A4;F3 |
| mocetinostat | 1 | 336 | 6 | CFD;BST2;TSPAN8;MT1F;NCAM1;ISG15 |
| XMD16-144 | 2 | 222.6667 | 7 | BST2;ISG15;S 100A9;S 100A8;CFD;SERPINI1;RAMP 1 |
| (+)-JQ1 | 1 | 332 | 4 | MT1F;ISG15;DKK3;MT1E |
| SB590885 | 2 | 221.3333 | 7 | CFD;BST2;GPX3;ISG15;DKK3;TF;NCAM1 |
| BYL719 | 1 | 319 | 4 | CFD;MLPH;MT1F;MT1E |
| YM-201636 | 1 | 318 | 5 | TF;GPX3;CCL5;MT1F;MT1E |
| XMD-1150 | 3 | 181 | 6 | CSTA;ISG15;MT1E;GPX3;MT1F;NCAM1 |
| CP466722 | 2 | 210.6667 | 7 | CFD;WIF1;ISG15;PRSS8;MT1F;SERPINI1;DKK1 |
| dabrafenib | 1 | 314 | 6 | TSPAN8;GPX3;MT1F;ISG15;PRSS8;SLC4A4 |
| foretinib | 1 | 303 | 4 | ISG15;SERPINI1;RAMP1;S100A8 |
| GDC-0941 | 3 | 170.1429 | 9 | CFD;CSTA;GPX3;CCL5;S100A9;S100A8;TSPAN8;ISG15;SLC4A4 |
| PIK-93 | 3 | 169.5714 | 9 | CFD;CSTA;MLPH;TF;MZB1;DKK3;CCL5;MT1F;MT1E |
| PFI-1 | 1 | 287 | 4 | TSPAN8;MID1;F3;DKK3 |
| sirolimus | 3 | 162 | 8 | TF;ISG15;MT1E;BST2;GPX3;CCL5;MT1F;DKK1 |
| XMD-1499 | 1 | 272 | 4 | TF;MLPH;S 100A9;S 100A8 |
| ON-01910 | 3 | 154.1429 | 8 | MT1F;F3;S100A8;MT1E;BST2;NCAM1;CCL5;NEFH |
| linsitinib | 1 | 267 | 4 | CCL5;MT1F;NEFH;MT1E |
| AKT-inhibitor-1-2 | 1 | 266 | 4 | TSPAN8;GPX3;S100A9;S100A8 |
| ruxolitinib | 2 | 176.3333 | 5 | MT1F;ISG15;MT1E;CSTA;MLPH |
| RAF_265 | 2 | 171.6667 | 8 | BST2;TSPAN8;ISG15;SLC4A4;TF;MLPH;MT1F;MT1E |
| olaparib | 1 | 255 | 4 | CSTA;TSPAN8;NCAM1;NEFH |
| GSK-461364 | 3 | 144.7143 | 9 | BST2;MLPH;ISG15;CSTA;TSPAN8;PRSS8;MT1F;DKK3;MT1E |
| erlotinib | 1 | 248 | 4 | CFD;BST2;CSTA;WIF1 |
| HY-11007 | 1 | 247 | 3 | MT1F;DKK3;MT1E |
| QL-XI-92 | 1 | 245 | 4 | CFD;CSTA;TF;SERPINI1 |
| AST1306 | 1 | 235 | 3 | TSPAN8;ISG15;NEFH |
| GDC-0980 | 3 | 132.5714 | 7 | MT1F;DKK1;MT1E;TF;RAMP1;ISG15;SLC4A4 |
| lapatinib | 2 | 153.6667 | 7 | CFD;ITGA8;S100A8;DKK3;CSTA;ISG15;S100A9 |
| PI-103 | 1 | 227 | 4 | CFD;GPX3;DKK1;DKK3 |
| vemurafenib | 2 | 143.3333 | 6 | MLPH;WIF1;CCL5;CFD;MT1F;ISG15 |
| SGI-1776 | 1 | 214 | 3 | BST2;MT1F;SLC4A4 |
| NPK76-II-72-1 | 1 | 211 | 4 | MT1F;S100A9;S100A8;MT1E |
| BIX-01294 | 1 | 209 | 3 | TSPAN8;DKK1;S100A9 |
| KIN236 | 1 | 207 | 3 | MZB1;S100A9;S100A8 |
| PLX-4720 | 1 | 201 | 5 | CFD;MLPH;GPX3;ISG15;S 100A8 |
| saracatinib | 1 | 196 | 4 | MT1F;NCAM1;ISG15;RAMP1 |
| torin-2 | 1 | 187 | 5 | CFD;GPX3;MT1F;PRSS8;DKK3 |
| KIN001-265 | 1 | 174 | 4 | CSTA;TF;CCL5;SLC4A4 |
| HG-14-10-04 | 2 | 108.6667 | 6 | CFD;MLPH;ISG15;MID1;SLC4A4;DKK3 |
| OSI-930 | 1 | 161 | 3 | CFD;MLPH;CCL5 |
| GSK-2334470 | 1 | 158 | 3 | TSPAN8;CCL5;F3 |
| methylstat | 1 | 152 | 4 | CFD;TSPAN8;CCL5;PRSS8 |
| resveratrol | 1 | 147 | 3 | NEFH;DKK1;RAMP1 |
| cabozantinib | 1 | 145 | 4 | BST2;MLPH;ISG15;SLC4A4 |
| AZD-6482 | 1 | 140 | 4 | MLPH;MT1F;RAMP1;MT1E |
| rondual-kinase- inhibitor | 1 | 136 | 3 | CSTA;MLPH;S100A9 |
| SB-525334 | 1 | 117 | 2 | CCL5;S100A8 |
| pazopanib | 1 | 115 | 3 | BST2;SLC4A4;DKK1 |
| AZD-8055 | 2 | 71.66667 | 6 | CFD;ISG15;F3;CSTA;DKK1;DKK3 |
| ZSTK-474 | 1 | 107 | 4 | CFD;GPX3;S 100A9;S 100A8 |
| AZ-628 | 1 | 95 | 5 | CFD;NCAM1;ISG15;NEFH;RAMP1 |
| QL-XII-47 | 3 | 53.85714 | 7 | MT1F;DKK1;S100A8;MT1E;F3;S100A9;RAMP1 |
| nintedanib | 1 | 83 | 3 | CSTA;S100A9;S100A8 |
| tozasertib | 1 | 80 | 3 | MLPH;GPX3;ISG15 |
| enzastaurin | 1 | 70 | 2 | TSPAN8;DKK1 |
| QL-X-138 | 1 | 58 | 3 | CFD;GPX3;F3 |
| GSK1059615 | 1 | 53 | 3 | BST2;MT1F;MT1E |
| everolimus | 1 | 44 | 3 | CFD;MLPH;DKK1 |
| NVP-AEW541 | 1 | 36 | 3 | TF;WIF1;ISG15 |
| forskolin | 1 | 20 | 3 | CCL5;F3;DKK3 |
| torin-1 | 1 | 18 | 4 | TF;MT1F;DKK3;MT1E |
| A66 | 1 | 9 | 3 | TSPAN8;DKK1;F3 |
| ponatinib | 1 | 4 | 3 | CFD;CSTA;TSPAN8 |

Four PDX models (MC-PRX-01, MC-PRX-03, MC-PRX-07, MC-PRX-08) from AA/P non-responders and one PDX model (MC-PRX-04) from AA/P responder were successfully established using the AA/P treatment naive metastatic biopsies (visit 1) from the PROMOTE patients. Differential expression analysis of the RNAseq data from AA/P non-responders and responder PDXs identified 377 upregulated and 763 downregulated genes in PDX from AA/P non-responders (FDR ≤ 0.05 and fold change ≥ 2) (Table 5). Volcano plots for differentially expressed genes (DEG) were presented in Figure 4A. The most significantly enriched pathways using the DEG were related to mitosis and G2/M checkpoints. Upregulated genes were then subjected to drug discovery analysis through the Enrichr portal. One hundred seventeen drugs with a mean of 4.0 and a median of 2 signatures per drug (Figure 3C, Table 6) were identified. No drug was identified using the down-regulated genes in PDX. Four drugs (palbociclib, mitoxantrone, PHA-793887 and PD-0325901) ranked top either by total number of signatures or by Rank Score, were overlapped with the top drugs identified using the patients' DE genes (Figure 3D, Table 4).

**Table 5. Differential expression genes from PROMOTE PDX models.**

| logFC | logCPM | LR | PValue | FDR | Gene_Name |
|---|---|---|---|---|---|
| 2.002071 | 5.360753 | 50.88438 | 9.80E-13 | 1.51E-08 | CBR4 |
| -5.57596 | 0.924567 | 47.97948 | 4.31E-12 | 3.33E-08 | ABLIM2 |
| 2.985655 | 5.108241 | 41.7563 | 1.03E-10 | 5.32E-07 | SLC15A2 |
| -6.4003 | 4.089833 | 39.82405 | 2.78E-10 | 1.07E-06 | CDCA7L |
| -4.07441 | 3.838753 | 36.29217 | 1.70E-09 | 4.23E-06 | PLP2 |
| 2.292276 | 3.337748 | 35.82853 | 2.15E-09 | 4.23E-06 | AP006222.2 |
| -4.93091 | 2.126479 | 35.6244 | 2.39E-09 | 4.23E-06 | XYLT1 |
| -3.62702 | 3.129904 | 35.60052 | 2.42E-09 | 4.23E-06 | RIOX1 |
| 3.479597 | 6.076746 | 35.56835 | 2.46E-09 | 4.23E-06 | PCAT1 |
| 3.977875 | 2.104089 | 34.32547 | 4.66E-09 | 7.20E-06 | ALPK2 |
| -8.50343 | 5.290736 | 34.08453 | 5.28E-09 | 7.41E-06 | PJA2 |
| -1.84286 | 4.923499 | 33.39249 | 7.53E-09 | 9.69E-06 | RALGPS1 |
| 2.222764 | 2.368661 | 33.05269 | 8.97E-09 | 1.07E-05 | BDH2 |
| -2.79494 | 1.670617 | 32.75705 | 1.04E-08 | 1. 15E-05 | SLC6A9 |
| 5.29571 | 7.531377 | 32.41319 | 1.25E-08 | 1.28E-05 | PSCA |
| -6.65741 | 1.998675 | 31.5883 | 1.91E-08 | 1.84E-05 | FOXL2NB |
| -2.35925 | 4.489245 | 30.72728 | 2.97E-08 | 2.70E-05 | NIPSNAP3A |
| 1.417545 | 4.527788 | 30.445 | 3.43E-08 | 2.95E-05 | CEP83 |
| -4.66915 | 2.155925 | 30.32903 | 3.65E-08 | 2.96E-05 | ST3GAL6 |
| -1.31674 | 6.658673 | 29.91583 | 4.51E-08 | 3.47E-05 | FLII |
| -4.90544 | 5.07293 | 29.82926 | 4.72E-08 | 3.47E-05 | FAM171A1 |
| -2.60292 | 3.444844 | 29.52529 | 5.52E-08 | 3.87E-05 | SNX7 |
| -1.73537 | 3.429306 | 28.91019 | 7.58E-08 | 5.09E-05 | MAPK7 |
| -10.9547 | 2.303238 | 28.73765 | 8.29E-08 | 5.33E-05 | C5orf63 |
| -3.5399 | 3.726017 | 28.62149 | 8.80E-08 | 5.44E-05 | NEXMIF |
| -3.37063 | 4.297511 | 28.53698 | 9.19E-08 | 5.46E-05 | EML1 |
| 1.674677 | 0.921569 | 27.89912 | 1.28E-07 | 7.10E-05 | AC067931.1 |
| -1.93402 | 5.228379 | 27.88421 | 1.29E-07 | 7.10E-05 | PIK3CB |
| -1.5645 | 4.344507 | 26.99948 | 2.04E-07 | 0.000108 | ABHD17C |
| 1.478961 | 8.711648 | 26.90201 | 2.14E-07 | 0.00011 | ELOVL5 |
| -1.88659 | 4.055647 | 26.61804 | 2.48E-07 | 0.000124 | ZDHHC13 |
| -1.68676 | 5.905507 | 26.45871 | 2.69E-07 | 0.00013 | SCP2 |
| -7.26838 | 0.372453 | 26.38461 | 2.80E-07 | 0.00013 | AC007342.4 |
| -2.64037 | 4.485834 | 26.3471 | 2.85E-07 | 0.00013 | TTC9 |
| -1.84254 | 4.419918 | 25.98591 | 3.44E-07 | 0.000152 | GPRC5C |
| -10.7076 | 2.06258 | 25.86434 | 3.66E-07 | 0.000156 | DCLK1 |
| -3.96389 | 0.927854 | 25.82736 | 3.73E-07 | 0.000156 | DUSP5 |
| 4.111409 | 3.053256 | 25.63509 | 4.12E-07 | 0.000164 | ACKR2 |
| -4.70205 | 3.783726 | 25.62655 | 4.14E-07 | 0.000164 | CLSTN2 |
| -2.58749 | 6.355091 | 25.14658 | 5.31E-07 | 0.000198 | SREBF1 |
| 4.302299 | 3.643366 | 25.11829 | 5.39E-07 | 0.000198 | DDIT4L |
| -4.05725 | 2.428047 | 25.07618 | 5.51E-07 | 0.000198 | PAK6 |
| -3.58455 | 4.072291 | 25.07612 | 5.51E-07 | 0.000198 | SH3KBP1 |
| -3.80566 | 1.676603 | 25.00053 | 5.73E-07 | 0.000201 | MSX1 |
| -6.9121 | 6.818851 | 24.83526 | 6.24E-07 | 0.000214 | CD44 |
| -3.71319 | 4.054593 | 24.51689 | 7.37E-07 | 0.000247 | PPM1L |
| -3.35668 | 3.444594 | 24.25582 | 8.44E-07 | 0.000277 | RTL5 |
| -7.8608 | 1.681119 | 24.05291 | 9.37E-07 | 0.000299 | FOXL2 |
| -1.42841 | 4.730971 | 24.02793 | 9.49E-07 | 0.000299 | TOP3A |
| -3.24521 | 4.142807 | 23.73789 | 1.10E-06 | 0.000341 | MICA |
| -1.18969 | 5.736064 | 23.65028 | 1.16E-06 | 0.00035 | SCAMP2 |
| -5.04935 | 1.78706 | 23.595 | 1.19E-06 | 0.000353 | AC108519.1 |
| -1.95818 | 6.24606 | 23.35739 | 1.35E-06 | 0.000382 | ALDH9A1 |
| -2.07442 | 4.281887 | 23.34198 | 1.36E-06 | 0.000382 | SOCS6 |
| 1.150195 | 6.026284 | 23.33739 | 1.36E-06 | 0.000382 | C7orf50 |
| -2.58568 | 6.177811 | 23.28663 | 1.40E-06 | 0.000383 | SPECC1 |
| -7.74142 | 3.200371 | 23.24704 | 1.42E-06 | 0.000383 | ECHDC3 |
| -3.33283 | 1.741093 | 23.22914 | 1.44E-06 | 0.000383 | KCNK5 |
| 2.517261 | 1.930147 | 23.11214 | 1.53E-06 | 0.0004 | MINDY4 |
| -7.65887 | 1.480066 | 23.03481 | 1.59E-06 | 0.00041 | FIGN |
| -7.60849 | 4.489428 | 22.87821 | 1.73E-06 | 0.000437 | CHD1 |
| 1.919776 | 1.55106 | 22.81392 | 1.78E-06 | 0.00044 | COA6-AS 1 |
| 3.511594 | 2.622531 | 22.80215 | 1.80E-06 | 0.00044 | TRIM54 |
| 2.086762 | 2.163335 | 22.72439 | 1.87E-06 | 0.000451 | AC004982.1 |
| 2.226208 | 1.187789 | 22.59449 | 2.00E-06 | 0.000471 | AC109326.1 |
| -4.14385 | 2.099909 | 22.55751 | 2.04E-06 | 0.000471 | B3GLCT |
| 2.390898 | 0.898779 | 22.55461 | 2.04E-06 | 0.000471 | LINC01979 |
| -2.43781 | -0.16465 | 22.50166 | 2.10E-06 | 0.000477 | RFLNB |
| 1.628582 | 0.30872 | 22.40365 | 2.21E-06 | 0.000495 | RPS3AP6 |
| 1.534698 | 8.982873 | 22.34834 | 2.27E-06 | 0.000502 | RPS3A |
| -2.4003 | 3.427215 | 22.32202 | 2.31E-06 | 0.000502 | RELL1 |
| 1.649623 | 3.023556 | 22.2242 | 2.43E-06 | 0.00052 | AC107068.1 |
| -2.09462 | 4.694309 | 22.12267 | 2.56E-06 | 0.000539 | ZFYVE9 |
| 1.385312 | 4.584088 | 22.10436 | 2.58E-06 | 0.000539 | AC083843.3 |
| 1.587172 | 2.373598 | 21.9815 | 2.75E-06 | 0.000565 | AP002387.2 |
| 2.323616 | 1.445328 | 21.95773 | 2.79E-06 | 0.000565 | AC011337.1 |
| 1.803808 | 3.135094 | 21.93418 | 2.82E-06 | 0.000565 | AC084198.2 |
| -7.53928 | 3.346191 | 21.91325 | 2.85E-06 | 0.000565 | MAN2A1 |
| -5.88488 | 0.220497 | 21.83943 | 2.96E-06 | 0.00058 | FUOM |
| 2.112113 | 0.409178 | 21.79721 | 3.03E-06 | 0.000585 | AC239798.2 |
| -1.73359 | 5.042723 | 21.72589 | 3.15E-06 | 0.0006 | CCDC149 |
| 4.503962 | 4.447025 | 21.67378 | 3.23E-06 | 0.000605 | PTGS2 |
| -6.40062 | 0.229365 | 21.66242 | 3.25E-06 | 0.000605 | AC007342.5 |
| 1.377848 | 1.90803 | 21.60882 | 3.34E-06 | 0.000615 | FAT2 |
| -2.7552 | 3.985678 | 21.54912 | 3.45E-06 | 0.000627 | ANKRD9 |
| 2.037365 | 1.253521 | 21.47493 | 3.58E-06 | 0.000644 | AC009318.1 |
| -2.10314 | 0.241795 | 21.45109 | 3.63E-06 | 0.000644 | LY6G5C |
| -3.46741 | 0.519824 | 21.41015 | 3.71E-06 | 0.000651 | PLXNA4 |
| 2.036435 | 5.24722 | 21.35225 | 3.82E-06 | 0.000658 | MUC20 |
| 1.538801 | 0.718679 | 21.3456 | 3.84E-06 | 0.000658 | RPS3AP47 |
| -6.62631 | 1.84235 | 21.25926 | 4.01E-06 | 0.000681 | PRSS16 |
| -3.61322 | 2.810181 | 21.17863 | 4.18E-06 | 0.000694 | RPP25 |
| 2.352444 | 1.993644 | 21.17254 | 4.20E-06 | 0.000694 | RP11-423O2.5 |
| -5.2214 | 4.598568 | 21.16059 | 4.22E-06 | 0.000694 | PTPRN2 |
| -7.45574 | 0.568674 | 21.13411 | 4.28E-06 | 0.000696 | PABPC1L2B |
| -3.217 | 1.510916 | 21.08674 | 4.39E-06 | 0.000706 | EVX1 |
| -6.60334 | 2.451362 | 21.06297 | 4.44E-06 | 0.000708 | FBXL7 |
| 1.480703 | 3.482367 | 21.04232 | 4.49E-06 | 0.000708 | ZNF34 |
| -2.12372 | 5.939336 | 20.93976 | 4.74E-06 | 0.000734 | FGFRL1 |
| 2.179076 | 1.583095 | 20.9347 | 4.75E-06 | 0.000734 | EAF2 |
| 3.539767 | 1.839837 | 20.82782 | 5.02E-06 | 0.000747 | PGM5 |
| -5.86154 | 2.248957 | 20.82407 | 5.03E-06 | 0.000747 | GPC6 |
| -2.06712 | 4.498254 | 20.81988 | 5.05E-06 | 0.000747 | CTBP1-DT |
| -7.86292 | 2.502469 | 20.80941 | 5.07E-06 | 0.000747 | FER |
| -1.18909 | 4.763599 | 20.80889 | 5.07E-06 | 0.000747 | TMEM 11 |
| -5.34496 | 2.372494 | 20.77467 | 5.17E-06 | 0.000753 | STARD4-AS1 |
| 1.473034 | 4.410726 | 20.70831 | 5.35E-06 | 0.00077 | RPS3AP26 |
| 2.241328 | 3.265197 | 20.69487 | 5.39E-06 | 0.00077 | MUC20P1 |
| -4.68033 | 3.652875 | 20.57558 | 5.73E-06 | 0.000809 | ZFHX4 |
| 1.255051 | 7.137284 | 20.56278 | 5.77E-06 | 0.000809 | C8orf33 |
| -1.16409 | 7.708203 | 20.54864 | 5.81E-06 | 0.000809 | NET1 |
| -1.67935 | 5.967689 | 20.51291 | 5.92E-06 | 0.000817 | EFCAB14 |
| -6.11235 | 2.933494 | 20.47982 | 6.03E-06 | 0.000824 | NREP |
| -1.88076 | 3.319947 | 20.40233 | 6.28E-06 | 0.00085 | PDSS2 |
| -1.68552 | 6.213794 | 20.35031 | 6.45E-06 | 0.000866 | KPNA4 |
| 1.639137 | 6.49905 | 20.16265 | 7.11E-06 | 0.000947 | RSRP1 |
| -1.86652 | 5.534709 | 20.12468 | 7.26E-06 | 0.000958 | FBXL5 |
| -1.53803 | 4.620014 | 20.04863 | 7.55E-06 | 0.000988 | FAM57A |
| -6.52335 | 5.479502 | 19.99226 | 7.78E-06 | 0.001003 | HDAC9 |
| -8.57191 | 1.574919 | 19.9884 | 7.79E-06 | 0.001003 | FAM50B |
| -2.54504 | 4.281672 | 19.90071 | 8.16E-06 | 0.001041 | DNAJC 1 |
| -6.75583 | 2.389489 | 19.84724 | 8.39E-06 | 0.001062 | RGMB |
| -1.98829 | 4.445428 | 19.74859 | 8.83E-06 | 0.001109 | RSRC1 |
| -7.4478 | 0.554177 | 19.6691 | 9.21E-06 | 0.001147 | AC091179.1 |
| -1.24933 | 3.876937 | 19.60004 | 9.55E-06 | 0.001175 | ZNF93 |
| 2.230667 | 4.047201 | 19.59191 | 9.59E-06 | 0.001175 | GLIPR2 |
| 1.425777 | 0.657396 | 19.52728 | 9.92E-06 | 0.001206 | AC104791.2 |
| -5.70716 | 1.249931 | 19.4453 | 1.04E-05 | 0.001249 | AC005829.2 |
| -1.89461 | 5.814148 | 19.378 | 1.07E-05 | 0.001284 | CD24P4 |
| -1.10069 | 5.622267 | 19.26507 | 1. 14E-05 | 0.001352 | PATL1 |
| -1.92264 | 2.869088 | 19.21895 | 1.17E-05 | 0.001367 | DERA |
| 1.502167 | 1.297176 | 19.21395 | 1.17E-05 | 0.001367 | AC010468.1 |
| -1.70452 | 5.702947 | 19.13621 | 1.22E-05 | 0.001414 | KEAP1 |
| -1.54685 | 7.138898 | 19.11258 | 1.23E-05 | 0.00142 | NSD2 |
| -2.19505 | 9.337631 | 18.94226 | 1.35E-05 | 0.001542 | GOLM1 |
| -5.20361 | 0.301206 | 18.91636 | 1.37E-05 | 0.001544 | PCDH18 |
| 2.00208 | 2.677327 | 18.91089 | 1.37E-05 | 0.001544 | ZNF252P-AS 1 |
| 2.698023 | 1.769992 | 18.88896 | 1.39E-05 | 0.001551 | MAP1A |
| -8.11193 | 1.922105 | 18.83568 | 1.42E-05 | 0.001583 | RGMB-AS1 |
| -4.52037 | 3.779835 | 18.80502 | 1.45E-05 | 0.001597 | DTNA |
| -5.94125 | 1.66393 | 18.72863 | 1.51E-05 | 0.001651 | HFE |
| -3.49764 | 1.503708 | 18.70783 | 1.52E-05 | 0.001657 | KRTAP5-1 |
| -4.878 | 2.131226 | 18.69006 | 1.54E-05 | 0.001661 | MAPK8IP1P2 |
| -5.95236 | 2.567526 | 18.58509 | 1.62E-05 | 0.001743 | SPART |
| -2.44252 | 2.747783 | 18.50293 | 1.70E-05 | 0.001802 | SATB2 |
| -2.42163 | 3.743041 | 18.46563 | 1.73E-05 | 0.001802 | SLC1A4 |
| 1.155329 | 5.365207 | 18.46061 | 1.73E-05 | 0.001802 | RPS6KC1 |
| 1.362857 | 5.63089 | 18.45131 | 1.74E-05 | 0.001802 | MEX3A |
| -6.77059 | 4.756164 | 18.44439 | 1.75E-05 | 0.001802 | CNN3 |
| 1.687882 | 5.679565 | 18.444 | 1.75E-05 | 0.001802 | PTP4A3 |
| 3.072173 | 7.96157 | 18.37259 | 1.82E-05 | 0.001858 | AZGP1 |
| -1.4765 | 4.868174 | 18.35126 | 1.84E-05 | 0.001867 | KDSR |
| 2.347814 | 1.844049 | 18.27228 | 1.91E-05 | 0.001925 | MRPS18AP1 |
| -6.10715 | 1.674426 | 18.26443 | 1.92E-05 | 0.001925 | MSRA |
| -4.69687 | 3.65473 | 18.22871 | 1.96E-05 | 0.001925 | CAMK1D |
| 1.52382 | 2.310723 | 18.22448 | 1.96E-05 | 0.001925 | AL021707.2 |
| -3.29057 | 4.597682 | 18.22262 | 1.97E-05 | 0.001925 | LRRK1 |
| -5.48292 | 3.048819 | 18.2108 | 1.98E-05 | 0.001925 | LINC00649 |
| -2.02959 | 3.265147 | 18.17834 | 2.01E-05 | 0.001925 | DCBLD1 |
| -1.17769 | 4.451956 | 18.17602 | 2.01E-05 | 0.001925 | KIAA0753 |
| -4.16256 | 3.11102 | 18.17512 | 2.01E-05 | 0.001925 | ARAP2 |
| -3.49226 | 7.020733 | 18.17141 | 2.02E-05 | 0.001925 | SLC12A2 |
| 1.182929 | 5.002768 | 18.14024 | 2.05E-05 | 0.001945 | PIK3R4 |
| 1.622097 | 1.227981 | 18.10955 | 2.09E-05 | 0.001964 | AC020898.1 |
| 2.469079 | 1.389296 | 18.05333 | 2.15E-05 | 0.001994 | LMO7-AS1 |
| -1.79867 | 5.427091 | 18.04824 | 2.15E-05 | 0.001994 | VPS4B |
| 1.181172 | 4.371499 | 18.04305 | 2.16E-05 | 0.001994 | AASS |
| -6.90668 | 2.063839 | 18.03463 | 2.17E-05 | 0.001994 | SLIT2 |
| -1.03005 | 6.289575 | 18.01439 | 2.19E-05 | 0.002004 | ZBTB4 |
| 1.963635 | 6.797109 | 17.99786 | 2.21E-05 | 0.002009 | GABPB1-AS1 |
| -2.20558 | 0.553137 | 17.96459 | 2.25E-05 | 0.002019 | ID2-AS1 |
| -4.48116 | 0.211127 | 17.95215 | 2.27E-05 | 0.002019 | PCDHGA3 |
| 1.726934 | 2.799994 | 17.94449 | 2.27E-05 | 0.002019 | CLN3 |
| -1.58969 | 3.982006 | 17.92175 | 2.30E-05 | 0.002019 | RAVER1 |
| -8.44566 | 3.031304 | 17.91707 | 2.31E-05 | 0.002019 | LINC01748 |
| -1.79464 | 3.533401 | 17.91638 | 2.31E-05 | 0.002019 | GK |
| -1.17542 | 4.373693 | 17.90291 | 2.32E-05 | 0.002019 | LLGL1 |
| -1.91533 | 2.945797 | 17.90164 | 2.33E-05 | 0.002019 | PKN3 |
| -1.67646 | 5.762201 | 17.82454 | 2.42E-05 | 0.002087 | LAP3 |
| -2.95364 | 0.394433 | 17.81651 | 2.43E-05 | 0.002087 | AL450263.1 |
| -1.83914 | 6.194718 | 17.77765 | 2.48E-05 | 0.002105 | KPNA2 |
| -3.55246 | 2.432094 | 17.77577 | 2.49E-05 | 0.002105 | CCNJ |
| -1.71448 | 3.166493 | 17.76937 | 2.49E-05 | 0.002105 | MC1R |
| 1.012283 | 7.276454 | 17.75062 | 2.52E-05 | 0.002106 | RBM5 |
| -2.50422 | 4.522207 | 17.74766 | 2.52E-05 | 0.002106 | TRIM68 |
| 2.984006 | 2.348033 | 17.66743 | 2.63E-05 | 0.002185 | H19 |
| -1.0738 | 5.232225 | 17.65402 | 2.65E-05 | 0.002189 | PCMT1 |
| -3.13535 | 1.776403 | 17.64221 | 2.67E-05 | 0.00219 | GPR37 |
| -1.75646 | 5.115056 | 17.56432 | 2.78E-05 | 0.00227 | KANK2 |
| -3.28763 | 3.132039 | 17.5239 | 2.84E-05 | 0.002306 | P2RY1 |
| 1.037392 | 4.491509 | 17.50709 | 2.86E-05 | 0.002315 | COMMD6 |
| 1.539445 | 0.376589 | 17.48514 | 2.90E-05 | 0.002329 | AC133552.5 |
| 3.789552 | 4.465837 | 17.47332 | 2.91E-05 | 0.002332 | APOBR |
| 1.375997 | 0.483362 | 17.4628 | 2.93E-05 | 0.002333 | MORC2-AS1 |
| -3.48761 | 3.718431 | 17.45223 | 2.95E-05 | 0.002334 | RNF152 |
| 1.704858 | 6.845048 | 17.40034 | 3.03E-05 | 0.002386 | NUDT16 |
| 2.011277 | 2.060868 | 17.38006 | 3.06E-05 | 0.00239 | EXTL3-AS1 |
| -3.81606 | 1.438715 | 17.37792 | 3.06E-05 | 0.00239 | STOX1 |
| -2.34767 | 4.309307 | 17.35296 | 3.10E-05 | 0.002408 | AFAP1 |
| -1.49952 | 4.449197 | 17.34047 | 3.12E-05 | 0.002408 | TAPT1 |
| -2.68517 | 2.366634 | 17.33475 | 3.13E-05 | 0.002408 | CTSK |
| -2.19676 | 0.052 | 17.30607 | 3.18E-05 | 0.002433 | CLIC2 |
| -1.85572 | 4.351019 | 17.2358 | 3.30E-05 | 0.002499 | MEX3D |
| -1.44587 | 3.707402 | 17.23132 | 3.31E-05 | 0.002499 | RRAS2 |
| -1.61775 | 6.542036 | 17.22674 | 3.32E-05 | 0.002499 | DSC2 |
| 1.463504 | 3.4786 | 17.13604 | 3.48E-05 | 0.002609 | CLN5 |
| -3.5403 | 3.35766 | 17.07003 | 3.60E-05 | 0.002672 | PTPRG |
| -3.08837 | 8.376225 | 17.05968 | 3.62E-05 | 0.002672 | SAT1 |
| -2.28671 | 1.921047 | 17.05452 | 3.63E-05 | 0.002672 | KCNK6 |
| 1.905976 | 1.821986 | 17.03666 | 3.67E-05 | 0.002684 | TGM1 |
| -1.04977 | 6.592135 | 17.01226 | 3.71E-05 | 0.002696 | GALNT1 |
| -1.33241 | 6.739899 | 17.01016 | 3.72E-05 | 0.002696 | COPB1 |
| -1.40823 | 5.674433 | 16.99589 | 3.75E-05 | 0.002704 | SMC2 |
| -4.95293 | 3.630018 | 16.95967 | 3.82E-05 | 0.002743 | IRX2 |
| -6.1401 | 5.435997 | 16.94093 | 3.86E-05 | 0.002757 | HMGCS2 |
| -1.51057 | 4.680607 | 16.91537 | 3.91E-05 | 0.002782 | ZCCHC24 |
| 1.352858 | 1.306755 | 16.89265 | 3.96E-05 | 0.002803 | TRIM73 |
| -2.36972 | 3.543319 | 16.8757 | 3.99E-05 | 0.002815 | HIVEP3 |
| 1.218787 | 3.202032 | 16.84145 | 4.06E-05 | 0.002853 | LINC01347 |
| -1.11462 | 6.498918 | 16.80169 | 4.15E-05 | 0.002897 | SLC44A2 |
| 1.23091 | 2.724829 | 16.79543 | 4.16E-05 | 0.002897 | SMIM8 |
| 1.657164 | 1.221185 | 16.69073 | 4.40E-05 | 0.003047 | TTC23L |
| -3.04739 | 4.905259 | 16.67941 | 4.43E-05 | 0.003052 | CDH3 |
| -1.63168 | 4.064299 | 16.65457 | 4.48E-05 | 0.003078 | DLGAP5 |
| 1.708985 | 0.745072 | 16.6411 | 4.52E-05 | 0.003087 | SNHG25 |
| -1.48089 | 5.053981 | 16.63262 | 4.54E-05 | 0.003087 | EHBP1L1 |
| -1.39008 | 5.799096 | 16.61471 | 4.58E-05 | 0.003092 | FANCI |
| -1.70746 | 2.170192 | 16.61288 | 4.58E-05 | 0.003092 | ZNF653 |
| -1.89775 | 2.572361 | 16.58248 | 4.66E-05 | 0.003128 | AKIP1 |
| 2.670802 | 3.919295 | 16.56903 | 4.69E-05 | 0.003137 | PRNCR1 |
| 1.665506 | 0.698995 | 16.5106 | 4.84E-05 | 0.003221 | AL031281.2 |
| -4.73216 | 1.781021 | 16.46814 | 4.95E-05 | 0.00326 | MCOLN3 |
| -4.59717 | 3.533978 | 16.46498 | 4.96E-05 | 0.00326 | DPY19L1 |
| -1.39074 | 5.559701 | 16.46357 | 4.96E-05 | 0.00326 | C9orf152 |
| -2.34516 | 6.694064 | 16.42802 | 5.05E-05 | 0.003295 | ARHGAP21 |
| -2.08597 | 2.75472 | 16.41959 | 5.08E-05 | 0.003295 | PPIC |
| -1.33508 | 2.945762 | 16.41924 | 5.08E-05 | 0.003295 | PRC1 |
| -1.67851 | 4.78499 | 16.40675 | 5.11E-05 | 0.003303 | ANXA3 |
| 1.207389 | 2.654948 | 16.3864 | 5.17E-05 | 0.003324 | CCDC110 |
| -1.84074 | 4.70995 | 16.33188 | 5.32E-05 | 0.003391 | TRIM3 |
| -1.55094 | 8.889958 | 16.32589 | 5.33E-05 | 0.003391 | SCD |
| -5.67925 | 2.434914 | 16.32508 | 5.34E-05 | 0.003391 | MRAS |
| -2.80163 | 3.707802 | 16.30972 | 5.38E-05 | 0.003391 | SEPT6 |
| -2.67283 | 7.006345 | 16.25288 | 5.54E-05 | 0.00348 | CREB3L1 |
| -4.81957 | 3.325446 | 16.21936 | 5.64E-05 | 0.003525 | KHDRBS3 |
| -1.90511 | 5.886004 | 16.21339 | 5.66E-05 | 0.003525 | SMC4 |
| 1.21333 | 4.667822 | 16.08352 | 6.06E-05 | 0.003737 | FBXW7 |
| 1.123257 | 6.634444 | 16.08343 | 6.06E-05 | 0.003737 | SECISBP2L |
| -5.04267 | 1.755037 | 16.07993 | 6.07E-05 | 0.003737 | TMPRSS13 |
| -3.9479 | 3.658679 | 16.06473 | 6.12E-05 | 0.003748 | MBNL3 |
| -2.62487 | 5.276671 | 16.0595 | 6.14E-05 | 0.003748 | PARP8 |
| -1.2563 | 5.41608 | 16.03547 | 6.22E-05 | 0.00378 | TMEM109 |
| -3.43067 | 2.936781 | 16.01667 | 6.28E-05 | 0.003803 | PAM |
| -5.35498 | 0.035655 | 15.98096 | 6.40E-05 | 0.00386 | AC108690.1 |
| -1.65763 | 1.882829 | 15.93066 | 6.57E-05 | 0.003949 | HASPIN |
| -1.18439 | 3.129478 | 15.91717 | 6.62E-05 | 0.003962 | AC004706.3 |
| -3.76833 | 1.115833 | 15.89391 | 6.70E-05 | 0.003995 | MARCH3 |
| 1.210353 | 1.354108 | 15.84742 | 6.87E-05 | 0.00406 | CKLF |
| -2.00261 | 1.480371 | 15.84176 | 6.89E-05 | 0.00406 | GPR143 |
| 1.037249 | 5.199563 | 15.82627 | 6.94E-05 | 0.004067 | ZNF827 |
| 1.622815 | 0.792 | 15.82412 | 6.95E-05 | 0.004067 | AC010504.1 |
| -5.15599 | 1.924753 | 15.81409 | 6.99E-05 | 0.004073 | AC106791.1 |
| -4.59283 | 2.098901 | 15.80392 | 7.03E-05 | 0.00408 | LINC00847 |
| -1.6404 | 7.450548 | 15.79596 | 7.06E-05 | 0.004082 | LMAN1 |
| -1.95192 | 5.672608 | 15.76794 | 7.16E-05 | 0.004127 | CHPT1 |
| -1.42237 | 4.427558 | 15.69535 | 7.44E-05 | 0.004272 | NDC1 |
| -2.80073 | 1.711161 | 15.68106 | 7.50E-05 | 0.004289 | USP32P3 |
| -1.2501 | 4.768281 | 15.66927 | 7.54E-05 | 0.0043 | DNASE2 |
| 2.647378 | 2.016422 | 15.6538 | 7.61E-05 | 0.004319 | RPGRIP1 |
| 1.263994 | 0.351119 | 15.63123 | 7.70E-05 | 0.004355 | AC002558.3 |
| -5.8113 | 2.333713 | 15.6204 | 7.74E-05 | 0.004364 | TTC22 |
| 1.360901 | 4.395155 | 15.59438 | 7.85E-05 | 0.004408 | CCDC58 |
| -1.39453 | 2.970802 | 15.5853 | 7.89E-05 | 0.004408 | RTTN |
| 1.136128 | 6.024499 | 15.57168 | 7.94E-05 | 0.004414 | ABCE1 |
| -1.28321 | 6.387858 | 15.5348 | 8.10E-05 | 0.00447 | ARHGEF11 |
| 3.229576 | 2.194458 | 15.53409 | 8.10E-05 | 0.00447 | SYNPO2 |
| -5.33607 | 2.803779 | 15.52334 | 8.15E-05 | 0.00448 | PLPP2 |
| -2.00935 | 3.749733 | 15.49247 | 8.28E-05 | 0.004537 | DUSP23 |
| -2.21113 | -0.09372 | 15.4728 | 8.37E-05 | 0.004553 | AC004233.2 |
| -3.1506 | 2.111555 | 15.46991 | 8.38E-05 | 0.004553 | C3orf67 |
| -1.86947 | 5.433679 | 15.45296 | 8.46E-05 | 0.004553 | GFM1 |
| -3.27507 | 4.689508 | 15.44926 | 8.48E-05 | 0.004553 | CPAMD8 |
| -2.94145 | 1.302563 | 15.44923 | 8.48E-05 | 0.004553 | DNAJC6 |
| 1.631434 | 1.771251 | 15.44621 | 8.49E-05 | 0.004553 | RP11-782C8.1 |
| -1.90426 | 0.423396 | 15.43197 | 8.55E-05 | 0.004571 | FAM57B |
| -2.43865 | 2.637911 | 15.38544 | 8.77E-05 | 0.004669 | DOK3 |
| -1.93667 | 4.89651 | 15.35003 | 8.93E-05 | 0.004741 | ATP9B |
| 1.622375 | 7.907127 | 15.30683 | 9.14E-05 | 0.004834 | ATP2C1 |
| -1.4281 | 5.720608 | 15.28592 | 9.24E-05 | 0.004871 | SLBP |
| 1.777086 | 3.745051 | 15.26749 | 9.33E-05 | 0.004889 | MHENCR |
| 1.77393 | 0.93518 | 15.26604 | 9.34E-05 | 0.004889 | AL355377.4 |
| -4.32751 | 6.753095 | 15.2483 | 9.43E-05 | 0.004909 | ST6GAL 1 |
| 1.047059 | 6.390322 | 15.24556 | 9.44E-05 | 0.004909 | ZNF7 |
| -8.2658 | 2.065 | 15.23129 | 9.51E-05 | 0.00493 | SH3GL2 |
| 1.33064 | 3.990583 | 15.2207 | 9.56E-05 | 0.004937 | ARHGAP10 |
| -1.52843 | 5.905948 | 15.21514 | 9.59E-05 | 0.004937 | RNF4 |
| -1.45446 | 4.058653 | 15.20986 | 9.62E-05 | 0.004937 | PPP1R3B |
| -4.11281 | 2.456233 | 15.19574 | 9.69E-05 | 0.004957 | ZNF559 |
| -1.43965 | 7.255209 | 15.18047 | 9.77E-05 | 0.004981 | CD164 |
| 1.346384 | 6.106902 | 15.15928 | 9.88E-05 | 0.00501 | PABPC1L |
| -3.12356 | 5.562201 | 15.1514 | 9.92E-05 | 0.00501 | LDLR |
| 1.352739 | 7.187487 | 15.1507 | 9.93E-05 | 0.00501 | ARGLU1 |
| -1.78952 | 7.022219 | 15.11018 | 0.000101 | 0.005102 | SYNE2 |
| -1.1419 | 4.916436 | 15.0989 | 0.000102 | 0.005116 | GEMIN4 |
| -1.51255 | 3.219438 | 15.08656 | 0.000103 | 0.005133 | MTRF1L |
| 2.165293 | 1.434835 | 15.05575 | 0.000104 | 0.005173 | SLC16A6P1 |
| -1.84498 | 1.326708 | 15.0539 | 0.000104 | 0.005173 | ULK4 |
| -1.48367 | 4.699521 | 15.03879 | 0.000105 | 0.005198 | EFL1 |
| -2.34568 | 0.769825 | 15.00023 | 0.000107 | 0.005284 | AF001548.2 |
| -1.16734 | 7.382264 | 14.99581 | 0.000108 | 0.005284 | IQGAP1 |
| -2.49812 | 2.250361 | 14.98188 | 0.000109 | 0.005306 | PCDHGB2 |
| -2.25704 | 4.930305 | 14.96631 | 0.000109 | 0.005318 | TLE1 |
| -2.1965 | 5.597293 | 14.96561 | 0.000109 | 0.005318 | GALNT10 |
| -3.96682 | 3.357493 | 14.9543 | 0.00011 | 0.005333 | ENPP1 |
| 1.108697 | 7.317529 | 14.9343 | 0.000111 | 0.00537 | HP1BP3 |
| -1.99237 | 1.775789 | 14.92948 | 0.000112 | 0.00537 | STRIP2 |
| -4.61221 | 4.034129 | 14.88139 | 0.000114 | 0.005492 | NEFH |
| -1.04074 | 5.698027 | 14.87067 | 0.000115 | 0.005506 | STIM1 |
| -1.65969 | 4.784799 | 14.84438 | 0.000117 | 0.005566 | ZNF236 |
| -1.51686 | 3.868788 | 14.83558 | 0.000117 | 0.005575 | DHRS7B |
| -1.16428 | 5.044155 | 14.80302 | 0.000119 | 0.005655 | COPS3 |
| -4.90528 | 2.928963 | 14.77566 | 0.000121 | 0.00572 | DCBLD2 |
| 1.804778 | 7.675614 | 14.76494 | 0.000122 | 0.005732 | ZNF589 |
| -1.62503 | 5.706161 | 14.76017 | 0.000122 | 0.005732 | MAN2B2 |
| -2.77293 | 0.673534 | 14.71393 | 0.000125 | 0.005856 | RIN3 |
| -1.1632 | 5.785271 | 14.68803 | 0.000127 | 0.005919 | TIMELESS |
| -1.62529 | 2.621686 | 14.68023 | 0.000127 | 0.005926 | AP001062.1 |
| 1.198249 | 8.321701 | 14.66823 | 0.000128 | 0.005946 | LUC7L3 |
| -1.55704 | 5.650764 | 14.65732 | 0.000129 | 0.005963 | ECT2 |
| -4.13231 | 2.967053 | 14.62639 | 0.000131 | 0.006025 | FAM221A |
| 1.354135 | 3.965933 | 14.61732 | 0.000132 | 0.006036 | NABP1 |
| 1.373847 | 1.995722 | 14.59458 | 0.000133 | 0.006091 | FMC1 |
| 1.418494 | 5.402291 | 14.53223 | 0.000138 | 0.006278 | DENND3 |
| -1.02893 | 6.245883 | 14.51729 | 0.000139 | 0.006309 | DLST |
| -1.43403 | 5.628219 | 14.50745 | 0.00014 | 0.006324 | MAN2B 1 |
| 1.952537 | 3.482429 | 14.48209 | 0.000141 | 0.006391 | BANK1 |
| -1.53093 | 3.85826 | 14.45711 | 0.000143 | 0.006457 | CZIB |
| -1.85439 | 2.927482 | 14.44203 | 0.000145 | 0.00649 | AC139795.2 |
| -1.20908 | 4.527284 | 14.42153 | 0.000146 | 0.006509 | CCNB2 |
| -2.96404 | 5.214378 | 14.42131 | 0.000146 | 0.006509 | NHSL1 |
| -3.01381 | 6.334802 | 14.42027 | 0.000146 | 0.006509 | EMB |
| 1.391009 | 3.818129 | 14.41276 | 0.000147 | 0.006516 | LINC01355 |
| 2.704239 | 1.020213 | 14.35427 | 0.000151 | 0.006702 | FAM225B |
| -1.34859 | 5.513242 | 14.33423 | 0.000153 | 0.006755 | SMCR8 |
| -1.44441 | 4.816006 | 14.32159 | 0.000154 | 0.006781 | PDCD10 |
| -4.08082 | 1.792628 | 14.28954 | 0.000157 | 0.006878 | TSPY26P |
| 1.55378 | 3.081088 | 14.25165 | 0.00016 | 0.006998 | MINCR |
| -4.5435 | 1.96526 | 14.22612 | 0.000162 | 0.007053 | STARD4 |
| -4.08658 | 1.095529 | 14.21983 | 0.000163 | 0.007057 | AC005064.1 |
| 1.283694 | 4.791247 | 14.19215 | 0.000165 | 0.007123 | FBXL3 |
| -1.90086 | 4.753741 | 14.19133 | 0.000165 | 0.007123 | PIGN |
| 1.565413 | 0.566548 | 14.18655 | 0.000166 | 0.007123 | RP11-782C8.5 |
| 2.006382 | 5.378217 | 14.15566 | 0.000168 | 0.007221 | KLKP 1 |
| -1.34559 | 4.494877 | 14.14549 | 0.000169 | 0.00723 | SLC29A1 |
| -4.00707 | 2.59052 | 14.14283 | 0.000169 | 0.00723 | MS4A8 |
| -1.55016 | 1.240712 | 14.13366 | 0.00017 | 0.007245 | AQP4 |
| -1.82314 | 1.505566 | 14.12147 | 0.000171 | 0.007252 | AC092944.1 |
| -1.39787 | 4.940848 | 14.09397 | 0.000174 | 0.007319 | MFSD1 |
| 1.224509 | 2.360136 | 14.03579 | 0.000179 | 0.007528 | MIR4453HG |
| -4.20113 | 5.072584 | 14.01961 | 0.000181 | 0.007572 | ADCY2 |
| -1.21053 | 4.403689 | 14.01473 | 0.000181 | 0.007572 | AP5M1 |
| -1.70832 | 6.96366 | 13.99953 | 0.000183 | 0.007613 | IGF2R |
| -1.57496 | 4.961734 | 13.9793 | 0.000185 | 0.007675 | UEVLD |
| -2.09393 | 4.391187 | 13.96951 | 0.000186 | 0.007694 | ZFP3 |
| -3.48387 | 2.693425 | 13.95995 | 0.000187 | 0.007713 | HEIH |
| -4.33846 | 4.4739 | 13.94102 | 0.000189 | 0.00777 | SATB1 |
| -1.22005 | 5.333589 | 13.93034 | 0.00019 | 0.007793 | CDK1 |
| -1.46053 | 3.831086 | 13.91948 | 0.000191 | 0.007818 | PXYLP 1 |
| 1.439656 | 1.874131 | 13.88508 | 0.000194 | 0.007907 | KLHL31 |
| 1.548002 | 1.125848 | 13.885 | 0.000194 | 0.007907 | ASPDH |
| -2.97119 | 6.275163 | 13.88311 | 0.000195 | 0.007907 | ATF5 |
| -1.3756 | 5.486641 | 13.87568 | 0.000195 | 0.007918 | BAHCC1 |
| -2.41656 | 4.213716 | 13.86739 | 0.000196 | 0.007932 | JAG1 |
| -2.5885 | 0.51637 | 13.84821 | 0.000198 | 0.007977 | KCND1 |
| 1.215641 | 4.494589 | 13.84688 | 0.000198 | 0.007977 | TSEN2 |
| -1.12689 | 6.176228 | 13.83963 | 0.000199 | 0.007987 | ABCA3 |
| -2.22847 | 7.198248 | 13.79551 | 0.000204 | 0.008156 | ANXA5 |
| 1.020015 | 4.646879 | 13.74981 | 0.000209 | 0.008335 | COPS7A |
| -5.03872 | 6.857946 | 13.73086 | 0.000211 | 0.008385 | FAT1 |
| -2.60491 | 2.61071 | 13.7289 | 0.000211 | 0.008385 | TSHZ2 |
| -2.38653 | 0.659763 | 13.72313 | 0.000212 | 0.00839 | MID1IP1-AS1 |
| -1.99287 | 3.319788 | 13.70329 | 0.000214 | 0.008435 | FBXO5 |
| 1.559225 | 2.363464 | 13.65167 | 0.00022 | 0.008648 | TUBA3D |
| -1.41922 | 6.632908 | 13.63515 | 0.000222 | 0.008688 | NRDC |
| -2.07942 | 4.890651 | 13.63358 | 0.000222 | 0.008688 | EVC |
| -1.53667 | 5.973208 | 13.62006 | 0.000224 | 0.008729 | RRM1 |
| 1.794264 | 0.910633 | 13.61002 | 0.000225 | 0.008753 | AL355377.3 |
| -1.77283 | 4.940728 | 13.57324 | 0.000229 | 0.008886 | CADM4 |
| -2.74734 | 1.410671 | 13.57228 | 0.00023 | 0.008886 | FKBP9P 1 |
| 1.434619 | 3.804136 | 13.5619 | 0.000231 | 0.008897 | HSPBAP1 |
| -2.43221 | 4.201249 | 13.5606 | 0.000231 | 0.008897 | FAM171B |
| 2.427724 | 1.959515 | 13.55409 | 0.000232 | 0.008906 | AC110619.1 |
| -3.15205 | 4.050294 | 13.52835 | 0.000235 | 0.009006 | TCIM |
| -1.8112 | 1.979074 | 13.4757 | 0.000242 | 0.00924 | ACBD7 |
| 2.013848 | 8.466435 | 13.38988 | 0.000253 | 0.009648 | SRRM1 |
| 1.19006 | 1.876767 | 13.34779 | 0.000259 | 0.009843 | CR392039.5 |
| 1.016675 | 2.963385 | 13.3232 | 0.000262 | 0.009949 | DPH6 |
| -3.59555 | 2.008892 | 13.28981 | 0.000267 | 0.010102 | TNFSF12 |
| -6.23313 | 3.916343 | 13.27386 | 0.000269 | 0.010164 | SSTR1 |
| -4.02564 | 1.937954 | 13.26571 | 0.00027 | 0.010183 | AC126544.1 |
| -1.32379 | 5.720064 | 13.24549 | 0.000273 | 0.010215 | SOD2 |
| -1.02441 | 6.621456 | 13.24196 | 0.000274 | 0.010215 | OSBP |
| -1.34551 | 5.053461 | 13.2408 | 0.000274 | 0.010215 | SHMT1 |
| -6.82087 | 10.07621 | 13.2331 | 0.000275 | 0.010215 | PLA2G2A |
| -1.70758 | 0.59093 | 13.23261 | 0.000275 | 0.010215 | FZD9 |
| -1.5687 | 6.423426 | 13.21993 | 0.000277 | 0.01026 | DNMT1 |
| 1.265987 | 8.690659 | 13.20319 | 0.000279 | 0.010303 | ALCAM |
| -1.40448 | 5.536798 | 13.2031 | 0.000279 | 0.010303 | RNH1 |
| -1.9987 | 0.913027 | 13.19865 | 0.00028 | 0.010303 | SLC25A21 |
| -1.71475 | 5.919902 | 13.19211 | 0.000281 | 0.010314 | TMEM30B |
| -1.65139 | 0.58932 | 13.17267 | 0.000284 | 0.010397 | MROH8 |
| -2.45335 | 4.671415 | 13.13717 | 0.000289 | 0.010557 | MAFB |
| -1.4031 | 7.598136 | 13.13525 | 0.00029 | 0.010557 | WDR1 |
| -1.9795 | 6.449078 | 13.12751 | 0.000291 | 0.010576 | LETM1 |
| -4.07261 | 0.369533 | 13.11287 | 0.000293 | 0.010634 | CADM2 |
| 1.11283 | 6.111432 | 13.08487 | 0.000298 | 0.010752 | MDN1 |
| -1.69132 | 2.220335 | 13.08278 | 0.000298 | 0.010752 | KCNJ11 |
| 1.674967 | 3.564689 | 13.07895 | 0.000299 | 0.010752 | CLEC2D |
| -1.26391 | 5.072342 | 13.04602 | 0.000304 | 0.010892 | TSG101 |
| -2.10417 | 3.757667 | 13.03967 | 0.000305 | 0.010904 | WNT7B |
| -1.25853 | 4.636933 | 13.03369 | 0.000306 | 0.010913 | SLC35A2 |
| -1.93535 | 2.498946 | 13.00055 | 0.000311 | 0.011047 | OTUD1 |
| -2.20415 | 3.965381 | 12.99939 | 0.000312 | 0.011047 | BRCA2 |
| -4.02499 | 1.616439 | 12.99799 | 0.000312 | 0.011047 | KRTAP5-AS1 |
| 2.797476 | 2.132173 | 12.99071 | 0.000313 | 0.011065 | IL5RA |
| 1.532886 | 2.558576 | 12.98478 | 0.000314 | 0.011074 | AL450384.2 |
| -2.69375 | 3.447651 | 12.96945 | 0.000317 | 0.01114 | PIK3AP1 |
| -1.30041 | 4.25527 | 12.95136 | 0.00032 | 0.011202 | TARSL2 |
| 1.048286 | 3.066348 | 12.95056 | 0.00032 | 0.011202 | NEK11 |
| -2.05739 | 1.827765 | 12.93728 | 0.000322 | 0.011256 | PCDHGC3 |
| -3.20763 | 1.520723 | 12.93024 | 0.000323 | 0.01126 | SAMD 13 |
| 1.161531 | 3.950024 | 12.92817 | 0.000324 | 0.01126 | NT5C |
| 1.119992 | 2.063775 | 12.91643 | 0.000326 | 0.01128 | AP002360.1 |
| -1.04981 | 5.133726 | 12.90176 | 0.000328 | 0.011343 | SELENOS |
| -1.51941 | 6.266863 | 12.87527 | 0.000333 | 0.011479 | MBP |
| 1.452768 | 3.908841 | 12.86832 | 0.000334 | 0.011496 | NPHP3 |
| -5.21222 | 2.874699 | 12.86141 | 0.000335 | 0.011513 | EGFL7 |
| -4.26213 | 2.614897 | 12.84715 | 0.000338 | 0.011567 | CHST2 |
| -1.12073 | 4.15582 | 12.84382 | 0.000339 | 0.011567 | CCNB1 |
| -3.64851 | 1.414331 | 12.83965 | 0.000339 | 0.011567 | AC093001.1 |
| 1.678161 | 6.464423 | 12.8276 | 0.000342 | 0.011567 | AF117829.1 |
| -1.01296 | 5.756292 | 12.82737 | 0.000342 | 0.011567 | SEL1L3 |
| 1.522078 | 2.454178 | 12.8249 | 0.000342 | 0.011567 | MARCH1 |
| 1.143551 | 1.463006 | 12.82387 | 0.000342 | 0.011567 | C6orf163 |
| 1.137691 | 4.094382 | 12.81022 | 0.000345 | 0.011595 | LYPLAL1 |
| -1.92067 | 5.832512 | 12.80505 | 0.000346 | 0.011595 | INSIG1 |
| -1.32712 | 4.988302 | 12.80401 | 0.000346 | 0.011595 | RNF141 |
| -1.56429 | 2.091571 | 12.80263 | 0.000346 | 0.011595 | PARS2 |
| -1.75636 | 4.960624 | 12.799 | 0.000347 | 0.011595 | MID1IP1 |
| 1.059156 | 5.638847 | 12.76815 | 0.000353 | 0.01175 | COG4 |
| 3.689692 | 2.353578 | 12.76609 | 0.000353 | 0.01175 | THRSP |
| 1.326826 | 0.825035 | 12.73432 | 0.000359 | 0.011925 | AL031666.3 |
| 1.453972 | 2.131547 | 12.72239 | 0.000361 | 0.011976 | AL353795.3 |
| -2.38291 | 3.154504 | 12.71541 | 0.000363 | 0.01198 | FBXL17 |
| -3.37616 | 3.796934 | 12.71377 | 0.000363 | 0.01198 | S100A16 |
| -2.99287 | 4.365825 | 12.70236 | 0.000365 | 0.012017 | ASTN2 |
| 1.350572 | 3.552791 | 12.69843 | 0.000366 | 0.012017 | LINC02035 |
| -2.01123 | 4.660447 | 12.69603 | 0.000366 | 0.012017 | ZNF544 |
| -1.17801 | 4.756696 | 12.67685 | 0.00037 | 0.012115 | SPTY2D1 |
| -2.14708 | 0.513679 | 12.66616 | 0.000372 | 0.012159 | ANXA2R |
| -1.36754 | 6.650306 | 12.65437 | 0.000375 | 0.012206 | ZMYND11 |
| 1.978224 | 1.82293 | 12.64643 | 0.000376 | 0.012206 | AQP1 |
| -1.25177 | 5.469468 | 12.64628 | 0.000376 | 0.012206 | CTR9 |
| -1.01782 | 5.720662 | 12.64321 | 0.000377 | 0.012206 | ZNF592 |
| -1.06878 | 5.92012 | 12.60601 | 0.000385 | 0.012375 | AP1M2 |
| -4.03495 | 2.859742 | 12.60571 | 0.000385 | 0.012375 | ABCC3 |
| -1.07171 | 5.175829 | 12.60567 | 0.000385 | 0.012375 | TMEM41B |
| 1.421969 | 4.163915 | 12.59232 | 0.000387 | 0.012436 | CEP44 |
| -3.26721 | 2.087027 | 12.58579 | 0.000389 | 0.012436 | PDE8B |
| -1.61566 | 3.992721 | 12.58495 | 0.000389 | 0.012436 | SLC35D1 |
| -2.69838 | 1.027304 | 12.57281 | 0.000391 | 0.012491 | AC010615.1 |
| -1.98796 | 0.884305 | 12.56511 | 0.000393 | 0.012517 | AC011477.3 |
| -6.38765 | 5.227899 | 12.54224 | 0.000398 | 0.012603 | CD177 |
| -2.89449 | 0.9778 | 12.53734 | 0.000399 | 0.012603 | NAT8L |
| 1.564341 | 0.540616 | 12.5322 | 0.0004 | 0.012603 | AC008443.5 |
| -1.64677 | 5.892071 | 12.53047 | 0.0004 | 0.012603 | MAEA |
| -1.72668 | 6.664176 | 12.52926 | 0.000401 | 0.012603 | GAK |
| -1.411 | 5.796389 | 12.52341 | 0.000402 | 0.012617 | TACC3 |
| -1.90403 | 4.242839 | 12.5076 | 0.000405 | 0.012683 | RAB23 |
| 1.70641 | 1.725606 | 12.50612 | 0.000406 | 0.012683 | P2RX7 |
| 2.059053 | 0.899105 | 12.49729 | 0.000408 | 0.012697 | SYT15 |
| -4.5409 | 4.959774 | 12.4965 | 0.000408 | 0.012697 | ANGPTL4 |
| -2.42582 | 1.022993 | 12.48608 | 0.00041 | 0.012713 | SERPINB8 |
| -1.40147 | 4.580602 | 12.48385 | 0.00041 | 0.012713 | PHLPP1 |
| -4.64619 | 3.563182 | 12.47955 | 0.000411 | 0.012713 | MUC3A |
| -4.10639 | 2.684405 | 12.47916 | 0.000412 | 0.012713 | KLHL3 |
| -4.35376 | 3.014627 | 12.47113 | 0.000413 | 0.012742 | SIRPA |
| -1.30135 | 4.096666 | 12.45772 | 0.000416 | 0.012795 | ZNF407 |
| -6.37797 | 4.494953 | 12.454 | 0.000417 | 0.012795 | IGFBP3 |
| -1.43302 | 3.114128 | 12.4495 | 0.000418 | 0.012795 | SKA1 |
| 1.100302 | 6.565991 | 12.43912 | 0.00042 | 0.01281 | KAT2A |
| 1.253904 | 0.383911 | 12.43888 | 0.00042 | 0.01281 | AL136221.1 |
| 2.056054 | 3.374815 | 12.4234 | 0.000424 | 0.012892 | RMST |
| 1.32753 | 3.487615 | 12.41393 | 0.000426 | 0.012904 | EXOSC5 |
| -1.44465 | 0.546639 | 12.41221 | 0.000427 | 0.012904 | TIGD3 |
| 1.410808 | 4.45983 | 12.40387 | 0.000428 | 0.012904 | TBKBP1 |
| -1.27539 | 5.994998 | 12.4003 | 0.000429 | 0.012904 | NPC2 |
| 2.306233 | 4.396852 | 12.40026 | 0.000429 | 0.012904 | LINC00910 |
| -7.77997 | 5.111601 | 12.39963 | 0.000429 | 0.012904 | ERG |
| 1.706749 | 4.733024 | 12.38671 | 0.000432 | 0.012969 | CD27-AS1 |
| -1.1743 | 4.942753 | 12.37473 | 0.000435 | 0.013025 | IGSF9 |
| 1.912131 | 2.242467 | 12.37143 | 0.000436 | 0.013025 | AC139495.3 |
| 1.894715 | 0.367439 | 12.36582 | 0.000437 | 0.013039 | CR381670.2 |
| -4.53508 | 3.844447 | 12.33575 | 0.000444 | 0.013199 | NINL |
| 1.976016 | 2.477356 | 12.31044 | 0.00045 | 0.013336 | RASSF4 |
| -1.87652 | 4.270152 | 12.30935 | 0.000451 | 0.013336 | SIAH2 |
| -2.0822 | 1.264345 | 12.29077 | 0.000455 | 0.013389 | AC012618.3 |
| 1.542786 | 0.517156 | 12.28974 | 0.000455 | 0.013389 | C9orf131 |
| -1.22182 | 7.994591 | 12.28855 | 0.000456 | 0.013389 | USP22 |
| 1.747151 | 1.721301 | 12.28539 | 0.000457 | 0.013389 | ANKLE1 |
| -1.40418 | 4.40281 | 12.28423 | 0.000457 | 0.013389 | NAGK |
| -4.62894 | 2.862297 | 12.25855 | 0.000463 | 0.013523 | CHST9 |
| -2.7186 | 4.812725 | 12.2455 | 0.000466 | 0.013592 | STARD13 |
| -2.24067 | 4.964824 | 12.22821 | 0.000471 | 0.013693 | CHD7 |
| -3.47587 | 0.894599 | 12.22394 | 0.000472 | 0.013698 | ILDR2 |
| -1.47186 | 6.212186 | 12.2154 | 0.000474 | 0.013735 | MAN2A2 |
| -1.00658 | 5.279114 | 12.18854 | 0.000481 | 0.013882 | TEX261 |
| -2.16917 | 3.878245 | 12.15609 | 0.000489 | 0.014083 | RAP2B |
| -1.22632 | 6.293211 | 12.15391 | 0.00049 | 0.014083 | NUP98 |
| 1.084495 | 3.541164 | 12.15136 | 0.000491 | 0.014083 | C2orf92 |
| 1.927776 | 1.40153 | 12.13965 | 0.000494 | 0.014145 | RNF122 |
| 1.857484 | 0.53792 | 12.10352 | 0.000503 | 0.014395 | PTPRVP |
| -2.66625 | 6.336725 | 12.08761 | 0.000508 | 0.014492 | ARHGAP29 |
| 1.114449 | 9.746004 | 12.07051 | 0.000512 | 0.014583 | RPL28 |
| -1.1951 | 7.01776 | 12.04606 | 0.000519 | 0.014737 | DHX15 |
| -6.20628 | 5.575257 | 12.04004 | 0.000521 | 0.014753 | MUC12 |
| -1.42153 | 4.882381 | 12.03713 | 0.000522 | 0.014753 | TGFBR1 |
| 1.546982 | 1.534101 | 12.0327 | 0.000523 | 0.014761 | AC090739.1 |
| -4.61763 | 1.386016 | 12.02622 | 0.000525 | 0.014786 | HCG11 |
| -5.17537 | 2.028567 | 12.01694 | 0.000527 | 0.014816 | CREB5 |
| -4.0247 | 2.454638 | 12.01567 | 0.000528 | 0.014816 | C5orf38 |
| 1.091047 | 5.198481 | 12.00894 | 0.000529 | 0.014842 | GABARAPL1 |
| -2.70172 | 0.90309 | 11.99821 | 0.000533 | 0.014901 | LINC02489 |
| -1.95927 | 3.835578 | 11.98691 | 0.000536 | 0.014964 | OSTM1 |
| 1.464045 | 3.247458 | 11.9823 | 0.000537 | 0.014971 | AC091729.3 |
| 1.345371 | 3.181383 | 11.97937 | 0.000538 | 0.014971 | CRADD |
| -1.6251 | 4.087152 | 11.9644 | 0.000542 | 0.015064 | SMPD1 |
| -1.15915 | 5.646808 | 11.9604 | 0.000543 | 0.01507 | ATPAF1 |
| -3.09363 | 4.122707 | 11.94783 | 0.000547 | 0.015118 | PLIN2 |
| 1.085906 | 4.843583 | 11.94778 | 0.000547 | 0.015118 | ZNF330 |
| -1.00704 | 6.520014 | 11.92721 | 0.000553 | 0.015258 | TNPO2 |
| 2.78655 | 3.042269 | 11.90287 | 0.00056 | 0.015432 | ATP8B4 |
| -2.66366 | 1.031471 | 11.88437 | 0.000566 | 0.01553 | LURAPIL |
| -1.5894 | 4.981417 | 11.87713 | 0.000568 | 0.015535 | HOOK1 |
| -2.1711 | 4.483276 | 11.87058 | 0.00057 | 0.015553 | GUCY1B1 |
| 1.541075 | 5.560859 | 11.86849 | 0.000571 | 0.015553 | RPL32P3 |
| 1.211046 | 0.659736 | 11.86125 | 0.000573 | 0.015586 | NSRP1P1 |
| 1.448501 | 3.228945 | 11.85089 | 0.000576 | 0.015635 | KIAA1257 |
| -1.87411 | 5.021835 | 11.84888 | 0.000577 | 0.015635 | CNKSR3 |
| -1.53169 | 4.05798 | 11.84086 | 0.000579 | 0.015647 | TIMM21 |
| -2.3084 | 1.896038 | 11.81599 | 0.000587 | 0.015808 | FAHD2B |
| -1.47901 | 0.325238 | 11.81484 | 0.000588 | 0.015808 | FRMD3 |
| -3.20253 | 2.270006 | 11.81208 | 0.000588 | 0.015808 | VWDE |
| -2.44946 | 1.867942 | 11.80264 | 0.000591 | 0.015861 | SYP |
| -1.14748 | 4.774522 | 11.79103 | 0.000595 | 0.015897 | ASNA1 |
| -1.64466 | 2.427542 | 11.78785 | 0.000596 | 0.015897 | PRXL2C |
| 1.22896 | 1.247501 | 11.78604 | 0.000597 | 0.015897 | AC009061.2 |
| -1.14947 | 3.953324 | 11.78549 | 0.000597 | 0.015897 | ABHD5 |
| 1.211078 | 5.50589 | 11.76223 | 0.000604 | 0.016069 | TTLL3 |
| -1.11917 | 7.103859 | 11.75514 | 0.000607 | 0.016103 | ADD1 |
| -1.05728 | 5.835174 | 11.74257 | 0.000611 | 0.016184 | MGST3 |
| -3.57021 | 3.48894 | 11.72276 | 0.000617 | 0.016329 | B3GAT1 |
| -1.38149 | 3.719695 | 11.71138 | 0.000621 | 0.016378 | SEPSECS |
| -1.25514 | 4.120801 | 11.71085 | 0.000621 | 0.016378 | RMC1 |
| -1.44392 | 4.768828 | 11.70193 | 0.000624 | 0.016395 | TADA2B |
| -1.41213 | 5.338557 | 11.67719 | 0.000633 | 0.016564 | UVSSA |
| -1.09631 | 6.118526 | 11.67134 | 0.000635 | 0.016588 | REEP3 |
| -2.50914 | 3.603884 | 11.65623 | 0.00064 | 0.016695 | CPPED1 |
| -4.0818 | 3.960569 | 11.65315 | 0.000641 | 0.016695 | NECTIN3 |
| 1.236139 | 7.092955 | 11.6471 | 0.000643 | 0.016721 | TBC1D8 |
| -1.84109 | 4.337645 | 11.607 | 0.000657 | 0.016985 | TRIM66 |
| -1.06998 | 6.172846 | 11.60525 | 0.000658 | 0.016985 | PSEN1 |
| -3.9684 | 3.812333 | 11.59992 | 0.00066 | 0.016985 | LRRC31 |
| -1.57138 | 4.356555 | 11.59926 | 0.00066 | 0.016985 | CTDP1 |
| -4.24402 | 2.567296 | 11.58654 | 0.000664 | 0.017046 | N4BP2L1 |
| 1.551443 | 0.980035 | 11.58267 | 0.000666 | 0.017046 | AC012184.3 |
| 1.602361 | 2.053706 | 11.58207 | 0.000666 | 0.017046 | AC107027.3 |
| 1.019891 | 5.949844 | 11.58024 | 0.000667 | 0.017046 | BTAF1 |
| 1.44131 | 1.722092 | 11.57212 | 0.000669 | 0.017092 | PINK1-AS |
| 2.685195 | 2.142769 | 11.55681 | 0.000675 | 0.017205 | MT3 |
| -3.40662 | 2.425772 | 11.54699 | 0.000679 | 0.017268 | EIF5A2 |
| -1.57818 | 4.831617 | 11.51632 | 0.00069 | 0.017503 | NBEA |
| -2.62539 | 4.568092 | 11.51567 | 0.00069 | 0.017503 | SPTSSB |
| 1.286206 | 3.744298 | 11.50977 | 0.000692 | 0.017517 | RTEL1-TNFRSF6B |
| -1.54643 | 5.782387 | 11.50816 | 0.000693 | 0.017517 | KIAA0232 |
| -1.53075 | 4.983892 | 11.48591 | 0.000701 | 0.017664 | ZNF514 |
| -1.28481 | 4.873449 | 11.48535 | 0.000701 | 0.017664 | TES |
| 1.667825 | 0.565872 | 11.48346 | 0.000702 | 0.017664 | C1orf162 |
| -1.25276 | 6.807936 | 11.4623 | 0.00071 | 0.017815 | ACAT1 |
| -1.67922 | 2.937113 | 11.4607 | 0.000711 | 0.017815 | HOXC4 |
| -2.70512 | 0.229994 | 11.45857 | 0.000712 | 0.017815 | TMEM17 |
| -2.3539 | 3.079387 | 11.45515 | 0.000713 | 0.017819 | FAXC |
| 1.458221 | 1.767444 | 11.44771 | 0.000716 | 0.017862 | AP002907.1 |
| 1.384945 | 0.491748 | 11.40684 | 0.000732 | 0.018215 | AC093788.1 |
| -1.15286 | 4.711913 | 11.40488 | 0.000733 | 0.018215 | STIL |
| -1.04042 | 5.577378 | 11.40241 | 0.000733 | 0.018215 | BAG5 |
| -1.00415 | 7.16387 | 11.35408 | 0.000753 | 0.018665 | MYOIC |
| -1.66918 | 5.117851 | 11.34051 | 0.000758 | 0.018771 | TMEM129 |
| -2.08796 | 4.273857 | 11.32232 | 0.000766 | 0.018896 | ACOX3 |
| -2.97092 | 2.624038 | 11.30959 | 0.000771 | 0.01897 | F2RL3 |
| -3.89346 | 5.648202 | 11.3079 | 0.000772 | 0.01897 | TUBA1A |
| -1.58189 | 4.407448 | 11.30616 | 0.000773 | 0.01897 | ERV3-1 |
| 1.266696 | 2.628394 | 11.28732 | 0.00078 | 0.019133 | FRG1JP |
| 2.59477 | 3.451492 | 11.27248 | 0.000787 | 0.019256 | POU5F1B |
| -1.32939 | 6.561788 | 11.26604 | 0.000789 | 0.019267 | MYL12A |
| -1.30003 | 3.832604 | 11.26556 | 0.00079 | 0.019267 | BTBD10 |
| -2.77203 | 2.736291 | 11.25262 | 0.000795 | 0.019371 | CDKN2B |
| 1.195382 | 2.269096 | 11.23123 | 0.000804 | 0.019507 | AC109322.1 |
| 2.244067 | 1.808466 | 11.23088 | 0.000804 | 0.019507 | MYLK-AS1 |
| -1.40476 | 4.954789 | 11.22417 | 0.000807 | 0.019547 | RGS12 |
| -1.63627 | 6.109391 | 11.21991 | 0.000809 | 0.019561 | PCYOX1 |
| -1.23992 | 3.77759 | 11.21482 | 0.000811 | 0.019584 | LCORL |
| 1.256013 | 2.945448 | 11.21012 | 0.000814 | 0.019584 | ZNF321P |
| -3.58354 | 1.388711 | 11.20839 | 0.000814 | 0.019584 | LINC00472 |
| -1.38757 | 5.422769 | 11.20613 | 0.000815 | 0.019584 | NT5DC1 |
| -3.44029 | 1.45992 | 11.17224 | 0.00083 | 0.019883 | RELN |
| -1.23081 | 5.040032 | 11.16921 | 0.000832 | 0.019885 | SUSD6 |
| 1.206504 | 3.902745 | 11.16626 | 0.000833 | 0.019886 | RBM48 |
| -2.37755 | 1.312542 | 11.15517 | 0.000838 | 0.019974 | FLVCR2 |
| -1.70117 | 4.469414 | 11.1515 | 0.00084 | 0.019983 | PDE3B |
| -1.13159 | 3.436585 | 11.14198 | 0.000844 | 0.020055 | MTFMT |
| -1.16591 | 5.264863 | 11.12796 | 0.00085 | 0.020145 | STIM2 |
| -5.04527 | 3.757881 | 11.11805 | 0.000855 | 0.020219 | ANGPTL2 |
| -1.04602 | 5.252681 | 11.11376 | 0.000857 | 0.020219 | TMED8 |
| 1.308171 | 2.688863 | 11.11262 | 0.000857 | 0.020219 | AL022311.1 |
| -2.24742 | 1.175014 | 11.10489 | 0.000861 | 0.020273 | C19orf71 |
| 1.082447 | 4.539131 | 11.09837 | 0.000864 | 0.020313 | RHOBTB3 |
| 1.222287 | 1.804552 | 11.0748 | 0.000875 | 0.020542 | AC084824.1 |
| 1.69118 | 1.955022 | 11.06519 | 0.00088 | 0.020617 | AL390195.2 |
| -1.26879 | 6.27915 | 11.05888 | 0.000883 | 0.020656 | USP47 |
| 2.053128 | 2.258128 | 11.04369 | 0.00089 | 0.020795 | FUT2 |
| 1.431082 | 9.937659 | 11.02732 | 0.000898 | 0.020941 | RPL11 |
| -1.44168 | 3.007313 | 11.02272 | 0.0009 | 0.020941 | MOB3C |
| -3.335 | 1.62395 | 11.02227 | 0.0009 | 0.020941 | SFMBT2 |
| 1.834948 | 1.465327 | 11.0165 | 0.000903 | 0.020975 | AC044860.1 |
| -1.4167 | 6.706817 | 11.00754 | 0.000907 | 0.021045 | TMEM59 |
| 1.337094 | 0.286784 | 11.00365 | 0.000909 | 0.021056 | AC233280.1 |
| 1.264768 | 6.809627 | 10.99767 | 0.000912 | 0.021056 | KIF5C |
| 1.032548 | 8.108609 | 10.99706 | 0.000913 | 0.021056 | EEF1D |
| -1.60117 | 3.202902 | 10.98459 | 0.000919 | 0.021148 | DENND5B |
| -3.98919 | 1.16253 | 10.97972 | 0.000921 | 0.021173 | PTPRR |
| -2.60796 | 5.504153 | 10.96951 | 0.000926 | 0.021256 | STC2 |
| -1.29961 | 4.350616 | 10.9669 | 0.000928 | 0.021256 | REV3L |
| 1.199916 | 5.581796 | 10.94147 | 0.00094 | 0.021518 | RAB17 |
| -1.77093 | 3.812581 | 10.93802 | 0.000942 | 0.021526 | SGPP1 |
| 1.025266 | 3.807329 | 10.93503 | 0.000944 | 0.021529 | EMG1 |
| -1.79801 | 3.710167 | 10.92429 | 0.000949 | 0.021622 | TIPARP |
| 1.434115 | 3.483281 | 10.92035 | 0.000951 | 0.021637 | NR6A1 |
| -3.16208 | 4.334283 | 10.90894 | 0.000957 | 0.021738 | LFNG |
| -1.59192 | 5.512706 | 10.90446 | 0.000959 | 0.021758 | FAAH |
| -1.04427 | 5.269078 | 10.90178 | 0.000961 | 0.021758 | SLC25A44 |
| -1.60719 | 4.188751 | 10.89693 | 0.000963 | 0.021784 | CRYZ |
| -3.15499 | 4.433482 | 10.88245 | 0.000971 | 0.021858 | TMEM178A |
| -3.55639 | 5.657633 | 10.87833 | 0.000973 | 0.021875 | GSTCD |
| -1.36728 | 5.132073 | 10.87155 | 0.000977 | 0.021924 | SWAP70 |
| -1.13106 | 4.964499 | 10.86526 | 0.00098 | 0.021934 | MPDU1 |
| -1.68467 | 4.02782 | 10.85401 | 0.000986 | 0.022036 | LRRC42 |
| -1.41912 | 3.901137 | 10.84528 | 0.00099 | 0.022108 | NHLRC3 |
| -1.96867 | 5.565852 | 10.83484 | 0.000996 | 0.022201 | LMNB 1 |
| -1.15356 | 4.7779 | 10.81557 | 0.001007 | 0.022337 | AC027237.1 |
| -4.17744 | 2.992091 | 10.80786 | 0.001011 | 0.022369 | CPM |
| 1.502404 | 1.131757 | 10.80762 | 0.001011 | 0.022369 | TRIM74 |
| 2.381598 | 1.895964 | 10.80289 | 0.001013 | 0.022391 | DNM1P51 |
| -2.89644 | 3.882802 | 10.78968 | 0.001021 | 0.02249 | CRYBG3 |
| -1.36607 | 4.750283 | 10.78035 | 0.001026 | 0.02251 | PTPN9 |
| -1.11447 | 3.542924 | 10.77931 | 0.001026 | 0.02251 | POC1A |
| -1.00378 | 6.382382 | 10.77925 | 0.001026 | 0.02251 | CD151 |
| -3.53006 | 2.074286 | 10.77746 | 0.001027 | 0.02251 | ZNF711 |
| -1.42792 | 4.530897 | 10.75466 | 0.00104 | 0.022757 | EPHB3 |
| 3.453651 | 3.896307 | 10.74964 | 0.001043 | 0.022787 | ADGRL2 |
| 1.335179 | 2.337108 | 10.73923 | 0.001049 | 0.022883 | ABHD1 |
| -4.0784 | 2.351547 | 10.72669 | 0.001056 | 0.022983 | UST |
| -5.18646 | 4.091663 | 10.72414 | 0.001057 | 0.022983 | ANOS1 |
| -1.09644 | 4.471213 | 10.7233 | 0.001058 | 0.022983 | PLEKHG3 |
| -1.4395 | 5.588424 | 10.71395 | 0.001063 | 0.023067 | HEXB |
| 1.508317 | 5.550152 | 10.70219 | 0.00107 | 0.023155 | SLC25A27 |
| 1.496929 | 6.195945 | 10.69915 | 0.001072 | 0.023155 | TMCC1 |
| 1.113373 | 2.97095 | 10.69162 | 0.001076 | 0.023217 | ZCWPW1 |
| 1.307395 | 6.06693 | 10.68501 | 0.00108 | 0.023268 | PDZRN3 |
| 1.294911 | 6.220523 | 10.67511 | 0.001086 | 0.02336 | TOP1MT |
| -1.29502 | 1.39708 | 10.66424 | 0.001092 | 0.023442 | CYP51A1 |
| -1.42357 | 7.023579 | 10.66268 | 0.001093 | 0.023442 | TSPAN3 |
| 1.502414 | 6.855078 | 10.63305 | 0.001111 | 0.023732 | IQANK1 |
| -1.02596 | 5.562845 | 10.62464 | 0.001116 | 0.023807 | PGM1 |
| 1.147073 | 5.803102 | 10.6066 | 0.001127 | 0.023954 | TRIQK |
| -1.18808 | 4.800916 | 10.60654 | 0.001127 | 0.023954 | CARS |
| 1.206323 | 0.275964 | 10.6056 | 0.001127 | 0.023954 | AL450998.3 |
| -1.22763 | 4.747963 | 10.60077 | 0.00113 | 0.023984 | PGM2 |
| 1.179602 | 6.945743 | 10.57421 | 0.001147 | 0.024231 | MRPL3 |
| 1.235325 | 1.852672 | 10.55603 | 0.001158 | 0.024404 | AC138028.2 |
| -1.30721 | 4.127487 | 10.53972 | 0.001168 | 0.024586 | SCAPER |
| -3.02376 | 4.570218 | 10.53538 | 0.001171 | 0.024586 | BICDL1 |
| 1.368726 | 4.089805 | 10.53475 | 0.001172 | 0.024586 | AC003681.1 |
| 1.384129 | 3.450774 | 10.52052 | 0.001181 | 0.024742 | AL160006.1 |
| -1.44968 | 5.452787 | 10.51277 | 0.001186 | 0.024742 | WRNIP 1 |
| 1.267916 | 2.254049 | 10.51121 | 0.001187 | 0.024742 | AC005839.1 |
| 1.099766 | 4.307724 | 10.51055 | 0.001187 | 0.024742 | CENPC |
| -1.25133 | 1.37745 | 10.49341 | 0.001198 | 0.024884 | AP006621.5 |
| 2.153384 | 1.781897 | 10.49228 | 0.001199 | 0.024884 | PRRT3-AS1 |
| 1.366967 | 0.428946 | 10.49025 | 0.0012 | 0.024884 | AC023424.3 |
| -1.01018 | 4.172396 | 10.48998 | 0.0012 | 0.024884 | CDC40 |
| -1.25668 | 4.30363 | 10.45346 | 0.001224 | 0.025313 | HRAS |
| -1.52971 | 2.785133 | 10.44752 | 0.001228 | 0.025361 | PORCN |
| 2.044792 | 0.429548 | 10.42377 | 0.001244 | 0.025655 | ANKK 1 |
| 1.17349 | 2.998499 | 10.40312 | 0.001258 | 0.025901 | LINC00893 |
| -1.13505 | 4.163583 | 10.40122 | 0.001259 | 0.025901 | TMEM9B |
| -1.93198 | 4.7454 | 10.38517 | 0.00127 | 0.026058 | AEN |
| -1.07327 | 4.046928 | 10.37149 | 0.00128 | 0.026217 | STX10 |
| 1.398501 | 3.84963 | 10.35485 | 0.001291 | 0.026389 | LSM1 |
| -1.57058 | 2.558096 | 10.35294 | 0.001293 | 0.026389 | MBLAC2 |
| -1.35872 | 3.505149 | 10.35207 | 0.001293 | 0.026389 | ERMAP |
| -1.3897 | 4.679877 | 10.31746 | 0.001318 | 0.026747 | GINM1 |
| -1.92328 | 3.586537 | 10.31286 | 0.001321 | 0.026778 | CRMP1 |
| -2.74765 | 2.841714 | 10.30688 | 0.001325 | 0.02683 | ZNF35 |
| 1.589804 | 1.312972 | 10.30119 | 0.001329 | 0.026834 | AC010186.3 |
| -1.09881 | 6.273003 | 10.29936 | 0.001331 | 0.026834 | FDPS |
| -3.7404 | 3.002665 | 10.28764 | 0.001339 | 0.026935 | FZD8 |
| 1.483976 | 3.6037 | 10.28062 | 0.001344 | 0.026967 | LINC01238 |
| 1.476106 | 3.508909 | 10.27671 | 0.001347 | 0.026988 | KAZN |
| -1.12635 | 5.582945 | 10.26343 | 0.001357 | 0.027114 | POLG |
| 1.007759 | 5.94166 | 10.25554 | 0.001363 | 0.027195 | MCCC1 |
| 1.750909 | 1.980182 | 10.25206 | 0.001365 | 0.027211 | AL731563.3 |
| -1.20448 | 4.636709 | 10.23345 | 0.001379 | 0.027381 | MCUR1 |
| -2.29491 | 1.56579 | 10.22839 | 0.001383 | 0.027399 | PRDM5 |
| 2.865363 | 2.276671 | 10.22624 | 0.001385 | 0.027399 | ALDH1A2 |
| 2.820362 | 1.931096 | 10.22517 | 0.001385 | 0.027399 | UNC5A |
| -1.16741 | 5.112973 | 10.21975 | 0.001389 | 0.027444 | HIVEP 1 |
| 1.318508 | 1.525046 | 10.21712 | 0.001391 | 0.027448 | LINC01772 |
| -1.1449 | 5.309152 | 10.18543 | 0.001416 | 0.027853 | MAP2K1 |
| 1.173287 | 2.407519 | 10.17641 | 0.001422 | 0.027894 | AC012360.3 |
| -1.35484 | 6.298692 | 10.17594 | 0.001423 | 0.027894 | MXD4 |
| -1.67456 | 4.03784 | 10.17567 | 0.001423 | 0.027894 | VLDLR |
| -1.05832 | 3.566139 | 10.16782 | 0.001429 | 0.027909 | BEND3 |
| -4.65938 | 3.569692 | 10.16527 | 0.001431 | 0.027909 | CRYM |
| 1.808364 | 0.672057 | 10.16203 | 0.001434 | 0.027909 | LRRC17 |
| -2.11863 | 1.547204 | 10.15853 | 0.001436 | 0.027909 | SLC26A5 |
| -1.19119 | 5.687037 | 10.15836 | 0.001436 | 0.027909 | PPIF |
| 1.978192 | 3.262146 | 10.14732 | 0.001445 | 0.028017 | SNORA7B |
| 1.309919 | 0.363635 | 10.14665 | 0.001446 | 0.028017 | SMIM1 |
| 1.560344 | 3.380057 | 10.142 | 0.001449 | 0.028052 | TMEM154 |
| -1.52948 | 3.406835 | 10.13063 | 0.001458 | 0.02816 | ANKRD42 |
| 1.444339 | 1.026049 | 10.12854 | 0.00146 | 0.02816 | AL353135.2 |
| -1.09225 | 6.247047 | 10.12587 | 0.001462 | 0.02816 | AIFM1 |
| -1.25806 | 5.827681 | 10.12573 | 0.001462 | 0.02816 | TULP4 |
| -1.05854 | 5.34025 | 10.11145 | 0.001474 | 0.028318 | IL13RA1 |
| 3.039559 | 2.9897 | 10.11077 | 0.001474 | 0.028318 | RHOXF1P3 |
| -1.21728 | 4.204943 | 10.10854 | 0.001476 | 0.028318 | MOB3A |
| -1.53104 | 2.012428 | 10.09399 | 0.001488 | 0.028507 | ZBTB49 |
| -2.68733 | 3.853298 | 10.09172 | 0.001489 | 0.028507 | RIMKLA |
| 2.051234 | 1.305742 | 10.08935 | 0.001491 | 0.028508 | AL390957.1 |
| 1.165374 | 3.558857 | 10.07486 | 0.001503 | 0.028697 | LSM6 |
| -1.26013 | 2.058408 | 10.05638 | 0.001518 | 0.028859 | FSTL3 |
| 1.699869 | 1.127782 | 10.05576 | 0.001519 | 0.028859 | CCDC114 |
| 4.547983 | 4.597482 | 10.05241 | 0.001521 | 0.028871 | PCAT4 |
| -3.90263 | 1.744255 | 10.04178 | 0.00153 | 0.028877 | PLBD 1-AS 1 |
| -1.59751 | 2.196441 | 10.04115 | 0.001531 | 0.028877 | ZBTB8A |
| 1.049524 | 9.438227 | 10.03864 | 0.001533 | 0.028877 | RPL23 |
| -2.15693 | 2.875085 | 10.03778 | 0.001534 | 0.028877 | TIAM2 |
| -2.0998 | 7.230918 | 10.03746 | 0.001534 | 0.028877 | FURIN |
| 1.55539 | 0.980607 | 10.03627 | 0.001535 | 0.028877 | AC067930.8 |
| 1.197337 | 1.964481 | 10.03035 | 0.00154 | 0.028917 | ZNF436-AS1 |
| -2.39829 | 6.860533 | 10.02921 | 0.001541 | 0.028917 | FSTL1 |
| 1.605033 | 5.936972 | 10.02265 | 0.001546 | 0.028985 | SNHG4 |
| -1.26563 | 3.106249 | 10.0178 | 0.00155 | 0.028991 | KLHL25 |
| 1.200531 | 1.018671 | 10.00714 | 0.001559 | 0.029029 | AC087521.2 |
| 1.202736 | 3.479114 | 10.00649 | 0.00156 | 0.029029 | NEK3 |
| -6.3996 | 3.382798 | 10.00204 | 0.001564 | 0.029064 | NPY6R |
| -1.13236 | 4.801751 | 9.996363 | 0.001568 | 0.029119 | IDH3A |
| -5.35202 | 4.613127 | 9.979022 | 0.001583 | 0.02929 | GC |
| 1.112719 | 3.679947 | 9.978517 | 0.001584 | 0.02929 | C3orf62 |
| -1.18856 | 5.537795 | 9.977665 | 0.001585 | 0.02929 | ADGRA3 |
| -2.97936 | 6.038508 | 9.976529 | 0.001585 | 0.02929 | SIPA1L2 |
| -1.18669 | 4.583914 | 9.973085 | 0.001588 | 0.02929 | ZNF440 |
| -2.43151 | 2.621433 | 9.972323 | 0.001589 | 0.02929 | PLPP3 |
| -1.52203 | 0.961837 | 9.969193 | 0.001592 | 0.029291 | AL161891.1 |
| 2.93436 | 2.002195 | 9.965732 | 0.001595 | 0.029291 | KLF2P4 |
| -1.05476 | 6.944645 | 9.960754 | 0.001599 | 0.029335 | MPZL1 |
| -1.01343 | 4.651734 | 9.957498 | 0.001602 | 0.029342 | MRPS 11 |
| -1.26395 | 4.373694 | 9.955942 | 0.001603 | 0.029342 | COA7 |
| -3.55003 | 2.192897 | 9.948967 | 0.001609 | 0.029372 | HCP5 |
| 1.229998 | 0.499298 | 9.947549 | 0.001611 | 0.029372 | AC007308.6 |
| 1.595952 | 1.381727 | 9.924336 | 0.001631 | 0.029644 | AL159169.2 |
| 1.0323 | 4.486928 | 9.921808 | 0.001633 | 0.029645 | ZNF550 |
| -1.58631 | 5.38163 | 9.913267 | 0.001641 | 0.029713 | KIF4A |
| 2.308939 | 5.414191 | 9.907424 | 0.001646 | 0.029773 | CASC19 |
| 2.301078 | 3.496165 | 9.893353 | 0.001659 | 0.029863 | AC005534.1 |
| -1.08846 | 3.561824 | 9.893278 | 0.001659 | 0.029863 | EIF4E3 |
| -2.7218 | 0.63838 | 9.888458 | 0.001663 | 0.029877 | CCDC144CP |
| 2.156216 | 1.088237 | 9.888124 | 0.001663 | 0.029877 | KLK14 |
| -3.20918 | 3.003698 | 9.878396 | 0.001672 | 0.029931 | DPYSL4 |
| -1.18954 | 3.175479 | 9.87208 | 0.001678 | 0.029958 | NATD1 |
| 1.873146 | 2.498164 | 9.871953 | 0.001678 | 0.029958 | LINC00885 |
| -1.27507 | 2.266161 | 9.869112 | 0.001681 | 0.029958 | ZNF287 |
| 1.100516 | 0.393372 | 9.858629 | 0.00169 | 0.030018 | AC084824.3 |
| 1.189387 | 2.47468 | 9.85724 | 0.001692 | 0.030018 | NPIPB 13 |
| -1.4563 | 4.165586 | 9.853387 | 0.001695 | 0.030027 | CCNA2 |
| -1.69874 | 6.634399 | 9.823195 | 0.001723 | 0.030455 | NUCB2 |
| -1.3624 | 8.322145 | 9.818072 | 0.001728 | 0.030473 | DHCR24 |
| -1.65484 | 6.218028 | 9.817901 | 0.001728 | 0.030473 | SEC11C |
| -6.86409 | 7.051041 | 9.815337 | 0.001731 | 0.03048 | ALOX15B |
| -1.69728 | 5.499494 | 9.806688 | 0.001739 | 0.030589 | FAR1 |
| -2.42131 | 1.991271 | 9.797632 | 0.001747 | 0.030699 | PCDHGA5 |
| -1.38752 | 6.087961 | 9.780856 | 0.001763 | 0.030892 | OGDH |
| -3.49684 | 3.230886 | 9.778175 | 0.001766 | 0.030892 | DNAH5 |
| 1.460221 | 5.075841 | 9.778136 | 0.001766 | 0.030892 | SNHG5 |
| -1.5425 | 4.044725 | 9.774388 | 0.00177 | 0.03092 | MAD2L1 |
| -2.22619 | 0.933918 | 9.765351 | 0.001778 | 0.031037 | AL161910.1 |
| -1.15857 | 3.953021 | 9.75664 | 0.001787 | 0.031114 | PHF19 |
| -1.43971 | 1.740885 | 9.751077 | 0.001792 | 0.031173 | CDHRS |
| 1.160556 | 0.7859 | 9.74305 | 0.0018 | 0.031239 | AC079414.3 |
| 1.256566 | 4.762689 | 9.698458 | 0.001844 | 0.031823 | RAD51-AS1 |
| -2.17252 | 4.633672 | 9.695849 | 0.001847 | 0.031823 | C12orf75 |
| -1.66102 | 3.768082 | 9.695664 | 0.001847 | 0.031823 | SOD3 |
| -1.56407 | 1.263131 | 9.695076 | 0.001848 | 0.031823 | MGAT2 |
| -1.0996 | 3.986986 | 9.694578 | 0.001848 | 0.031823 | CXCL16 |
| 1.528017 | 0.569091 | 9.689689 | 0.001853 | 0.031838 | AL157938.3 |
| -2.71846 | 3.712905 | 9.68214 | 0.001861 | 0.031899 | TNFAIP8 |
| -4.30297 | 3.194148 | 9.677167 | 0.001866 | 0.031914 | PHLDA3 |
| 1.327121 | 0.565187 | 9.670296 | 0.001873 | 0.031941 | AL359922.2 |
| 1.082537 | 5.291605 | 9.669517 | 0.001874 | 0.031941 | FBXO32 |
| 1.846962 | 1.456905 | 9.665064 | 0.001878 | 0.031969 | AC112191.2 |
| 1.389045 | 2.890493 | 9.663834 | 0.001879 | 0.031969 | ARHGAP4 |
| -2.84284 | 2.100073 | 9.660704 | 0.001883 | 0.031988 | HHAT |
| -1.49645 | 5.275407 | 9.654269 | 0.001889 | 0.032047 | ASCC3 |
| -1.80056 | 3.091661 | 9.651649 | 0.001892 | 0.032047 | ARMT1 |
| 2.263504 | 2.618765 | 9.648961 | 0.001895 | 0.032047 | PKHD1L1 |
| -1.72979 | 1.93629 | 9.64875 | 0.001895 | 0.032047 | EPM2A |
| -1.57436 | 3.07733 | 9.647247 | 0.001896 | 0.032047 | PARD6G |
| 1.008652 | 6.833066 | 9.634978 | 0.001909 | 0.032192 | TARBP1 |
| 1.030468 | 6.079754 | 9.62874 | 0.001916 | 0.032196 | DOCK1 |
| -1.18917 | 3.979073 | 9.625182 | 0.001919 | 0.032196 | ALDH16A1 |
| 1.449621 | 3.959588 | 9.62471 | 0.00192 | 0.032196 | RP11-152F13.3 |
| 1.06686 | 6.326912 | 9.615345 | 0.00193 | 0.032291 | KAT6A |
| -1.04151 | 3.509277 | 9.612536 | 0.001933 | 0.032305 | TTC31 |
| -1.19902 | 6.62118 | 9.599043 | 0.001947 | 0.03244 | CTBP1 |
| 1.288692 | 4.701157 | 9.597507 | 0.001948 | 0.03244 | CROCCP2 |
| -1.91184 | 2.05058 | 9.571551 | 0.001976 | 0.032857 | GRK4 |
| 1.059574 | 6.156103 | 9.56589 | 0.001982 | 0.032923 | ANKRD10 |
| -2.29292 | 1.51169 | 9.561981 | 0.001986 | 0.032957 | ARSB |
| -2.41446 | 0.914369 | 9.558535 | 0.00199 | 0.032976 | PCDHGB4 |
| 1.129694 | 3.217959 | 9.557016 | 0.001992 | 0.032976 | MMAA |
| 3.266214 | 5.618647 | 9.547262 | 0.002002 | 0.033026 | PGC |
| -1.25349 | 3.71981 | 9.545418 | 0.002004 | 0.033026 | NACC2 |
| 1.278484 | 1.339748 | 9.5444 | 0.002006 | 0.033026 | CLEC11A |
| -1.07554 | 5.526142 | 9.541911 | 0.002008 | 0.033036 | SCAF8 |
| -1.62622 | 2.009898 | 9.525878 | 0.002026 | 0.03329 | FAM86EP |
| 1.6935 | 3.323564 | 9.514885 | 0.002038 | 0.033419 | AC018809.2 |
| 1.183514 | 2.32496 | 9.502107 | 0.002052 | 0.033606 | AC244090.1 |
| 1.582386 | 5.326226 | 9.500769 | 0.002054 | 0.033606 | MAPT |
| 1.088037 | 1.599809 | 9.48714 | 0.002069 | 0.033785 | FAM86HP |
| -3.8145 | 4.808532 | 9.482903 | 0.002074 | 0.033827 | PKDCC |
| -1.39197 | 4.219241 | 9.472355 | 0.002086 | 0.033986 | CA5B |
| -1.25196 | 5.226375 | 9.467886 | 0.002091 | 0.034033 | RELCH |
| -1.76823 | 4.423362 | 9.462971 | 0.002097 | 0.034089 | USP43 |
| -1.23866 | 4.767039 | 9.460308 | 0.0021 | 0.034102 | PSMD5 |
| -1.14933 | 2.784098 | 9.447749 | 0.002114 | 0.0343 | PRRG2 |
| -2.21892 | 1.841081 | 9.443499 | 0.002119 | 0.0343 | MIR924HG |
| 1.583065 | 5.10205 | 9.442599 | 0.00212 | 0.0343 | COL28A1 |
| 2.044166 | 1.907344 | 9.442033 | 0.002121 | 0.0343 | TMC1 |
| -1.39662 | 6.479321 | 9.41555 | 0.002152 | 0.034689 | AAK1 |
| -2.03906 | 3.045985 | 9.415499 | 0.002152 | 0.034689 | TMEM42 |
| -1.04756 | 4.237924 | 9.413689 | 0.002154 | 0.034689 | ZNF700 |
| -2.80058 | 2.270673 | 9.410326 | 0.002158 | 0.034701 | JAZF1 |
| -2.99226 | 2.023294 | 9.40567 | 0.002163 | 0.034701 | ACHE |
| 1.908875 | 2.061994 | 9.404865 | 0.002164 | 0.034701 | ST3GAL5 |
| -1.35485 | 0.20711 | 9.404564 | 0.002164 | 0.034701 | LTC4S |
| -1.72319 | 4.170873 | 9.400639 | 0.002169 | 0.034701 | EVI5L |
| -2.69508 | 1.695778 | 9.4001 | 0.00217 | 0.034701 | RNF180 |
| 1.209407 | 0.881599 | 9.399686 | 0.00217 | 0.034701 | NTAN1P2 |
| -1.08824 | 5.245896 | 9.387714 | 0.002184 | 0.03484 | PHKA1 |
| -1.1828 | 6.063604 | 9.386792 | 0.002186 | 0.03484 | IDI1 |
| 1.441004 | 3.949788 | 9.386704 | 0.002186 | 0.03484 | RP13-996F3.3 |
| -1.12396 | 7.191096 | 9.378997 | 0.002195 | 0.034914 | KIAA1217 |
| 2.112204 | 0.953545 | 9.374772 | 0.0022 | 0.03492 | SLC6A14 |
| -1.13483 | 5.6575 | 9.373018 | 0.002202 | 0.03492 | ULK3 |
| 1.255471 | 0.367861 | 9.367855 | 0.002208 | 0.034983 | AL031666.2 |
| -1.36193 | 6.525069 | 9.351494 | 0.002228 | 0.035215 | H2AFJ |
| -1.68739 | 0.697846 | 9.342296 | 0.002239 | 0.035329 | AL583810.1 |
| 1.144331 | 2.625062 | 9.327571 | 0.002257 | 0.035578 | AC245060.5 |
| -2.39726 | 3.03513 | 9.316199 | 0.002271 | 0.035719 | ARHGEF6 |
| -3.17344 | 1.481613 | 9.315965 | 0.002272 | 0.035719 | NHSL2 |
| 1.281675 | 0.5501 | 9.314715 | 0.002273 | 0.035719 | AL020996.1 |
| 1.592614 | 7.607668 | 9.312189 | 0.002276 | 0.035732 | NRBP2 |
| 3.085882 | 3.327048 | 9.297821 | 0.002294 | 0.035957 | SLCO1A2 |
| 1.787306 | 2.459348 | 9.296958 | 0.002295 | 0.035957 | AC010210.1 |
| -1.28548 | 3.109001 | 9.293015 | 0.0023 | 0.035961 | LRRC75A |
| -1.0374 | 2.703344 | 9.28888 | 0.002305 | 0.035961 | AC068831.7 |
| -1.45042 | 4.452415 | 9.286537 | 0.002308 | 0.035961 | ZBED3 |
| -1.06805 | 4.489975 | 9.286122 | 0.002309 | 0.035961 | TNIP2 |
| -4.71495 | 5.249672 | 9.285636 | 0.00231 | 0.035961 | ROBO1 |
| -1.12974 | 3.794199 | 9.282498 | 0.002314 | 0.035987 | HSPA14 |
| -2.58564 | 1.799583 | 9.273252 | 0.002325 | 0.036133 | DNASE2B |
| 1.984904 | 1.745685 | 9.270382 | 0.002329 | 0.036153 | AL109613.1 |
| -1.37739 | 2.742877 | 9.263628 | 0.002337 | 0.0362 | SAAL 1 |
| -1.74402 | 5.242942 | 9.262486 | 0.002339 | 0.0362 | STXBP1 |
| -1.09944 | 5.482327 | 9.245708 | 0.00236 | 0.03649 | TMEM181 |
| -2.31258 | 4.572273 | 9.244213 | 0.002362 | 0.03649 | P4HA2 |
| 1.581881 | 5.897921 | 9.231437 | 0.002379 | 0.036635 | GPCPD1 |
| -1.61978 | 5.382305 | 9.226314 | 0.002386 | 0.036701 | ZMPSTE24 |
| -1.04703 | 4.137829 | 9.222191 | 0.002391 | 0.036748 | CKS2 |
| 1.242922 | 4.554184 | 9.217153 | 0.002398 | 0.036812 | MBNL2 |
| 1.418517 | 11.73215 | 9.206279 | 0.002412 | 0.036942 | RPL8 |
| -1.26865 | 4.651259 | 9.205149 | 0.002413 | 0.036942 | PCGF5 |
| -3.23667 | 4.326784 | 9.204989 | 0.002414 | 0.036942 | ZNF350-AS1 |
| -1.80519 | 3.36736 | 9.202788 | 0.002416 | 0.036942 | TRIM21 |
| -1.14756 | 6.747708 | 9.195514 | 0.002426 | 0.036942 | ANKRD52 |
| 2.6444 | 1.026493 | 9.19535 | 0.002426 | 0.036942 | ZNF730 |
| -1.26195 | 1.705013 | 9.194397 | 0.002428 | 0.036942 | ST20-AS 1 |
| 1.433073 | 0.286259 | 9.190406 | 0.002433 | 0.036986 | PRSS48 |
| -1.06342 | 6.76835 | 9.186154 | 0.002439 | 0.037031 | LRPAP1 |
| -5.09022 | 4.756337 | 9.178044 | 0.002449 | 0.037117 | FMOD |
| -1.77226 | 6.595205 | 9.174556 | 0.002454 | 0.037117 | ANXA2 |
| -1.21685 | 3.806804 | 9.17317 | 0.002456 | 0.037117 | RTKN2 |
| -1.06356 | 4.901036 | 9.160779 | 0.002473 | 0.037296 | CC2D1B |
| -2.21206 | 2.776953 | 9.157172 | 0.002477 | 0.037333 | NCR3LG1 |
| 1.783234 | 4.353115 | 9.146309 | 0.002492 | 0.037519 | ACSF2 |
| 1.289284 | 2.413628 | 9.142393 | 0.002498 | 0.037563 | LINC00339 |
| -1.28355 | 4.532297 | 9.130004 | 0.002515 | 0.037689 | HAUS3 |
| 1.340884 | 0.598325 | 9.127444 | 0.002518 | 0.037689 | RP11-439A17.9 |
| -1.47105 | 3.990927 | 9.127366 | 0.002518 | 0.037689 | UPRT |
| -2.90945 | 3.063349 | 9.121128 | 0.002527 | 0.037781 | AL590560.2 |
| -1.09764 | 5.34675 | 9.119348 | 0.002529 | 0.037781 | UGP2 |
| 1.364957 | 0.834515 | 9.117174 | 0.002532 | 0.03779 | AC005899.8 |
| -1.41351 | 5.447751 | 9.114092 | 0.002536 | 0.037817 | ULK2 |
| 1.366635 | 5.003483 | 9.101978 | 0.002553 | 0.038032 | CFAP70 |
| 1.44477 | 2.937217 | 9.09832 | 0.002558 | 0.038046 | FAM49A |
| 1.209373 | 3.473606 | 9.096983 | 0.00256 | 0.038046 | AC004982.2 |
| -1.12123 | 4.255046 | 9.096009 | 0.002562 | 0.038046 | FAM160A2 |
| -1.22226 | 3.408992 | 9.093537 | 0.002565 | 0.038061 | STAG3L4 |
| 1.25662 | 0.363542 | 9.081441 | 0.002582 | 0.03824 | AL133243.4 |
| -1.14963 | 3.20586 | 9.064608 | 0.002606 | 0.038486 | SLC25A15 |
| -2.20457 | 2.76303 | 9.064475 | 0.002606 | 0.038486 | ARHGAP44 |
| -1.0668 | 5.333852 | 9.05952 | 0.002613 | 0.038553 | TSPAN6 |
| 1.234452 | 4.063441 | 9.049936 | 0.002627 | 0.038598 | MAK16 |
| -1.279 | 5.80509 | 9.047428 | 0.002631 | 0.038598 | INF2 |
| 1.047328 | 2.434868 | 9.047113 | 0.002631 | 0.038598 | ZFHX2-AS1 |
| 1.69453 | 2.167206 | 9.038154 | 0.002644 | 0.038711 | AC073655.2 |
| -1.23741 | 3.396466 | 9.031824 | 0.002653 | 0.038711 | KATNA1 |
| -1.02887 | 3.910311 | 9.03165 | 0.002653 | 0.038711 | MKKS |
| -2.09076 | 2.253149 | 9.031383 | 0.002654 | 0.038711 | AC026748.3 |
| -1.52714 | 3.639672 | 9.029034 | 0.002657 | 0.038711 | SLC26A1 |
| 1.798686 | 2.805694 | 9.028952 | 0.002657 | 0.038711 | RNF32 |
| -2.78064 | 4.973335 | 9.023352 | 0.002666 | 0.038711 | UCP2 |
| -1.07137 | 5.441901 | 9.023155 | 0.002666 | 0.038711 | TET3 |
| -1.06659 | 3.79483 | 9.021173 | 0.002669 | 0.038711 | TSSC4 |
| -1.26485 | 2.985328 | 9.018476 | 0.002673 | 0.038726 | TRIM59 |
| -1.4172 | 2.496573 | 8.998021 | 0.002703 | 0.039115 | ELAC1 |
| 1.10861 | 2.302374 | 8.993801 | 0.002709 | 0.039115 | LBHD1 |
| 1.277815 | 6.948756 | 8.987372 | 0.002719 | 0.03917 | AFMID |
| 2.817241 | 0.640491 | 8.981993 | 0.002727 | 0.039249 | AC018865.1 |
| 2.56844 | 1.124481 | 8.972651 | 0.002741 | 0.039392 | LINC01694 |
| -1.36111 | 7.27774 | 8.968882 | 0.002746 | 0.039392 | HK1 |
| 1.154761 | 5.49728 | 8.96854 | 0.002747 | 0.039392 | TIMMDC1 |
| 1.160687 | 2.960828 | 8.964042 | 0.002753 | 0.039453 | SRP14-AS1 |
| -1.6555 | 2.168875 | 8.960716 | 0.002758 | 0.039454 | ORC1 |
| -2.64347 | 0.858883 | 8.960577 | 0.002759 | 0.039454 | LRRC43 |
| 2.420691 | 2.134405 | 8.940129 | 0.00279 | 0.039748 | LINC01270 |
| -1.33726 | 5.542861 | 8.939736 | 0.00279 | 0.039748 | RAB25 |
| -2.06551 | 5.858451 | 8.935577 | 0.002797 | 0.039748 | ZNF736 |
| -1.66462 | 2.494645 | 8.935094 | 0.002797 | 0.039748 | CEP85L |
| -1.15555 | 3.940692 | 8.931876 | 0.002802 | 0.039748 | SIRT3 |
| -1.3008 | 1.456414 | 8.931844 | 0.002802 | 0.039748 | MTX1P1 |
| -1.67836 | 3.920477 | 8.929325 | 0.002806 | 0.039767 | MLLT3 |
| -1.46925 | 3.991987 | 8.924998 | 0.002813 | 0.039825 | NCAPG |
| -4.04978 | 3.661997 | 8.920917 | 0.002819 | 0.039852 | FGD1 |
| -3.19493 | 2.837048 | 8.920405 | 0.00282 | 0.039852 | SMIM10L2A |
| -1.87378 | 0.657082 | 8.917126 | 0.002825 | 0.039887 | AL683813.1 |
| -1.90719 | 1.032796 | 8.911913 | 0.002833 | 0.039964 | FLVCR1-DT |
| 2.899385 | 2.44663 | 8.908825 | 0.002838 | 0.039974 | CLEC7A |
| -3.32104 | 2.170331 | 8.899093 | 0.002853 | 0.040111 | TFF3 |
| -1.38413 | 2.194143 | 8.898573 | 0.002854 | 0.040111 | C5orf49 |
| 1.149759 | 4.622057 | 8.894834 | 0.00286 | 0.040157 | MERTK |
| 1.141906 | 2.611642 | 8.890046 | 0.002867 | 0.040225 | RPL13AP5 |
| -1.1751 | 5.524332 | 8.878248 | 0.002886 | 0.040426 | RANBP9 |
| -1.07126 | 5.833475 | 8.877671 | 0.002887 | 0.040426 | FAM193A |
| 3.101759 | 4.311527 | 8.872734 | 0.002895 | 0.040477 | FAR2P4 |
| -2.34536 | 2.16934 | 8.872058 | 0.002896 | 0.040477 | PCDHGB5 |
| -3.13228 | 1.100492 | 8.868276 | 0.002902 | 0.040524 | BBS12 |
| -1.45573 | 3.341938 | 8.865899 | 0.002905 | 0.04054 | RCCD1 |
| 1.104436 | 2.420016 | 8.863316 | 0.00291 | 0.040547 | AC007566.1 |
| -1.13167 | 4.356251 | 8.862283 | 0.002911 | 0.040547 | SLC35C1 |
| -2.23329 | 1.518988 | 8.857552 | 0.002919 | 0.040593 | MCTP1 |
| 1.954016 | 2.975155 | 8.854461 | 0.002924 | 0.040612 | GPER1 |
| -1.68402 | 2.673277 | 8.839168 | 0.002948 | 0.040916 | PALMD |
| -1.2511 | 3.356428 | 8.834167 | 0.002956 | 0.040992 | NOP14-AS1 |
| 1.645256 | 6.274728 | 8.83097 | 0.002962 | 0.040994 | TRIM2 |
| 1.898752 | 6.92785 | 8.82951 | 0.002964 | 0.040994 | TRGC1 |
| -3.93141 | 2.687652 | 8.806468 | 0.003002 | 0.041384 | LGI2 |
| -3.16707 | 3.860107 | 8.805369 | 0.003003 | 0.041384 | GHR |
| -1.07508 | 5.696135 | 8.790099 | 0.003029 | 0.041695 | SEPT8 |
| -1.81196 | 0.499913 | 8.7681 | 0.003065 | 0.042086 | OTUD7A |
| -1.02038 | 3.177471 | 8.766554 | 0.003068 | 0.042086 | PARP3 |
| -1.79717 | 5.959167 | 8.752759 | 0.003091 | 0.042368 | GABBR1 |
| -1.91606 | 2.310414 | 8.75064 | 0.003095 | 0.04238 | CYP46A1 |
| -2.56533 | 1.328808 | 8.743226 | 0.003108 | 0.042433 | AC226118.1 |
| 1.054822 | 1.470941 | 8.741533 | 0.00311 | 0.042433 | AC067852.3 |
| -1.0399 | 4.815305 | 8.74117 | 0.003111 | 0.042433 | ALMS1 |
| -1.97992 | 1.054023 | 8.736819 | 0.003118 | 0.042448 | ANKRD18CP |
| -2.05722 | 1.583892 | 8.731636 | 0.003127 | 0.042485 | KIF26B |
| -2.56304 | 1.087276 | 8.729375 | 0.003131 | 0.0425 | DLL1 |
| -2.89657 | 1.751634 | 8.724952 | 0.003139 | 0.042566 | LINC01133 |
| 2.173181 | 1.385928 | 8.721229 | 0.003145 | 0.042616 | AK5 |
| -1.27237 | 2.980176 | 8.715956 | 0.003154 | 0.042701 | RHOG |
| -1.25084 | 4.652129 | 8.704132 | 0.003175 | 0.042867 | CPT2 |
| -2.42995 | 1.90217 | 8.698389 | 0.003185 | 0.042964 | AC139795.1 |
| -1.28384 | 6.292271 | 8.692988 | 0.003194 | 0.043054 | HOOK2 |
| 2.286828 | 1.566277 | 8.675021 | 0.003226 | 0.043389 | C 12orf74 |
| -1.02685 | 6.134278 | 8.672911 | 0.00323 | 0.043389 | TSPYL1 |
| -4.87666 | 7.289967 | 8.670377 | 0.003234 | 0.043389 | CYP1B1 |
| -1.22866 | 1.849693 | 8.669375 | 0.003236 | 0.043389 | PCSK4 |
| 1.865064 | 1.107443 | 8.665866 | 0.003242 | 0.043434 | AL031727.1 |
| -1.28006 | 4.158969 | 8.657463 | 0.003257 | 0.043498 | POMT2 |
| 1.513882 | 6.856655 | 8.657184 | 0.003258 | 0.043498 | MUC20-OT1 |
| -1.37437 | 5.069993 | 8.655571 | 0.003261 | 0.043498 | PIP5K1C |
| -1.06034 | 4.756458 | 8.65274 | 0.003266 | 0.043522 | MRPL18 |
| -2.17876 | 2.878032 | 8.650183 | 0.00327 | 0.043529 | AC104825.1 |
| -1.06695 | 2.536776 | 8.638587 | 0.003291 | 0.043699 | BLOC1S4 |
| -3.31973 | 4.100309 | 8.637906 | 0.003292 | 0.043699 | NRSN2 |
| 1.897838 | 7.574682 | 8.632488 | 0.003302 | 0.043747 | HACD2 |
| 1.579616 | 0.796156 | 8.628079 | 0.00331 | 0.043813 | ASB9P1 |
| 1.642349 | 2.554562 | 8.613308 | 0.003337 | 0.044019 | ADCY10P1 |
| 1.280671 | 0.640389 | 8.608196 | 0.003347 | 0.044105 | AC002310.1 |
| -2.1217 | 3.753453 | 8.601688 | 0.003359 | 0.044225 | DIPK2A |
| 3.25951 | 5.114954 | 8.597186 | 0.003367 | 0.044257 | MUC15 |
| -1.29927 | 5.015285 | 8.59549 | 0.00337 | 0.044257 | SEC24A |
| 1.222373 | 0.531605 | 8.59405 | 0.003373 | 0.044257 | AC011479.3 |
| 1.364443 | 1.797514 | 8.592615 | 0.003375 | 0.044257 | UBE3D |
| 1.076416 | 2.420585 | 8.590229 | 0.00338 | 0.044277 | RYR3 |
| 1.024957 | 2.135795 | 8.57422 | 0.00341 | 0.04456 | AC007114.2 |
| -1.17231 | 3.26659 | 8.570987 | 0.003416 | 0.04456 | PHF11 |
| 1.163153 | 4.38497 | 8.570687 | 0.003416 | 0.04456 | CNKSR1 |
| -1.19598 | 2.70534 | 8.569197 | 0.003419 | 0.04456 | TOE1 |
| 1.136856 | 0.443765 | 8.568929 | 0.003419 | 0.04456 | AC138696.2 |
| -1.39647 | 3.81148 | 8.567867 | 0.003421 | 0.04456 | MAP4K2 |
| -1.07648 | 2.284409 | 8.563171 | 0.00343 | 0.044637 | AC010422.8 |
| -2.17533 | 3.917088 | 8.558139 | 0.00344 | 0.044723 | ENTPD2 |
| -2.97423 | 2.572865 | 8.552145 | 0.003451 | 0.044764 | RNF217 |
| 1.440165 | 0.908603 | 8.55166 | 0.003452 | 0.044764 | AC104667.2 |
| -1.26988 | 4.748533 | 8.548832 | 0.003457 | 0.044764 | RWDD1 |
| 1.069003 | 1.673387 | 8.546143 | 0.003463 | 0.044793 | Z95115.1 |
| 1.024738 | 3.781777 | 8.541721 | 0.003471 | 0.044793 | AL135999.1 |
| -2.02883 | 1.47562 | 8.534477 | 0.003485 | 0.044819 | PCDHGA1 |
| -2.20574 | 2.687841 | 8.533609 | 0.003486 | 0.044819 | SYCP3 |
| -1.81187 | 6.375115 | 8.533373 | 0.003487 | 0.044819 | NCAPD3 |
| -1.08182 | 4.191128 | 8.533361 | 0.003487 | 0.044819 | ZNF510 |
| -1.40067 | 3.753206 | 8.532928 | 0.003488 | 0.044819 | PIP4K2A |
| -1.49096 | 5.598024 | 8.528854 | 0.003496 | 0.044882 | PAQR6 |
| -1.47275 | 4.28076 | 8.522245 | 0.003508 | 0.045008 | JUNB |
| 2.085026 | 2.048899 | 8.518517 | 0.003516 | 0.045063 | AC018714.2 |
| -1.44543 | 5.734586 | 8.516199 | 0.00352 | 0.045078 | NIN |
| -1.70626 | 3.508769 | 8.514861 | 0.003523 | 0.045078 | KIAA1211 |
| 1.731116 | 2.872577 | 8.512322 | 0.003527 | 0.045104 | NEB |
| 1.553004 | 2.396269 | 8.510439 | 0.003531 | 0.045113 | RP11-152F13.7 |
| 1.158059 | 2.136873 | 8.503473 | 0.003545 | 0.045204 | MYO1F |
| -1.32176 | 5.775675 | 8.502036 | 0.003547 | 0.045204 | ENTPD5 |
| 2.176797 | 4.916862 | 8.500385 | 0.003551 | 0.045204 | MYLK |
| 2.425765 | 1.830557 | 8.499266 | 0.003553 | 0.045204 | BCL2L15 |
| 1.972191 | 5.092142 | 8.497646 | 0.003556 | 0.045207 | S1PR3 |
| -1.75733 | 0.456836 | 8.486195 | 0.003579 | 0.045455 | APOM |
| 1.169887 | 1.040115 | 8.484328 | 0.003582 | 0.045465 | AC018628.2 |
| -1.345 | 5.141987 | 8.47901 | 0.003593 | 0.04556 | TMX3 |
| 1.000568 | 5.503607 | 8.464942 | 0.003621 | 0.045873 | STARD3NL |
| -1.17334 | 4.085529 | 8.463576 | 0.003623 | 0.045873 | ANKEF1 |
| 1.138894 | 5.808501 | 8.44157 | 0.003667 | 0.046129 | TMEM184C |
| -6.00924 | 5.17095 | 8.437612 | 0.003675 | 0.046162 | CEL |
| -3.40445 | 3.36859 | 8.437327 | 0.003676 | 0.046162 | CEP126 |
| -1.41429 | 3.411043 | 8.424695 | 0.003702 | 0.046396 | HPS5 |
| -2.00098 | 6.212064 | 8.423681 | 0.003704 | 0.046396 | ENDOD1 |
| 1.604476 | 1.765653 | 8.4209 | 0.003709 | 0.046423 | AL713852.1 |
| -1.40131 | 4.135602 | 8.416729 | 0.003718 | 0.046423 | PEX10 |
| -2.34137 | 2.972741 | 8.413757 | 0.003724 | 0.04646 | GPR161 |
| 1.048579 | 1.8578 | 8.410902 | 0.00373 | 0.04646 | AC024560.4 |
| -2.21075 | 2.728126 | 8.405726 | 0.00374 | 0.046552 | MIR22HG |
| 1.086234 | 5.637666 | 8.403904 | 0.003744 | 0.046552 | ZXDC |
| -1.56983 | 3.392768 | 8.402908 | 0.003746 | 0.046552 | DUSP8 |
| 1.253018 | 5.949717 | 8.401258 | 0.00375 | 0.046557 | PALLD |
| -1.77821 | 5.159831 | 8.395927 | 0.003761 | 0.046626 | ATR |
| -1.28881 | 0.702134 | 8.394243 | 0.003764 | 0.046626 | HIST1H2AM |
| -1.31952 | 4.473019 | 8.390696 | 0.003771 | 0.046626 | BTBD3 |
| 1.075063 | 5.36593 | 8.389798 | 0.003773 | 0.046626 | MTFR1L |
| -2.03664 | 0.453478 | 8.382282 | 0.003789 | 0.046782 | BEGAIN |
| -1.00093 | 4.124205 | 8.369969 | 0.003815 | 0.047025 | AHCYL2 |
| -1.20085 | 1.272192 | 8.352205 | 0.003852 | 0.047373 | SRXN1 |
| 2.189764 | 1.942954 | 8.344189 | 0.003869 | 0.047536 | AL356056.2 |
| -1.76615 | 2.933863 | 8.342287 | 0.003873 | 0.047536 | NDN |
| -1.62419 | 6.944278 | 8.32126 | 0.003918 | 0.047811 | UGDH |
| 1.037562 | 6.092255 | 8.320243 | 0.003921 | 0.047811 | JRK |
| -1.23997 | 5.591038 | 8.315958 | 0.00393 | 0.047811 | NACC1 |
| 1.14565 | 1.984294 | 8.313779 | 0.003935 | 0.047811 | RIMKLBP2 |
| -1.49601 | 1.225319 | 8.310158 | 0.003942 | 0.047811 | TTC4 |
| 1.02219 | 2.749625 | 8.309838 | 0.003943 | 0.047811 | RP11-231C14.4 |
| 1.101238 | 0.899488 | 8.308955 | 0.003945 | 0.047811 | PRMT5-AS1 |
| -1.08999 | 4.552457 | 8.306257 | 0.003951 | 0.047811 | MIS18BP1 |
| -1.36519 | 4.942971 | 8.304089 | 0.003956 | 0.047811 | IL4R |
| 1.702641 | 0.93194 | 8.301109 | 0.003962 | 0.047811 | AC117382.1 |
| -5.6633 | 4.696558 | 8.291247 | 0.003984 | 0.047959 | PLTP |
| -2.79186 | 1.450399 | 8.285678 | 0.003996 | 0.048055 | MCUB |
| -2.03982 | 2.615426 | 8.284812 | 0.003998 | 0.048055 | EMP1 |
| 1.825483 | 1.35623 | 8.277638 | 0.004014 | 0.048147 | FAM225A |
| -2.26812 | 4.838547 | 8.276297 | 0.004017 | 0.048147 | ZNF518B |
| -1.80451 | 3.991867 | 8.275312 | 0.004019 | 0.048147 | CNTLN |
| 1.98905 | 1.812965 | 8.273024 | 0.004024 | 0.048147 | GRAMD2A |
| -3.50083 | 1.500715 | 8.271344 | 0.004028 | 0.048147 | AC124045.1 |
| -1.17331 | 3.719043 | 8.270535 | 0.004029 | 0.048147 | GSKIP |
| 1.371308 | 6.383371 | 8.265307 | 0.004041 | 0.048221 | ASAP1 |
| -1.02864 | 4.618141 | 8.264476 | 0.004043 | 0.048221 | DESI2 |
| 1.523188 | 3.87552 | 8.261671 | 0.004049 | 0.048258 | WDR97 |
| -1.03379 | 5.683006 | 8.250328 | 0.004074 | 0.04849 | NSMAF |
| -1.80718 | 3.13537 | 8.247379 | 0.004081 | 0.04849 | ID2 |
| -3.36431 | 5.716391 | 8.241783 | 0.004094 | 0.048587 | LRRN1 |
| -1.17376 | 5.459112 | 8.240655 | 0.004096 | 0.048587 | MORC4 |
| 1.004141 | 0.547977 | 8.239548 | 0.004099 | 0.048587 | AL391095.3 |
| 1.693669 | 1.50325 | 8.236355 | 0.004106 | 0.048635 | AC069547.1 |
| -2.85337 | 2.778355 | 8.228453 | 0.004124 | 0.048735 | BMP8B |
| 1.642135 | 0.801193 | 8.226319 | 0.004129 | 0.048741 | AC005899.7 |
| 1.758631 | 0.195034 | 8.219573 | 0.004144 | 0.048827 | AC096741.1 |
| -1.15732 | 5.276258 | 8.219087 | 0.004145 | 0.048827 | LNPEP |
| -1.23299 | 3.634122 | 8.218111 | 0.004147 | 0.048827 | TP53I3 |
| 1.64952 | 4.644138 | 8.210901 | 0.004164 | 0.048949 | DZIP1 |
| -1.12474 | 4.25041 | 8.210811 | 0.004164 | 0.048949 | CAMLG |
| -1.02504 | 4.707904 | 8.205497 | 0.004176 | 0.048974 | JKAMP |
| -1.03264 | 3.611001 | 8.204381 | 0.004179 | 0.048974 | GINS1 |
| -1.03387 | 5.759232 | 8.202934 | 0.004182 | 0.048975 | HDAC6 |
| -1.41455 | 1.798832 | 8.201609 | 0.004185 | 0.048975 | RNASEH2A |
| -1.40674 | 1.969168 | 8.199821 | 0.004189 | 0.048986 | HIST2H2BD |
| -1.25785 | 4.833383 | 8.189763 | 0.004213 | 0.049146 | NBPF15 |
| 1.49741 | 2.877519 | 8.187037 | 0.004219 | 0.049146 | CSPG4P9 |
| -2.04375 | 3.389099 | 8.184193 | 0.004226 | 0.04916 | CCL28 |
| 1.180642 | 2.99348 | 8.183797 | 0.004227 | 0.04916 | LRP2BP |
| -1.9422 | 3.785306 | 8.179063 | 0.004238 | 0.049251 | AP1S2 |
| -3.54368 | 3.398933 | 8.173297 | 0.004251 | 0.049371 | AC010624.3 |
| -1.12762 | 3.798317 | 8.167198 | 0.004265 | 0.049444 | SUSD1 |
| -1.06794 | 5.668585 | 8.16652 | 0.004267 | 0.049444 | ZADH2 |
| 1.793988 | 0.802514 | 8.162767 | 0.004276 | 0.04951 | CHAD |
| -1.02177 | 6.238164 | 8.155187 | 0.004294 | 0.049654 | SIN3A |
| -3.48308 | 3.214578 | 8.152538 | 0.0043 | 0.049654 | XDH |
| -1.57869 | 3.210051 | 8.147317 | 0.004312 | 0.049747 | EHHADH |
| 1.366408 | 1.596867 | 8.138888 | 0.004333 | 0.049941 | AC110285.6 |
| -2.40491 | 1.485182 | 8.137393 | 0.004336 | 0.049945 | SMC1B |
| -8.63808 | 9.252364 | 8.136003 | 0.004339 | 0.049946 | VIM |

**Table 6. Summary of drugs search return from EnrichR-L1000 Chem Perturbation Down using upregulated genes in PDX models derived from AA/P non-responders.**

| Drug Name | Number of L1000 Signature | Rank Score | Number Gene Target | Gene targets |
|---|---|---|---|---|
| torin-1 | 14 | 467.2906672 | 50 | CCNA2;CCNB2;SATB1;PRC1;CDK1;CKS2;TACC3;PLIN2;MEX3D;DLGAP5;LMNB1;DNMT1;R RM1;NCAPG;SMC4;SKA1;CCNB1;KIF4A;ECT2;MAD2L1;NET1;SCD;UCP2;ENPP1;SLC1A4;D HCR24;FANCI;IDI1;HSPA14;SMC2;TIMELESS;STC2;FDPS;P4HA2;INSIG1;CYP51A1;LDLR;A BCC3;CHD7;SIRT3;ALDH9A1;IL13RA1;KPNA2;STIL;ANXA3;ARHGAP29;FBXO5;DUSP5;TFF 3;CD44 |
| palbociclib | 52 | 459.8153277 | 118 | ABCC3;SCP2;ALMS1;ACOX3;KDSR;CHST2;IGF2R;HK1;ACAT1;FANCI;RRM1;NCAPG;SMC4 ;SMC2;CCNA2;PRC1;KIF4A;CDK1;TACC3;ECT2;DLGAP5;MAD2L1;CCNB2;PTPRN2;TLE1;S CD;TFF3;FSTL1;CCNB1;HFE;IGFBP3;CHD7;AAK1;ADCY2;ABHD5;CD44;ALDH9A1;FAM171 A1;TMEM109;HAUS3;CHPT1;EPM2A;RNH1;SATB1;CYPS1A1;FSTL3;DNAJC6;CREB3L1;ST3 GAL6;ATP9B;AP1M2;NET1;CKS2;KEAP1;MGAT2;LMNB1;ANXA3;DCBLD2;SLBP;PPIF;TIM ELESS:CPM;P4HA2;SHMT1;INSIG1;SLIT2;PGM1;PPIC;DHCR24;KPNA2;CNN3;FBXO5;ENPP1 ;ARHGAP29;LDLR;MSRA;RAB25;GOLM1;FAM57A;USP22;ULK2;FMOD;MPDU1;IDI1;MBP;H SPA14;PCMT1;DNAJC1;CADM4;DTNA;FAT1;FDPS;COPS3;TIPARP;DNASE2;ERG;PLP2;CDC 40;MCTP1;DPYSL4;KATNA1;DUSP5;PAK6;ASCC3;ATF5;SATB2;MSX1;ST6GAL1;RELN;PLA 2G2A;GALNT1;SYNE2;SOD2;STIL;SKA1;DNMT1;CDH3;SLC1A4 |
| mitoxantrone | 35 | 458.3696921 | 86 | CCNA2;IDI1;TLE1;TIPARP;USP22;CKS2;RRAS2;ARHGAP29;SMC4;DLGAP5;PRC1;CDK1;TA CC3;KPNA2;ECT2;HSPA14;FSTL1;LMNB1;MAD2L1;ANXA3;NCAPG;NEFH;PAM;PPIC;FANC I;RRM1;PTPRN2;SMC2;CCNB2;KIF4A;PPIF;TIMELESS;DHCR24;SCP2;TSPAN6;ADCY2;BRC A2;AP1M2;ALDH9A1;GOLM1;SKA1;TFF3;KHDRBS3;SLC1A4;FBXO5;DNAJC1;RNH1;CTDP1 ;CADM4;CHD7;IL13RA1;EPM2A;ACHE;CD164;HOOK1;ST3GAL6;IQGAP1;ATF5;IGF2R;DUS P5;FAM57A;CD44;DCLK1;CNN3;SATB1;CYPS1A1;STC2;CCNB1;DCBLD2;NET1;SATB2;CHS T2;JAG1;IGFBP3;FAM171A1;HMGCS2;PHKA1;ROBO1;PGM1;MEX3D;TUBA1A;TMEM109;IN SIG1;SCD;TES;GALNT1 |
| WYE-125132 | 12 | 452.1770452 | 61 | FANCI;NET1;CCNA2;RRM1;PRC1;KIF4A;CHD7;CDK1;TACC3;DLGAP5;LMNB1;FDPS;HFE;A RAP2;ENPP1;ABHD5;BRCA2;AP1M2;FSTL3;DUSP5;CCNJ;TIPARP;INSIG1;RRAS2;CHD1;SIR T3;SCAF8;BAG5;MAFB;TFF3;CHST2;IDI1;TIMELESS;CKS2;KPNA2;SMC2;MAD2L1;IGFBP3; FAM57A;PLA2G2A;HSPA14;CNN3;CCNB2;SMC4;PPIC;SLBP;DNMT1;NCAPG;FSTL1;CCNB1; ATF5;SLC1A4;ECT2;PPIF;ANXA2;TLE1;SIAH2;RAB25;PALMD;GEMIN4;PGM1 |
| OSI-027 | 10 | 438.5474096 | 50 | FANCI;SMC4;SMC2;CCNA2;CCNB2;SCP2;PRC1;TSPAN6;KIF4A;TIMELESS;CDK1;TACC3;E CT2;DLGAP5;MAD2L1;ABCC3;CPM;RELN;INSIG1;STC2;CREB3L1;TFF3;USP22;CKS2;CRYM ;MPZL1;LMNB1;NCAPG;ARHGAP29;FBXO5;STIL;DTNA;INF2;MFSD1;SOD2;SLC29A1;IDH3 A;DUSP5;ANXA3;IGFBP3;DCBLD2;NET1;CCNB1;IDI1;RRM1;SLC1A4;MBP;ATF5;DNMT1;K PNA2 |
| ZSTK-474 | 10 | 436.973607 | 33 | FANCI;RRM1;NCAPG;SMC4;SMC2;LMNB1;CCNA2;CCNB2;PRC1;KIF4A;CDK1;CKS2;TACC 3;ECT2;DLGAP5;MAD2L1;IDI1;ANXA3;RRAS2;ARHGAP29;STIL;DERA;DUSP5;FBXO5;DHC R24;TIMELESS;SCD;INSIG1;PLA2G2A;KPNA2;TMEM109;NUP98;CNN3 |
| MK-2206 | 5 | 432.0322581 | 31 | SCD;P4HA2;IGFBP3;INSIG1;CYP51A1;RRAS2;DHCR24;LDLR;CD44;SATB1;TFF3;CCNA2;RR M1;PRC1;PPIF;CDK1;CKS2;TACC3;NCAPG;KPNA2;DLGAP5;MAD2L1;FANCI;SLC1A4;SMC2 ;LMNB1;TIMELESS;MSX1;NET1;IDI1;CNN3 |
| PI-103 | 11 | 424.3463605 | 44 | FANCI;RRM1;NCAPG;SMC4;CRYZ;CCNA2;CCNB2;TUBA1A;CKS2;ATF5;ECT2;DLGAP5;MA D2L1;CTBP1;GOLM1;LMNB1;SLC25A15;PRC1;KIF4A;CDK1;PPIF;TIMELESS;CHPT1;DUSP5; IDI1;KHDRBS3;ANXA3;FAM57A;RRAS2;MEX3D;CDH3;FAT1;TACC3;SMC2;FBXO5;KPNA2; NUCB2;NET1;CCNB1;ENPP1;SCD;CYP51A1;DHCR24;LDLR |
| GSK-2126458 | 17 | 423.0243151 | 63 | FANCI;RRM1;NCAPG;SMC4;LMNB1;CCNA2;CCNB2;PRC1;KIF4A;TIMELESS;CDK1;TACC3; KPNA2;DLGAP5;MAD2L1;GHR;SATB1;SLC1A4;ADCY2;AP1M2;PPIC;FBXO5;MEX3D;SLC29 A1;SMC2;NET1;TFF3;CKS2;NUP98;SLIT2;DUSP5;TIPARP;INSIG1;KEAP1;RRAS2;SOD2;BAG 5;MAFB;PPIF;PLIN2;ANGPTL4;LDLR;CHST2;DNMT1;ATF5;GPR37;ANXA3;STIL;DHCR24;S KA1;CNN3;ECT2;IDI1;HSPA14;CCNB1;SCD;TUBA1A;ARHGEF6;IGFBP3;ARHGAP29;TMEM1 09;PLA2G2A;CYP51A1 |
| HG-14-8-02 | 1 | 423 | 8 | IDI1;MAFB;ANXA3;SCD;TACC3;ARHGAP29;DHCR24;KPNA2 |
| foretinib | 4 | 422 | 29 | FANCI;DUSP5;ANXA3;IGFBP3;NCAPG;SMC4;NET1;CCNA2;PRC1;CDK1;CKS2;TACC3;KPN A2;ECT2;DLGAP5;MAD2L1;STIL;DHCR24;SMC2;CNN3;LMNB1;KIF4A;TIMELESS;HSPA14; CCNB1;ORC1;CCNB2;TLE1;CHPT1 |
| AZD-8055 | 9 | 421.4246575 | 34 | CCNA2;RRM1;ANXA3;PRC1;CDK1;CKS2;TACC3;NCAPG;SMC4;NEFH;DLGAP5;MAD2L1;ID I1;SCD;CYP51A1;SLC1A4;DHCR24;ALOX15B;CCNB2;CCNB1;KIF4A;LMNB1;NUP98;HSPA14 ;SMC2;PPIF;KPNA2;FANCI;TIMELESS;SKA1;FBXO5;MBP;MSX1;ECT2 |
| rocilinostat | 1 | 419 | 15 | NCAPG;MEX3D;FSTL1;LMNB1;CCNA2;PRC1;KIF4A; TIMELESS;CDK1;TACC3; TMEM109;FB XO5;DLGAP5;PGM1;MAD2L1 |
| radicicol | 4 | 416 | 31 | CCNA2;CCNB2;PRC1;CKS2;CDK1;TACC3;NCAPG;DHCR24;LMNB1;MAD2L1;KIF4A;FBXO5; SKA1;DLGAP5;TNFAIP8;CADM4;ENPP1;DNASE2;BRCA2;BAHCC1;ALDH9A1;CRYZ;CNN3; FANCI;DNMT1;STIL;RRM1;IGFBP3;SMC2;ORC1;TIMELESS |
| dovitinib | 9 | 391.7495108 | 44 | NET1;CCNA2;ANXA3;CDK1;TACC3;ARHGAP29;ECT2;SMC4;CD44;LMNB1;DCBLD2;MAD2 L1;KANK2;IDI1;CCNB2;SCD;PRC1;PPIC;GPR37;CYP51A1;PPIF;TFF3;DLGAP5;FANCI;IGFBP 3;NCAPG;VLDLR;ATF5;SMC2;DNMT1;RRM1;KIF4A;TIMELESS;FBXO5;SLC25A15;CCNB1; CKS2;MAFB;PLA2G2A;DHCR24;KPNA2;HSPA14;CNN3;NUCB2 |
| fostamatinib | 1 | 386 | 12 | FANCI;CCNA2;DNMT1;PRC1;TIMELESS;CDK1;TACC3;NCAPG;SMC4;DLGAP5;SMC2;MAD2 L1 |
| PHA-793887 | 16 | 382.3664454 | 50 | CCNA2;FANCI;CCNB2;PRC1;CDK1;TACC3;KPNA2;SMC4;DLGAP5;PPIC;RRM1;ANXA3;NCA PG;SMC2;MAD2L1;INSIG1;SHMT1;LMNB1;STC2;KIF4A;TIMELESS;IGFBP3;HSPA14;FSTL1; CCNB1;DNMT1;CKS2;IDI1;DUSPS5;TIPARP;ARHGAP29;DHCR24;LDLR;CNN3;SCD;CYP51A1 ;PAK6;NEFH;FAM57A;FBXO5;PLP2;ECT2;PTPRN2;KEAP1;ATF5;NET1;PALMD;SLC29A1; MBP;STIL |
| WZ-4-145 | 7 | 378.1259843 | 39 | CCNA2;PRC1;PPIF;CKS2;CDK1;NCAPG;NUP98;KPNA2;DLGAP5;MAD2L1;FANCI;SLBP;RR M1;IGFBP3;SMC4;SMC2;ORC1;HMGCS2;PPIC;NET1;ANXA3;KIF4A;TACC3;RNF4;ECT2;DH CR24;KIAA0232;HSPA14;FSTL1;SEL1L3;LMNB1;SCD;CCNB2;CCNB1; TIMELESS; FBXO5;STIL ;CNN3;VLDLR |
| gefitinib | 2 | 372.3333333 | 19 | CCNA2;CDK1;CKS2;TACC3;NCAPG;PDE8B;KPNA2;HSPA14;SMC4;DLGAP5;PPIC;MAD2L1; FANCI;IDI1;RRM1;LMNB1;PRC1;KIF4A;PPIF |
| geldanamycin | 3 | 369.1428571 | 23 | FANCI;STIL;NCAPG;SMC4;CCNA2;CCNB2;PRC1;TIMELESS;CDK1;TACC3;PLIN2;FBXO5; ECT2;CRYM;DLGAP5;MAD2L1;RRM1;CCNB1;KIF4A;KPNA2;CDH3;STC2;SKA1 |

The gene targets potentially modulated by these four drugs were examined from the L1000 signatures. These drugs shared highly similar profile of gene expression modulation. Using patient's RNAseq data as input, 89 DEGs are modulated by at least one of the four drugs, among them, 50 genes were shared among all drugs (Figure 5A). Meanwhile, 20 genes were shared among all drugs out of 87 DEGs targeted by at least one drugs using PDX RNAseq data as input (Figure 5B). Combining the overlapped genes from patient and PDX, 11 genes were identified that were shared between the two datasets as commonly targeted genes by all 4 drugs (Figure 3D, Figure 5C). Of these 11 genes (CCNA2, CCNB1, CCNB2, PRC1, SMC2, DLGAP5, ECT2, FBXO5, CDK1, NCAPG, KIF4A), all except 1 gene (PRC1) were significantly upregulated in AA/P non-responders from all metastatic sites in the PROMOTE patient data (Figure 3E), and all were significantly upregulated in PDX tumors derived from AA/P non-responders (Figure 3F). The 11 genes mostly belonged to the mitotic and G2M checkpoint pathways (Figure 4B). The efficacy of these 4 drugs, and the effect of these drugs on 11 gene expression, was experimentally tested.

### Identified drugs sensitized abiraterone treatment and reversed Abi-resistant expression profile

To examine whether the four identified drugs can sensitize abiraterone treatment in prostate cancer, Abi resistant prostate cancer cell models in 22Rv1 and LNCaP (22Rv1-AbiRes and LNCap-AbiRes) were generated. The resistant phenotypes were tested using the cytotoxicity assay (Figure 1A). The expression of AR, AR variants V7 and del567es, and AR regulated hallmark genes, such as TMPRSS2, FKBP5, NKX3.1, and PSA were also increased in the resistant cells compared to the parental lines (Figure 1B), so as the 11 genes potentially modulated by the four identified drugs (Figure 1C). To test the therapeutic effects of the identified drugs, both parental and resistant cell lines were treated with abiraterone in the presence and absence of the four drugs. Concentrations corresponding to IC50 of each drug, as determined by single treatment preliminary experiments except for PD-0325901 in LNCaP cells which harbors a Q56P mutation, conferring resistance to this MEK inhibitor, were used in the experiment. As shown in Figure 6A-B, the four drugs significantly reversed the resistant phenotype when combined with Abi in both parental and Abi-resistant 22RV1 cells, and the effect was more prominent in the resistant cell lines. In LNCaP cell, all except for PD-0325901 when combined with Abi, dramatically decreased viability in the resistant cell lines while the effect was not as significant in the parental Abi sensitive cells. The sensitization effects of the identified drugs were further tested in PDX derived organoids in a dose-dependent fashion. MC-PRX-01 (Figure 6C) and MC-PRX-05 (Figure 6D) PDX models were derived from AA/P non-responder pre-treatment tumors. The two organoids showed Abi resistant phenotype with less than 10% inhibition of organoid growth by abiraterone alone at the highest tested concentration. However, the four drugs all dramatically sensitized the organoids to abiraterone, shown as reduction in organoid viability in a dose-dependent fashion. Among the four, mitoxantrone was superior to others, given the range of doses tested in this experiment.

Expression of the 11 potential drug targeting genes was further validated using qRT-PCR in parental and Abi-resistant prostate cancer lines as well as in the PDX derived organoids. As shown in Figure 7, the expression of the 11 genes was generally suppressed after treatment of the four drugs either alone or in combination with Abi. On the other hand, the four drugs exhibited different effects on the expression modulation of these 11 genes. Among the four drugs, mitoxantrone most consistently suppressed the expression of the 11 genes in both 22Rv1 parental and AbiRes cells (Figure 7A-B), as well as the two organoid models (Figure 7E-F). It also exhibited better suppression effects in LNCaP Abi-resistant cells compared to that of parental cells (Figure 7C-D). Palbociclib also consistently reduced the expression of the 11 target genes in 22Rv1, LNCaP and MC-PRX-01, while less effective in the other organoid model. The other two drugs, PD-0325901 and PHA-793887, showed less consistency in the suppression of these 11 genes, especially in LNCaP which harbors a MEK Q56P mutant, resulting in resistance to MEK inhibitor in this case.

### TOP2 inhibitors sensitized abiraterone in Abi-resistant PDX models

Among the four drugs, mitoxantrone, is currently the only FDA approved drug for CRPC patients. It was the most potent in the cytotoxicity experiments and showed more significant effects on the 11 gene suppression (Figure 6 and Figure 7). Therefore, the efficacy of mitoxantrone, as well as doxorubicin, another TOP2 inhibitor commonly used in the treatment of breast cancer, was further evaluated either alone or combined with abiraterone in two PDX models derived from AA/P non-responder patient samples. MC-PRX-01 was derived from a pre-treatment sample (Figure 8A-B), and MC-PRX-06 was derived from a post-treatment sample (Figure 8D-E). These two models were used to test the effect of TOP2 inhibitors in both Abi primary resistant and acquired resistant settings. Both PDX models responded minimally to Abi treatment alone. On the other hand, mitoxantrone/doxorubicin treatment alone were able to decrease the tumor growth by more than 50%. Abi and TOP2 inhibitor combination treatments almost completely abolished tumor growth in 8 out of 10 mice in MC-PRX-01, and in 3 out of 8 mice in MC-PRX-06. In all cases, no significant toxicities were observed as evidenced by consistent mice body weight ((Figure 8C and 8F).

The expression of the 11 drug targeting genes in the PDX tumors harvested after various treatments was further examined. Expression of the 11 genes was significantly downregulated after drug treatment in both mitoxantrone and doxorubicin treated tumors (Figure 8G and 8H), consistent with the effect of TOP2 inhibitor on the 11 gene expression in cell lines and organoids.

### 11 gene panel as biomarkers for individualized treatment

The 11 genes were upregulated in AA/P non-responders' tumor samples and PDX models derived from AA/P non-responders (Figure 3E-F). Whether these genes can be used as predictive markers associated with AA/P resistance and, furthermore, whether patients with these markers might have poor prognosis, and could benefit from alternative therapies such as mitoxantrone, was evaluated.

Unsupervised machine learning by k-means clustering analysis was performed using the 11 genes on 68 PROMOTE baseline patient samples from all biopsy sites (Figure 9A, top panel). Based on the elbow method, the patient can be best clustered into 3 subgroups (Figure 10). One of the clusters exhibited apparently higher expression, based on the sum of z-scores of the 11 genes of that cluster compared to the other two clusters. This cluster was designated as the "high-expression cluster", which included the samples from which the AA/P non-responding PDX models were derived. The other two clusters were collectively referred to as "low-expression cluster". Moreover, the high-expression cluster identified by the gene panels was associated with significantly poor overall survival (OS) with a p-value of 0.00164 (Figure 9A, middle panel). A similar finding was also observed using another prognostic endpoint, time to treatment change (TTTC) (Figure 9A, bottom panel), with a p-value of 0.017. Similar clustering and Kaplan-Meier analysis were performed on biopsy samples from bone metastasis (Figure 11).

A second independent cohort, the Stand Up To Cancer (SU2C) was also analyzed (see, for example, Abida et al., Proceedings of the National Academy of Sciences 116, 11428-11436 (2019)). The trial has two treatment arms, enzalutamide and abiraterone. To best resemble the PROMOTE cohort, the Abi-naïve biopsy samples from the Abi treatment arm that had overall survival data were used (53 samples, Table 7). Using the 11 gene panel and clustering strategy that was applied in the PROMOTE cohort, the expression data of the SU2C cohort can also be clustered into high and low expression subgroups. The patients in high expression cluster exhibited worse survival outcome as compared with the low expression clusters with a p-value of 0.0208 (Figure 9B). In order to examine the possible prognostic prediction values of the gene panel in the primary tumor settings, the TCGA prostate cancer cohort was further examined. Due to the low mortality rate in primary prostate cancer, progression-free survival (PFS) was used as the clinical outcome for evaluation. Interestingly, the gene panel also showed that patients in the high expression subgroup had worse PFS outcome in the TCGA cohort (Figure 9C). Moreover, the gene panel was evaluated in other TCGA cancer types, including breast cancer, cervical cancer, and colon cancer. None of these cancer types showed statistically significant associations between the expression clusters based on the 11 gene expression and the outcome (PFS), suggesting that this gene panel is more specifically related to prostate cancer prognosis (Figure 12).

**Table 7. SU2C sub-cohort included in this study.**

| SAMPLE_ID |
|---|
| TP_2077_Tumor |
| TP_2078_Tumor |
| SC_9137_Tumor |
| TP_2081_T |
| SC_9144_Tumor |
| SC_9171_Tumor |
| SC_9173_Tumor |
| MO_1499_T |
| MO_1510_T |
| SC_9176_T |
| SC_9146_T |
| SC_9195_T |
| MO_1553_T |
| SC_9203_T |
| MO_1084-Tumor |
| MO_1094-Tumor |
| SC_9011-Tumor |
| MO_1124-Tumor |
| SC_9026-Tumor |
| SC_9027-Tumor |
| SC_9039-Tumor |
| SC_9036-Tumor |
| SC_9037-Tumor |
| SC_9043-Tumor |
| MO_1176-Tumor |
| MO_1179-Tumor |
| MO_1184-Tumor |
| MO_1192-Tumor |
| SC_9050-Tumor |
| SC_9055-Tumor |
| SC_9056-Tumor |
| MO_1219-Tumor |
| SC_9057-Tumor |
| MO_1241-Tumor |
| SC_9065-Tumor |
| SC_9066-Tumor |
| SC_9068-Tumor |
| TP_2054-TM |
| SC_9080-TM |
| SC_9081-TM |
| SC_9082-TM |
| MO_1336-TM |
| MO_1337-TM |
| SC_9086-TM |
| SC_9089-TM |
| SC_9090_T |
| TP_2060-TM |
| TP_2064-TM |
| SC_9092-TM |
| MO_1410-Tumor |
| TP_2069_Tumor |
| SC_9121_Tumor |
| MO_1447_Tumor |

### Multivariate analysis of the 11 gene panel-expression clusters reveals its potential utility to predict outcomes and selection of alternative therapies

The effect of the gene panel in the context of other genomic alterations and clinical variables in the PROMOTE samples was examined using the Spearman correlation of the expression of the 11 genes with a number of clinical variables, including PSA and testosterone levels, as well as three widely used gene panels or scores that are known to be associated with prostate cancer progression, including AR activity score (20 genes) (see, for example, Hieronymus et al., Cancer Cell 10, 321-330 (2006)), cell cycle progression (CCP) (31 genes) (see, for example, Cuzick et al. The Lancet Oncology 12, 245-255 (2011)), and neuroendocrine prostate cancer (NEPC) (70 genes) (see, for example, Beltran et al., Nat. Med., 22, 298-305 (2016)). The 11 genes identified here were highly correlated with the CCP score (Figure 13A-B), despite the fact that only three genes (CDK1, DLGAP5, and PRC1) were shared between the CCP gene panel and the 11 gene panel identified here. Mann-Whitney test of CCP scores between high- and low-expression subgroup (Figure 9A) indicated a significant difference with p-values as low as 9.5x10⁻¹⁵ (Figure 13E). Compared to the low-expression group, the high-expression subgroup appeared to be associated with a higher proportion of non-bone tissue biopsy origin, a higher proportion of ETS fusions, and AR activity score, but not PSA, NEPC score or mutation burden (Figure 13C-I). However, the high-expression subgroup carried higher fraction of copy number variations (p-value=0.024), indicating increased genomic instability that was likely responsible for the association with the worse outcome (Figure 13J). No mutations were identified in the 11 genes. However, gene gain and loss were observed essentially in all 11 genes, with three genes (CCNA2, CDK1, and KIF4A) most different between the high- and low-expression groups (Figure 13K).

The prognostic prediction values of the gene panels were further analyzed by COX proportional hazard models. Univariate analysis identified log-PSA as the only clinical variable associated with prognosis. Expression of AR-V7, CCP score and NEPC score were also identified to be significantly associated with overall survival as described elsewhere (see, for example, Cuzick et al., The Lancet Oncology, 12, 245-255 (2011); Beltran et al., Nat. Med., 22, 298-305 (2016); Hu et al., Cancer Res., 69, 16-22 (2009); and Sommariva et al., European Urology, 69, 107-115 (2016)). The 11 gene expression clusters, compared with other gene panels tested here, are more significantly associated with overall survival (Figure 14A) with a p-value < 0.005 and hazard ratio of 2.57 (95% CI 1.35-4.90). A multivariate COX model based on the 11 gene panel, AR activity score, CCP score, NEPC score, or log10(PSA) individually or pairwise combinations between any of the two was then built (Figure 14B). The expression of AR-V7 was not included because of missing data in half of the samples due to sample quality. A multivariate COX model was employed and evaluated the goodness of fit for COX using two statistics, p-values of log-likelihood ratio test and Akaike information criterion (AIC), which compares relative goodness-of-fit of the model but punish for overfitting with additional covariates that contribute minimally to the goodness-of-fit. Since the 11 gene panel-determined subgroups seemed to be independent of the PSA or NEPC (Figure 13G-H), combination of the 11 gene panel with either log10(PSA) and/or NEPC score both improved the model fitting. Moreover, the multivariate model that includes the 11 gene panel still out-performed the NEPC score or AR activity score when combined with the same covariates (Figure 14B). The performance between the CCP score and the 11 gene panel was similar. However, the CCP score fit better than the 11 gene panel when combined with log10(PSA) and/or NEPC score as covariates despite lower AIC.

Similar analyses were performed using the data from the Abi-treated SU2C cohort. As shown in Figure 14C, the 11 gene panel was significant with a p-value of 0.0263 and a hazard ratio of 2.77 (95% CI 1.13-6.80) in the univariate COX model. Except for the 11 gene panel, only the NEPC score was significantly associated with outcome. The NEPC score has been extensively validated in the SU2C cohort previously. In the SU2C cohort, patients within the 11-gene high cluster tend to have a high NEPC score in general when compared with patients within the low expression cluster (Mann-Whitney p-value 3.8×10-4), (Figure 15A-B). A similar association was also observed between the 11 gene expression cluster and the CCP score (Mann-Whitney p-value 7.5×10-12). Multivariate COX model in the SU2C cohort indicated that the 11 gene panel was slightly better to predict overall survival than the CCP score or AR activity score either alone or in combination with NEPC score Figure 14D.

### Example 2: CDK inhibitors and Abi resistance

All patient specimens were collected as part of the PROMOTE study as described elsewhere (see, for example, Champoux. Annu. Rev. Biochem., 70, 369-413 (2001); and Kellner et al., Lancet Oncol., 3, 235-243 (2002)). Briefly, pathology confirmed metastatic tumor biopsy tissues of mCRPC was renal capsule xenografted and 25 mg testosterone pellet was subcutaneously implanted into 6-8 weeks old male CB17 NOD-SCID mice (Jackson laboratories, Bar Harbor, Maine; Charles River Laboratories, Raleigh, North Carolina) and observed for at least 6 months or till the tumor reached 1.0-1.5 cm at maximal length, when sub-xenografts were expanded by subcutaneous implantation with harvested PDX tumor mixed with Matrigel (Corning, Corning, New York) with no exogenous testosterone supplied. Early generation PDX tumors were collected for cryo-storage and next-generation sequencing. Pathology confirmed PDX model generated from AA/P non-responder bone metastatic tumor biopsy tissues were employed to test the *in vivo* tumor response to abiraterone ± Mito/Dox/Palb/PHA. After PDX tumor reached approximately 100 mm³, mice were randomized to 6 groups. Each group of mice (n = 5) was administered, for 35 consecutive days, with vehicle control daily, 200 mg/kg abiraterone (dissolved in 5% benzyl alcohol and 95% safflower oil) per oral 5 days/week, 150 mg/kg palbociclib (50 mmol/L, Sodium lactate, 5%Tween80) per oral 5 days/week, 20 mg/kg PHA-793887 (5% dextrose, 5%DMSO) intravenous three times per week, a combination of Abi plus palbociclib, or a combination of Abi plus PHA-793887. Body weight and tumor volumes (width² × length/2) were measured two to three times per week with a digital caliper, and the average tumor volumes were determined. At the end of the treatment period, mice were euthanized and the tumors were removed, dissected and frozen at -80°C for further analysis.

While both PHA and Palb inhibited tumor growth, Palb appeared to be effective as single treatment, while PHA-alone did not exhibit statistically significant inhibition at tested dosage, but did appear to sensitize the tumor to Abi treatment (Figures 22A - 22C). In all cases, no significant toxicities were observed as evidenced by mice body weight (Figure 22D).

### Example 3: Biomarkers predicting abiraterone treatment prognosis and TOP2 inhibitor synergistic effect in castration resistant prostate cancer

DKFZ early onset prostate cancer cohort was downloaded from the cBioPortal as log2 transformed RPKM values. Patients with RNAseq data and biochemical relapse (BCR) was included. The final analyzable cohort included 105 samples with 24 relapsed and 81 non-relapsed (Table 8).

**Table 8. SU2C and DKFZ sub-cohort included in this study.**

| **SU2C** | **DKFZ** |
|---|---|
| TP_2077_Tumor | ICGC_PCA001_T01 |
| TP_2078_Tumor | ICGC_PCA003_T01 |
| SC_9137_Tumor | ICGC_PCA004_T01 |
| TP_2081_T | ICGC_PCA006_T01 |
| SC_9144_Tumor | ICGC_PCA008_T01 |
| SC_9171_Tumor | ICGC_PCA009_T01 |
| SC_9173_Tumor | ICGC_PCA010_T01 |
| MO_1499_T | ICGC_PCA011_T01 |
| MO_1510_T | ICGC_PCA012_T01 |
| SC_9176_T | ICGC_PCA014_T01 |
| SC_9146_T | ICGC_PCA015_T01 |
| SC_9195_T | ICGC_PCA021_T01 |
| MO_1553_T | ICGC_PCA025_T01 |
| SC_9203_T | ICGC_PCA026_T01 |
| MO_1084-Tumor | ICGC_PCA027_T02 |
| MO_1094-Tumor | ICGC_PCA028_T01 |
| SC_9011-Tumor | ICGC_PCA030_T01 |
| MO_1124-Tumor | ICGC_PCA031_T01 |
| SC_9026-Tumor | ICGC_PCA032_T01 |
| SC_9027-Tumor | ICGC_PCA033_T01 |
| SC_9039-Tumor | ICGC_PCA034_T01 |
| SC_9036-Tumor | ICGC_PCA034_T02 |
| SC_9037-Tumor | ICGC_PCA034_T03 |
| SC_9043-Tumor | ICGC_PCA034_T04 |
| MO_1176-Tumor | ICGC_PCA034_T05 |
| MO_1179-Tumor | ICGC_PCA034_T06 |
| MO_1184-Tumor | ICGC_PCA035_T01 |
| MO_1192-Tumor | ICGC_PCA035_T02 |
| SC_9050-Tumor | ICGC_PCA035_T03 |
| SC_9055-Tumor | ICGC_PCA035_T04 |
| SC_9056-Tumor | ICGC_PCA035_T05 |
| MO_1219-Tumor | ICGC_PCA035_T06 |
| SC_9057-Tumor | ICGC_PCA037_T01 |
| MO_1241-Tumor | ICGC_PCA037_T05 |
| SC_9065-Tumor | ICGC_PCA037_T06 |
| SC_9066-Tumor | ICGC_PCA038_T01 |
| SC_9068-Tumor | ICGC_PCA038_T02 |
| TP_2054-TM | ICGC_PCA038_T03 |
| SC_9080-TM | ICGC_PCA038_T04 |
| SC_9081-TM | ICGC_PCA038_T05 |
| SC_9082-TM | ICGC_PCA038_T06 |
| MO_1336-TM | ICGC_PCA039_T01 |
| MO_1337-TM | ICGC_PCA041_T01 |
| SC_9086-TM | ICGC_PCA041_T02 |
| SC_9089-TM | ICGC_PCA041_T03 |
| SC_9090_T | ICGC_PCA041_T04 |
| TP_2060-TM | ICGC_PCA041_T05 |
| TP_2064-TM | ICGC_PCA044_T01 |
| SC_9092-TM | ICGC_PCA044_T02 |
| MO_1410-Tumor | ICGC_PCA044_T03 |
| TP_2069_Tumor | ICGC_PCA049_T01 |
| SC_9121_Tumor | ICGC_PCA056_T01 |
| MO_1447_Tumor | ICGC_PCA057_T01 |
| | ICGC_PCA058_T01 |
| | ICGC_PCA059_T01 |
| | ICGC_PCA061_T01 |
| | ICGC_PCA062_T01 |
| | ICGC_PCA064_T01 |
| | ICGC_PCA069_T01 |
| | ICGC_PCA070_T01 |
| | ICGC_PCA071_T01 |
| | ICGC_PCA072_T01 |
| | ICGC_PCA084_T01 |
| | ICGC_PCA090_T01 |
| | ICGC_PCA091_T01 |
| | ICGC_PCA092_T01 |
| | ICGC_PCA093_T01 |
| | ICGC_PCA094_T01 |
| | ICGC_PCA095_T01 |
| | ICGC_PCA096_T01 |
| | ICGC_PCA099_T01 |
| | ICGC_PCA100_T01 |
| | ICGC_PCA105_T01 |
| | ICGC_PCA106_T01 |
| | ICGC_PCA110_T01 |
| | ICGC_PCA111_T01 |
| | ICGC_PCA112_T01 |
| | ICGC_PCA113_T01 |
| | ICGC_PCA118_T01 |
| | ICGC_PCA124_T01 |
| | ICGC_PCA125_T01 |
| | ICGC_PCA127_T01 |
| | ICGC_PCA128_T01 |
| | ICGC_PCA134_T01 |
| | ICGC_PCA138_T01 |
| | ICGC_PCA139_T01 |
| | ICGC_PCA144_T01 |
| | ICGC_PCA145_T01 |
| | ICGC_PCA148_T01 |
| | ICGC_PCA155_T01 |
| | ICGC_PCA156_T01 |
| | ICGC_PCA158_T01 |
| | ICGC_PCA159_T01 |
| | ICGC_PCA161_T01 |
| | ICGC_PCA162_T01 |
| | ICGC_PCA168_T01 |
| | ICGC_PCA169_T01 |
| | ICGC_PCA170_T01 |
| | ICGC_PCA172_T01 |
| | ICGC_PCA175_T01 |
| | ICGC_PCA176_T01 |
| | ICGC_PCA184_T01 |
| | ICGC_PCA187_T01 |
| | ICGC_PCA192_T01 |
| | ICGC_PCA198_T01 |

Using the 11 gene panel and clustering strategy that was applied to the PROMOTE dataset (see, Example 1), the expression data of the SU2C cohort can also be clustered into high and low expression subgroups, and that patients in high expression cluster also exhibited worse overall survival as compared to the low expression cluster (p-value = 0.0208). When including samples in the enzalutamide treatment arm (9 additional samples with available data), only one more sample was clustered into high-expression subgroup and survival benefits were essentially the same as abiraterone-arm only (Figures 23A - 23B).

In order to examine whether the panel also has a prognostic value of disease relapse in the primary tumor, the German Cancer Research Center (DKFZ) early-onset prostate cancer cohort was further examined. Progression-free survival (PFS) was used as the clinical outcome for the TCGA cohort and biochemical relapse (BCR) for the DKFZ cohort. The results also showed that the patients with high expression or in the high expression subgroup had a worse PFS outcome in the TCGA cohort (p-value = 6.78 × 10⁻⁶) and a worse BCR outcome in the DKFZ cohort (p-value = 6.55 × 10⁻⁵) (Figures 23C - 23D). These results also indicated the potential value of our gene panel in predicting risk of disease progression in the primary tumor.

## Claims

1. An *ex vivo* method for assessing a mammal having prostate cancer, wherein said method comprises:
(a) detecting, in a sample from said mammal, a presence or absence of an increased level of expression of a cyclin A2 (CCNA2) polypeptide, a cyclin B1 (CCNB1) polypeptide, a CCNB2 polypeptide, a protein regulator of cytokinesis 1 (PRC1) polypeptide, a structural maintenance of chromosomes protein 2 (SMC2) polypeptide, a discs large-associated protein 5 (DLGAP5) polypeptide, an epithelial cell transforming 2 (ECT2) polypeptide, a F-box only protein 5 (FBXO5) polypeptide, a cyclin dependent kinase 1 (CDK1) polypeptide, a non-SMC condensin I complex subunit G (NCAPG) polypeptide, and a kinesin family member 4A (KIF4A) polypeptide;
(b) classifying said mammal as being unlikely to respond to an anti-androgen agent if said presence of said increased level is detected; and
(c) classifying said mammal as being likely to respond to said anti-androgen agent if said absence of said increased level is detected.

2. The method of claim 1, wherein said method comprises detecting said presence of said increased level of said polypeptides, and wherein said method comprises classifying said mammal as being unlikely to respond to said anti-androgen agent.

3. The method of claim 1, wherein said method comprises detecting said absence of said increased level of said polypeptides, and wherein said method comprises classifying said mammal as being likely to respond to said anti-androgen agent.

4. The method of any one of claims 1-3, wherein said detecting comprises a clustering analysis, optionally wherein said clustering analysis is a machine learning based clustering analysis.

5. A cancer drug for use in a method for treating a mammal having prostate cancer, wherein said method comprises:
(a) detecting, in a sample obtained from said mammal, an increased level of expression of a CCNA2 polypeptide, a CCNB1 polypeptide, a CCNB2 polypeptide, a PRC1 polypeptide, a SMC2 polypeptide, a DLGAP5 polypeptide, an ECT2 polypeptide, a FBXO5 polypeptide, a CDK1 polypeptide, a NCAPG polypeptide, and a KIF4A polypeptide; and
(b) administering said cancer drug to said mammal, wherein said cancer drug is not an anti-androgen agent.

6. The cancer drug for use of claim 5, wherein said cancer drug is selected from the group consisting of docetaxel, cabazitaxel, mitoxantrone, estramustine, doxorubicin, palbociclib, ribociclib, abemaciclib, PD-0325901, and PHA-793887, or a combination thereof.

7. An anti-androgen agent for use in a method for treating a mammal having prostate cancer, wherein said method comprises:
(a) detecting, in a sample obtained from said mammal, an absence of an increased level of expression of a CCNA2 polypeptide, a CCNB1 polypeptide, a CCNB2 polypeptide, a PRC1 polypeptide, a SMC2 polypeptide, a DLGAP5 polypeptide, an ECT2 polypeptide, a FBXO5 polypeptide, a CDK1 polypeptide, a NCAPG polypeptide, and a KIF4A polypeptide; and
(b) administering said anti-androgen agent to said mammal.

8. A DNA topoisomerase 2-alpha (TOP2A) inhibitor for use in a method for treating a mammal having prostate cancer, wherein said method comprises:
(a) detecting, in a sample obtained from said mammal, a presence of an increased level of expression of a CCNA2 polypeptide, a CCNB1 polypeptide, a CCNB2 polypeptide, a PRC1 polypeptide, a SMC2 polypeptide, a DLGAP5 polypeptide, an ECT2 polypeptide, a FBXO5 polypeptide, a CDK1 polypeptide, a NCAPG polypeptide, and a KIF4A polypeptide;
(b) administering said TOP2A inhibitor to said mammal to increase the sensitivity of prostate cancer cells within said mammal to an anti-androgen agent; and
(c) administering said anti-androgen agent to said mammal.

9. The TOP2A inhibitor for use of claim 8, wherein said TOP2A inhibitor is selected from the group consisting of mitoxantrone, doxorubicin, teniposide, daunorubicin, amsacrine, ellipticines, aurintricarboxylic acid, and HU-331.

10. A cyclin-dependent kinase (CDK) 4/6 inhibitor for use in a method for treating a mammal having prostate cancer, wherein said method comprises:
(a) detecting, in a sample obtained from said mammal, a presence of an increased level of expression of a CCNA2 polypeptide, a CCNB1 polypeptide, a CCNB2 polypeptide, a PRC1 polypeptide, a SMC2 polypeptide, a DLGAP5 polypeptide, an ECT2 polypeptide, a FBXO5 polypeptide, a CDK1 polypeptide, a NCAPG polypeptide, and a KIF4A polypeptide;
(b) administering said CDK 4/6 inhibitor to said mammal to increase the sensitivity of prostate cancer cells within said mammal to an anti-androgen agent; and
(c) administering said anti-androgen agent to said mammal.

11. The CDK 4/6 inhibitor for use of claim 10, wherein said CDK 4/6 inhibitor is selected from the group consisting of palbociclib, abemaciclib, and ribociclib.

12. A pan-cyclin-dependent kinase (CDK) inhibitor for use in a method for treating a mammal having prostate cancer, wherein said method comprises:
(a) detecting, in a sample obtained from said mammal, a presence of an increased level of a CCNA2 polypeptide, a CCNB1 polypeptide, a CCNB2 polypeptide, a PRC1 polypeptide, a SMC2 polypeptide, a DLGAP5 polypeptide, an ECT2 polypeptide, a FBXO5 polypeptide, a CDK1 polypeptide, a NCAPG polypeptide, and a KIF4A polypeptide;
(b) administering said pan-CDK inhibitor to said mammal to increase the sensitivity of prostate cancer cells within said mammal to an anti-androgen agent; and
(c) administering said anti-androgen agent to said mammal.

13. The pan-CDK inhibitor for use of claim 12, wherein said pan-CDK inhibitor is PHA-793887.

14. The method of any one of claims 1-4, the cancer drug for use of any one of claims 5-6, the anti-androgen agent for use of claim 7, the TOP2A inhibitor for use of any one of claims 8-9, the CDK 4/6 inhibitor for use of any one of claims 10-11, or the pan-CDK inhibitor for use of any one of claims 12-13, wherein said mammal is a human, and/or wherein said sample comprises cancer cells of said prostate cancer, optionally wherein said prostate cancer is a metastatic prostate cancer.

15. The method of any one of claims 1-4 and 14, the anti-androgen agent for use of any one of claims 7 and 14, the TOP2A inhibitor for use of any one of claims 8-9 and 14, the CDK 4/6 inhibitor for use of any one of claims 10-11 and 14, or the pan-CDK inhibitor for use of any one of claims 12-14, wherein said anti-androgen agent is selected from the group consisting of leuprolide, goserelin, triptorelin, histrelin, degarelix, abiraterone, ketoconazole, flutamide, bicalutamide, nilutamide, enzalutamide, apalutamide, and darolutamide.

## Patentansprüche

1. Ex-vivo-Verfahren zur Beurteilung eines Säugers mit Prostatakrebs, wobei das Verfahren umfasst:
(a) Detektieren einer Anwesenheit oder Abwesenheit eines erhöhten Expressionsniveaus eines Cyclin-A2 (CCNA2)-Polypeptids, eines Cyclin-B1 (CCNB1)-Polypeptids, eines CCNB2-Polypeptids, eines Proteinregulators des Cytokinesis-1 (PRC1)-Polypeptids, einer strukturellen Aufrechterhaltung des Chromosomen-Protein-2 (SMC2)-Polypeptids, eines Discs Large-Associated Protein-5 (DLGAP5)-Polypeptids, eines Epithelialzell-transformierenden-2 (ECT2)-Polypeptids, eines Nur-F-Box-Protein-5 (FBXO5)-Proteins, eines cyclinabhängige Kinase 1 (CDK1)-Polypeptids, eines Nicht-SMC-Kondensin-I-Komplex-Untereinheit-G (NCAPG)-Polypeptids und eines Kinesinfamilien-4A (KIF4A)-Polypeptids in einer Probe des Säugers;
(b) Klassifizieren des Säugers als wahrscheinlich nicht auf ein Anti-Androgenmittel ansprechend, wenn die Anwesenheit des erhöhten Niveaus detektiert wird; und
(c) Klassifizieren des Säugers als wahrscheinlich auf das Anti-Androgenmittel ansprechend, wenn die Abwesenheit des erhöhten Niveaus detektiert wird.

2. Verfahren nach Anspruch 1, wobei das Verfahren Detektieren der Anwesenheit des erhöhten Niveaus der Polypeptide umfasst, und wobei das Verfahren Klassifizieren des Säugers als wahrscheinlich nicht auf das Anti-Androgenmittel ansprechend umfasst.

3. Verfahren nach Anspruch 1, wobei das Verfahren Detektieren der Abwesenheit des erhöhten Niveaus der Polypeptide umfasst, und wobei das Verfahren Klassifizieren des Säugers als wahrscheinlich auf das Anti-Androgenmittel ansprechend umfasst.

4. Verfahren nach einem der Ansprüche 1-3, wobei das Detektieren eine Clusteranalyse umfasst, wobei die Clusteranalyse gegebenenfalls eine auf maschinellem Lernen basierende Clusteranalyse ist.

5. Krebsarzneimittel zur Verwendung in einem Verfahren zur Behandlung eines Säugers mit Prostatakrebs, wobei das Verfahren umfasst:
(a) Detektieren eines erhöhten Expressionsniveaus eines CCNA2-Polypeptids, eines CCNB1-Polypeptids, eines CCNB2-Polypeptids, eines PRC1-Polypeptids, eines SMC2-Polypeptids, eines DLGAP5-Polypeptids, eines ECT2-Polypeptids, eines FBXO5-Polypeptids, eines CDK1-Polypeptids, eines NCAPG-Polypeptids und eines KIF4A-Polypeptids in einer von dem Säuger erhaltenen Probe;
(b) Verabreichen des Krebsarzneimittels an den Säuger, wobei das Krebsarzneimittel kein Anti-Androgenmittel ist.

6. Krebsarzneimittel zur Verwendung nach Anspruch 5, wobei das Krebsarzneimittel ausgewählt ist aus der Gruppe bestehend aus Docetaxel, Cabazitaxel, Mitoxantron, Estramustin, Doxorubicin, Palbociclib, Ribociclib, Abemaciclib, PD-0325901 und PHA-793887 oder einer Kombination davon.

7. Anti-Androgenmittel zur Verwendung in einem Verfahren zur Behandlung eines Säugers mit Prostatakrebs, wobei das Verfahren umfasst:
(a) Detektieren einer Abwesenheit eines erhöhten Expressionsniveaus eines CCNA2-Polypeptids, eines CCNB1-Polypeptids, eines CCNB2-Polypeptids, eines PRC1-Polypeptids, eines SMC2-Polypeptids, eines DLGAP5-Polypeptids, eines ECT2-Polypeptids, eines FBXO5-Polypeptids, eines CDK1-Polypeptids, eines NCAPG-Polypeptids und eines KIF4A-Polypeptids in einer von dem Säuger erhaltenen Probe;
(b) Verabreichen des Anti-Androgenmittels an den Säuger.

8. DNA-Topoisomerase-2-alpha (TOP2A)-Inhibitor zur Verwendung in einem Verfahren zur Behandlung eines Säugers mit Prostatakrebs, wobei das Verfahren umfasst:
(a) Detektieren einer Anwesenheit eines erhöhten Expressionsniveaus eines CCNA2-Polypeptids, eines CCNB1-Polypeptids, eines CCNB2-Polypeptids, eines PRC1-Polypeptids, eines SMC2-Polypeptids, eines DLGAP5-Polypeptids, eines ECT2-Polypeptids, eines FBXO5-Polypeptids, eines CDK1-Polypeptids, eines NCAPG-Polypeptids und eines KIF4A-Polypeptids in einer von dem Säuger erhaltenen Probe;
(b) Verabreichen des TOP2A-Inhibitors an den Säuger, um die Empfindlichkeit von Prostatakrebszellen innerhalb des Säugers gegenüber einem Anti-Androgenmittel zu erhöhen; und
(c) Verabreichen des Anti-Androgenmittels an den Säuger.

9. TOP2A-Inhibitor zur Verwendung nach Anspruch 8, wobei der TOP2A-Inhibitor ausgewählt ist aus der Gruppe bestehend aus Mitoxantron, Doxorubicin, Teniposid, Daunorubicin, Amsacrin, Ellipticinen, Aurintricarbonsäure und HU-331.

10. Cyclin-abhängige Kinase (CDK)-4/6-Inhibitor zur Verwendung in einem Verfahren zur Behandlung eines Säugers mit Prostatakrebs, wobei das Verfahren umfasst:
(a) Detektieren einer Anwesenheit eines erhöhten Expressionsniveaus eines CCNA2-Polypeptids, eines CCNB1-Polypeptids, eines CCNB2-Polypeptids, eines PRC1-Polypeptids, eines SMC2-Polypeptids, eines DLGAP5-Polypeptids, eines ECT2-Polypeptids, eines FBXO5-Polypeptids, eines CDK1-Polypeptids, eines NCAPG-Polypeptids und eines KIF4A-Polypeptids in einer von dem Säuger erhaltenen Probe;
(b) Verabreichen des CDK-4/6-Inhibitors an den Säuger, um die Empfindlichkeit von Prostatakrebszellen innerhalb des Säugers gegenüber einem Anti-Androgenmittel zu erhöhen; und
(c) Verabreichen des Anti-Androgenmittels an den Säuger.

11. CDK-4/6-Inhibitor zur Verwendung nach Anspruch 10, wobei der CDK-4/6-Inhibitor ausgewählt ist aus der Gruppe bestehend aus Palbociclib, Abemaciclib und Ribociclib.

12. Pan-Cyclin-abhängige Kinase(CDK)-Inhibitor zur Verwendung in einem Verfahren zur Behandlung eines Säugers mit Prostatakrebs, wobei das Verfahren umfasst:
(a) Detektieren einer Anwesenheit eines erhöhten Niveaus eines CCNA2-Polypeptids, eines CCNB1-Polypeptids, eines CCNB2-Polypeptids, eines PRC1-Polypeptids, eines SMC2-Polypeptids, eines DLGAP5-Polypeptids, eines ECT2-Polypeptids, eines FBXO5-Polypeptids, eines CDK1-Polypeptids, eines NCAPG-Polypeptids und eines KIF4A-Polypeptids in einer von dem Säuger erhaltenen Probe;
(b) Verabreichen des Pan-CDK-Inhibitors an den Säuger, um die Empfindlichkeit von Prostatakrebszellen innerhalb des Säugers gegenüber einem Anti-Androgenmittel zu erhöhen; und
(c) Verabreichen des Anti-Androgenmittels an den Säuger.

13. Pan-CDK-Inhibitor zur Verwendung nach Anspruch 12, wobei der Pan-CDK-Inhibitor PHA-793887 ist.

14. Verfahren nach einem der Ansprüche 1-4, Krebsarzneimittel zur Verwendung nach einem der Ansprüche 5-6, Anti-Androgenmittel zur Verwendung nach Anspruch 7, TOP2A-Inhibitor zur Verwendung nach einem der Ansprüche 8-9, CDK-4/6-Inhibitor zur Verwendung nach einem der Ansprüche 10-11 oder Pan-CDK-Inhibitor zur Verwendung nach einem der Ansprüche 12-13, wobei der Säuger ein Mensch ist, und/oder wobei die Probe Krebszellen des Prostatakrebses umfasst, wobei der Prostatakrebs gegebenenfalls ein metastasierter Prostatakrebs ist.

15. Verfahren nach einem der Ansprüche 1-4 und 14, Anti-Androgenmittel zur Verwendung nach einem der Ansprüche 7 und 14, TOP2A-Inhibitor zur Verwendung nach einem der Ansprüche 8-9 und 14, CDK-4/6-Inhibitor zur Verwendung nach einem der Ansprüche 10-11 und 14 oder Pan-CDK-Inhibitor zur Verwendung nach einem der Ansprüche 12-14, wobei das Anti-Androgenmittel ausgewählt ist aus der Gruppe bestehend aus Leuprolid, Goserelin, Triptorelin, Histrelin, Degarelix, Abirateron, Ketoconazol, Flutamid, Bicalutamid, Nilutamid, Enzalutamid, Apalutamid und Darolutamid.

## Revendications

1. Procédé *ex vivo* pour l'évaluation d'un mammifère ayant un cancer de l'endomètre, ledit procédé comprenant :
(a) la détection, dans un échantillon dudit mammifère, d'une présence ou d'une absence d'un niveau augmenté d'expression d'un polypeptide de cycline A2 (CCNA2), d'un polypeptide de cycline B1 (CCNB1), d'un polypeptide CCNB2, d'un polypeptide de régulateur protéique de la cytokinèse 1 (PRC1), d'un polypeptide de maintien structurel des chromosomes 2 (SMC2), d'un polypeptide de protéine associée aux grands disques 5 (DLGAP5), d'un polypeptide de transformation des cellules épithéliales 2 (ECT2), d'un polypeptide de protéine F-box uniquement 5 (FBXO5), d'un polypeptide de kinase dépendante de la cycline 1 (CDK1), d'un polypeptide de sous-unité G du complexe de condensine I non-SMC (NCAPG) et d'un polypeptide de membre de la famille des kinésines 4A (KIF4A) ;
(b) la classification dudit mammifère comme étant peu susceptible de répondre à un agent anti-androgène si ladite présence dudit niveau augmenté est détectée ; et
(c) la classification dudit mammifère comme étant susceptible de répondre audit agent anti-androgène si ladite absence dudit niveau augmenté est détectée.

2. Procédé selon la revendication 1, dans lequel ledit procédé comprend la détection de ladite présence dudit niveau augmenté desdits polypeptides, et dans lequel ledit procédé comprend la classification dudit mammifère comme étant peu susceptible de répondre audit agent anti-androgène.

3. Procédé selon la revendication 1, dans lequel ledit procédé comprend la détection de ladite absence dudit niveau augmenté desdits polypeptides, et dans lequel ledit procédé comprend la classification dudit mammifère comme étant susceptible de répondre audit agent anti-androgène.

4. Procédé selon l'une quelconque des revendications 1 à 3, dans lequel ladite détection comprend une analyse de regroupement, facultativement dans lequel ladite analyse de regroupement est une analyse de regroupement basée sur un apprentissage automatique.

5. Médicament anticancéreux pour utilisation dans un procédé pour le traitement d'un mammifère ayant un cancer de la prostate, ledit procédé comprenant :
(a) la détection, dans un échantillon obtenu à partir dudit mammifère, d'un niveau d'expression augmenté d'un polypeptide CCNA2, d'un polypeptide CCNB1, d'un polypeptide CCNB2, d'un polypeptide PRC1, d'un polypeptide SMC2, d'un polypeptide DLGAP5, d'un polypeptide ECT2, d'un polypeptide FBXO5, d'un polypeptide CDK1, d'un polypeptide NCAPG et d'un polypeptide KIF4A ; et
(b) l'administration dudit médicament anticancéreux audit mammifère, où ledit médicament anticancéreux n'est pas un agent anti-androgène.

6. Médicament anticancéreux destiné à être utilisé selon la revendication 5, dans lequel ledit médicament anticancéreux est choisi dans le groupe constitué par docétaxel, cabazitaxel, mitoxantrone, estramustine, doxorubicine, palbociclib, ribociclib, abémaciclib, PD-0325901 et PHA-793887, ou une combinaison de ceux-ci.

7. Agent anti-androgène pour utilisation dans un procédé pour traiter un mammifère ayant un cancer de la prostate, ledit procédé comprenant :
(a) la détection, dans un échantillon obtenu chez ledit mammifère, d'une absence d'un niveau d'expression augmenté d'un polypeptide CCNA2, d'un polypeptide CCNB1, d'un polypeptide CCNB2, d'un polypeptide PRC1, d'un polypeptide SMC2, d'un polypeptide DLGAP5, d'un polypeptide ECT2, d'un polypeptide FBXO5, d'un polypeptide CDK1, d'un polypeptide NCAPG et d'un polypeptide KIF4A ; et
(b) l'administration dudit agent anti-androgène audit mammifère.

8. Inhibiteur d'ADN topoisomérase 2-alpha (TOP2A) destiné à être utilisé dans un procédé de traitement d'un mammifère ayant un cancer de la prostate, ledit procédé comprenant :
(a) la détection, dans un échantillon obtenu chez ledit mammifère, d'une présence d'un niveau d'expression augmenté d'un polypeptide CCNA2, d'un polypeptide CCNB1, d'un polypeptide CCNB2, d'un polypeptide PRC1, d'un polypeptide SMC2, d'un polypeptide DLGAP5, d'un polypeptide ECT2, d'un polypeptide FBXO5, d'un polypeptide CDK1, d'un polypeptide NCAPG et d'un polypeptide KIF4A ;
(b) l'administration dudit inhibiteur de TOP2A audit mammifère pour augmenter la sensibilité de cellules de cancer de la prostate chez ledit mammifère à un agent anti-androgène ; et
(c) l'administration dudit agent anti-androgène audit mammifère.

9. Inhibiteur de TOP2A pour utilisation selon la revendication 8, dans lequel ledit inhibiteur de TOP2A est choisi dans le groupe constitué par mitoxantrone, doxorubicine, téniposide, daunorubicine, amsacrine, ellipticines, acide aurintricarboxylique et HU-331.

10. Inhibiteur de kinase dépendante des cyclines (CDK) 4/6 destiné à être utilisé dans un procédé pour le traitement d'un mammifère ayant un cancer de la prostate, ledit procédé comprenant :
(a) la détection, dans un échantillon obtenu chez ledit mammifère, d'une présence d'un niveau d'expression augmenté d'un polypeptide CCNA2, d'un polypeptide CCNB1, d'un polypeptide CCNB2, d'un polypeptide PRC1, d'un polypeptide SMC2, d'un polypeptide DLGAP5, d'un polypeptide ECT2, d'un polypeptide FBXO5, d'un polypeptide CDK1, d'un polypeptide NCAPG et d'un polypeptide KIF4A ;
(b) l'administration dudit inhibiteur de CDK 4/6 audit mammifère afin d'augmenter la sensibilité des cellules de cancer de la prostate chez ledit mammifère à un agent anti-androgène ; et
(c) l'administration dudit agent anti-androgène audit mammifère.

11. Inhibiteur de CDK 4/6 pour utilisation selon la revendication 10, dans lequel ledit inhibiteur de CDK 4/6 est choisi dans le groupe constitué par palbociclib, abémaciclib et ribociclib.

12. Inhibiteur de kinase dépendante de la pan-cycline (CDK) destiné à être utilisé dans un procédé pour le traitement d'un mammifère ayant un cancer de la prostate, ledit procédé comprenant :
(a) la détection, dans un échantillon obtenu dudit mammifère, d'une présence d'un niveau augmenté d'un polypeptide CCNA2, d'un polypeptide CCNB1, d'un polypeptide CCNB2, d'un polypeptide PRC1, d'un polypeptide SMC2, d'un polypeptide DLGAP5, d'un polypeptide ECT2, d'un polypeptide FBXO5, d'un polypeptide CDK1, d'un polypeptide NCAPG et d'un polypeptide KIF4A ;
(b) l'administration dudit inhibiteur de pan-CDK audit mammifère pour augmenter la sensibilité des cellules de cancer de la prostate chez ledit mammifère à un agent anti-androgène ; et
(c) l'administration dudit agent anti-androgène audit mammifère.

13. Inhibiteur de pan-CDK pour utilisation selon la revendication 12, dans lequel ledit inhibiteur de pan-CDK est PHA-793887.

14. Procédé selon l'une quelconque des revendications 1 à 4, médicament anticancéreux pour utilisation selon l'une quelconque des revendications 5 à 6, agent anti-androgène pour utilisation de la revendication 7, inhibiteur de TOP2A pour utilisation selon l'une quelconque des revendications 8 à 9, inhibiteur de CDK 4/6 pour utilisation selon l'une quelconque des revendications 10 à 11, ou inhibiteur de pan-CDK pour utilisation selon l'une quelconque des revendications 12 à 13, dans lequel ledit mammifère est un humain, et/ou dans lequel ledit échantillon comprend des cellules cancéreuses dudit cancer de la prostate, facultativement où ledit cancer de la prostate est un cancer de la prostate métastasique.

15. Procédé selon l'une quelconque des revendications 1 à 4 et 14, agent anti-androgène pour utilisation selon l'une quelconque des revendications 7 et 14, inhibiteur de TOP2A pour utilisation selon l'une quelconque des revendications 8 à 9 et 14, inhibiteur de CDK 4/6 pour utilisation selon l'une quelconque des revendications 10 à 11 et 14, ou inhibiteur de pan-CDK pour utilisation selon l'une quelconque des revendications 12 à 14, dans lequel ledit agent anti-androgène est choisi dans le groupe constitué par leuprolide, goséréline, triptoréline, histréline, dégarélix, abiratérone, kétoconazole, flutamide, bicalutamide, nilutamide, enzalutamide, apalutamide et darolutamide.
